(19) Europäisches Patentamt
European Patent Office
Office européen des brevets

(11) EP 4 214 198 B1

(12) # EUROPEAN PATENT SPECIFICATION

(45) Date of publication and mention of the grant of the patent:
**08.07.2026 Bulletin 2026/28**

(21) Application number: **21868717.6**

(22) Date of filing: **17.09.2021**

(51) International Patent Classification (IPC):
***C07D 401/12*** (2006.01) ***C07D 401/06*** (2006.01)
***C07D 471/04*** (2006.01) ***C07D 487/04*** (2006.01)
***C07D 403/06*** (2006.01) ***C07D 403/12*** (2006.01)
***A61K 31/444*** (2006.01) ***A61K 31/47*** (2006.01)
***A61K 31/506*** (2006.01) ***A61K 31/519*** (2006.01)
***A61P 37/00*** (2006.01) ***A61P 35/00*** (2006.01)
***A61P 19/02*** (2006.01) ***A61P 25/00*** (2006.01)
***A61P 3/00*** (2006.01)

(52) Cooperative Patent Classification (CPC):
**C07D 471/04; A61K 31/444; A61K 31/47; A61K 31/506; A61K 31/519; A61P 3/00; A61P 19/02; A61P 25/00; A61P 35/00; A61P 37/00; C07D 401/12; C07D 401/14; C07D 487/14; C07D 491/056; C07D 519/00**

(86) International application number:
**PCT/CN2021/119057**

(87) International publication number:
**WO 2022/057895 (24.03.2022 Gazette 2022/12)**

(54) **HETEROAROMATIC COMPOUNDS AND USES THEREOF**
HETEROAROMATISCHE VERBINDUNGEN UND VERWENDUNGEN DAVON
COMPOSÉS HÉTÉROAROMATIQUES ET LEURS UTILISATIONS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **21.09.2020 CN 202010998486**
**14.09.2021 CN 202111071969**

(43) Date of publication of application:
**26.07.2023 Bulletin 2023/30**

(73) Proprietor: **HUTCHMED Limited**
**Shanghai 201203 (CN)**

(72) Inventors:
- **SU, Wei-Guo**
**Shanghai 201203 (CN)**
- **ZHANG, Weihan**
**Shanghai 201203 (CN)**
- **YANG, Haibin**
**Shanghai 201203 (CN)**

(74) Representative: **Sagittarius IP**
**Marlow International**
**Parkway**
**Marlow SL7 1YL (GB)**

(56) References cited:
**WO-A1-2012/151523 WO-A1-2012/151541**
**WO-A1-2017/015267 WO-A1-2018/184115**
**WO-A1-2019/174601 WO-A2-2008/064265**
**WO-A2-2018/083635 US-A1- 2019 308 949**

- **MEYERS, MARVIN J. ET AL.: "Structure-based Drug Design Enables Conversion of a DFG-in Binding CSF-1R Kinase Inhibitor to a DFG-out Binding Mode", BIOORGANIC & MEDICINAL CHEMISTRY LETTERS, vol. 20, no. 5, 1 March 2010 (2010-03-01), pages 1542 - 1547, XP026911300, ISSN: 0960-894X, DOI: 10.1016/j.bmcl.2010.01.078**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 214 198 B1

## Description

### Technical Field

The present invention relates to heteroaromatic compounds, pharmaceutical compositions comprising same, methods for preparing same and uses thereof.

### Background Art

Members of type III tyrosine kinase receptor family include CSF-1R, PDGFRα, PDGFRβ, FLT3 and c-KIT. The members of this family are all composed of an extracellular immunoglobulin-like domain, a transmembrane domain, a juxtamembrane domain and a protein kinase domain, wherein the kinase domain is highly conserved (Nat Rev Cancer. 2012, 12(11):753-66). The phosphorylation signal mediated thereby participates in numerous cell biological functions and plays an important role in the occurrence of diseases. Specifically, there are reports indicating that mutations in the kinase domain of PDGFRα and c-KIT would lead to gastrointestinal tumors (J Pathol. 2011, 223(2): 251-261). In addition, it is found that FLT-3 tandem duplication (FLT3-ITD) is a key pathogenic factor in approximately 20% of patients with acute lymphoblastic leukemia (Biomark Insights. 2015, 10(Suppl 3):1-14).

CSF-1R, i.e. CSF-1 receptor (colony stimulating factor 1 receptor), is encoded by the oncogene c-fms. The human c-fms gene is located at 5q33.3 of chromosome 5, downstream of the β-type platelet-derived growth factor receptor (PDGF_Rβ) gene, and the two genes are connected end to end. Human CSF-1R is a single-chain, transmembrane receptor tyrosine kinase, a transmembrane glycoprotein composed of 972 amino acids, with a molecular weight of 150 Kd. It consists of an extramembrane region with 512 amino acids, a transmembrane region with 25 amino acids, and an intracellular cytoplasmic region with 435 amino acids. The extracellular region has 5 disulfide bonds and 11 possible glycosylation sites, and the intracellular region has a Gly-X-Gly-X-X-Gly motif. Lysine at position 616 is a binding site for ATP, flanked by a kinase insertion region with 72 amino acids. It is speculated that it has the function of recognizing specific substrates (Cold Spring Harb Perspect Biol. 2014, 6(6)).

CSF-1, also called M-CSF (macrophage colony stimulating factor), is encoded by the CSF-1 gene. CSF-1 exerts its biological effects by binding to the only cell surface receptor CSF-1R thereof. After binding to CSF-1, CSF-1R undergoes changes in its conformation and forms a dimer or polymer. After dimerization, the tyrosine kinase activity of the receptor is activated, and the tyrosines at positions 544, 559, 699, 708, 723, 809, 923, etc. are phosphorylated, and subsequently interact with multiple intracellular signaling pathways such as Ras, MAPK, PI3K, JAK, etc. to produce various biological effects in cells (J Cell Biochem. 1988, 38(3):179-87).

The tumor microenvironment is a complex ecosystem, and provides support for the occurrence, growth and metastasis of tumors. Macrophages are particularly abundant in immune cells that migrate to the tumor site, and exist in all stages of tumor development. Studies have shown that tumor-associated macrophages (TAMs) play an important role in the occurrence, growth and metastasis of tumors. For primary tumors, macrophages can stimulate the neovascularization, aid the extravasation, survival and continuous growth of tumor cells, thereby promoting tumor cell metastasis. TAM also exerts an immunosuppressive effect, preventing natural killer cells and T cells from attacking tumor cells (Immunity. 2014, 41(1):49-61). CSF-1R is expressed in macrophages, and the survival and differentiation of macrophages depends on the CSF-1/CSF-1R signaling pathway. The CSF-1/CSF-1R signaling pathway interferes with tumor progression by regulating TAMs to reduce tumor invasiveness and proliferation, as a consequence, the CSF1/CSF1R signaling pathway is a potential target for cancer treatment. Overexpression of CSF-1 or CSF-1R is related to tumor malignant invasiveness and poor prognosis. Studies have shown that the application of CSF-1R inhibitors can affect the exchange of inflammatory factors between TAMs and glioma cells, which significantly reduces the volume of glioblastoma, and reduces tumor invasiveness and proliferation (Nat Med. 2013, 19(10):1264-72). In addition, aberrantly high expression of CSF-1 is the main pathogenesis of tenosynovial giant cell tumor (a type of rare non-metastatic tumor with giant cell tumor and pigmented villonodular synovitis in tendon sheath). Patients with tenosynovial giant cell tumor have obvious clinical benefits after using CSF-1R inhibitors (N Engl J Med. 2015, 373(5):428-37).

In addition to tumors, the CSF-1R signaling pathway plays an important role in autoimmune diseases and inflammatory diseases, including systemic lupus erythematosus, arthritis, atherosclerosis and obesity (Arthritis Res Ther. 2016, 18:75; Nat Rev Immunol. 2008, 8(7):533-44; J Immunother Cancer. 2017, 5(1):53). Therefore, the development of CSF-1R inhibitors may also be used to treat such diseases.

Furthermore, increasing studies have shown that inflammation in the nervous system and abnormal activation of brain microglia cells are important pathogenic factors of related neurodegenerative diseases, especially Alzheimer's disease (Neurobiol Aging. 2000, 21:383-421). Among others, the signaling pathway mediated by CSF-1R plays a leading role in the activation and proliferation of microglia cells in the brain. Studies have shown that the expression of CSF-1R in tissue samples from Alzheimer's patients is significantly upregulated, accompanied by the abnormal activation and proliferation of microglia (Brain Res., 1994, 639:171-4). Animal model studies have shown that blocking CSF-1R signaling

can effectively inhibit microglial proliferation, thereby effectively alleviating the disease progression in mouse models of Alzheimer's disease (Brain. 2016, 139:891-907). In models of other neurodegenerative diseases, such as amyotrophic lateral sclerosis, the therapeutic effects of targeting CSF-1R on the disease has been preliminarily demonstrated in a proof-of-concept study (Sci Rep., 2016, 6:25663). Currently, a number of CSF-1R inhibitor candidates are in clinical trials on neurodegenerative diseases. Bioorg. Med. Chem. Lett., 2010, 20:1543-1547 discloses further CSF-1R inhibitors.

Novel CSF-1R inhibitors are needed for the treatment of diseases, such as cancer, autoimmune diseases, inflammatory diseases or neurodegenerative diseases. The present invention addresses these needs.

**Summary of the Invention**

Provided is a compound of formula (I):

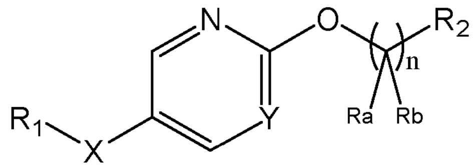

(I)

or a pharmaceutically acceptable salt thereof, or a solvate, a racemic mixture, an enantiomer, a diastereomer or a tautomer thereof, wherein

$R_1$ is chosen from:

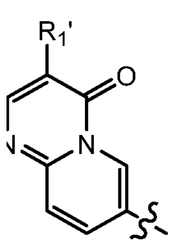,
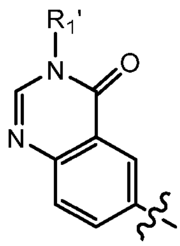,
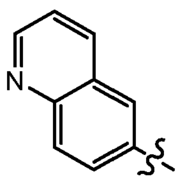,
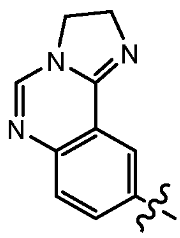

and

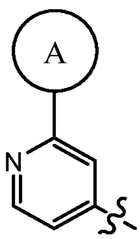;

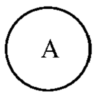

is chosen from phenyl and 5-6 membered heteroaryl, each of which is optionally substituted with one or more groups independently chosen from: -CN, halogen, $C_{1-6}$ alkyl, -O($C_{1-6}$ alkyl), $C_{1-6}$ haloalkyl, -O($C_{1-6}$ haloalkyl), -$C_{1-6}$ alkylene-CN, and -$C_{1-6}$ alkylene-OH;

$R_1$' is chosen from H, halogen, -CN, $C_{1-6}$ alkyl, $C_{1-6}$ haloalkyl, -$C_{1-6}$ alkylene-CN, - $C_{1-6}$ alkylene-OH, -O($C_{1-6}$ alkyl), -O($C_{1-6}$ haloalkyl), $C_{3-8}$ cycloalkyl, 4-8 membered heterocyclyl, and $NR_4R_5$; $R_4$ and $R_5$ are each independently chosen from H, halogen, -CN, $C_{1-6}$ alkyl, $C_{1-6}$ haloalkyl, -$C_{1-6}$ alkylene-CN, -$C_{1-6}$ alkylene-OH, and -O($C_{1-6}$ alkyl); or $R_4$ and $R_5$ together with the N atom to which they are attached form a 4-8 membered heterocyclic ring;

X is O or $CR_6R_7$; $R_6$ and $R_7$ are each independently chosen from H, halogen, $C_{1-6}$ alkyl, $C_{1-6}$ haloalkyl, -$C_{1-6}$ alkylene-CN, -$C_{1-6}$ alkylene-OH, -O($C_{1-6}$ alkyl) and -O($C_{1-6}$ haloalkyl);

Y is N or $CR_3$; $R_3$ is chosen from H, -CN, halogen, $C_{1-6}$ alkyl, -O($C_{1-6}$ alkyl), -$C_{1-6}$ alkylene-CN, -$C_{1-6}$ alkylene-OH, $C_{1-6}$ haloalkyl, and -O($C_{1-6}$ haloalkyl);

$R_a$ and $R_b$ are each independently chosen from H, halogen, -CN, $C_{1-6}$ alkyl, $C_{1-6}$ haloalkyl, -$C_{1-6}$ alkylene-CN, -$C_{1-6}$ alkylene-OH, -O($C_{1-6}$ alkyl) and -O($C_{1-6}$ haloalkyl);

n is 0, 1, 2, 3, or 4;

$R_2$ is phenyl or 5-10 membered heteroaryl, each of which is optionally substituted with one or more groups independently chosen from: -CN, halogen, $C_{1-6}$ alkyl, $C_{2-6}$ alkenyl, $C_{2-6}$ alkynyl, -O($C_{1-6}$ alkyl), $C_{1-6}$ haloalkyl, -O($C_{1-6}$ haloalkyl), -$C_{1-6}$ alkylene-CN, -$C_{1-6}$ alkylene-OH, $C_{3-8}$ cycloalkyl, 4-8 membered heterocyclyl, and 5-6 membered heteroaryl, wherein the $C_{3-8}$ cycloalkyl, 4-8 membered heterocyclyl, or 5-6 membered heteroaryl, as a substituent of $R_2$, is each optionally substituted with one or more groups independently chosen from: -CN, halogen, $C_{1-6}$ alkyl, -O($C_{1-6}$ alkyl), $C_{1-6}$ haloalkyl, -O($C_{1-6}$ haloalkyl), -$C_{1-6}$ alkylene-CN, and -$C_{1-6}$ alkylene-OH;

or, when Y is $CR_3$ and n is not 0, $R_3$ and one $R_a$ together with the carbon atoms to which they are attached and the atom(s) among the carbon atoms form a 4-8 membered heterocyclic ring.

Also provided is a pharmaceutical composition, comprising the compound of formula (I) (e.g., a compound of any of the examples as described herein) and/or the pharmaceutically acceptable salt thereof according to the present invention, and optionally comprising a pharmaceutically acceptable excipient (e.g., a pharmaceutically acceptable carrier).

Also provided is a compound of formula (I) (e.g., a compound of any of the examples as described herein) and/or the pharmaceutically acceptable salt thereof according to the present invention for use as a pharmaceutical, e.g. in treating a disease mediated by CSF-1R or at least in part by CSF-1R in a subject.

Also provided is a compound of formula (I) (e.g., a compound of any of the examples as described herein) and/or the pharmaceutically acceptable salt thereof according to the present invention for use in treating an autoimmune disease, an inflammatory disease, a neurodegenerative disease, cancer, a metabolic disease, obesity, or an obesity-related disease in a subject.

## Detailed Description of the Invention

### Definitions

As used in the present application, the following words, phrases and symbols are generally intended to have the meanings as set forth below, except to the extent that the context in which they are used indicates otherwise.

A dash ("-") that is not between two letters or symbols is used to indicate a point of attachment for a substituent. For example, -O($C_{1-6}$ alkyl) refers to the attachment of $C_{1-6}$ alkyl to the rest of the molecule through an oxygen atom. When the point of attachment for a substituent is well known to the person of ordinary skill in the art ("POSITA"), "-" can be omitted, for example, a halogen substituent.

The term "alkyl" as used herein refers to a straight or branched saturated hydrocarbon radical containing 1-18 carbon atoms, preferably 1-10 carbon atoms, particularly preferably 1-6 carbon atoms, further preferably 1-4 (such as 1-3 or 1-2) carbon atoms. For example, "$C_{1-6}$ alkyl" refers to an alkyl containing 1-6 carbon atoms. Examples of alkyl include, but are not limited to, methyl ("Me"), ethyl ("Et"), n-propyl ("n-Pr"), i-propyl ("i-Pr"), n-butyl ("n-Bu"), i-butyl ("i-Bu"), s-butyl ("s-Bu") and t-butyl ("t-Bu").

The term "alkylene" as used herein refers to a straight or branched saturated divalent hydrocarbon radical containing 1-18 carbon atoms, preferably 1-10 carbon atoms, particularly preferably 1-6 carbon atoms, further preferably 1-4 (such as 1-3 or 1-2) carbon atoms. For example, "$C_{1-6}$ alkylene" refers to a straight or branched alkylene containing 1-6 carbon atoms, for example, straight alkylene-$(CH_2)_n$-, wherein n is an integer from 1 to 6, such as -$CH_2$-, -$CH_2$-$CH_2$-, -$CH_2$-$CH_2$-$CH_2$-, and the like, or a branched alkylene, for example, -$CH_2$-$CH(CH_3)$-$CH_2$-, -$CH(CH_3)$-$CH_2$-, -$C(CH_3)_2$-, and the like.

The term "alkenyl" as used herein refers to a straight or branched unsaturated hydrocarbon radical containing one or more, for example 1, 2, or 3 carbon-carbon double bonds (C=C) and 2-10 carbon atoms, preferably 2-6 carbon atoms, more preferably 2-4 carbon atoms. For example, "$C_{2-6}$ alkenyl" refers to an alkenyl containing 1, 2, or 3, preferably 1 or 2 carbon-carbon double bonds and 2-6 carbon atoms. Examples of alkenyl include, but are not limited to, vinyl, propenyl, allyl and 2-butenyl. The point of attachment for the alkenyl can be on or not on the double bonds.

The term "alkynyl" as used herein refers to a straight or branched unsaturated hydrocarbon radical containing one or more, for example 1, 2, or 3, carbon-carbon triple bonds (C≡C) and 2-10 carbon atoms, preferably 2-6 carbon atoms, more preferably 2-4 carbon atoms. For example, "$C_{2-6}$ alkynyl" refers to an alkynyl containing 1, 2, or 3, preferably 1 or 2 carbon-carbon triple bonds and 2-6 carbon atoms. Examples of alkynyl include, but are not limited to, ethynyl, 2-propynyl and 2-butynyl. The point of attachment for the alkynyl can be on or not on the triple bonds.

The term "halogen" or "halo" as used herein refers to fluoro, chloro, bromo, and iodo, preferably fluoro, chloro and

bromo, more preferably fluoro and chloro.

The term "haloalkyl" as used herein refers to an alkyl radical, as defined herein, in which one or more, for example 1, 2, 3, 4, 5, or 6, hydrogen atoms are replaced with halogen atoms, and when more than one hydrogen atoms are replaced with halogen atoms, the halogen atoms may be the same or different from each other. $C_{1-6}$ haloalkyl refers to an alkyl radical having 1-6 carbon atoms, in which one or more hydrogen atoms, for example 1, 2, 3, 4, 5 or 6 hydrogen atoms are replaced with halogen atoms. Examples of haloalkyl include, but are not limited to, $-CF_3$, $-CHF_2$, $-CH_2F$, $-CH_2CF_3$, $-CH(CF_3)_2$, and the like.

The term "cycloalkyl" as used herein refers to saturated or partially unsaturated cyclic hydrocarbon radical having 3-12 ring carbon atoms (such as 3-8 ring carbon atoms, 5-7 ring carbon atoms, 4-7 ring carbon atoms, 5-6 ring carbon atoms or 3-6 ring carbon atoms); which may have one or more rings, such as 1, 2, or 3 rings, preferably 1 or 2 rings. For example, "$C_{3-8}$ cycloalkyl" refers to a cycloalkyl containing 3-8 ring carbon atoms. The cycloalkyl may include a fused or bridged ring, or a spirocyclic ring. The rings of the cycloalkyl may be saturated or have one or more, for example, one or two double bonds (i.e. partially unsaturated), but not fully conjugated, and not an aryl as defined herein. Examples of cycloalkyl include, but are not limited to, cyclopropyl, cyclobutyl, cyclopentyl, cyclohexyl, cycloheptyl, cyclooctyl, bicyclo[4.1.0]heptanyl, bicyclo[3.1.1]heptanyl, spiro[3.3]heptanyl, spiro[2.2]pentanyl, cyclopropenyl, cyclobutenyl, cyclopentenyl, cyclopentadienyl, cyclohexenyl, cycloheptenyl, cyclooctenyl and bicyclo[3.1.1]hept-2-ene. In an embodiment of the present invention, the ring of cycloalkyl is saturated.

The term "heterocyclyl" or "heterocycle" as used herein refers to: saturated or partially unsaturated monocyclic, bicyclic or tricyclic radicals having 3-12 ring atoms (such as 3-8 ring atoms, 4-8 ring atoms, 5-7 ring atoms, 4-6 ring atoms, 3-6 ring atoms or 5-6 ring atoms), and containing one or more (such as 1, 2 or 3, preferably 1 or 2) ring heteroatoms independently chosen from N, O and S in the rings, with the remaining ring atoms being carbon. The heterocycle also includes those wherein the N or S heteroatom are optionally oxidized to various oxidation states. The point of attachment of heterocyclyl can be on the N heteroatom or carbon. For example, "3-12 membered heterocyclyl" or "3-12 membered heterocycle" refers to a heterocyclyl having 3-12 ring atoms, and containing at least one heteroatom chosen from N, O and S; "4-8 membered heterocyclyl" or "4-8 membered heterocycle" refers to a heterocyclyl having 4-8 ring atoms, and containing at least one heteroatom chosen from N, O and S. The heterocycle or heterocyclyl may include a fused or bridged ring, or a spirocyclic ring. The rings of the heterocycle or heterocyclyl may be saturated or have one or more, for example, one or two double bonds (i.e. partially unsaturated), but not fully conjugated, and not a heteroaryl as defined herein. In an embodiment of the present invention, the rings of heterocycle or heterocyclyl are saturated. Examples of heterocyclyl include, but are not limited to: oxiranyl, aziridinyl, oxetanyl, azetidinyl, pyrrolidyl, tetrahydrofuranyl, tetrahydropyranyl, dioxolanyl, dioxanyl, morpholinyl, thiomorpholinyl, piperidyl, piperazinyl, pyrazolidinyl, dihydrooxadiazolyl, and oxaspiro[3.3]heptanyl.

The term "aryl" or "aromatic ring" as used herein refers to carbocyclic hydrocarbon radical of 6 to 14 carbon atoms consisting of one ring or more fused rings, wherein at least one ring is an aromatic ring. Examples of aryl include, but are not limited to: phenyl, naphthyl, 1,2,3,4-tetrahydronaphthyl, indenyl, indanyl, azulenyl, preferably phenyl and naphthyl, most preferably phenyl.

The term "heteroaryl" or "heteroaromatic ring" as used herein refers to: aromatic hydrocarbyl (i.e., 5-14 membered heteroaryl, 5-12 membered heteroaryl, 5-10 membered heteroaryl, 5-6 membered heteroaryl or 6 membered heteroaryl) having 5-14 ring atoms (such as 5-12 ring atoms, 5-10 ring atoms, 5-6 ring atoms or 6 ring atoms), and containing one or more (such as 1, 2, 3 or 4, preferably 1, 2 or 3, more preferably 1 or 2) ring heteroatoms independently chosen from N, O and S in the rings, with the remaining ring atoms being carbon atoms; which may have one or more rings, such as 1, 2, or 3 rings, preferably 1 or 2 rings. For example, the heteroaryl includes:

monocyclic aromatic hydrocarbyl having 5, 6 or 7 ring atoms (preferably 5 or 6 ring atoms, namely, 5-6 membered heteroaryl), and containing one or more, for example 1, 2, 3 or 4, preferably 1, 2 or 3, more preferably 1 or 2 ring heteroatoms independently chosen from N, O and S (preferably N and O) in the ring, with the remaining ring atoms being carbon atoms; and

bicyclic aromatic hydrocarbyl having 8-12 ring atoms (preferably 9 or 10 ring atoms), and at least one of the rings contains one or more, such as 1, 2, 3 or 4, preferably 1, 2 or 3 ring heteroatoms independently chosen from N, O and S (preferably N), with the remaining ring atoms being carbon atoms, wherein at least one ring is aromatic ring. For example, bicyclic heteroaryl include a 5-6 membered heteroaryl ring fused with a 5-6 membered cycloalkyl ring.

When the total number of S and O atoms in the heteroaryl group exceeds 1, said S and O heteroatoms are not adjacent to one another.

Heteroaryl also include those in which the N ring atom is in the form of N-oxide, for example N-oxide pyridine.

Examples of heteroaryl include, but are not limited to: 5-6 membered heteroaryl, such as pyridyl, N-oxide pyridyl, pyrazinyl, pyrimidyl, triazinyl (such as 1,3,5-triazinyl), pyrazolyl, imidazolyl, oxazolyl, isoxazolyl, oxadiazolyl (such as 1,2,4-oxadiazolyl, 1,2,5-oxadiazolyl and 1,3,4-oxadiazolyl), thiazolyl, isothiazolyl, thiadiazolyl, tetrazolyl, triazolyl (such as

1,2,3-triazolyl and 1,2,4-triazolyl), thienyl, furanyl, pyranyl, pyrrolyl, and pyridazinyl; and bicyclic heteroaryl, such as benzodioxolyl, benzoxazolyl, benzoisoxazolyl, benzothienyl, benzothiazolyl, benzoisothiazolyl, imidazopyridyl (such as imidazo[1,2-a]pyridyl), imidazopyridazinyl (such as imidazo[1,2-b]pyridazinyl), pyrrolopyridyl (such as 1H-pyrrolo[2,3-b] pyridyl), pyrrolopyrimidyl (such as pyrrolo[3,4-d]pyrimidyl), pyrazolopyridyl (such as 1H-pyrazolo[3,4-b]pyridyl), pyrazolopyrimidyl (such as pyrazolo[1,5-a]pyrimidyl), triazolopyridyl (such as [1,2,4]triazolo[4,3-a]pyridyl and [1,2,4]triazolo[1,5-a]pyridyl), triazolopyridazinyl (such as [1,2,4]triazolo[4,3-b]pyridazinyl), tetrazolopyridyl (such as tetrazolo[1,5-a]pyridyl), benzofuranyl, benzoimidazolinyl, indolyl, indazolyl, purinyl, quinolinyl, isoquinolinyl, and quinazolinyl.

The term "hydroxyl" as used herein refers to -OH group.

The term "oxo" as used herein refers to =O group.

The term "cyano" as used herein refers to -CN group.

The term "optional" or "optionally" as used herein means that the subsequently described event or circumstance may or may not occur, and the description includes instances wherein the event or circumstance occur and instances in which it does not occur. For example, "optionally substituted alkyl" includes "unsubstituted alkyl" and "substituted alkyl" defined herein. It will be understood by the POSITA, with respect to any group containing one or more substituents, that such groups are not intended to introduce any substitution or substitution patterns that are sterically impractical, chemically incorrect, synthetically non-feasible and/or inherently unstable.

The term "substituted" or "substituted with...", as used herein, means that one or more hydrogen atoms on the designated atom or group are replaced with one or more substituents chosen from the indicated group of substituents, provided that the designated atom's normal valence is not exceeded. When a substituent is oxo (i.e., =O), then two hydrogens on a single atom are replaced by the oxo. Combinations of substituents and/or variables are permitted only when they result in chemically correct and stable compounds. A chemically correct and stable compound is meant to imply a compound that is sufficiently robust to survive sufficient isolation from a reaction mixture, and then can be formulated into a formulation having at least practical utility.

Unless otherwise specified, substituents are named into the core structure. For example, it is to be understood that when (cycloalkyl)alkyl is listed as a possible substituent, the point of attachment of this substituent to the core structure is in the alkyl portion.

The term "substituted with one or more groups" as used herein means that one or more hydrogens on the designated atom or group are independently replaced with one or more substituents chosen from indicated group. In some embodiments, "substituted with one or more groups" means the designated atom or group is replaced with 1, 2, 3 or 4 substituents independently chosen from designated group.

It will be understood by the POSITA that some of the compounds of formula (I) may contain one or more chiral centers and therefore exist in two or more stereoisomeric forms. The racemates of these isomers, the individual isomers and mixtures enriched in one enantiomer, as well as diastereomers when there are two chiral centers, and mixtures partially enriched with specific diastereomers are within the scope of the present invention. It will be further understood by the POSITA that the present invention includes all the individual stereoisomers (e.g. enantiomers, e.g. (R) isomers or (S) isomers), racemic mixtures or partially resolved mixtures of the compounds of formula (I) and, where appropriate, the individual tautomeric forms thereof.

In some embodiments, the present invention provides compounds of various stereoisomeric purities, that is, enantiomeric or diastereomeric purity expressed in different "ee" or "de" values. In some embodiments, the compound of formula (I) described herein has an enantiomeric purity of at least 60% ee (e.g., 60%, 65%, 70%, 75%, 80%, 85%, 90%, 91%, 92%, 93%, 94%, 95%, 96%, 97%, 98%, 99%, 99.5%, 99.9% ee, or any value between these listed values). In some embodiments, the compound of formula (I) described herein has an enantiomeric purity of greater than 99.9% ee, extending up to 100% ee. In some embodiments, the compound of formula (I) described herein has a diastereomeric purity of at least 60% de (e.g., 60%, 65%, 70%, 75%, 80%, 85%, 90%, 91%, 92%, 93%, 94%, 95%, 96%, 97%, 98%, 99%, 99.5%, 99.9% de, or any value between these listed values). In some embodiments, the compound of formula (I) described herein has a diastereomeric purity of greater than 99.9% de.

The term "enantiomeric excess" or "ee" refers to the amount of one enantiomer relative to the other. For a mixture of R and S enantiomers, the percentage of enantiomeric excess is defined as $| R - S | *100$, where R and S are the mole or weight fractions of the respective enantiomers in the mixture, $R + S = 1$. If the optical rotation of a chiral substance is known, the percentage of enantiomeric excess is defined as ([a]obs/[a]max)* 100, wherein [a]obs is the optical rotation of the enantiomeric mixture, and [a]max is the optical rotation of the pure enantiomer.

The term "diastereomeric excess" or "de" refers to the amount of one diastereomer relative to the other, and is defined by analogy based on the enantiomeric excess. Therefore, for a mixture of diastereomers D1 and D2, the percentage of diastereomeric excess is defined as $| D1 - D2 | *100$, wherein D1 and D2 are the mole or weight fractions of the respective diastereomers in the mixture, $D1 + D2 = 1$.

The diastereomeric excess and enantiomeric excess can be measured by a number of analytical techniques (including nuclear magnetic resonance spectroscopy, chiral column chromatography and/or optical polarimetry) according to conventional protocols well known to a person skilled in the art.

The racemates can be used as such or can be resolved into their individual isomers. The resolution can afford stereochemically pure compounds or mixtures enriched in one or more isomers. Methods for separation of isomers are well known (cf. Allinger N. L. and Eliel E. L. in "Topics in Stereochemistry", Vol. 6, Wiley Interscience, 1971) and include physical methods such as chromatography using a chiral adsorbent. Individual isomers can be prepared in chiral form from chiral precursors. Alternatively, individual isomers can be separated chemically from a mixture by forming diastereomeric salts with a chiral acid, such as the individual enantiomers of 10-camphorsulfonic acid, camphoric acid, alpha-bromocamphoric acid, tartaric acid, diacetyltartaric acid, malic acid, pyrrolidone-5-carboxylic acid, and the like, fractionally crystallizing the salts, and then freeing one or both of the resolved bases, optionally repeating the process, so as obtain either or both substantially free of the other; i.e., in a form having an optical purity of > 95%. Alternatively, the racemates can be covalently linked to a chiral compound (auxiliary) to produce diastereomers which can be separated by chromatography or by fractional crystallization after which time the chiral auxiliary is chemically removed to afford the pure enantiomers.

The term "tautomer" as used herein refers to constitutional isomers of compounds generated by rapid movement of an atom in two positions in a molecule. Tautomers readily interconvert into each other, e.g., enol form and ketone form are typical tautomers.

A "pharmaceutically acceptable salt" is intended to mean a salt of a free acid or base of a compound of formula (I) that is non-toxic, biologically tolerable, or otherwise biologically suitable for administration to the subject. For example, the pharmaceutically acceptable salt is an acid addition salt including such as a salt derived from an inorganic acid and an organic acid. Said inorganic acid includes such as hydrochloric acid, hydrobromic acid, hydroiodic acid, sulfuric acid, phosphoric acid, and nitric acid; said organic acid includes such as p-toluenesulfonic acid, salicylic acid, methanesulfonic acid, oxalic acid, succinic acid, citric acid, malic acid, lactic acid, fumaric acid, and the like. For examples, see, generally, S. M. Berge, et al., "Pharmaceutical Salts", J. Pharm. Sci., 1977, 66:1-19, and Handbook of Pharmaceutical Salts, Properties, Selection, and Use, Stahl and Wermuth, Eds., Wiley-VCH and VHCA, Zurich, 2002.

In addition, if a compound of the present invention herein is obtained as an acid addition salt, the free base can be obtained by basifying a solution of the acid addition salt. Conversely, if the product is a free base, an acid addition salt, particularly a pharmaceutically acceptable acid addition salt, may be produced by dissolving the free base in a suitable solvent and treating the solution with an acid, in accordance with conventional procedures for preparing acid addition salts from base compounds. The POSITA will recognize various synthetic methodologies that may be used without undue experimentation to prepare non-toxic pharmaceutically acceptable acid addition salts or base addition salts.

The term "solvates" means solvent addition forms that contain either stoichiometric or non-stoichiometric amounts of solvent. Some compounds have a tendency to trap a fixed molar ratio of solvent molecules in the solid state, thus forming a solvate. If the solvent is water, the solvate formed is a hydrate, when the solvent is alcohol, the solvate formed is an alcoholate. Hydrates are formed by the combination of one or more molecules of water, with one molecule of the substances in which the water retains its molecular state as $H_2O$, such combination being able to form one or more hydrates, for example, hemihydrate, monohydrate, and dihydrate.

As used herein, the terms "group(s)" and "radical(s)" are synonymous and are intended to indicate functional groups or fragments of molecules attachable to other fragments of molecules.

The term "active ingredient" is used to indicate a chemical substance which has biological activity, such as the compound of formula (I) of the present invention (e.g., a compound of any of the examples as described herein) and/or a pharmaceutically acceptable salt thereof. In some embodiments, an "active ingredient" is a chemical substance having pharmaceutical utility, and its pharmaceutical activity can be determined by appropriate *in vitro* or *in vivo* trials (for example, preclinical or clinical trials).

The terms "treating" or "treatment" of a disease or disorder, in the context of achieving therapeutic benefit, refer to administering one or more pharmaceutical substances, especially the compound of formula (I) or a pharmaceutically acceptable salt thereof described herein to a subject that has the disease or disorder, or has a symptom of a disease or disorder, or has a predisposition toward a disease or disorder, with the purpose to cure, heal, alleviate, relieve, alter, remedy, ameliorate, improve, or affect the disease or disorder, the symptoms of the disease or disorder, or the predisposition toward the disease or disorder. In some embodiment, the disease or disorder is an autoimmune disease or inflammatory disease. In some embodiments, the disease or disorder is a neurodegenerative disease.

The terms "treating", "contacting" and "reacting", in the context of a chemical reaction, mean adding or mixing two or more reagents under appropriate conditions to produce the indicated and/or the desired product. It should be appreciated that the reaction which produces the indicated and/or the desired product may not necessarily result directly from the combination of two reagents which were initially added, i.e., there may be one or more intermediates which are produced in the mixture which ultimately lead to the formation of the indicated and/or the desired product.

The term "effective amount" as used herein refers to an amount of the compound of the present invention sufficient to generally bring about a therapeutic benefit in patients in need of treatment for a disease or disorder mediated by CSF-1R activity or at least in part by CSF-1R. Effective amounts of the active ingredient of the present disclosure may be ascertained by methods such as modeling, dose escalation studies or clinical trials, and by taking into consideration

factors, e.g., the route of administration, the pharmacokinetics of the agent, the severity of the disease or disorder, the subject's previous or ongoing therapy, the subject's health status and response to drugs, and the judgment of the attending physician.

An exemplary dose is in the range of from about 0.0001 to about 200 mg of active agent per kg of subject's body weight per day, such as from about 0.001 to 100 mg/kg/day, or about 0.01 to 35 mg/kg/day, or about 0.1 to 10 mg/kg daily in single or divided dosage units (e.g., BID, TID, QID). For a 70 kg person, an illustrative range of a suitable dose is from about 0.05 to about 7 g/day, or from about 0.2 to about 5 g/day.

The term "inhibition" or "inhibiting" refers to a decrease in the baseline activity of a biological activity. The term "inhibition of CSF-1R activity" refers to a decrease in the activity of CSF-1R as a direct or indirect response to the presence of the compound of formula (I) and/or a pharmaceutically acceptable salt thereof described herein, relative to the activity of CSF-1R in the absence of the compound of formula (I) and/or a pharmaceutically acceptable salt thereof. The decrease in activity may be due to the direct interaction of the compound of formula (I) and/or a pharmaceutically acceptable salt thereof described herein with CSF-1R, or due to the interaction of the compound of formula (I) and/or a pharmaceutically acceptable salt thereof described herein, with one or more other factors that in turn affect the CSF-1R activity. For example, the presence of the compound of formula (I) and/or a pharmaceutically acceptable salt thereof described herein may decrease the CSF-1R activity by directly binding to the CSF-1R, by directly or indirectly influencing another factor, or by directly or indirectly decreasing the amount of CSF-1R present in the cell or organism.

The term "subject" as used herein means mammals and non-mammals. Mammals means any member of the mammalia class including, but not limited to, humans; non-human primates such as chimpanzees and other apes and monkey species; farm animals such as cattle, horses, sheep, goats, and swine; domestic animals such as rabbits, dogs, and cats; laboratory animals including rodents, such as rats, mice, and guinea pigs; and the like. Examples of non-mammals include, but are not limited to, birds, and the like. The term "subject" does not denote a particular age or sex. In some embodiments, the subject is a human.

The term "about" as used herein means approximately, in the region of, roughly, or around. When the term "about" is used in conjunction with a numerical range, it adjusts the range by extending the limit above or below the numerical value given. In general, the term "about" is used herein to modify a numerical value above or below the stated value by a variance of 20%.

Technical and scientific terms used herein and not specifically defined have the meaning commonly understood by the POSITA to which the present disclosure pertains.

## Detailed Description of Embodiments

Embodiment 1. A compound of formula (I):

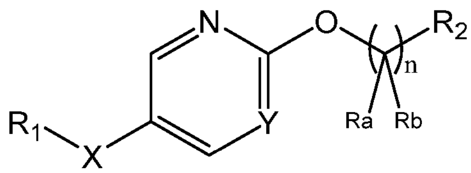

(I)

or a pharmaceutically acceptable salt thereof, or a solvate, a racemic mixture, an enantiomer, a diastereomer or a tautomer thereof, wherein

$R_1$ is chosen from:

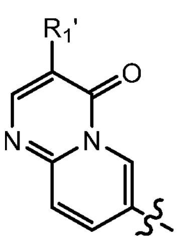,
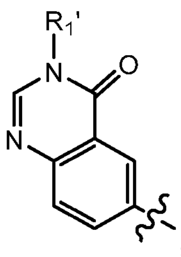,
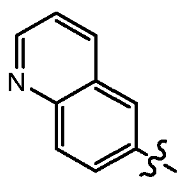,
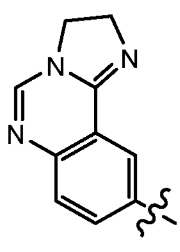

and

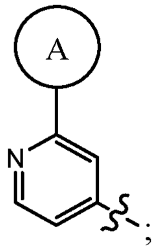

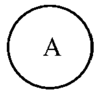

is chosen from phenyl and 5-6 membered heteroaryl, each of which is optionally substituted with one or more groups independently chosen from: -CN, halogen, $C_{1-6}$ alkyl, -O($C_{1-6}$ alkyl), $C_{1-6}$ haloalkyl, -O($C_{1-6}$ haloalkyl), -$C_{1-6}$ alkylene-CN, and -$C_{1-6}$ alkylene-OH;

$R_1$' is chosen from H, halogen, -CN, $C_{1-6}$ alkyl, $C_{1-6}$ haloalkyl, -$C_{1-6}$ alkylene-CN, - $C_{1-6}$ alkylene-OH, -O($C_{1-6}$ alkyl), -O($C_{1-6}$ haloalkyl), $C_{3-8}$ cycloalkyl, 4-8 membered heterocyclyl, and $NR_4R_5$; $R_4$ and $R_5$ are each independently chosen from H, halogen, -CN, $C_{1-6}$ alkyl, $C_{1-6}$ haloalkyl, -$C_{1-6}$ alkylene-CN, -$C_{1-6}$ alkylene-OH, and -O($C_{1-6}$ alkyl); or $R_4$ and $R_5$ together with the N atom to which they are attached form a 4-8 membered heterocyclic ring;

X is O or $CR_6R_7$; $R_6$ and $R_7$ are each independently chosen from H, halogen, $C_{1-6}$ alkyl, $C_{1-6}$ haloalkyl, -$C_{1-6}$ alkylene-CN, -$C_{1-6}$ alkylene-OH, -O($C_{1-6}$ alkyl) and -O($C_{1-6}$ haloalkyl);

Y is N or $CR_3$; $R_3$ is chosen from H, -CN, halogen, $C_{1-6}$ alkyl, -O($C_{1-6}$ alkyl), -$C_{1-6}$ alkylene-CN, -$C_{1-6}$ alkylene-OH, $C_{1-6}$ haloalkyl, and -O($C_{1-6}$ haloalkyl);

$R_a$ and $R_b$ are each independently chosen from H, halogen, -CN, $C_{1-6}$ alkyl, $C_{1-6}$ haloalkyl, -$C_{1-6}$ alkylene-CN, -$C_{1-6}$ alkylene-OH, -O($C_{1-6}$ alkyl) and -O($C_{1-6}$ haloalkyl);

n is 0, 1, 2, 3, or 4;

$R_2$ is phenyl or 5-10 membered heteroaryl, each of which is optionally substituted with one or more groups independently chosen from: -CN, halogen, $C_{1-6}$ alkyl, $C_{2-6}$ alkenyl, $C_{2-6}$ alkynyl, -O($C_{1-6}$ alkyl), $C_{1-6}$ haloalkyl, -O($C_{1-6}$ haloalkyl), -$C_{1-6}$ alkylene-CN, -$C_{1-6}$ alkylene-OH, $C_{3-8}$ cycloalkyl, 4-8 membered heterocyclyl, and 5-6 membered heteroaryl, wherein the $C_{3-8}$ cycloalkyl, 4-8 membered heterocyclyl, or 5-6 membered heteroaryl, as a substituent of $R_2$, is each optionally substituted with one or more groups independently chosen from: -CN, halogen, $C_{1-6}$ alkyl, -O($C_{1-6}$ alkyl), $C_{1-6}$ haloalkyl, -O($C_{1-6}$ haloalkyl), -$C_{1-6}$ alkylene-CN, and -$C_{1-6}$ alkylene-OH;

or, when Y is $CR_3$ and n is not 0, $R_3$ and one $R_a$ together with the carbon atoms to which they are attached and the atom(s) among the carbon atoms form a 4-8 membered heterocyclic ring.

Embodiment 2. The compound, or the pharmaceutically acceptable salt thereof, or the solvate, the racemic mixture, the enantiomer, the diastereomer or the tautomer thereof according to embodiment 1, wherein $R_1$ is chosen from

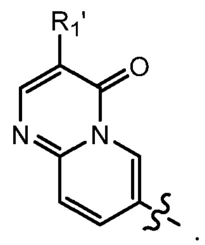

Embodiment 3. The compound, or the pharmaceutically acceptable salt thereof, or the solvate, the racemic mixture, the enantiomer, the diastereomer or the tautomer thereof according to embodiment 2, wherein $R_1$' is chosen from H, $C_{1-6}$ alkyl, $C_{3-8}$ cycloalkyl, and $NR_4R_5$; $R_4$ and $R_5$ are both H; or $R_4$ and $R_5$ together with the N atom to which they are attached form a 4-8 membered heterocyclic ring, preferably form a 5 or 6 membered heterocyclic ring which, in addition to the N atom to which $R_4$ and $R_5$ are attached, contains 0, 1, or 2 heteroatoms independently chosen from N, O or S, and more preferably form a morpholine ring.

Embodiment 4. The compound, or the pharmaceutically acceptable salt thereof, or the solvate, the racemic mixture, the enantiomer, the diastereomer or the tautomer thereof according to embodiment 3, wherein $R_1$' is chosen from H and $C_{1-6}$ alkyl, preferably $R_1$' is chosen from H and $C_{1-3}$ alkyl, and more preferably, $R_1$' is chosen from H and methyl.

Embodiment 5. The compound, or the pharmaceutically acceptable salt thereof, or the solvate, the racemic mixture, the enantiomer, the diastereomer or the tautomer thereof according to embodiment 1, wherein $R_1$ is chosen from

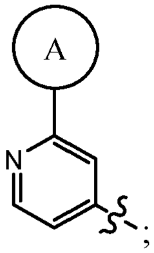

and

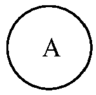

is 5-6 membered heteroaryl, which is optionally substituted with one or more groups independently chosen from: $C_{1-6}$ alkyl and -$C_{1-6}$ alkylene-OH; preferably,

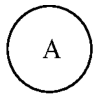

is pyrazolyl, which is optionally substituted with one or more groups independently chosen from: $C_{1-6}$ alkyl and -$C_{1-6}$ alkylene-OH; more preferably,

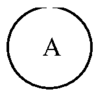

is pyrazolyl, which is optionally substituted with one or more groups independently chosen from: $C_{1-6}$ alkyl; further preferably,

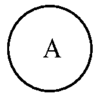

is pyrazolyl substituted with one $C_{1-3}$ alkyl; and most preferably,

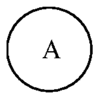

is 1-methyl-1*H*-pyrazol-4-yl.

Embodiment 6. The compound, or the pharmaceutically acceptable salt thereof, or the solvate, the racemic mixture, the enantiomer, the diastereomer or the tautomer thereof according to any one of embodiments 1-5, wherein X is O or $CH_2$; and preferably, X is O.

Embodiment 7. The compound, or the pharmaceutically acceptable salt thereof, or the solvate, the racemic mixture, the enantiomer, the diastereomer or the tautomer thereof according to any one of embodiments 1-6, wherein Y is N or $CR_3$; and $R_3$ is chosen from H, -CN, halogen, $C_{1-6}$ alkyl, -O($C_{1-6}$ alkyl), and -O($C_{1-6}$ haloalkyl).

Embodiment 8. The compound, or the pharmaceutically acceptable salt thereof, or the solvate, the racemic mixture, the enantiomer, the diastereomer or the tautomer thereof according to embodiment 7, wherein Y is $CR_3$, and $R_3$ is chosen from H, -CN, halogen, $C_{1-6}$ alkyl, -O($C_{1-6}$ alkyl), or -O($C_{1-6}$ haloalkyl); preferably, $R_3$ is -O($C_{1-6}$ alkyl); and more preferably, $R_3$ is -O($C_{1-3}$ alkyl).

Embodiment 9. The compound, or the pharmaceutically acceptable salt thereof, or the solvate, the racemic mixture, the enantiomer, the diastereomer or the tautomer thereof according to any one of embodiments 1-8, wherein $R_a$ and $R_b$ are both H, and n is 0, 1 or 2; and preferably, $R_a$ and $R_b$ are both H, and n is 1.

Embodiment 10. The compound, or the pharmaceutically acceptable salt thereof, or the solvate, the racemic

mixture, the enantiomer, the diastereomer or the tautomer thereof according to any one of embodiments 1-9, wherein $R_2$ is phenyl or 5-6 membered heteroaryl, each of which is optionally substituted with one or more groups independently chosen from: halogen, $C_{1-6}$ alkyl, $C_{2-6}$ alkynyl, -O($C_{1-6}$ alkyl), -$C_{1-6}$ alkylene-CN, -$C_{1-6}$ alkylene-OH, $C_{3-8}$ cycloalkyl, and 5-6 membered heteroaryl, wherein the $C_{3-8}$ cycloalkyl or 5-6 membered heteroaryl, as a substituent of $R_2$, is each optionally substituted with one or more halogen.

Embodiment 11. The compound, or the pharmaceutically acceptable salt thereof, or the solvate, the racemic mixture, the enantiomer, the diastereomer or the tautomer thereof according to embodiment 10, wherein $R_2$ is phenyl or pyridyl, each of which is optionally substituted with one or more groups independently chosen from: halogen, $C_{1-6}$ alkyl, -O($C_{1-6}$ alkyl), and $C_{3-8}$ cycloalkyl, wherein the $C_{3-8}$ cycloalkyl, as a substituent of $R_2$, is optionally substituted with one or more halogen.

Embodiment 12. The compound, or the pharmaceutically acceptable salt thereof, or the solvate, the racemic mixture, the enantiomer, the diastereomer or the tautomer thereof according to embodiment 1, wherein the compound has a structure of formula (I-1a):

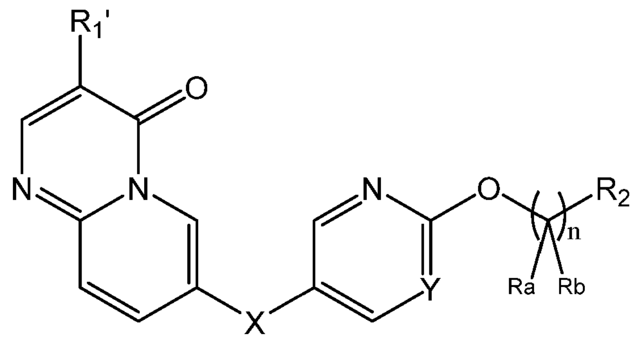

(I-1a)

wherein

$R_1$' is chosen from H, $C_{1-6}$ alkyl, $C_{3-8}$ cycloalkyl, and $NR_4R_5$; $R_4$ and $R_5$ are both H; or $R_4$ and $R_5$ together with the N atom to which they are attached form a 4-8 membered heterocyclic ring, preferably form a 5 or 6 membered heterocyclic ring which, in addition to the N atom to which $R_4$ and $R_5$ are attached, contains 0, 1, or 2 heteroatoms independently chosen from N, O or S, and more preferably form a morpholine ring;
X is O or $CH_2$;
Y is N or $CR_3$; $R_3$ is chosen from H, -CN, halogen, $C_{1-6}$ alkyl, -O($C_{1-6}$ alkyl), and - O($C_{1-6}$ haloalkyl);
$R_a$ and $R_b$ are both H;
n is 0, 1 or 2; and
$R_2$ is phenyl, which is optionally substituted with one or more groups independently chosen from: halogen, $C_{1-6}$ alkyl, $C_{2-6}$ alkynyl, -O($C_{1-6}$ alkyl), -$C_{1-6}$ alkylene-CN, -$C_{1-6}$ alkylene-OH, $C_{3-8}$ cycloalkyl, and 5-6 membered heteroaryl, wherein the $C_{3-8}$ cycloalkyl or 5-6 membered heteroaryl, as a substituent of $R_2$, is each optionally substituted with one or more halogen.

Embodiment 13. The compound, or the pharmaceutically acceptable salt thereof, or the solvate, the racemic mixture, the enantiomer, the diastereomer or the tautomer thereof according to embodiment 12, wherein

$R_1$' is chosen from H and $C_{1-6}$ alkyl;
X is O;
Y is $CR_3$; $R_3$ is chosen from H, -CN, halogen, $C_{1-6}$ alkyl, -O($C_{1-6}$ alkyl), and -O($C_{1-6}$ haloalkyl);
$R_a$ and $R_b$ are both H;
n is 1; and
$R_2$ is phenyl, which is optionally substituted with one or more groups independently chosen from: halogen, $C_{1-6}$ alkyl, -O($C_{1-6}$ alkyl), and $C_{3-8}$ cycloalkyl, wherein the $C_{3-8}$ cycloalkyl, as a substituent of $R_2$, is optionally substituted with one or more halogen.

Embodiment 14. The compound, or the pharmaceutically acceptable salt thereof, or the solvate, the racemic mixture, the enantiomer, the diastereomer or the tautomer thereof according to embodiment 1, wherein the compound has a structure of formula (I-1b):

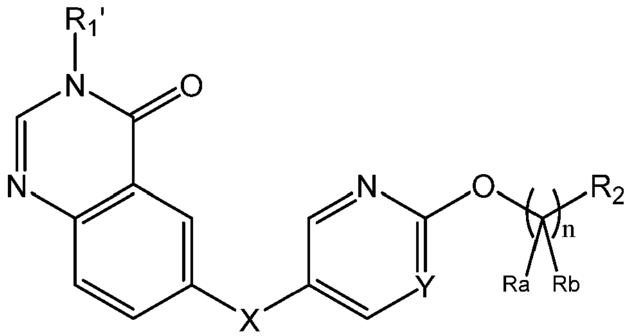

(I-1b)

wherein

$R_1$' is chosen from H and $C_{1-6}$ alkyl;
X is O;
Y is $CR_3$; $R_3$ is chosen from H, $C_{1-6}$ alkyl, -O($C_{1-6}$ alkyl), and -O($C_{1-6}$ haloalkyl);
$R_a$ and $R_b$ are both H;
n is 1 or 2; and
$R_2$ is phenyl, which is optionally substituted with one or more groups independently chosen from: halogen, $C_{1-6}$ alkyl, -O($C_{1-6}$ alkyl), and $C_{3-8}$ cycloalkyl, wherein the $C_{3-8}$ cycloalkyl, as a substituent of $R_2$, is optionally substituted with one or more halogen.

Embodiment 15. The compound, or the pharmaceutically acceptable salt thereof, or the solvate, the racemic mixture, the enantiomer, the diastereomer or the tautomer thereof according to embodiment 1, wherein the compound has a structure of formula (I-1c):

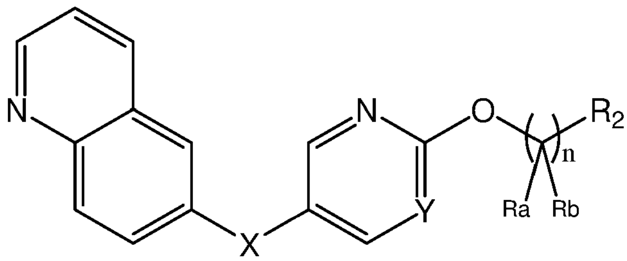

(I-1c)

wherein

X is O;
Y is $CR_3$; $R_3$ is chosen from H, $C_{1-6}$ alkyl, -O($C_{1-6}$ alkyl), and -O($C_{1-6}$ haloalkyl);
$R_a$ and $R_b$ are both H;
n is 1 or 2; and
$R_2$ is phenyl, which is optionally substituted with one or more groups independently chosen from: halogen, $C_{1-6}$ alkyl, -O($C_{1-6}$ alkyl), and $C_{3-8}$ cycloalkyl, wherein the $C_{3-8}$ cycloalkyl, as a substituent of $R_2$, is optionally substituted with one or more halogen.

Embodiment 16. The compound, or the pharmaceutically acceptable salt thereof, or the solvate, the racemic mixture, the enantiomer, the diastereomer or the tautomer thereof according to embodiment **1,** wherein the compound has a structure of formula (I-1d):

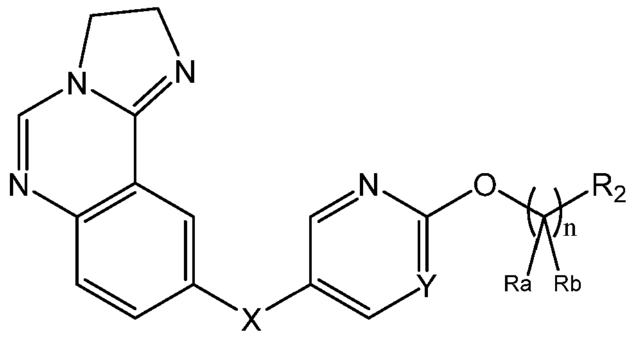

(I-1d)

wherein

X is O;
Y is $CR_3$; $R_3$ is chosen from H, $C_{1-6}$ alkyl, -O($C_{1-6}$ alkyl), and -O($C_{1-6}$ haloalkyl);
$R_a$ and $R_b$ are both H;
n is 1 or 2; and
$R_2$ is phenyl, which is optionally substituted with one or more groups independently chosen from: halogen, $C_{1-6}$ alkyl, -O($C_{1-6}$ alkyl), and $C_{3-8}$ cycloalkyl, wherein the $C_{3-8}$ cycloalkyl, as a substituent of $R_2$, is optionally substituted with one or more halogen.

Embodiment 17. The compound, or the pharmaceutically acceptable salt thereof, or the solvate, the racemic mixture, the enantiomer, the diastereomer or the tautomer thereof according to embodiment 1, wherein the compound has a structure of formula (I-1e):

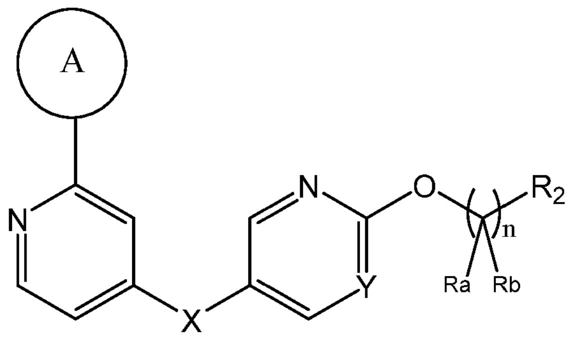

(I-1e)

wherein

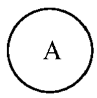

is 5-6 membered heteroaryl, which is optionally substituted with one or more groups independently chosen from: $C_{1-6}$ alkyl;
X is O or $CH_2$;
Y is $CR_3$; $R_3$ is chosen from H, $C_{1-6}$ alkyl, -O($C_{1-6}$ alkyl), and -O($C_{1-6}$ haloalkyl);
$R_a$ and $R_b$ are both H;
n is 1 or 2; and
$R_2$ is phenyl, which is optionally substituted with one or more groups independently chosen from: halogen, $C_{1-6}$ alkyl, -O($C_{1-6}$ alkyl), and $C_{3-8}$ cycloalkyl, wherein the $C_{3-8}$ cycloalkyl, as a substituent of $R_2$, is optionally substituted with one or more halogen.

Embodiment 18. The compound, or the pharmaceutically acceptable salt thereof, or the solvate, the racemic mixture, the enantiomer, the diastereomer or the tautomer thereof according to embodiment 17, wherein

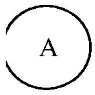

is pyrazolyl, which is optionally substituted with one or more groups independently chosen from: $C_{1-6}$ alkyl; preferably,

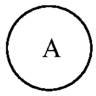

is pyrazolyl substituted with one $C_{1-3}$ alkyl; and more preferably,

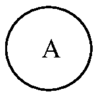

is 1-methyl-1H-pyrazol-4-yl.

Embodiment 19. The compound, or the pharmaceutically acceptable salt thereof, or the solvate, the racemic mixture, the enantiomer, the diastereomer or the tautomer thereof according to embodiment 1, wherein when Y is $CR_3$ and n is not 0, $R_3$ and one $R_a$ together with the carbon atoms to which they are attached and the atom(s) among the carbon atoms form a 5-6 membered heterocyclic ring.

Embodiment 20. The compound, or the pharmaceutically acceptable salt thereof, or the solvate, the racemic mixture, the enantiomer, the diastereomer or the tautomer thereof according to embodiment 19, wherein the compound has a structure of formula (I-2) or formula (I-3); and preferably, the compound has a structure of formula (I-2):

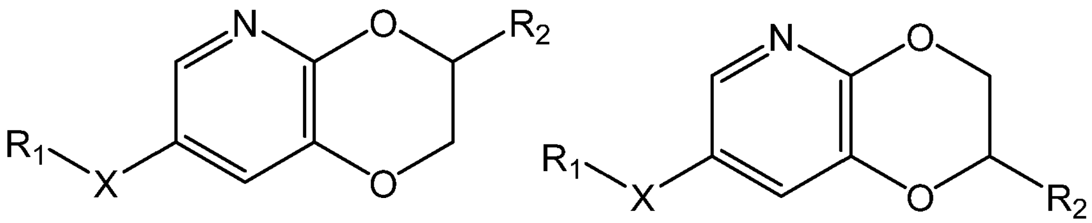

(I-2) (I-3) ,

wherein $R_1$, $R_2$ and X are as defined in embodiment 1.

Embodiment 21. The compound, or the pharmaceutically acceptable salt thereof, or the solvate, the racemic mixture, the enantiomer, the diastereomer or the tautomer thereof according to embodiment 20, wherein the compound has a structure of formula (I-2a):

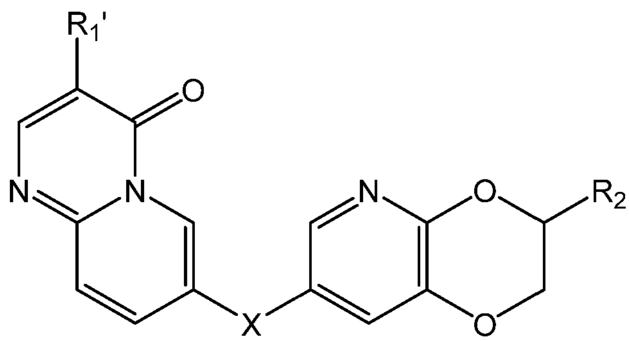

(I-2a)

wherein

$R_1$' is chosen from H and $C_{1-6}$ alkyl;
X is O; and
$R_2$ is phenyl or 5-6 membered heteroaryl, each of which is optionally substituted with one or more groups independently chosen from: halogen, $C_{1-6}$ alkyl, -O($C_{1-6}$ alkyl), and $C_{3-8}$ cycloalkyl, wherein the $C_{3-8}$ cycloalkyl, as a substituent of $R_2$, is optionally substituted with one or more halogen; and preferably, $R_2$ is phenyl or pyridyl, each of which is optionally substituted with one or more groups independently chosen from: - O($C_{1-6}$ alkyl) and $C_{3-8}$ cycloalkyl.

Embodiment 22. The compound, or the pharmaceutically acceptable salt thereof, or the solvate, the racemic mixture, the enantiomer, the diastereomer or the tautomer thereof according to embodiment 20, wherein the compound has a structure of formula (I-2b):

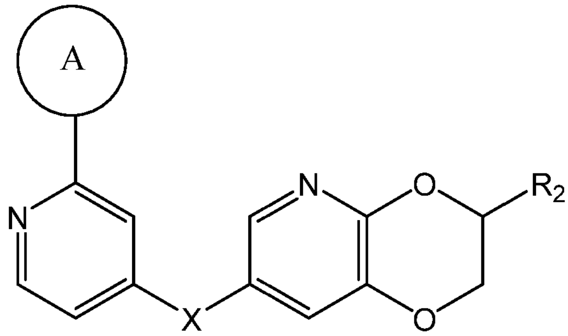

(I-2b)

wherein

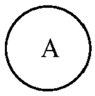

is 5-6 membered heteroaryl, which is optionally substituted with one or more groups independently chosen from: $C_{1-6}$ alkyl and -$C_{1-6}$ alkylene-OH; preferably,

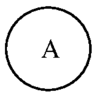

is pyrazolyl, which is optionally substituted with one or more groups independently chosen from: $C_{1-6}$ alkyl and -$C_{1-6}$ alkylene-OH; more preferably,

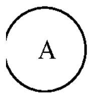

is pyrazolyl, which is optionally substituted with one or more groups independently chosen from: $C_{1-6}$ alkyl; further preferably,

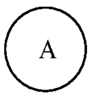

is pyrazolyl substituted with one $C_{1-3}$ alkyl; and most preferably,

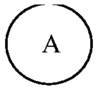

is 1-methyl-1*H*-pyrazol-4-yl;
X is O; and
$R_2$ is phenyl or 5-6 membered heteroaryl, each of which is optionally substituted with one or more groups independently chosen from: halogen, $C_{1-6}$ alkyl, -O($C_{1-6}$ alkyl), and $C_{3-8}$ cycloalkyl, wherein the $C_{3-8}$ cycloalkyl, as a substituent of $R_2$, is optionally substituted with one or more halogen; preferably, $R_2$ is phenyl or pyridyl, each of which is optionally substituted with one or more groups independently chosen from: -O($C_{1-6}$ alkyl) and $C_{3-8}$ cycloalkyl; and more preferably, $R_2$ is pyridyl, which is optionally substituted with one or more groups independently chosen from: -O($C_{1-6}$ alkyl) and $C_{3-8}$ cycloalkyl.

Embodiment 23. The compound of formula (I) according to embodiment 1, which is chosen from the following compounds or pharmaceutically acceptable salts thereof

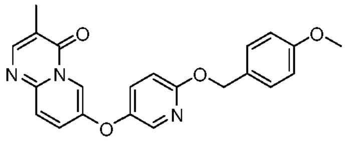

1

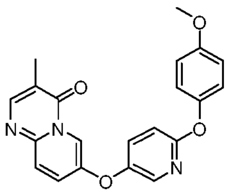

2

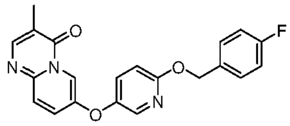

3

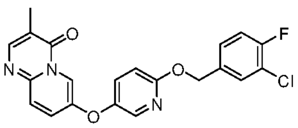

4

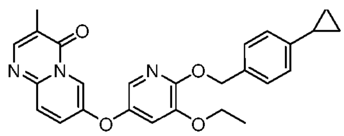

5

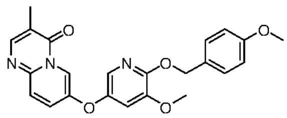

6

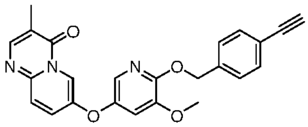

7

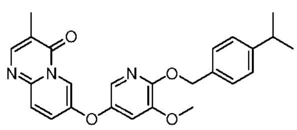

8

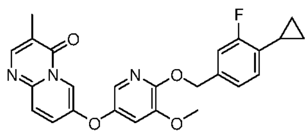

9

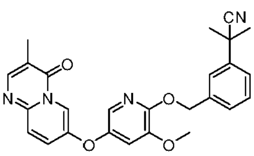

10

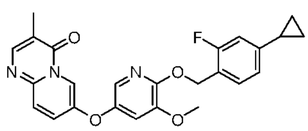

11

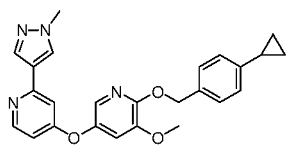

12

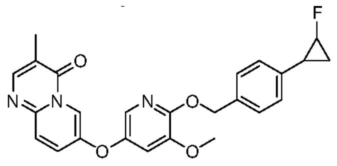
13

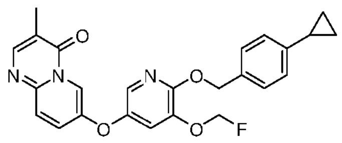
14

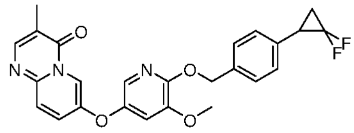
15

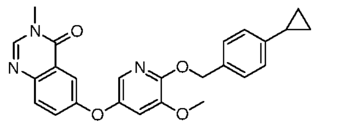
16

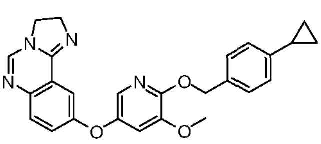
17

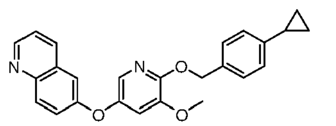
18

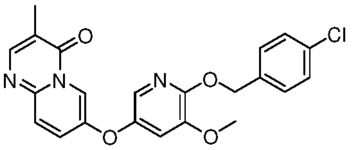
19

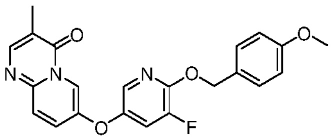
20

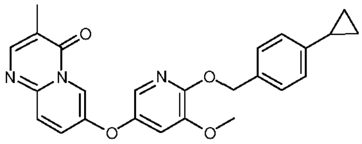
21

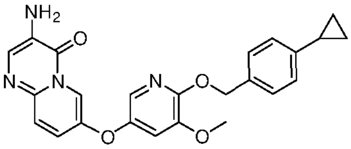
22

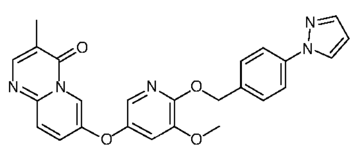
23

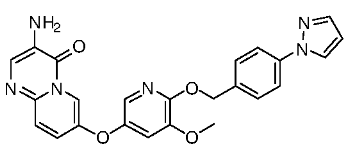
24

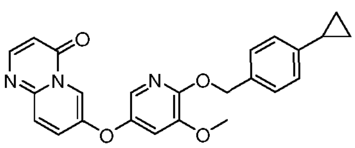
25

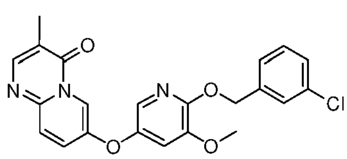
26

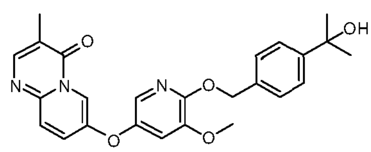
27

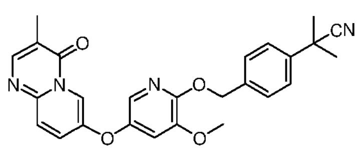
28

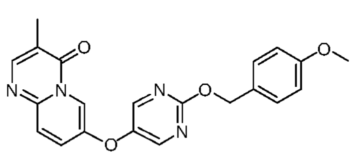
29

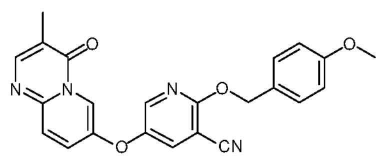
30

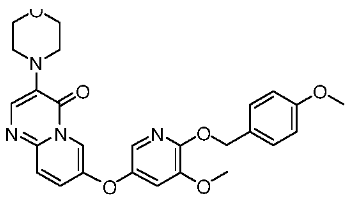

31

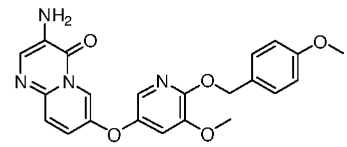

32

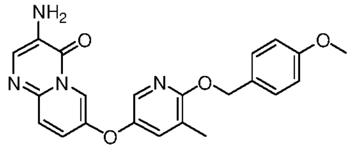

33

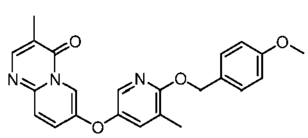

34

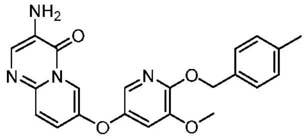

35

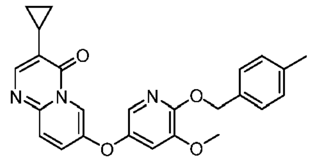

36

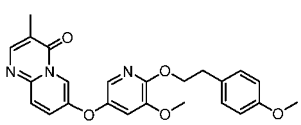

37

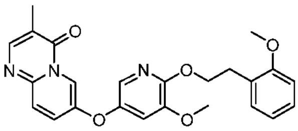

38

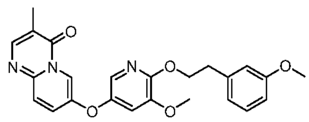

39

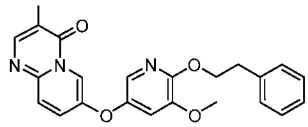

40

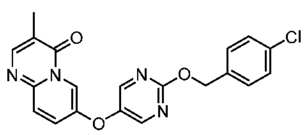

41

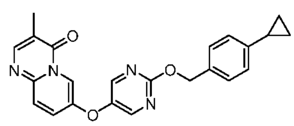

42

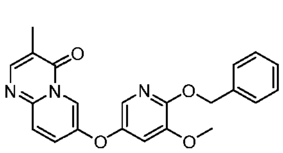

43

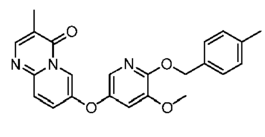

44

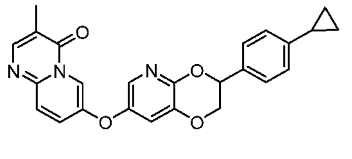

45

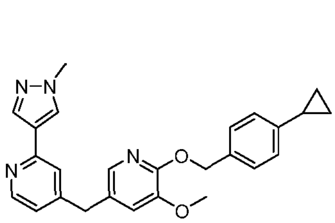

46

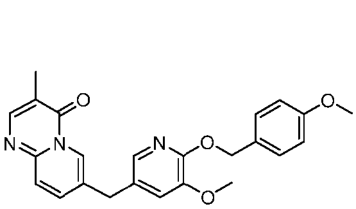

47

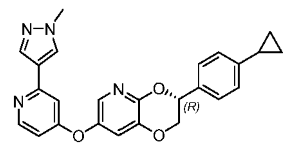 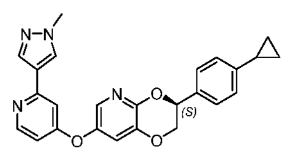 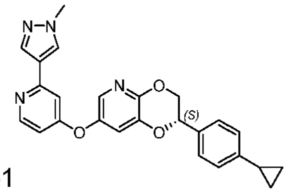 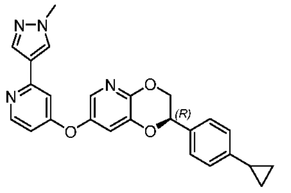

48-51

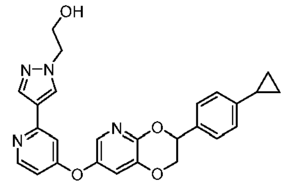

52

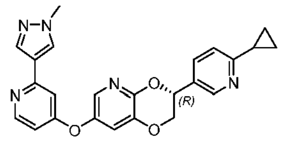 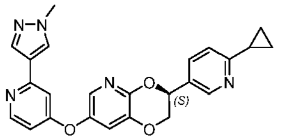

53 & 54

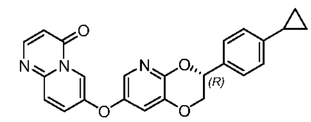 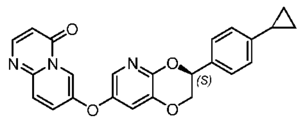

55 & 56

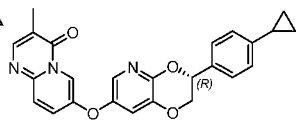 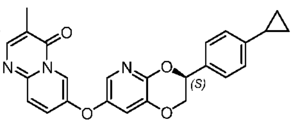

57 & 58

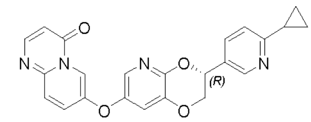 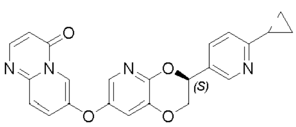

59 & 60

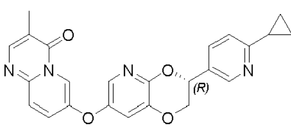 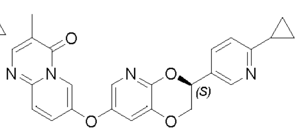

61 & 62

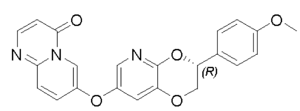 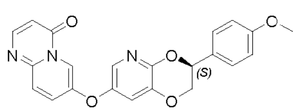

63 & 64

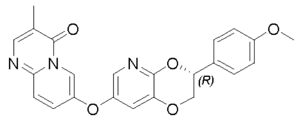 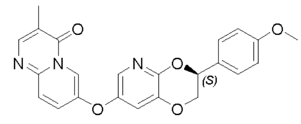

65 & 66

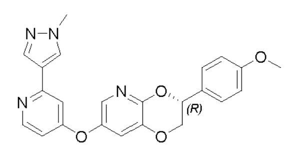 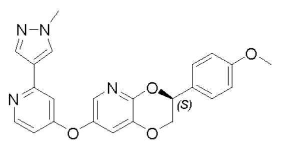

67 & 68

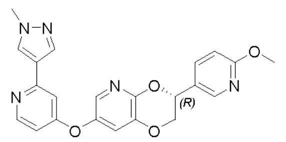 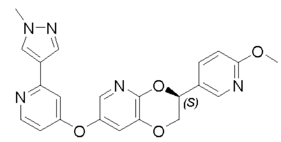

69 & 70

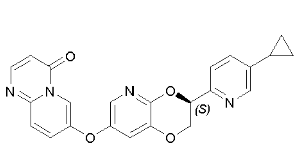 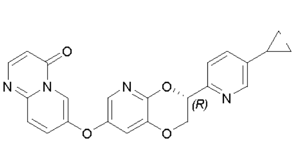

71 & 72

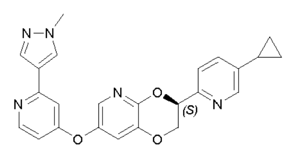 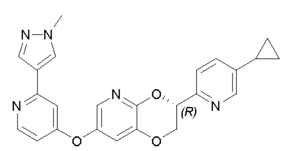

73 & 74

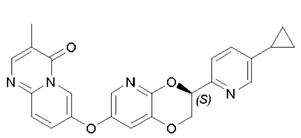 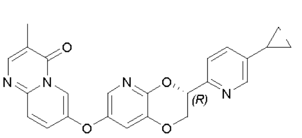

75 & 76

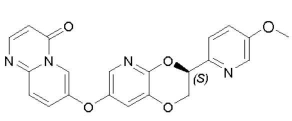 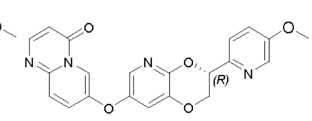

77 & 78

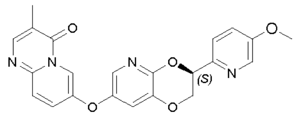 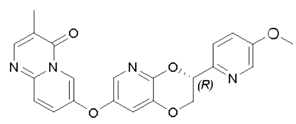

79 & 80

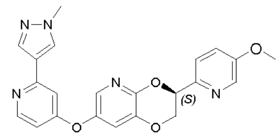 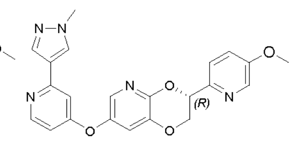

81 & 82

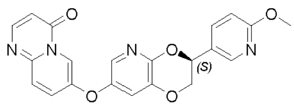

83

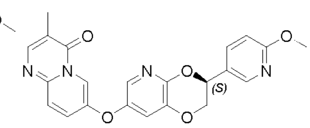

84

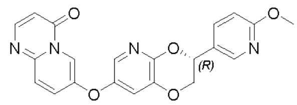

85

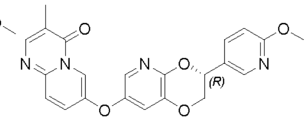

86

.

Embodiment 24. A pharmaceutical composition, comprising the compound or the pharmaceutically acceptable salt thereof according to any one of embodiments 1-23, and optionally comprising a pharmaceutically acceptable excipient.

Embodiment 25. A compound or a pharmaceutically acceptable salt thereof according to any one of embodiments 1-23 or a pharmaceutical composition according to embodiment 24, for use as a pharmaceutical, e.g. in treating a disease mediated by CSF-1R or at least in part by CSF-1R in a subject.

Embodiment 26. The compound for use or a pharmaceutically acceptable salt thereof or the pharmaceutical composition for use according to embodiment 25, wherein the disease is an autoimmune disease, an inflammatory disease, a neurodegenerative disease, cancer, a metabolic disease, obesity, or an obesity-related disease.

Embodiment 27. The compound for use or a pharmaceutically acceptable salt thereof or the pharmaceutical composition for use according to embodiment 26, wherein the autoimmune disease or inflammatory disease is chosen from rheumatoid arthritis, collagen-induced arthritis, osteoarthritis, pigmented villonodular synovitis (PVNS), systemic lupus erythematosus, multiple sclerosis, systemic scleroderma, autoimmune nephritis, inflammatory bowel disease, Crohn's disease, ulcerative colitis, psoriasis, atopic dermatitis, asthma, chronic obstructive pulmonary disease, Behcet's disease, idiopathic thrombocytopenic purpura, spinal arthritis, systemic juvenile idiopathic arthritis (SoJIA), pancreatitis, ischemia reperfusion injury of parenchymatous organs, organ-graft rejection, septicemia, systemic inflammatory response syndrome, and chemotherapy drugs induced organ injury; the neurodegenerative disease is chosen from Parkinson's disease (PD), multiple system atrophy, Alzheimer's disease (AD), frontotemporal lobar dementia, Huntington's disease (HD), corticobasal degeneration, spinocerebellar ataxia, amyotrophic lateral sclerosis (ALS), spinal muscular atrophy (SMA), and hereditary motor and sensory neuropathy (CMT); and the cancer is solid tumor or hematologic malignancy, such as ovarian cancer, lung cancer (including non-small cell lung cancer), brain tumor (including glioblastoma (GBM)), tenosynovial giant cell tumor, gastrointestinal stromal tumor (GIST), gastric cancer, esophageal cancer, colon cancer, colorectal cancer, pancreatic cancer, prostate cancer, breast cancer, cervical cancer, melanoma, mesothelioma, mesothelial carcinoma, renal cancer, liver cancer, thyroid carcinoma, head and neck cancer, urothelial carcinoma, bladder cancer, endometrial cancer, choriocarcinoma, adrenal carcinoma, sarcoma, leukemia, lymphoma, or myeloma.

Embodiment 28. A pharmaceutical combination, comprising a compound or a pharmaceutically acceptable salt thereof according to any one of embodiments 1-23, and at least one additional therapeutic agent.

Embodiment 29. The pharmaceutical combination according to embodiment 28, wherein the additional therapeutic agent is an anti-inflammatory agent or an anti-neoplastic agent; and preferably, the anti-neoplastic agent is chosen from a radiotherapeutic agent, a chemotherapeutic agent, an immune checkpoint inhibitor or agonist, and a targeted therapeutic agent.

In some embodiments, the disease mediated by CSF-1R or at least in part by CSF-1R is an autoimmune disease, an inflammatory disease, a neurodegenerative disease, cancer, a metabolic disease, obesity or an obesity-related disease.

In some embodiments, the disease mediated by CSF-1R or at least in part by CSF-1R is an autoimmune disease, an inflammatory disease or a neurodegenerative disease.

In some embodiments, the autoimmune disease or inflammatory disease is chosen from rheumatoid arthritis, collagen-induced arthritis, osteoarthritis, pigmented villonodular synovitis (PVNS), systemic lupus erythematosus, multiple sclerosis, systemic scleroderma, autoimmune nephritis, inflammatory bowel disease, Crohn's disease, ulcerative colitis, psoriasis, atopic dermatitis, asthma, chronic obstructive pulmonary disease, Behcet's disease, idiopathic thrombocytopenic purpura, spinal arthritis, systemic juvenile idiopathic arthritis (SoJIA), pancreatitis, ischemia reperfusion injury of parenchymatous organs, organ-graft rejection, septicemia, systemic inflammatory response syndrome and chemotherapy drugs induced organ injury.

In some embodiments, the neurodegenerative disease is chosen from Parkinson's disease (PD), multiple system atrophy, Alzheimer's disease (AD), frontotemporal lobar dementia, Huntington's disease (HD), corticobasal degeneration, spinocerebellar ataxia, amyotrophic lateral sclerosis (ALS), spinal muscular atrophy (SMA), and hereditary motor and sensory neuropathy (CMT).

***General Synthetic Methods***

The compound of formula (I) and/or a pharmaceutically acceptable salt thereof described herein can be synthesized using commercially available starting materials, by methods known in the art, or methods disclosed in the application. The synthetic routes shown in routes 1-3 illustrate the general synthetic methods of the compounds of the present invention.

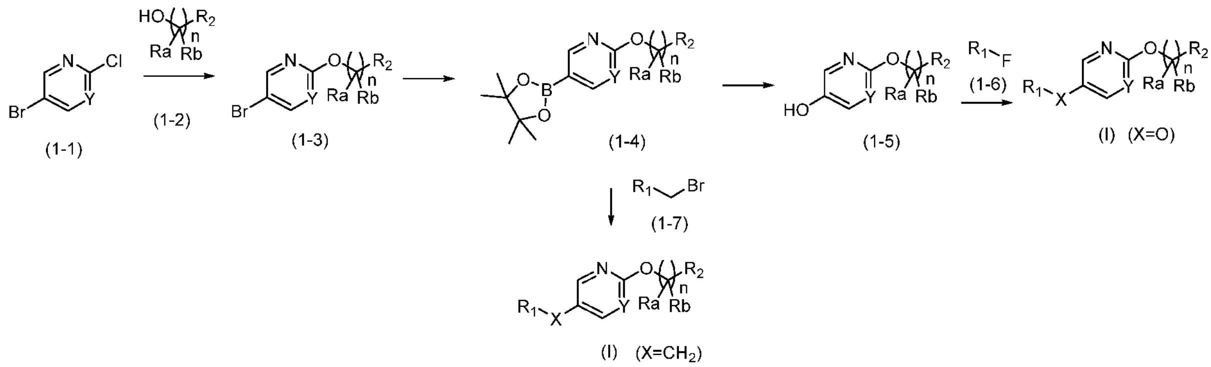


Route 1

As shown in route 1, under alkaline conditions (such as, but not limited to NaH), a compound of formula (1-1) is subjected to a substitution reaction with a compound of formula (1-2) to obtain a compound of formula (1-3). The compound of formula (1-3) is subjected to a catalysis of a palladium reagent (such as, but not limited to $Pd(dppf)Cl_2$) to produce a compound of formula (1-4), which is then subjected to an oxidation reaction (such as, but not limited to using $H_2O_2$ as an oxidant) and converted into a compound of formula (1-5). Under alkaline conditions (such as, but not limited to potassium carbonate), the compound of formula (1-5) is subjected to a substitution reaction with a compound of formula (1-6) to obtain the compound of formula (I) (wherein X=O). Alternatively, the compound of formula (1-4) is subjected to a reaction with a compound of formula (1-7) under the catalysis of a palladium reagent (such as, but not limited to $Pd(dppf)Cl_2$) to obtain the compound of formula (I) (wherein $X=CH_2$). $R_1$, $R_2$, $R_a$, $R_b$, Y, and n are as defined herein.

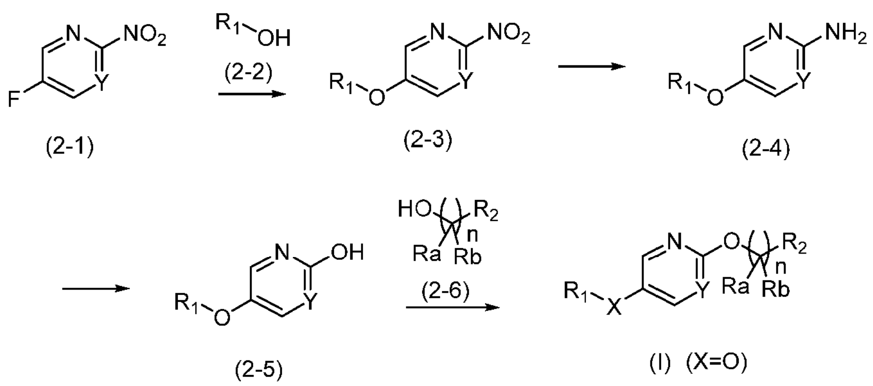


Route 2

As shown in route 2, under alkaline conditions (such as, but not limited to cesium carbonate), a compound of formula (2-1) is subjected to a substitution reaction with a compound of formula (2-2) to obtain a compound of formula (2-3). The compound of formula (2-3) is subjected to a reduction reaction (such as, but not limited to using iron as a reducing agent) to produce a compound of formula (2-4), which is then converted into a compound of formula (2-5) under the action of sulfuric acid and sodium nitrite. The compound of formula (2-5) is subjected to a Mitsunobu reaction with a compound of formula (2-6) to obtain the compound of formula (I) (wherein X=O). $R_1$, $R_2$, $R_a$, $R_b$, Y, and n are as defined herein.

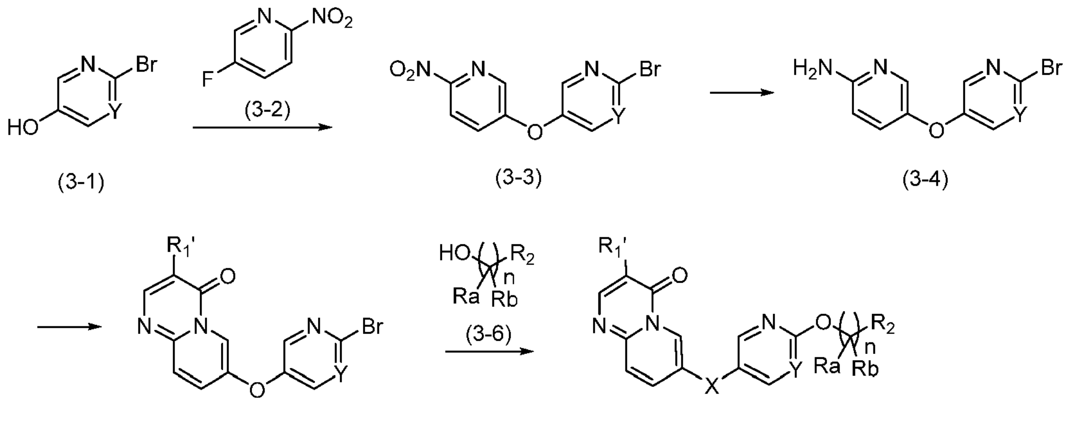

(3-1) (3-3) (3-4)
(3-5) (I-1a) (X=O)

Route 3

As shown in route 3, under alkaline conditions (such as, but not limited to cesium carbonate), a compound of formula (3-1) is subjected to a substitution reaction with a compound of formula (3-2) to obtain a compound of formula (3-3). The compound of formula (3-3) is subjected to a reduction reaction (such as, but not limited to using iron as a reducing agent) to produce a compound of formula (3-4), which is then subjected to a cyclization reaction (such as being subjected to a cyclization reaction with

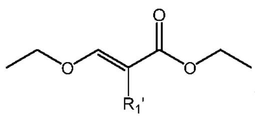
)

to produce a compound of formula (3-5). Under alkaline conditions (such as, but not limited to cesium carbonate), the compound of formula (3-5) is subjected to a substitution reaction with a compound of formula (3-6) to obtain the compound of formula (I-1a) (wherein X=O). $R_1$', $R_2$, $R_a$, $R_b$, Y, and n are as defined herein.

The substituents of the compounds thus obtained can be further modified to provide other desired compounds. Synthetic chemistry transformations are described, for example, in R. Larock, Comprehensive Organic Transformations, VCH Publishers (1989); L. Fieser and M. Fieser, Fieser and Fieser's Reagents for Organic Synthesis, John Wiley and Sons (1994); and L. Paquette, ed., Encyclopedia of Reagents for Organic Synthesis, John Wiley and Sons (1995) and subsequent editions thereof.

Before use, the compound of formula (I) and/or a pharmaceutically acceptable salt thereof described herein can be purified by column chromatography, high performance liquid chromatography, crystallization or other suitable methods.

## ***Pharmaceutical Compositions and Utility***

A pharmaceutical composition comprising the compound of formula (I) (e.g., a compound of any of the examples as described herein) or a pharmaceutically acceptable salt thereof described herein can be administered in various known manners, such as orally, parenterally, by inhalation spray, or via an implanted reservoir. The term "parenteral" as used herein includes subcutaneous, intracutaneous, intravenous, intramuscular, intraarticular, intraarterial, intrasynovial, intrasternal, intrathecal, intralesional and intracranial injection or infusion techniques.

An oral composition can be any orally acceptable dosage form including, but not limited to, tablets, capsules, pills, powder, emulsions, and aqueous suspensions, dispersions and solutions. Commonly used carriers for tablets include lactose and corn starch. Lubricating agents, such as magnesium stearate, are also typically added to tablets. For oral administration in a capsule form, useful diluents include lactose and dried corn starch. When aqueous suspensions or emulsions are administered orally, the active ingredient can be suspended or dissolved in an oily phase combined with emulsifying or suspending agents. If desired, certain sweetening, flavoring, or coloring agents can be added.

In some embodiments, the compound of formula (I) or a pharmaceutically acceptable salt thereof can be present in an amount of 1, 5, 10, 15, 20, 25, 50, 75, 80, 85, 90, 95, 100, 125, 150, 200, 250, 300, 400 and 500 mg in a tablet. In some embodiments, the compound of formula (I) or a pharmaceutically acceptable salt thereof can be present in an amount of 1, 5, 10, 15, 20, 25, 50, 75, 80, 85, 90, 95, 100, 125, 150, 200, 250, 300, 400 and 500 mg in a capsule.

A sterile injectable composition (e.g., aqueous or oleaginous suspension) can be formulated according to

techniques known in the art using suitable dispersing or wetting agents (for example, Tween 80) and suspending agents. The sterile injectable intermediate medium can also be a sterile injectable solution or suspension in a non-toxic parenterally acceptable diluent or solvent, for example, as a solution in 1,3-butanediol. Among the pharmaceutically acceptable vehicles and solvents that can be employed are mannitol, water, Ringer's solution and isotonic sodium chloride solution. In addition, sterile, fixed oils are conventionally employed as a solvent or suspending medium (e.g., synthetic mono- or di-glycerides). Fatty acids, such as oleic acid and its glyceride derivatives, and natural pharmaceutically acceptable oils, such as olive oil or castor oil, especially in their polyoxyethylated versions, can be used as injectable intermediate medium. These oil solutions or suspensions can also contain a long-chain alcohol diluent or dispersant, or carboxymethyl cellulose or similar dispersing agents.

An inhalation composition can be prepared according to techniques well known in the art of pharmaceutical formulation and can be prepared as solutions in saline, employing benzyl alcohol or other suitable preservatives, absorption promoters to enhance bioavailability, fluorocarbons, and/or other solubilizing or dispersing agents known in the art.

A topical composition can be formulated in form of oil, cream, lotion, ointment, and the like. Suitable carriers for the composition include vegetable or mineral oils, white petrolatum (white soft paraffin), branched chain fats or oils, animal fats and high molecular weight alcohols (greater than C12). In some embodiments, the pharmaceutically acceptable carrier is one in which the active ingredient is soluble. Emulsifiers, stabilizers, humectants and antioxidants may also be included as well as agents imparting color or fragrance, if desired. Additionally, transdermal penetration enhancers may be employed in those topical formulations. Examples of such enhancers can be found in U.S. Patent Nos. 3,989,816 and 4,444,762.

Creams may be formulated from a mixture of mineral oil, self-emulsifying beeswax and water in which mixture the active ingredient, dissolved in a small amount of an oil, such as almond oil, is admixed. An example of such a cream is one which includes, by weight, about 40 parts water, about 20 parts beeswax, about 40 parts mineral oil and about 1 part almond oil. Ointments may be formulated by mixing a solution of the active ingredient in a vegetable oil, such as almond oil, with warm soft paraffin and allowing the mixture to cool. An example of such an ointment is one which includes about 30% by weight almond oil and about 70% by weight white soft paraffin.

A pharmaceutically acceptable carrier refers to a carrier that is compatible with active ingredients of the composition (and in some embodiments, capable of stabilizing the active ingredients) and not deleterious to the subject to be treated. For example, solubilizing agents, such as cyclodextrins (which form specific, more soluble complexes with the compound of formula (I) and/or a pharmaceutically acceptable salt thereof described herein), can be utilized as pharmaceutical excipients for delivery of the active ingredients. Examples of other carriers include colloidal silicon dioxide, magnesium stearate, cellulose, sodium lauryl sulfate, and pigments such as D&C Yellow # 10.

Suitable in vitro assays can be used to evaluate the practical utility of the compound of formula (I) or a pharmaceutically acceptable salt thereof described herein in inhibiting the activity of CSF-1R. The compound of formula (I) or a pharmaceutically acceptable salt thereof described herein can further be examined for additional practical utility in treating an autoimmune disease, an inflammatory disease, a neurodegenerative disease or cancer, and the like by *in vivo* assays. For example, the compound of formula (I) or a pharmaceutically acceptable salt thereof described herein can be administered to an animal (e.g., a mouse model) having an autoimmune disease or an inflammatory disease and its therapeutic effects can be accessed. If the pre-clinical results are successful, the dosage range and administration route for animals, such as humans, can be projected.

The compound of formula (I) or a pharmaceutically acceptable salt thereof described herein can be used to achieve a beneficial therapeutic or prophylactic effect, for example, in subjects with an autoimmune disease or inflammatory disease.

The term "autoimmune disease" refers to a disease or disorder arising from and/or directed against an individual's own tissues or organs, or a co-segregate or manifestation thereof, or resulting condition therefrom. Examples of autoimmune diseases include, but are not limited to: chronic obstructive pulmonary disease (COPD), allergic rhinitis, lupus erythematosus, myasthenia gravis, multiple sclerosis (MS), rheumatoid arthritis (RA), collagen-induced arthritis, psoriasis, inflammatory bowel disease (IBD), Crohn's disease, ulcerative colitis, asthma, autoimmune nephritis, idiopathic thrombocytopenic purpura (ITP) and myeloproliferative disease, such as myelofibrosis, post-polycythemia vera/essential thrombocytosis myelofibrosis (post-PV/ET myelofibrosis).

The term "inflammatory disease" or "inflammatory disorder" refers to a pathological state that leads to inflammation, especially due to neutrophil chemotaxis. Non-limiting examples of inflammatory diseases include systemic inflammation and local inflammation, inflammation associated with immunosuppression, organ-graft rejection, allergic disease, inflammatory skin disease (including psoriasis and atopic dermatitis); systemic scleroderma and sclerosis; reactions associated with inflammatory bowel diseases (IBD, such as Crohn's disease and ulcerative colitis); ischemia reperfusion injury, including reperfusion injury of tissue caused by surgery, myocardial ischemia, such as myocardial infarction, cardiac arrest, reperfusion after heart operation and abnormal contractile response of coronary vessel after percutaneous transluminal coronary angioplasty, surgical tissue reperfusion injury of stroke and abdominal aortic

aneurysm; cerebral edema secondary to stroke; cranial trauma, and hemorrhagic shock; asphyxia; adult respiratory distress syndrome; acute lung injury; Behcet's disease; dermatomyositis; polymyositis; multiple sclerosis (MS); dermatitis; meningitis; encephalitis; uveitis; osteoarthritis; lupus nephritis; autoimmune disease such as rheumatoid arthritis (RA), Sjogren's syndrome, and vasculitis; diseases involving leukopedesis; central nervous system (CNS) inflammatory disease and multiple organ injury syndrome secondary to septicemia or trauma; alcoholic hepatitis; bacterial pneumonia; antigen-antibody complex mediated disease, including glomerulonephritis; pyaemia; sarcoidosis; immunopathologic responses to tissue/organ transplantation; lung inflammation, including pleurisy, alveolitis, vasculitis, pneumonia, chronic bronchitis, bronchiectasia, diffuse panbronchiolitis, hypersensitivity pneumonitis, idiopathic pulmonary fibrosis (IPF), cystic fibrosis, etc. Preferably indications include, but are not limited to, chronic inflammation, autoimmune diabetes, rheumatoid arthritis (RA), rheumatoid spondylitis, gouty arthritis and other arthrosis conditions, multiple sclerosis (MS), asthma, systemic lupus erythematosus, adult respiratory distress syndrome, Behcet's disease, psoriasis, chronic pulmonary inflammatory disease, graft versus host reaction, Crohn's disease, ulcerative colitis, inflammatory bowel disease (IBD), Alzheimer's disease and pyresis, and any diseases associated with inflammation and related conditions.

In some embodiments, the autoimmune disease or inflammatory disease is chosen from rheumatoid arthritis, collagen-induced arthritis, osteoarthritis, pigmented villonodular synovitis (PVNS), systemic lupus erythematosus, multiple sclerosis, systemic scleroderma, autoimmune nephritis, inflammatory bowel disease, Crohn's disease, ulcerative colitis, psoriasis, atopic dermatitis, asthma, chronic obstructive pulmonary disease, Behcet's disease, idiopathic thrombocytopenic purpura, spinal arthritis, systemic juvenile idiopathic arthritis (SoJIA), pancreatitis, ischemia reperfusion injury of parenchymatous organs, organ-graft rejection, septicemia, systemic inflammatory response syndrome and chemotherapy drugs induced organ injury.

The compound of formula (I) or a pharmaceutically acceptable salt thereof described herein can be used to achieve a beneficial therapeutic or prophylactic effect, for example, in subjects with a neurodegenerative disease.

The term "neurodegenerative diseases" refers to degenerative diseases or disorders of the nervous system caused by neuronal degeneration and apoptosis. Examples of neurodegenerative diseases include, but are not limited to: Parkinson's disease (PD), multiple system atrophy, Alzheimer's disease (AD), frontotemporal lobar dementia, Huntington's disease (HD), corticobasal degeneration, spinocerebellar ataxia, amyotrophic lateral sclerosis (ALS), spinal muscular atrophy (SMA), hereditary motor and sensory neuropathy (CMT), etc.

In some embodiments, the neurodegenerative disease is chosen from Parkinson's disease (PD), Alzheimer's disease (AD), Huntington's disease (HD), amyotrophic lateral sclerosis (ALS) and spinal muscular atrophy (SMA).

The compound of formula (I) or a pharmaceutically acceptable salt thereof described herein can be used to achieve a beneficial therapeutic or prophylactic effect, for example, in subjects with cancer.

As used herein, the term "cancer" refers to a cellular disorder characterized by uncontrolled or disregulated cell proliferation, decreased cellular differentiation, inappropriate ability to invade surrounding tissue, and/or ability to establish new growth at ectopic sites. The term "cancer" includes, but is not limited to, solid tumors and hematologic malignancies. The term "cancer" encompasses diseases of skin, tissues, organs, bone, cartilage, blood, and vessels. The term "cancer" encompasses primary cancer, and further metastatic cancer.

Non-limiting examples of solid tumors include pancreatic cancer; bladder cancer; colorectal cancer; colon cancer; breast cancer, including metastatic breast cancer; prostate cancer, including androgen-dependent and androgen-independent prostate cancer; testicular cancer; renal cancer, including, e.g., metastatic renal cell carcinoma; urothelial carcinoma; liver cancer; hepatocellular cancer; lung cancer, including, e.g., non-small cell lung cancer (NSCLC), bronchioloalveolar carcinoma (BAC), and adenocarcinoma of the lung; ovarian cancer, including, e.g., progressive epithelial or primary peritoneal cancer; cervical cancer; endometrial cancer; gastrointestinal stromal tumor (GIST); gastric cancer; esophageal cancer; head and neck cancer, including, e.g., squamous cell carcinoma of the head and neck; skin cancer, including, e.g., melanoma and basal carcinoma; neuroendocrine cancer, including metastatic neuroendocrine tumors; brain tumors, including, e.g., glioma, anaplastic oligodendroglioma, adult glioblastoma multiforme, and adult anaplastic astrocytoma; bone cancer; sarcoma, including, e.g., Kaposi's sarcoma; adrenal carcinoma; mesothelioma; mesothelial carcinoma; choriocarcinoma; muscle carcinoma; connective tissue carcinoma; tenosynovial giant cell tumor; and thyroid carcinoma.

Non-limiting examples of hematologic malignancies include acute myelogenous leukemia (AML); chronic myelogenous leukemia (CML), including accelerated phase CML and CML blastic phase (CML-BP); acute lymphocytic leukemia (ALL); chronic lymphocytic leukemia (CLL); Hodgkin's lymphoma; non-Hodgkin's lymphoma (NHL); follicular lymphoma; mantle cell lymphoma (MCL); B-cell lymphoma; T cell lymphoma; diffuse large B-cell lymphoma (DLBCL); multiple myeloma (MM); Waldenstrom macroglobulinemia; myelodysplastic syndrome (MDS), including refractory anemia (RA), refractory anemia with ring sideroblasts (RARS), refractory anemia with excess blasts (RAEB) and refractory anemia with excess blasts in transformation (RAEB-T); and myeloproliferative syndrome.

In some embodiments, the solid tumors include ovarian cancer, lung cancer (including non-small cell lung cancer), glioblastoma (GBM), tenosynovial giant cell tumor, gastrointestinal stromal tumor (GIST), gastric cancer, esophageal cancer, colon cancer, colorectal cancer, pancreatic cancer, prostate cancer, breast cancer, cervical cancer,

melanoma, mesothelioma, mesothelial carcinoma, renal cancer, liver cancer, thyroid carcinoma, head and neck cancer, urothelial carcinoma, bladder cancer, endometrial cancer, choriocarcinoma, adrenal carcinoma and sarcoma.

In some embodiments, typical hematologic malignancies include leukemia, for example acute lymphocytic leukemia (ALL), acute myelogenous leukemia (AML), chronic lymphocytic leukemia (CLL) and chronic myelogenous leukemia (CML); multiple myeloma (MM); and lymphoma, for example Hodgkin's lymphoma, non-Hodgkin's lymphoma (NHL), mantle cell lymphoma (MCL), follicular lymphoma, B-cell lymphoma, T-cell lymphoma and diffuse large B-cell lymphoma (DLBCL).

The term "metabolic diseases" refers to diseases or disorders caused by metabolic problems, including metabolic disorders and hypermetabolism. Examples of metabolic diseases include, but are not limited to: osteoporosis, diabetes, diabetic ketoacidosis, hyperglycemia and hyperosmolar syndrome, hypoglycemia, gout, protein-energy malnutrition, vitamin A deficiency disease, scurvy, vitamin D deficiency disease, etc.

The term "obesity-related diseases" refers to diseases or disorders related to, resulted from, or caused by obesity. Examples of obesity-related disease include, but are not limited to: diabetes, hypertension, insulin resistance syndrome, dyslipidemia, heart disease, cardiovascular disease (including atherosclerosis, abnormal heart rhythms, arrhythmias, myocardial infarction, congestive heart failure, coronary heart disease, and angina pectoris), cerebral infarction, cerebral hemorrhage, osteoarthritis, metabolic syndrome, non-alcoholic fatty liver disease, non-alcoholic steatohepatitis, and the like.

In addition, the compound of formula (I) (e.g., a compound of any of the examples as described herein) or a pharmaceutically acceptable salt thereof described herein can be administered in combination with additional therapeutic agents, for treating an autoimmune disease, an inflammatory disease or cancer. The additional therapeutic agents may be administered separately with the compound of formula (I) or a pharmaceutically acceptable salt thereof described herein or included with such an ingredient in a pharmaceutical composition according to the disclosure, such as a fixed-dose combination drug product. In some embodiments, the additional therapeutic agents are those that are known or discovered to be effective in the treatment of diseases mediated by CSF-1R or at least in part by CSF-1R, such as another CSF-1R inhibitor or a compound active against another target associated with the particular disease. The combination may serve to increase efficacy (e.g., by including in the combination a compound potentiating the potency or effectiveness of the compound of formula (I) or a pharmaceutically acceptable salt thereof described herein), decrease one or more side effects, or decrease the required dose of the compound of formula (I) or a pharmaceutically acceptable salt thereof described herein.

In some embodiments, the compound of formula (I) (e.g., a compound of any of the examples as described herein) or a pharmaceutically acceptable salt thereof described herein can be administered in combination with anti-inflammatory agents.

Examples of anti-inflammatory agent include, but are not limited to, adrenocortical hormones (such as fluticasone propionate, beclometasone dipropionate, momestasone furoate, triamcinolone acetonide or budesonide), disease modifying agents (such as antimalarial drugs, methotrexate, sulfasalazine, masalazine, azathioprine, 6-mercaptopurine, metronidazole, D-penicillamine), non-steroidal anti-inflammatory drugs (such as acetaminophen, aspirin, sodium salicylate, cromoglycate sodium, magnesium salicylate, choline magnesium trisalicylate, salsalate, ibuprofen, naproxen, diclofenac, diflunisal, etodolac, fenoprofen calcium, flurbiprofen, piroxicam, indomethacin, ketoprofen, ketorolac tromethamine, meclofenamic acid, meclofenamate sodium, mefenamic acid, nabumetone, oxaprozin, phenyl butyl nitrone (PBN), sulindac or tolmetin), COX-2 inhibitors, cytokine synthesis/release inhibitors (such as anti-cytokine antibody, anti-cytokine receptor antibody, etc.).

In some embodiments, the compound of formula (I) (e.g., a compound of any of the examples as described herein) or a pharmaceutically acceptable salt thereof described herein can be administered in combination with anti-neoplastic agents. The term "anti-neoplastic agent" as used herein refers to any agent that is administered to a subject suffering from cancer for the purposes of treating the cancer, includes, but is not limited to a radiotherapeutic agent, a chemotherapeutic agent, an immune checkpoint inhibitor or agonist, a targeted therapeutic agent, and the like.

Non-limiting examples of immune checkpoint inhibitors or agonists include PD-1 inhibitors, for example, anti-PD-1 antibodies, such as pembrolizumab, nivolumab and PDR001 (spartalizumab); PD-L1 inhibitors, for example, anti-PD-L1 antibodies, such as atezolizumab, durvalumab, and avelumab; CTLA-4 inhibitors, for example, anti-CTLA-4 antibodies, such as ipilimumab; and BTLA inhibitors, LAG-3 inhibitors, TIM3 inhibitors, TIGIT inhibitors, VISTA inhibitors, OX-40 agonists, and the like.

Non-limiting examples of chemotherapeutic agents include topoisomerase I inhibitors (e.g., irinotecan, topotecan, camptothecin and analogs or metabolites thereof, and doxorubicin); topoisomerase II inhibitors (e.g., etoposide, teniposide, mitoxantrone, idarubicin, and daunorubicin); alkylating agents (e.g., melphalan, chlorambucil, busulfan, thiotepa, ifosfamide, carmustine, lomustine, semustine, streptozocin, decarbazine, methotrexate, mitomycin C, and cyclophosphamide); DNA intercalators (e.g., cisplatin, oxaliplatin, and carboplatin); DNA intercalators and free radical generators such as bleomycin; nucleoside mimetics (e.g., 5-fluorouracil, capecitabine, gemcitabine, fludarabine, cytarabine, azacitidine, mercaptopurine, thioguanine, pentostatin, and hydroxyurea); paclitaxel, docetaxel, and related

analogs; vincristine, vinblastin, and related analogs; thalidomide and related analogs (e.g., CC-5013 and CC-4047).

Non-limiting examples of targeted therapeutic agents include: protein tyrosine kinase inhibitors (such as imatinib mesylate and gefitinib); proteasome inhibitors (such as bortezomib); NF-κB inhibitors, including IκB kinase inhibitors; IDO inhibitors; A2AR inhibitors; BRAF inhibitors (such as dabrafenib); MEK inhibitors (such as trametinib); mTOR inhibitors (such as rapamycin); anti-CD40 antibodies (such as APX005M, RO7009789); antibodies that bind to proteins overexpressed in cancer to down-regulate cell replication, such as anti-CD20 antibodies (such as rituximab, ibritumomab tiuxetan, and tositumomab), anti-Her2 monoclonal antibodies (such as trastuzumab), anti-EGFR antibodies (such as cetuximab) and anti-VEGF antibodies (such as bevacizumab); anti-angiogenic drugs, such as lenalidomide; and other protein or enzyme inhibitors, these proteins or enzymes are known to be upregulated, overexpressed or activated in cancers, and the inhibition of which can down-regulate cell replication.

## Examples

The examples below are intended to be purely exemplary and should not be considered to be limiting in any way. Efforts have been made to ensure the accuracy with respect to numbers used (for example, amounts, temperature, etc.), but the POSITA should understand that some experimental errors and deviations should be accounted for. Unless indicated otherwise, parts are parts by weight, temperature is in degrees Centigrade, and pressure is at or near atmospheric. All MS data were determined by Agilent 6120 or Agilent 1100. All NMR data were generated using a Varian 400 MHz NMR machine. All reagents, except intermediates, used in this invention are commercially available. All compound names except the reagents are generated by Chemdraw 18.0. The flash column chromatography is performed using a conventional silica gel column, unless specified otherwise or inconsistent with the context.

If there is any atom with empty valence(s) in any one of the structures disclosed herein, the empty balance(s) is (are) the hydrogen atom(s) which is (are) omitted for convenience purpose.

In the present application, in the case of inconsistency of the name and structure of a compound, when the two of which are both given for the compound, it is subject to the structure of the compound, unless the context shows that the structure of the compound is incorrect and the name is correct.

In the following examples, the abbreviations are used:

| | |
|---|---|
| AcOH | Acetic acid |
| $AC_2O$ | Acetic anhydride |
| AcOK | Potassium acetate |
| BINAP | (±)-2,2'-bis-(diphenylphosphino)-1,1'-binaphthyl |
| CuBr | Cuprous bromide |
| DIBAL-H | Diisobutylaluminium hydride |
| DIAD | Diisopropyl azodicarboxylate |
| DMF | N,N-dimethylformamide |
| DMSO | Dimethyl sulfoxide |
| DCM | Dichloromethane |
| DMA | *N,N*-dimethylacetylamide |
| DCE | Dichloroethane |
| $ddH_2O$ | Double distilled water |
| DMAP | 4-dimethylaminopyridine |
| EA | Ethyl acetate |
| EtOH | Ethanol |
| $Et_3N$ | Triethylamine |
| HATU | Hexafluorophosphate *O*-(7-azobenzotriazole-1-yl)-*N,N,N',N'*-tetramethyluronium |
| $H_2O_2$ | Hydrogen peroxide |
| i-PrOH | Isopropanol |
| $LiAlH_4$ | Lithium aluminum hydride |

(continued)

| | |
|---|---|
| MeOH | Methanol |
| MeI | Iodomethane |
| $Me_4tBuXPhos$ | 2-di-tert-butyl phosphin-3,4,5,6-tetramethyl-2',4',6'-triisopropyl biphenyl |
| MsCl | Methanesulfonyl chloride |
| NaOH | Sodium hydroxide |
| $Na_2S_2O_3$ | Sodium thiosulfate |
| $Na_2CO_3$ | Sodium carbonate |
| NaOMe | Sodium methoxide |
| NaH | Sodium hydride |
| NBS | N-bromosuccinimide |
| NMP | N-methyl-2-pyrrolidone |
| n-BuLi | n-Butyl lithium |
| $NH_4Cl$ | Ammonium chloride |
| PE | Petroleum ether |
| $Pd(OAc)_2$ | Palladium acetate |
| $Pd(PPh_3)_2Cl_2$ | Bis(triphenylphosphine)palladium dichloride |
| $Pd(dppf)Cl_2$ | [1,1'-bis(diphenylphosphino) ferrocene]palladium dichloride |
| $Pd(dppf)Cl_2 \cdot CH_2Cl_2$ | [1,1'-bis(diphenylphosphino) ferrocene]palladium dichloride dichloromethane complex |
| $Pd_2(dba)_3$ | Tris(dibenzylidene acetone)dipalladium |
| $Pd(PPh_3)_4$ | Tetra(triphenylphosphine)palladium |
| $Pin_2B_2$ | Bis(pinacolato)diboron |
| p-TsOH | p-toluenesulfonic acid |
| t-BuOK | Potassium t-butoxide |
| t-BuONa | Sodium tert-butoxide |
| TBAF | Tetrabutylammonium fluoride |
| TBSCl | Tert-butyldimethylchlorosilane |
| THF | Tetrahydrofuran |
| TEA | Triethylamine |
| XantPhos | 4,5-bis(diphenylphosphino)-9,9-dimethylxanthene |
| p-TLC | Preparative thin-layer chromatography |

**Example 1**

**Preparation of Intermediates**

**Intermediate 1: 5-bromo-2-chloro-3-methylpyridine**

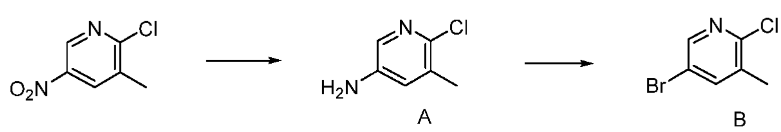

(A) 6-chloro-5-methylpyridin-3-amine

To a reaction flask, 2-chloro-3-methyl-5-nitropyridine (2 g, 11.6 mmol), iron powder (2.6 g, 46.5 mmol), $NH_4Cl$ (3.13 g, 58 mmol), ethanol (40 ml) and water (10 ml) were successively added, and the mixture was heated to 100°C and stirred for 3 hours. The reaction solution was concentrated; water was added; and the mixture was extracted with EA. The organic layer was washed with saturated brine, dried over anhydrous sodium sulfate and filtered. The filtrate was concentrated to obtain 1.5 g of the title product as a khaki solid. MS (m/z): 143.1 $[M+H]^+$

(B) 5-bromo-2-chloro-3-methylpyridine

To a reaction flask, 6-chloro-5-methylpyridin-3-amine (600 mg, 4.2 mmol), isopentyl nitrite (1.97 g, 16.9 mmol), CuBr (2.34 g, 16.9 mmol) and acetonitrile (20 ml) were successively added, and the mixture was heated to 70°C and stirred overnight. The reaction solution was concentrated and purified with flash column chromatography (eluted with petroleum ether:ethyl acetate = 4 : 1) to obtain 600 mg of the title product as a white solid. MS (m/z): 205.9 $[M+Na]^+$

**Intermediate 2: 7-hydroxy-3-methyl-4H-pyrido[1,2-a]pyrimidin-4-one**

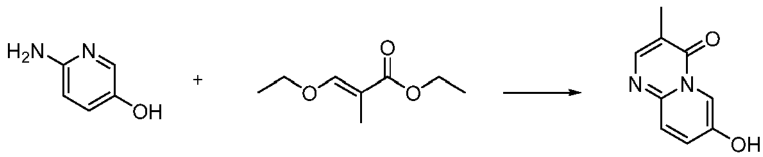

In a reaction flask, 6-aminopyridin-3-ol (3.3 g, 30.0 mmol) and ethyl (E)-3-ethoxy-2-methylacrylate (9.5 g, 60.0 mmol) were dissolved in acetic acid (50 ml), and the reaction solution was heated to reflux for 15 hours. The reaction solution was concentrated, and the residue was purified with flash column chromatography (eluted with a gradient of water:methanol = 100 : 0-0 : 100) to obtain 4.56 g of the title product as a light brown solid. MS (m/z): 177.0 $[M+H]^+$

The following intermediates were prepared according to the preparation procedure of intermediate 2 using corresponding starting materials and reagents under appropriate conditions that will be recognized by one skilled in the art.

| Intermediate | Structural formula | MS $(M+H)^+$ |
|---|---|---|
| **34** | 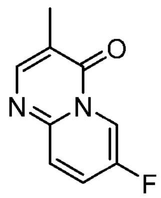 | **179.1** |

**Intermediate 3: (4-cyclopropyl-3-fluorophenyl)methanol**

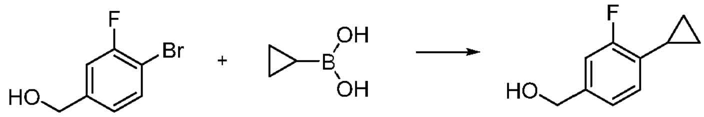

To a reaction flask, 4-bromo-3-fluorobenzyl alcohol (580 mg, 2.83 mmol), cyclopropylboronic acid (730 mg, 8.49 mmol), palladium acetate (63 mg, 0.28 mmol), tricyclohexyl phosphine (79 mg, 0.28 mmol), potassium phosphate (1200 mg, 5.66 mmol), toluene (40 ml) and water (5 ml) were added. The reaction solution was heated to reflux for 15 hours under nitrogen atmosphere. The reaction solution was concentrated, and the residue was purified with flash column chromatography (eluted with a gradient of water:methanol = 100 : 0-0 : 100) to obtain 266 mg of the title product as a white solid. MS (m/z): 149.1 $[M-OH]^+$

**Intermediate 4: 2-(3-(hydroxymethyl)phenyl)-2-methylpropanenitrile**

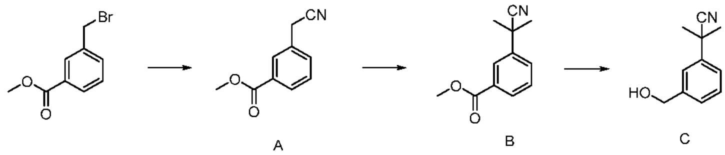

(A) methyl 3-(cyanomethyl)benzoate

To a reaction flask, methyl 3-(bromomethyl)benzoate (2290 mg, 10.0 mmol), sodium cyanide (735 mg, 15.0 mmol), DMF (5 ml) and water (0.5 ml) were added. The mixture was heated at 75°C for 5 hours. The reaction solution was cooled to room temperature; water (50 ml) was then added; and the mixture was extracted with ethyl acetate (50 ml) twice. The organic phases were combined, washed with saturated brine twice and then concentrated. The residue was purified with flash column chromatography (eluted with a gradient of water:methanol = 100 : 0-0 : 100) to obtain 1401 mg of the title product as a solid. MS (m/z): 176.1 $[M+H]^+$

(B) methyl 3-(2-cyanopropan-2-yl)benzoate

In a reaction flask, methyl 3-(cyanomethyl)benzoate (1401 mg, 8.0 mmol) was dissolved in DMSO (10 ml). In an ice bath, sodium hydride (960 mg, 24.0 mmol) was added to the reaction solution in portions. After the addition was completed, the reaction solution was stirred at room temperature for 20 minutes. Then iodomethane (3406 mg, 1.5 ml, 24.0 mmol) was added dropwise to the reaction solution. After the addition was completed, the reaction solution was stirred at room temperature for 4 hours. After the reaction was completed, water (50 ml) was added, and the mixture was extracted with ethyl acetate (50 ml) twice. The organic phases were combined, washed with saturated brine (30 ml) twice and then concentrated. The residue was purified with flash column chromatography (eluted with a gradient of petroleum ether:ethyl acetate = 100 : 0-0 : 100) to obtain 1260 mg of the title product as a pale yellow oil. MS (m/z): 204.1 $[M+H]^+$ (C) 2-(3-(hydroxymethyl)phenyl)-2-methylpropanenitrile

In a reaction flask, methyl 3-(2-cyanopropan-2-yl)benzoate (610 mg, 3.0 mmol) was dissolved in anhydrous tetrahydrofuran (30 ml). In an ice bath, borane dimethyl sulfide complex (2 M, 4.5 ml, 9.0 mmol) was added dropwise to the reaction solution. After the addition was completed, the reaction solution was stirred for another 15 hours. After the reaction was completed, the reaction was quenched by adding methanol dropwise in an ice bath. The reaction solution was concentrated, and the residue was purified with flash column chromatography (eluted with a gradient of water:methanol = 100 : 0-0 : 100) to obtain 370 mg of the title product as a pale yellow oil. MS (m/z): 158.1 $[M-OH]^+$

**Intermediate 5: (4-cyclopropyl-2-fluorophenyl)methanol**

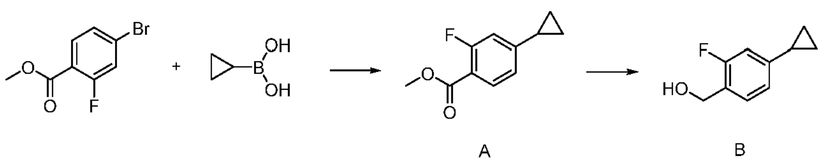

(A) methyl 4-cyclopropyl-2-fluorobenzoate

To a reaction flask, methyl 4-bromo-2-fluorobenzoate (2330 mg, 10.0 mmol), cyclopropylboronic acid (2577 mg, 30.0 mmol), palladium acetate (224 mg, 1.0 mmol), tricyclohexyl phosphine (280 mg, 1.0 mmol), potassium phosphate (4240 mg, 20.0 mmol), toluene (60 ml) and water (10 ml) were added. The reaction solution was heated to reflux for 15 hours under nitrogen atmosphere. The reaction solution was concentrated, and the residue was purified with flash column chromatography (eluted with a gradient of water:methanol = 100 : 0-0 : 100) to obtain 1760 mg of the title product as a white solid. MS (m/z): 195.1 $[M-OH]^+$

(B) (4-cyclopropyl-2-fluorophenyl)methanol

In a reaction flask, methyl 4-cyclopropyl-2-fluorobenzoate (971 mg, 4.0 mmol) was dissolved in anhydrous tetrahydrofuran (30 ml). In an ice bath, $LiAlH_4$ (455 mg, 12.0 mmol) was added in portions to the reaction solution. After the addition was completed, the reaction solution was stirred for another 15 hours. After the reaction was completed, the reaction was quenched by adding methanol dropwise in an ice bath. The reaction solution was concentrated, and the residue was purified with flash column chromatography (eluted with a gradient of water:methanol = 100 : 0-0 : 100) to obtain 430 mg of the title product as a pale yellow oil. MS (m/z): 149.1 $[M-OH]^+$

**Intermediate 6: 2-(1-methyl-1H-pyrazol-4-yl)pyridin-4-ol**

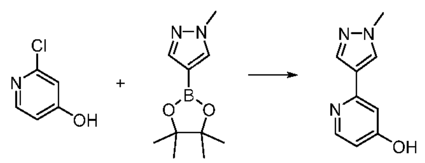

Under nitrogen atmosphere, 2-chloropyridin-4-ol (388 mg, 3.0 mmol), 1-methyl-4-(4,4,5,5-tetramethyl-1,3,2-dioxaborolan-2-yl)-1*H*-pyrazole (1248 mg, 6.0 mmol), potassium carbonate (829 mg, 6.0 mmol), Pd(dppf)$Cl_2$ (110 mg, 0.15 mmol), dioxane (20 ml) and water (5 ml) were added to a reaction flask, and the reaction solution was heated to reflux and stirred for 15 hours. The reaction solution was concentrated, and the residue was purified with flash column chromatography (eluted with a gradient of water:methanol = 100 : 0-0 : 100) to obtain 250 mg of the title product as a white solid. MS (m/z): 176.1$[M+H]^+$

**Intermediate 7: 5-fluoro-3-(fluoromethoxy)-2-nitropyridine**

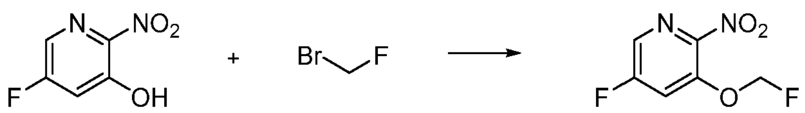

To a reaction flask, 5-fluoro-2-nitropyridin-3-ol (474 mg, 3.0 mmol), bromofluoromethane (373 mg, 3.3 mmol), potassium carbonate (498 mg, 3.6 mmol) and DMF (3 ml) were added. The mixture was stirred at room temperature for 48 hours. After the reaction was completed, water (20 ml) was added, and the mixture was extracted with ethyl acetate (50 ml) twice. The organic phases were combined, washed with saturated brine twice and then concentrated. The residue was purified with flash column chromatography (eluted with a gradient of water:methanol = 100 : 0-0 : 100) to obtain 520 mg of the title product as a pale yellow solid. MS (m/z): 191.1 $[M+H]^+$

**Intermediate 8: 5-bromo-2-chloronicotinonitrile**

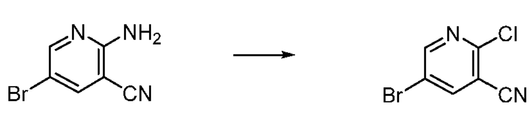

In a reaction flask, 2-amino-5-bromonicotinonitrile (1000 mg, 5.05 mmol) was dissolved in concentrated hydrochloric acid (10 ml). In an ice bath, aqueous sodium nitrite solution (418 mg, 6.06 mmol, dissolved in 3 ml of water) was added dropwise to the reaction solution. After the addition was completed, the reaction solution was stirred for another 15 hours. After the reaction was completed, the reaction solution was cooled to 0°C, and then 100 ml of water was added slowly to the reaction solution. The reaction solution was filtrated, and then the solid was washed with water and dried to obtain 900 mg of the title product. MS (m/z): 217.0, 219.0 $[M+H]^+$

**Intermediate 9: 2-(4-(hydroxymethyl)phenyl)-2-methylpropanenitrile**

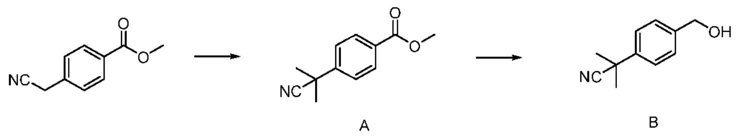

(A) methyl 4-(2-cyanopropan-2-yl)benzoate

In a reaction flask, methyl 4-(cyanomethyl)benzoate (1752 mg, 10.0 mmol) was dissolved in DMF (30 ml), and then sodium hydride (880 mg, 22.0 mmol) was added in portions to the reaction solution. After the addition was completed, the reaction solution was stirred at room temperature for half an hour, and then iodomethane (1.37 ml, 22.0 mmol) was added dropwise to reaction solution. After the addition was completed, the reaction solution was stirred at room temperature for another 4 hours. After the reaction was completed, water (50 ml) and ethyl acetate (100 ml) were added; liquid separation was carried out; and the aqueous phase was extracted with ethyl acetate twice. The organic phases were combined and concentrated. The residue was purified with flash column chromatography (eluted with a gradient of water:methanol = 100 : 0-0 : 100) to obtain 1600 mg of the title product as a white solid. MS (m/z): 204.1 $[M+H]^+$ (B) 2-(4-(hydroxymethyl)phenyl)-2-methylpropanenitrile

Under nitrogen atmosphere, methyl 4-(2-cyanopropan-2-yl)benzoate (1600 mg, 7.87 mmol) was dissolved in anhydrous tetrahydrofuran (40 ml) in a reaction flask. In an ice bath, DIBAL-H (10.5 ml, 15.7 mmol) was added dropwise to the reaction solution. After the addition was completed, the reaction solution was stirred for another 2 hours in an ice bath. After the reaction was completed, the reaction was quenched with saturated ammonium chloride. The reaction solution was concentrated, and the residue was purified with flash column chromatography (eluted with a gradient of water:methanol = 100 : 0-0 : 100) to obtain 1050 mg of the title product as a white solid. MS (m/z+Na): 198.1 $[M+H]^+$

The following intermediate was prepared according to the preparation procedure in step (B) of intermediate 9 using corresponding starting materials and reagents under appropriate conditions that will be recognized by one skilled in the art.

| Intermediate | Structural formula | MS $(M+H)^+$ |
|---|---|---|
| **36** | 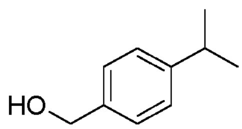 | **133.1 [M-OH]** |

**Intermediate 10: 2-(4-(hydroxymethyl)phenyl)propan-2-ol**

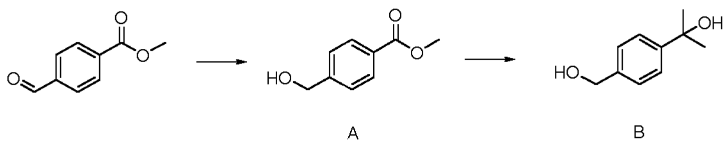

(A) methyl 4-(hydroxymethyl)benzoate

In a reaction flask, methyl 4-formylbenzoate (1642 mg, 10.0 mmol) was dissolved in THF (50 ml), and sodium borohydride was added in portions to the reaction solution in an ice bath. After the addition was completed, the reaction solution was stirred at room temperature for another 1 hour. After the reaction was completed, the reaction was quenched by adding water (5 ml) dropwise to the reaction solution in an ice bath. The mixture was concentrated, and the residue was purified with flash column chromatography (eluted with a gradient of water:methanol = 100 : 0-0 : 100) to obtain 1600 mg of the title product as a white solid. MS (m/z): 189.1 $[M+Na]^+$

(B) 2-(4-(hydroxymethyl)phenyl)propan-2-ol

Under nitrogen atmosphere, methyl 4-(hydroxymethyl)benzoate (1600 mg, 9.62 mmol) was dissolved in anhydrous tetrahydrofuran (40 ml) in a reaction flask. In an ice bath, methylmagnesium bromide (14.5 ml, 28.9 mmol) was added dropwise to reaction solution. After the addition was completed, the reaction solution was stirred for another 2 hours in an ice bath. After the reaction was completed, the reaction was quenched with saturated ammonium chloride. The reaction solution was concentrated, and the residue was purified with flash column chromatography (eluted with a gradient of water:methanol = 100 : 0-0 : 100) to obtain 1400 mg of the title product as a white solid. MS (m/z+Na): 189.1 $[M+H]^+$

**Intermediate 11: (4-(1*H*-pyrazol-1-yl)phenyl)methanol**

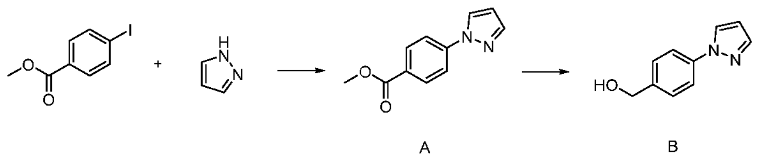

(A) methyl 4-(1*H*-pyrazol-1-yl)benzoate

To a reaction flask, methyl 4-iodobenzoate (1048 mg, 4.0 mmol), pyrazole (544 mg, 8.0 mmol), cesium carbonate (1303 mg, 4.0 mmol), copper oxide (32 mg, 0.4 mmol), ferric acetylacetonate (424 mg, 1.2 mmol) and DMF (10 ml) were added, and the mixture was heated to reflux for 15 hours. After the reaction was completed, the reaction solution was cooled to room temperature and then added ethyl acetate (20 ml). The mixture was filtered, and the resulting solid was washed with ethyl acetate. After the filtrate was concentrated, the residue was purified with flash column chromatography (eluted with a gradient of water:methanol = 100 : 0-0 : 100) to obtain 557 mg of the title product as a solid. MS (m/z): 203.1 $[M+H]^+$

(B) (4-(1*H*-pyrazol-1-yl)phenyl)methanol

In a reaction flask, methyl 4-(1*H*-pyrazol-1-yl)benzoate (557 mg, 2.76 mmol) was dissolved in anhydrous tetrahydrofuran (25 ml). In an ice bath, lithium aluminium hydride (314 mg, 8.28 mmol) was added in portions to the reaction solution. After the addition was completed, the reaction solution was stirred for another 15 hours. After the reaction was completed, the reaction was quenched by adding methanol dropwise in an ice bath. The reaction solution was concentrated, and the residue was purified with flash column chromatography (eluted with a gradient of water:methanol = 100 : 0-0 : 100) to obtain 390 mg of the title product. MS (m/z): 175.1 $[M+H]^+$

**Intermediate 12: tert-butyl (7-fluoro-4-oxo-4*H*-pyrido[1,2-a]pyrimidin-3-yl)carbamate**

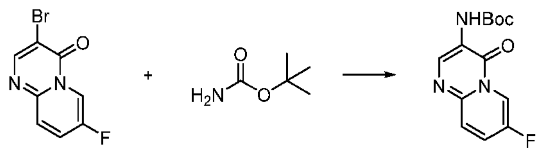

To a reaction flask, 3-bromo-7-fluoro-4*H*-pyrido[1,2-a]pyrimidin-4-one (486 mg, 2.0 mmol), tert-butyl carbamate (468 mg, 4.0 mmol), $Pd_2(dba)_3$ (91 mg, 0.1 mmol), cesium carbonate (716 mg, 2.2 mmol), Xantphos (116 mg, 0.2 mmol) and dioxane (20 ml) were added; and under nitrogen atmosphere, the reaction solution was heated to reflux for 15 hours. The reaction solution was concentrated, and the residue was purified with flash column chromatography (eluted with a gradient of water:methanol = 100 : 0-0 : 100) to obtain 490 mg of the title product as a pale yellow solid. MS (m/z): 280.1 $[M+H]^+$

**Intermediate 13: 6-((5-methoxy-6-nitropyridin-3-yl)oxy)-3-methylquinazolin-4(3*H*)-one**

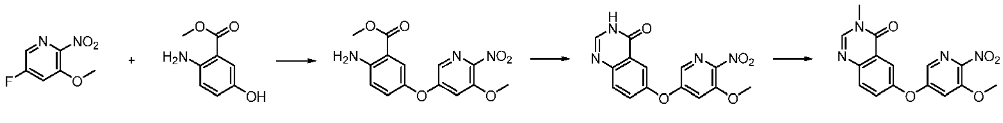

A B C

(A) methyl 2-amino-5-((5-methoxy-6-nitropyridin-3-yl)oxy)benzoate

To a reaction flask, 5-fluoro-3-methoxy-2-nitropyridine (516 mg, 3.0 mmol), methyl 2-amino-5-hydroxybenzoate (502 mg, 3.0 mmol), potassium carbonate (829 mg, 6.0 mmol) and DMF (10 ml) were added. The mixture was heated at 80°C for 4 hours. The reaction solution was cooled to room temperature; water (50 ml) was then added; and the mixture was extracted with ethyl acetate (50 ml) twice. The organic phases were combined, washed with saturated brine twice and then concentrated. The residue was purified with flash column chromatography (eluted with a gradient of water:methanol = 100 : 0-0 : 100) to obtain 900 mg of the title product as a pale yellow solid. MS (m/z): 320.0 $[M+H]^+$

(B) 6-((5-methoxy-6-nitropyridin-3-yl)oxy)quinazolin-4(3H)-one

In a reaction flask, methyl 2-amino-5-((5-methoxy-6-nitropyridin-3-yl)oxy)benzoate (900 mg, 2.82 mmol) was suspended in a mixed solvent of formic acid (5 ml) and formamide (5 ml), and the reaction solution was heated at 135°C for 5 hours. After the reaction was completed, the reaction solution was cooled to room temperature and was added water (100 ml) and ethyl acetate (50 ml); liquid separation was carried out; and the aqueous phase was extracted with ethyl acetate twice. The organic phases were combined and concentrated, and the residue was purified with flash column chromatography (eluted with a gradient of water:methanol = 100 : 0-0 : 100) to obtain 720 mg of the title product as a pale yellow solid. MS (m/z): 315.0 $[M+H]^+$

(C) 6-((5-methoxy-6-nitropyridin-3-yl)oxy)-3-methylquinazolin-4(3*H*)-one

To a reaction flask, 6-((5-methoxy-6-nitropyridin-3-yl)oxy)quinazolin-4(3*H*)-one (314 mg, 1.0 mmol), iodomethane (170 mg, 1.2 mmol), potassium carbonate (138 mg, 1.0 mmol) and DMF (3 ml) were added, and the mixture was stirred at room temperature for 3 hours. After the reaction was completed, water (20 ml) was added, and the mixture was extracted with ethyl acetate (20 ml) twice. The organic phases were combined, washed with saturated brine twice and then concentrated. The residue was purified with flash column chromatography (eluted with a gradient of water:methanol = 100 : 0-0 : 100) to obtain 300 mg of the title product as a pale yellow solid. MS (m/z): 329.0 $[M+H]^+$

**Intermediate 14: 9-((5-methoxy-6-nitropyridin-3-yl)oxy)-2,3-dihydroimidazo[1,2-c]quinazoline**

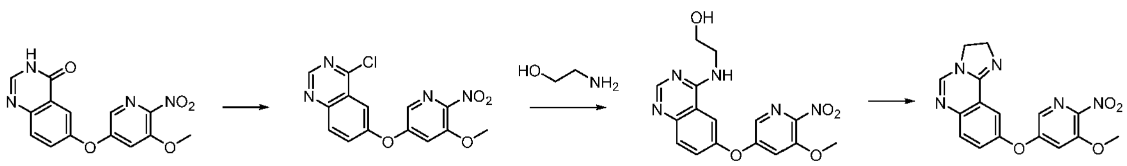

A B C

(A) 4-chloro-6-((5-methoxy-6-nitropyridin-3-yl)oxy)quinazoline

In a reaction flask, 6-((5-methoxy-6-nitropyridin-3-yl)oxy)quinazolin-4(3*H*)-one (157 mg, 0.5 mmol) was suspended in phosphorus oxychloride (5 ml), and the reaction solution was heated to reflux for 2 hours. After the reaction was completed, the reaction solution was concentrated to obtain 166 mg of the title product, which was used in the reaction of the next step directly. MS (m/z): 333.0 $[M+H]^+$

(B) 2-((6-((5-methoxy-6-nitropyridin-3-yl)oxy)quinazolin-4-yl)amino)ethan-1-ol

To a reaction flask, 4-chloro-6-((5-methoxy-6-nitropyridin-3-yl)oxy)quinazoline (166 mg, 0.5 mmol), ethanolamine (153 mg, 2.5 mmol) and dioxane (15 ml) were added. The reaction solution was heated to reflux for 15 hours. The reaction solution was concentrated, and the residue was purified with flash column chromatography (eluted with a gradient

of water:methanol = 100 : 0-0 : 100) to obtain 120 mg of the title product as a pale yellow solid. MS (m/z): 358.0 $[M+H]^+$

(C) 9-((5-methoxy-6-nitropyridin-3-yl)oxy)-2,3-dihydroimidazo[1,2-c]quinazoline

In a reaction flask, 2-((6-((5-methoxy-6-nitropyridin-3-yl)oxy)quinazolin-4-yl)amino)ethan-1-ol (120 mg, 0.335 mmol) was dissolved in dichloromethane (15 ml), and a solution of thionyl chloride (0.2 ml) in dichloromethane (2 ml) was added dropwise to the reaction solution in an ice bath. After the addition was completed, the reaction solution was stirred at room temperature for another 2 hours. The reaction solution was quenched with water and concentrated, and the residue was purified with flash column chromatography (eluted with a gradient of water:methanol = 100 : 0-0 : 100) to obtain 90 mg of the title product as a pale yellow solid. MS (m/z): 340.1$[M+H]^+$

**Intermediate 15: 7-fluoro-4*H*-pyrido[1,2-a]pyrimidin-4-one**

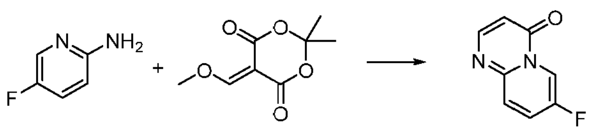

Under nitrogen atmosphere, 5-fluoro-2-aminopyridine (1.12 g, 10 mmol), 5-(methoxymethylene)-2,2-dimethyl-1,3-dioxane-4,6-dione (2.05 g, 11 mmol) and dioxane (20 ml) were successively added to a reaction flask, and the mixture was heated to reflux and stirred overnight. After cooled to room temperature, petroleum ether (60 ml) was added to the mixture, and the mixture was stirred for 2 hours and filtered. The solid was washed with petroleum ether and dried, and then diphenyl ether (20 ml) was added. Under nitrogen atmosphere, the mixture was heated to reflux and stirred for half an hour. After cooled to room temperature, petroleum ether (60 ml) was added to the mixture, and the mixture was stirred for 2 hours and then filtered. The solid was washed with petroleum ether, dried and then purified with flash column chromatography (eluted with a gradient of dichloromethane:methanol = 100 : 0-90 : 10) to obtain 800 mg of the title product as a brown solid. MS (m/z): 165.0 $[M+H]^+$

**Intermediate 16: 4-(bromomethyl)-2-(1-methyl-1*H*-pyrazol-4-yl)pyridine**

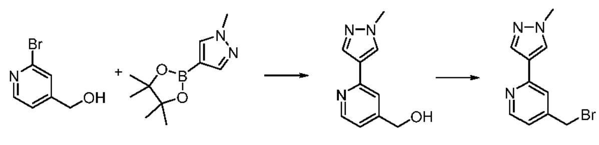

A B

(A) (2-(1-methyl-1*H*-pyrazol-4-yl)pyridin-4-yl)methanol

Under nitrogen atmosphere, (2-bromopyridin-4-yl)methanol (1.88 g, 10 mmol), 1-methyl-4-(4,4,5,5-tetramethyl-1,3,2-dioxaborolan-2-yl)-1*H*-pyrazole (2.5 g, 12 mmol), potassium carbonate (2.76 g, 20 mmol), $Pd(PPh_3)_2Cl_2$ (702 mg, 1 mmol) and dioxane/water (30 ml/6 ml) were successively added to a reaction flask, and the mixture was heated to 90°C and stirred for 6 hours. After cooled to room temperature, the reaction solution was concentrated, and the residue was purified with flash column chromatography (eluted with a gradient of water:methanol = 100 : 0-0 : 100) and flash column chromatography (eluted with a gradient of dichloromethane:methanol = 100 : 0-90 : 10) to obtain 1.5 g of the title product as a white solid. MS (m/z): 190.1 $[M+H]^+$

(B) 4-(bromomethyl)-2-(1-methyl-1*H*-pyrazol-4-yl)pyridine

Under nitrogen atmosphere, (2-(1-methyl-1*H*-pyrazol-4-yl)pyridin-4-yl)methanol (1.5 g, 7.9 mmol), carbon tetrabromide (3.94 g, 11.9 mmol) and dichloromethane (50 ml) were successively added to a reaction flask, and then triphenylphosphine (3.1 g, 11.9 mmol) was added in batches. The mixture was stirred at room temperature for 2 hours. Then the mixture was concentrated, and the residue was purified with flash column chromatography (eluted with a gradient of petroleum ether:ethyl acetate = 100 : 0-0 : 100) and flash column chromatography (eluted with a gradient of

water:methanol = 100 : 0-0 : 100) to obtain 1.0 g of the title product as a colorless oil. MS (m/z): 252.0 $[M+H]^+$

**Intermediate 17: 7-fluoro-3-morpholino-4*H*-pyrido[1,2-a]pyrimidin-4-one**

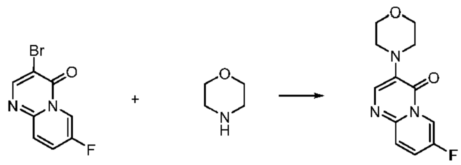

Under nitrogen atmosphere, 3-bromo-7-fluoro-4*H*-pyrido[1,2-a]pyrimidin-4-one (140 mg, 0.58 mmol), morpholine (76 mg, 0.87 mmol), $Pd_2(dba)_3$ (55 mg, 0.06 mmol), BINAP (68 mg, 0.11 mmol), t-BuONa (85 mg, 0.87 mmol) and toluene (8 ml) were successively added to a reaction flask, and the mixture was heated to 110°C and stirred for 3 hours. After concentration, the reaction solution was purified with flash column chromatography (eluted with a gradient of dichloromethane:methanol = 100 : 0-20 : 1) to obtain 80 mg of the title product as a yellow solid. MS (m/z): 250.0 $[M+H]^+$

**Intermediate 18: 3-cyclopropyl-7-fluoro-4H-pyrido[1,2-a]pyrimidin-4-one**

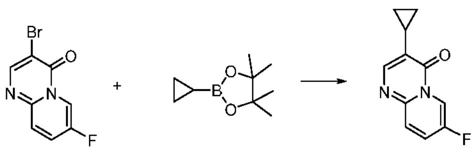

Under nitrogen atmosphere, 3-bromo-7-fluoro-4*H*-pyrido[1,2-a]pyrimidin-4-one (200 mg, 0.83 mmol), 2-cyclopropyl-4,4,5,5-tetramethyl-1,3,2-dioxaborolane (208 mg, 1.24 mmol), $Pd(OAc)_2$ (18 mg, 0.08 mmol), tricyclohexyl phosphine (45 mg, 0.16 mmol), potassium phosphate (530 mg, 2.48 mmol), toluene (8 ml) and water (1 ml) were successively added to a reaction flask, and the mixture was heated to 110°C and stirred for 24 hours. After concentration, the reaction solution was purified with flash column chromatography (eluted with petroleum ether:ethyl acetate = 1 : 1) to obtain 40 mg of the title product as a yellow solid. MS (m/z): 205.1 $[M+H]^+$

**Intermediate 19: 7-((6-bromopyridin-3-yl)oxy)-3-methyl-4*H*-pyrido[1,2-a]pyrimidin-4-one**

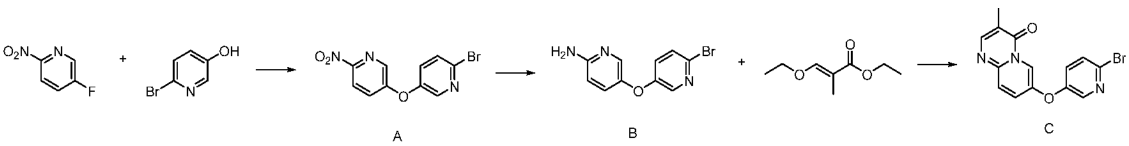

(A) 2-bromo-5-((6-nitropyridin-3-yl)oxy)pyridine

5-fluoro-2-nitropyridine (1.42 g, 10.0 mmol), 6-bromopyridin-3-ol (1.74 g, 10.0 mmol), potassium carbonate (2.76 g, 20.0 mmol) and DMF (20 ml) were added to a reaction flask. The mixture was heated at 100°C for 15 hours. The reaction solution was cooled to room temperature; water (100 ml) was then added; and the mixture was extracted with ethyl acetate (100 ml) twice. The organic phases were combined, washed with saturated brine twice and then concentrated. The residue was purified with flash column chromatography (eluted with a gradient of water:methanol = 100 : 0-0 : 100) to obtain 2.5 g of the title product as a yellow solid. MS (m/z): 296.0, 298.0 $[M+H]^+$

(B) 5-((6-bromopyridin-3-yl)oxy)pyridin-2-amine

2-bromo-5-((6-nitropyridin-3-yl)oxy)pyridine (2.5 g, 8.4 mmol), iron powder (1.88 g, 33.6 mmol), ammonium chloride (2.25 g, 42.0 mmol), ethanol (80 ml) and water (20 ml) were added to a reaction flask, and the reaction solution was

heated to reflux for 2 hours. After cooled to room temperature, the reaction solution was filtered, and the solid was washed with methanol. After the resulting filtrate was concentrated, the residue was purified with flash column chromatography (eluted with a gradient of water:methanol = 100 : 0-0 : 100) to obtain 2.25 g of the title product as a pale yellow solid. MS (m/z): 266.0, 268.0 $[M+H]^+$

(C) 7-((6-bromopyridin-3-yl)oxy)-3-methyl-4*H*-pyrido[1,2-a]pyrimidin-4-one

5-((6-bromopyridin-3-yl)oxy)pyridin-2-amine (2.25 g, 8.45 mmol), ethyl (E)-3-ethoxy-2-methylacrylate (2.7 g, 16.9 mmol) and acetic acid (20 ml) were added to a reaction flask, and the reaction solution was heated at 120°C for 15 hours. The reaction solution was concentrated, and the residue was purified with flash column chromatography (eluted with a gradient of water:methanol = 100 : 0-0 : 100) to obtain 2.5 g of the title product as a pale yellow solid. MS (m/z): 332.0, 334.0 $[M+H]^+$

**Intermediate 20: 5-methoxy-6-((4-methoxybenzyl)oxy)pyridin-3-ol**

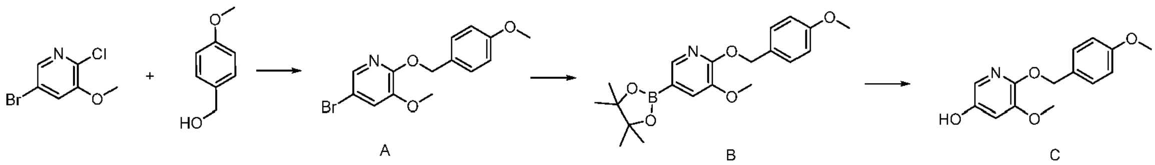

(A) 5-bromo-3-methoxy-2-((4-methoxybenzyl)oxy)pyridine

To a reaction flask, 5-bromo-2-chloro-3-methoxypyridine (500 mg, 2.26 mmol), (4-methoxyphenyl)methanol (342 mg, 2.48 mmol) and DMF (10 ml) were successively added, and then NaH (108 mg, 2.71 mmol) was added. The mixture was stirred at room temperature overnight. The reaction solution was poured into water, and the mixture was extracted with EA. The organic layer was washed with saturated brine, dried over anhydrous sodium sulfate and filtered. The filtrate was concentrated, and the residue was purified with flash column chromatography (petroleum ether:ethyl acetate = 3 : 1) to obtain 600 mg of the title product as a white solid. MS (m/z): 346.0 $[M+Na]^+$

(B) 3-methoxy-2-((4-methoxybenzyl)oxy)-5-(4,4,5,5-tetramethyl-1,3,2-dioxaborolan-2-yl)pyridine

Under nitrogen atmosphere, 5-bromo-3-methoxy-2-((4-methoxybenzyl)oxy)pyridine (500 mg, 1.54 mmol), $Pin_2B_2$ (588 mg, 2.32 mmol), $Pd(dppf)Cl_2$ (113 mg, 0.15 mmol), potassium acetate (455 mg, 4.64 mmol) and dioxane (15 ml) were successively added to a reaction flask, and the mixture was heated to 90°C and stirred for 3 hours. After concentration, the reaction solution was purified with flash column chromatography (petroleum ether:ethyl acetate = 5 : 1) to obtain 450 mg of the title product as a white solid. MS (m/z): 372.2 $[M+Na]^+$

(C) 5-methoxy-6-((4-methoxybenzyl)oxy)pyridin-3-ol

To a reaction flask, 3-methoxy-2-((4-methoxybenzyl)oxy)-5-(4,4,5,5-tetramethyl-1,3,2-dioxaborolan-2-yl)pyridine (371 mg, 1 mmol), NaOH (80 mg, 2 mmol), THF (5 ml) and water (1 ml) were successively added, and then $H_2O_2$ (0.5 ml) was added. The mixture was stirred at room temperature for 3 hours. The reaction solution was added $Na_2S_2O_3$ aqueous solution, and the mixture was extracted with EA. The organic layer was washed with saturated brine, dried over anhydrous sodium sulfate and filtered. The filtrate was concentrated, and the residue was purified with flash column chromatography (petroleum ether:ethyl acetate = 1 : 1) to obtain 200 mg of the title product as a white solid. MS (m/z): 284.1 $[M+Na]^+$

The following intermediates were prepared according to the preparation procedure of intermediate 20 using corresponding starting materials and reagents under appropriate conditions that will be recognized by one skilled in the art.

| Intermediate | Structural formula | MS $(M+H)^+$ |
|---|---|---|
| **21** | 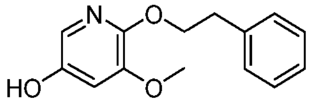 | **246.1** |

(continued)

| Intermediate | Structural formula | MS (M+H)$^+$ |
|---|---|---|
| **22** | 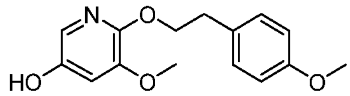 | **276.1** |
| **23** | 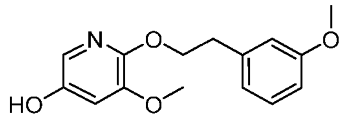 | **276.1** |
| **24** | 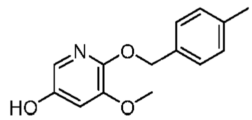 | **246.1** |
| **25** | 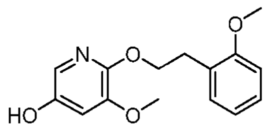 | **276.1** |
| **26** | 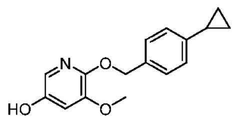 | **272.1** |
| **27** | 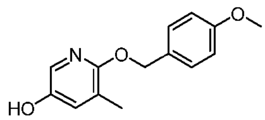 | **246.1** |
| **28** | 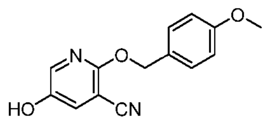 | **255.1[M-1]** |
| **35** | 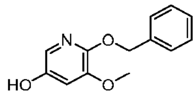 | **232.1** |
| **37** | 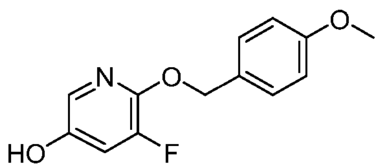 | **272.1[M+Na]** |
| **38** | 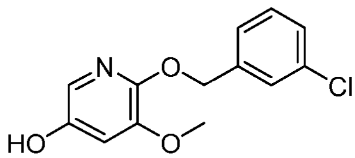 | **266.1** |
| **39** | 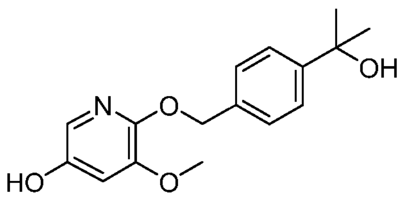 | **290.0** |

(continued)

| Intermediate | Structural formula | MS (M+H)+ |
|---|---|---|
| **40** | 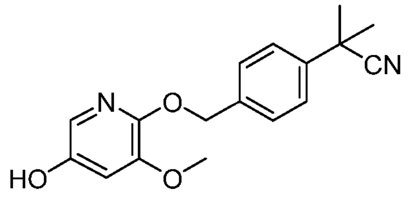 | **299.1** |
| **41** | 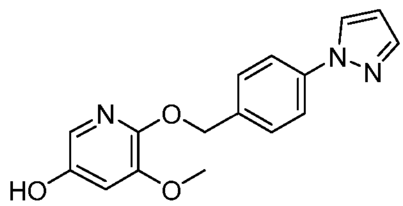 | **298.1** |
| **47** | 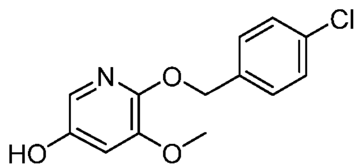 | **266.1** |

**Intermediate 29: 2-((4-cyclopropylbenzyl)oxy)pyrimidin-5-ol**

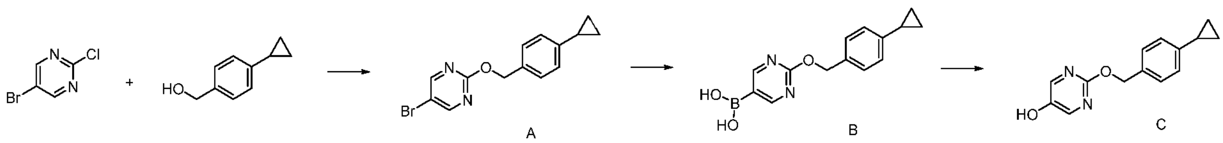

A B C

(A) 5-bromo-2-((4-cyclopropylbenzyl)oxy)pyrimidine

To a reaction flask, 5-bromo-2-chloropyrimidine (192 mg, 1 mmol), (4-cyclopropylphenyl)methanol (148 mg, 1 mmol) and DMF (5 ml) were successively added, and then NaH (48 mg, 1.2 mmol) was added. The mixture was stirred at room temperature overnight. Water was added to the reaction solution, and the mixture was extracted with EA. The organic layer was washed with saturated brine, concentrated and purified with flash column chromatography (petroleum ether:ethyl acetate = 2 : 1) to obtain 300 mg of the title product as a white solid. MS (m/z): 304.9[M+1]+

(B) (2-((4-cyclopropylbenzyl)oxy)pyrimidin-5-yl)boronic acid

Under nitrogen atmosphere, 5-bromo-2-((4-cyclopropylbenzyl)oxy)pyrimidine (220 mg, 0.72 mmol), $Pin_2B_2$ (275 mg, 1.08 mmol), Pd(dppf)$Cl_2$ (53 mg, 0.07 mmol), potassium acetate (212 mg, 2.17 mmol) and dioxane (8 ml) were successively added to a reaction flask, and the mixture was heated to 90°C and stirred for 3 hours. The reaction solution was concentrated and purified with flash column chromatography (petroleum ether:ethyl acetate = 1 : 1) to obtain 180 mg of the title product as a white solid. MS (m/z): 271.1[M+1]+

(C) 2-((4-cyclopropylbenzyl)oxy)pyrimidin-5-ol

To a reaction flask, (2-((4-cyclopropylbenzyl)oxy)pyrimidin-5-yl)boronic acid (180 mg, 0.67 mmol), NaOH (60 mg, 1.33 mmol), THF (10 ml) and water (2 ml) were successively added, and then $H_2O_2$ (1 ml) was added. The mixture was stirred at room temperature for 3 hours. $Na_2S_2O_3$ aqueous solution was added to the reaction solution, and the mixture was extracted with EA. The organic layer was washed with saturated brine, concentrated and purified with flash column chromatography (petroleum ether:ethyl acetate = 1 : 1) to obtain 120 mg of the title product as a white solid. MS (m/z): 243.1 [M+1]+

The following intermediates were prepared according to the preparation procedure of intermediate 29 using corresponding starting materials and reagents under appropriate conditions that will be recognized by one skilled in the art.

| Intermediate | Structural formula | MS (M+H)$^+$ |
|---|---|---|
| **30** | 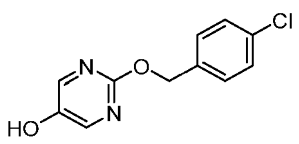 | **237.0** |
| **31** | 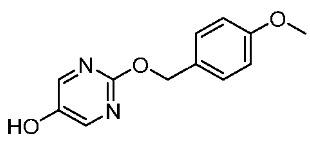 | **233.1** |

**Intermediate 32: 7-((5-ethoxy-6-hydroxypyridin-3-yl)oxy)-3-methyl-4*H*-pyrido[1,2-a]pyrimidin-4-one**

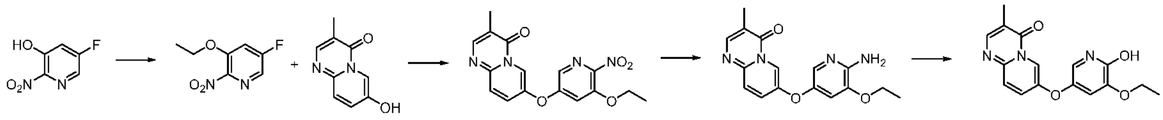

A B C D

(A) 3-ethoxy-5-fluoro-2-nitropyridine

Under nitrogen atmosphere, 5-fluoro-2-nitropyridin-3-ol (1 g, 6.3 mmol), iodoethene (0.8 ml, 10 mmol), potassium carbonate (1.38 g, 10 mmol) and DMF (15 ml) were successively added to a reaction flask, and the mixture was heated to 50°C and stirred overnight. After cooled to room temperature, the mixture was added water (60 ml), stirred for 1 hour and filtered. The solid was washed with water and dried to obtain 850 mg of the title product as a brown solid. MS (m/z): 187.0 [M+H]$^+$

(B) 7-((5-ethoxy-6-nitropyridin-3-yl)oxy)-3-methyl-4*H*-pyrido[1,2-a]pyrimidin-4-one

Under nitrogen atmosphere, 3-ethoxy-5-fluoro-2-nitropyridine (850 mg, 4.6 mmol), 7-hydroxy-3-methyl-4*H*-pyrido[1,2-a]pyrimidin-4-one (810 mg, 4.6 mmol), cesium carbonate (2.25 g, 6.9 mmol) and DMF (10 ml) were successively added to a reaction flask, and the mixture was heated to 80°C and stirred overnight. After cooled to room temperature, the mixture was added water (60 ml) and extracted with ethyl acetate (100 ml) twice. The organic phases were combined, washed with saturated brine (80 ml) twice and then concentrated. The residue was purified with flash column chromatography (eluted with a gradient of dichloromethane:methanol = 100 : 0-90 : 10) to obtain 900 mg of the title product as a black solid. MS (m/z): 343.1 [M+H]$^+$

(C) 7-((6-amino-5-ethoxypyridin-3-yl)oxy)-3-methyl-4*H*-pyrido[1,2-a]pyrimidin-4-one

Under nitrogen atmosphere, 7-((5-ethoxy-6-nitropyridin-3-yl)oxy)-3-methyl-4*H*-pyrido[1,2-a]pyrimidin-4-one (900 mg, 2.6 mmol), iron powder (582 mg, 10.4 mmol), ammonium chloride (695 mg, 13 mmol) and ethanol/water (16 ml/4 ml) were successively added to a reaction flask, and the mixture was heated to reflux and stirred for 2 hours. After cooled to room temperature, the reaction solution was filtered, and the solid was washed with methanol. After the resulting filtrate was concentrated, the residue was purified with flash column chromatography (eluted with a gradient of water (+0.05% formic acid):methanol = 100 : 0-0 : 100) to obtain 550 mg of the title product as a yellow solid. MS (m/z): 313.1 [M+H]$^+$

(D) 7-((5-ethoxy-6-hydroxypyridin-3-yl)oxy)-3-methyl-4*H*-pyrido[1,2-a]pyrimidin-4-one

Under nitrogen atmosphere, 7-((6-amino-5-ethoxypyridin-3-yl)oxy)-3-methyl-4*H*-pyrido[1,2-a]pyrimidin-4-one (550 mg, 1.76 mmol), sulfuric acid aqueous solution (10 ml, 20% (by weight)) and sodium nitrite (242 mg, 3.5 mmol) were

successively added to a reaction flask, and the mixture was stirred at room temperature for 2 hours. After concentration, the reaction solution was purified with flash column chromatography (eluted with a gradient of water:methanol = 100 : 0-0 : 100) to obtain 500 mg of the title product as a yellow solid. MS (m/z): 314.1 [M+H]+

The following intermediates were prepared according to the preparation procedure of intermediate 32 using corresponding starting materials and reagents under appropriate conditions that will be recognized by one skilled in the art.

| Interme diate | Structural formula | MS (M+H)+ |
|---|---|---|
| **33** | 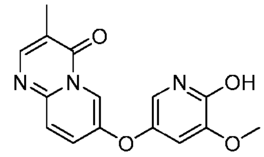 | **300.0** |
| **42** | 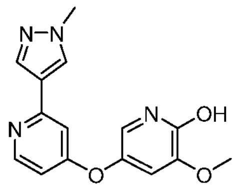 | **299.1** |
| **43** | 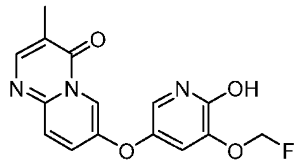 | **318.1** |
| **44*** | 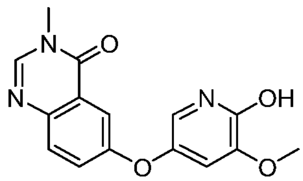 | **300.0** |
| **45*** | 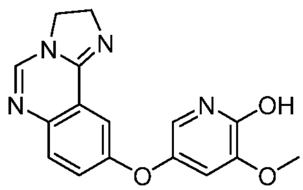 | **311.1** |
| **46** | 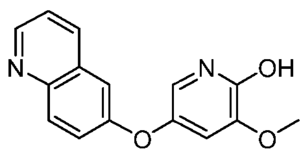 | **269.1** |
| *: Intermediate 44 and 45 were prepared according to the preparation procedure in steps (C) and (D) of intermediate 32 using intermediates 13 and 14 as starting materials, respectively. | | |

**Intermediate 48: 3-bromo-7-fluoro-4*H*-pyrido[1,2-a]pyrimidin-4-one**

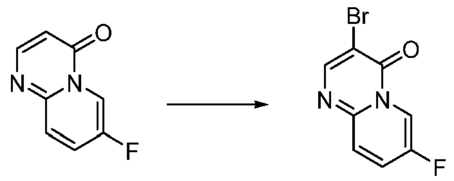

7-fluoro-4*H*-pyrido[1,2-a]pyrimidin-4-one (920 mg, 5.6 mmol), *N*-bromosuccinimide (998 mg, 5.6 mmol) and DMF (20 ml) were added to a reaction flask, and the reaction was stirred at room temperature for 2 hours. After the reaction was completed, water (30 ml) and ethyl acetate (40 ml) were added. The organic phases were separated, and the aqueous phase was extracted with ethyl acetate. The organic phases were combined, washed with saturated brine and then concentrated. The residue was purified with flash column chromatography (eluted with a gradient of petroleum ether:ethyl acetate = 100 : 0-0 : 100) to obtain 1.1 g of the title product as a white solid. MS (m/z): 244.0 $[M+H]^+$

**Intermediate 49: (3-methyl-4-oxo-4*H*-pyrido[1,2-a]pyrimidin-7-yl)boronic acid**

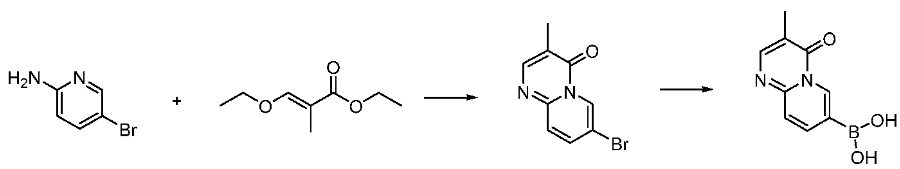

(A) 7-bromo-3-methyl-4*H*-pyrido[1,2-a]pyrimidin-4-one

5-bromopyridin-2-amine (1.0 g, 5.78 mmol), ethyl 3-ethoxy-2-methylacrylate (1.0 g, 6.36 mmol) and acetic acid (30 ml) were added to a reaction flask and the mixture was heated to reflux overnight. The reaction solution was concentrated, and the residue was purified with flash column chromatography (eluted with a gradient of water:methanol = 100 : 0-0 : 100) to obtain 1.23 g of the product as a white solid. MS (m/z): 239.1 $[M+H]^+$

(B) (3-methyl-4-oxo-4*H*-pyrido[1,2-a]pyrimidin-7-yl)boronic acid

7-bromo-3-methyl-4*H*-pyrido[1,2-a]pyrimidin-4-one (1.23 g, 5.14 mmol), $Pin_2B_2$ (1.96 g, 7.716 mmol), Pd(dppf) $Cl_2 \cdot CH_2Cl_2$ (420 mg, 0.51 mmol), KOAc (1.51 g, 15.43 mmol) and dioxane (40.0 ml) were added to a reaction flask and stirred at 100°C overnight. The reaction solution was concentrated. The residue was purified with flash column chromatography (eluted with a gradient of water:methanol = 100 : 0-0 : 100) to obtain 766 mg of the product as a white solid. MS (m/z): 205.1 $[M+H]^+$

**Intermediate 50: 4-fluoro-2-(1-methyl-1*H*-pyrazol-4-yl)pyridine**

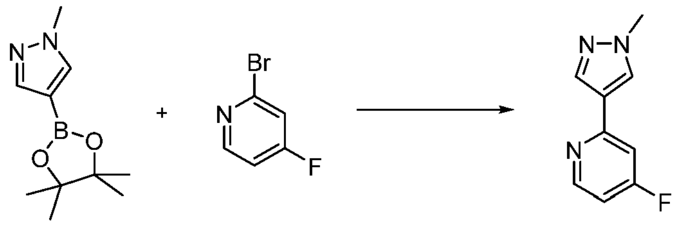

2-bromo-4-fluoropyridine (528 mg, 3.0 mmol), 1-methyl-4-(4,4,5,5-tetramethyl-1,3,2-dioxaborolan-2-yl)-1*H*-pyrazole (936 mg, 4.5 mmol), potassium carbonate (829 mg, 6.0 mmol), Pd(dppf)$Cl_2$ (110 mg, 0.15 mmol), dioxane (20 ml) and water (5 ml) were added to a reaction flask. Under nitrogen atmosphere, the reaction solution was heated to reflux and stirred for 15 hours. After the reaction was completed, the reaction solution was concentrated to dryness, and the residue was purified with flash column chromatography (eluted with a gradient of water:methanol = 100 : 0-0 : 100) to obtain 450 mg of the title product as a white solid. MS (m/z): 178.2$[M+H]^+$

**Intermediate 51: 2-(4-(4-fluoropyridin-2-yl)-1*H*-pyrazol-1-yl)ethan-1-ol**

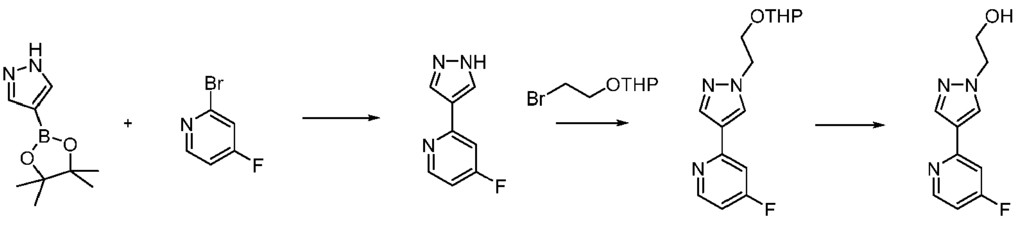

A B C

(A) 4-fluoro-2-(1*H*-pyrazol-4-yl)pyridine

The title product was prepared according to the preparation procedure of intermediate 50 using corresponding starting materials and reagents. MS (m/z): 164.2 [M+H]$^+$

(B) 4-fluoro-2-(1-(2-((tetrahydro-2*H*-pyran-2-yl)oxy)ethyl)-1*H*-pyrazol-4-yl)pyridine

In a reaction flask, 4-fluoro-2-(1*H*-pyrazol-4-yl)pyridine (420 mg, 2.57 mmol) was dissolved in DMF (5 ml). Sodium hydride (113 mg, 2.82 mmol) was added in portions to the reaction solution. After the addition was completed, the reaction solution was stirred at room temperature for half an hour. Subsequently, 2-(2-bromoethoxy)tetrahydro-2*H*-pyran (565 mg, 2.7 mmol) was dissolved in 2 ml of DMF and then added dropwise to the reaction solution. After the addition was completed, the reaction solution was stirred at room temperature for 15 hours. After the reaction was completed, the reaction solution was concentrated. The residue was purified with flash column chromatography (eluted with a gradient of water:methanol = 100 : 0-0 : 100) to obtain 630 mg of the title product as a pale yellow solid. MS (m/z): 292.2 [M+H]$^+$

(C) 2-(4-(4-fluoropyridin-2-yl)-1*H*-pyrazol-1-yl)ethan-1-ol

In a reaction flask, 4-fluoro-2-(1-(2-((tetrahydro-2*H*-pyran-2-yl)oxy)ethyl)-1*H*-pyrazol-4-yl)pyridine (630 mg, 2.16 mmol) and hydrochloric acid (1 ml) were dissolved in methanol (10 ml). After the addition was completed, the reaction solution was stirred at room temperature for 2 hours. After the reaction was completed, the reaction solution was concentrated. The residue was purified with flash column chromatography (eluted with a gradient of water:methanol = 100 : 0-0 : 100) to obtain 400 mg of the title product as a pale yellow solid. MS (m/z): 208.2 [M+H]$^+$

**Intermediate 52: 3-(4-cyclopropylphenyl)-2,3-dihydro-[1,4]dioxino[2,3-b]pyridin-7-ol**

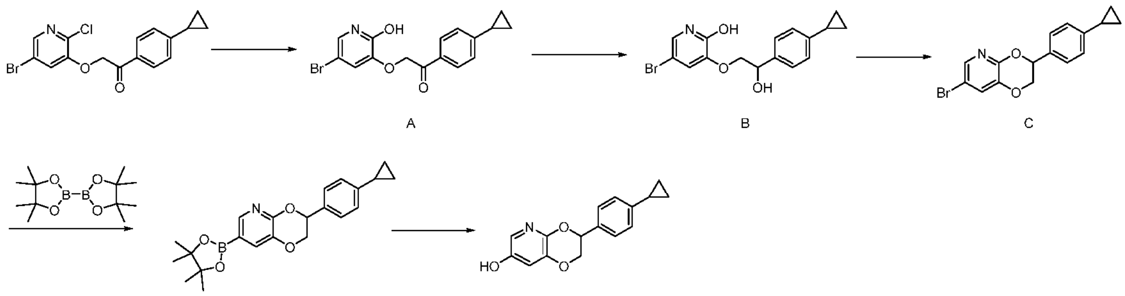

A B C

D E

(A) 2-((5-bromo-2-hydroxypyridin-3-yl)oxy)-1-(4-cyclopropylphenyl)ethan-1-one

2-((5-bromo-2-chloropyridin-3-yl)oxy)-1-(4-cyclopropylphenyl)ethan-1-one (366 mg, 1.0 mmol), sodium acetate (820 mg, 10.0 mmol) and acetic acid (15 ml) were added to a 20 ml microwave tube. The mixture was reacted in a microwave reactor, and the reaction was heated at 160°C for 4 hours. After the reaction was completed, the reaction solution was concentrated to dryness, and the residue was purified with flash column chromatography (eluted with a

gradient of water:methanol = 100 : 0-0 : 100) to obtain 210 mg of a white solid mixture. MS (m/z): 348.0, 350.0 $[M+H]^+$

(B) 5-bromo-3-(2-(4-cyclopropylphenyl)-2-hydroxyethoxy)pyridin-2-ol

In a reaction flask, 2-((5-bromo-2-hydroxypyridin-3-yl)oxy)-1-(4-cyclopropylphenyl)ethan-1-one (210 mg, 0.60 mmol) was dissolved in methanol (20 ml), and then sodium borohydride (246 mg, 1.20 mmol) was added in portions in an ice bath. After the addition was completed, the reaction solution was stirred at room temperature for 2 hours. The reaction solution was adjusted with dilute hydrochloric acid to a pH value of about 5-6. The reaction solution was concentrated to dryness, and the residue was purified with flash column chromatography (eluted with a gradient of water:methanol = 100 : 0-0 : 100) to obtain 180 mg of the title product as a white solid. MS (m/z-$H_2O$): 332.2, 334.2 $[M+H]^+$

(C) 7-bromo-3-(4-cyclopropylphenyl)-2,3-dihydro-[1,4]dioxino[2,3-b]pyridine

To a reaction flask, 5-bromo-3-(2-(4-cyclopropylphenyl)-2-hydroxyethoxy)pyridin-2-ol (180 mg, 0.51 mmol), triphenylphosphine (268 mg, 1.02 mmol), anhydrous tetrahydrofuran (20 ml) and diisopropyl azodicarboxylate (206 mg, 1.02 mmol) were successively added, and the reaction solution was stirred at room temperature for 2 hours. After the reaction was completed, the reaction was quenched by adding water (2 ml). The reaction solution was concentrated, and the residue was purified with flash column chromatography (eluted with a gradient of water:methanol = 100 : 0-0 : 100) to obtain 130 mg of the title product as a white solid. MS (m/z): 332.2, 334.2 $[M+H]^+$ (D) 3-(4-cyclopropylphenyl)-7-(4,4,5,5-tetramethyl-1,3,2-dioxaborolan-2-yl)-2,3-dihydro-[1,4]dioxino[2,3-b]pyridine

Under nitrogen atmosphere, 7-bromo-3-(4-cyclopropylphenyl)-2,3-dihydro-[1,4]dioxino[2,3-b]pyridine (130 mg, 0.391 mmol), $Pin_2B_2$ (199 mg, 0.782 mmol), potassium acetate (77 mg, 0.782 mmol), Pd(dppf)$Cl_2$ (24 mg, 0.04 mmol) and dioxane (30 ml) were successively added to a reaction flask, and the mixture was heated to reflux and stirred for 15 hours. After cooled to room temperature, the reaction solution was concentrated, and ethyl acetate (100 ml) was added. The mixture was stirred for 1 hour and filtered. The solid was washed with ethyl acetate. The filtrate was concentrated to obtain a crude product, and the crude product was used in the reaction of the next step directly. MS (m/z): 380.0 $[M+H]^+$

(E) 3-(4-cyclopropylphenyl)-2,3-dihydro-[1,4]dioxino[2,3-b]pyridin-7-ol

The crude (148 mg, 0.39 mmol) prepared in the previous step, THF (5 ml) and sodium hydroxide aqueous solution (31 mg, dissolved in 2 ml of water) were added to a reaction flask, and then hydrogen peroxide (1 ml, 30%wt) was added dropwise in an ice bath. After warmed to room temperature, the mixture was stirred for 1 hour. After cooling in an ice bath, saturated sodium thiosulfate aqueous solution was added dropwise. The mixture was stirred for 5 minutes, and concentrated after no peroxide was detected with a potassium iodide-starch test paper. The residue was purified with flash column chromatography (eluted with a gradient of petroleum ether:ethyl acetate = 100 : 0-0 : 100) to obtain 86 mg of the title product as a white solid. MS (m/z): 270.0 $[M+H]^+$

**Intermediate 53: 3-(6-cyclopropylpyridin-3-yl)-2,3-dihydro-[1,4]dioxino[2,3-b]pyridin-7-ol**

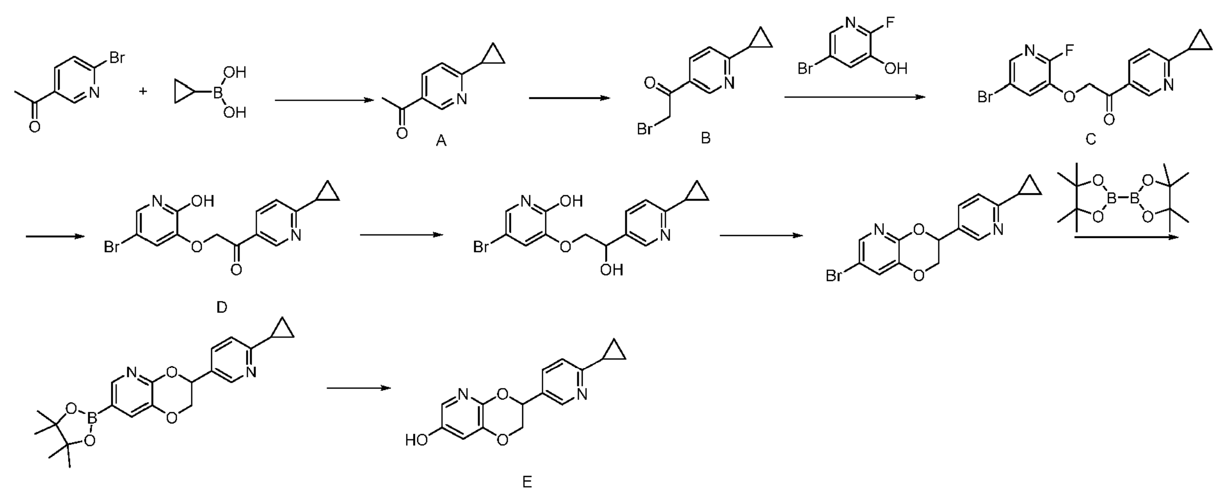

(A) 1-(6-cyclopropylpyridin-3-yl)ethan-1-one

To a reaction flask, 1-(6-bromopyridin-3-yl)ethan-1-one (8.0 g, 40.0 mmol), cyclopropylboronic acid (10.3 g, 120.0 mmol), palladium acetate (450 mg, 2.0 mmol), tricyclohexyl phosphine (1.12 g, 4.0 mmol), potassium phosphate (17 g, 80.0 mmol), toluene (150 ml) and water (30 ml) were added. The reaction solution was heated to reflux for 15 hours under nitrogen atmosphere. After the reaction was completed, the reaction solution was concentrated to dryness, and the residue was purified with flash column chromatography (eluted with a gradient of water:methanol = 100 : 0-0 : 100) to obtain 4.8 g of the title product as a white solid. MS (m/z): 162.2 $[M+H]^+$

(B) 2-bromo-1-(6-cyclopropylpyridin-3-yl)ethan-1-one

To a reaction flask, 1-(6-cyclopropylpyridin-3-yl)ethan-1-one (3.22 g, 20.0 mmol) and hydrobromic acid acetic acid solution (20 ml) were added. Liquid bromine (3.2 g, 20.0 mmol) was added dropwise to the mixture at room temperature. The reaction solution was stirred at room temperature for 3 hours. After the reaction was completed, the reaction solution was neutralized with saturated sodium bicarbonate. Then the mixture was concentrated to dryness, and the residue was purified with flash column chromatography (eluted with a gradient of water:methanol = 100 : 0-0 : 100) to obtain 4.3 g of the product as a pale yellow oil. MS (m/z): 240.0, 242.0 $[M+H]^+$

(C) 2-((5-bromo-2-fluoropyridin-3-yl)oxy)-1-(6-cyclopropylpyridin-3-yl)ethan-1-one

In a reaction flask, 5-bromo-2-fluoropyridin-3-ol (3436 mg, 17.9 mmol) was dissolved in DMF (50 ml). Sodium hydride (716 mg, 17.9 mmol) was added in portions to the reaction solution. After the addition was completed, the reaction solution was stirred at room temperature for half an hour. Subsequently, 2-bromo-1-(6-cyclopropylpyridin-3-yl)ethan-1-one (4300 mg, 17.9 mmol) was dissolved in 5 ml of DMF and then added dropwise to the reaction solution. After the addition was completed, the reaction solution was stirred at room temperature for 2 hours. After the reaction was completed, water (100 ml) was added, and the mixture was extracted with ethyl acetate (200 ml) twice. The organic phases were combined, washed with saturated brine twice and then concentrated. The residue was purified with flash column chromatography (eluted with a gradient of water:methanol = 100 : 0-0 : 100) to obtain 4.65 g of the title product as a pale yellow solid. MS (m/z): 367.0, 369.0 $[M+H]^+$

(D) 2-((5-bromo-2-hydroxypyridin-3-yl)oxy)-1-(6-cyclopropylpyridin-3-yl)ethan-1-one

2-((5-bromo-2-fluoropyridin-3-yl)oxy)-1-(6-cyclopropylpyridin-3-yl)ethan-1-one (4.65 g, 13.2 mmol), sodium acetate (10.82 g, 132.0 mmol) and acetic acid (100 ml) were added to a reaction flask. The mixture was heated at 140°C for 10 hours. After the reaction was completed, the reaction solution was concentrated to dryness, and the residue was purified with flash column chromatography (eluted with a gradient of water:methanol = 100 : 0-0 : 100) to obtain 3.1 g of a white solid mixture. MS (m/z): 349.0, 351.0 $[M+H]^+$

(E) 3-(6-cyclopropylpyridin-3-yl)-2,3-dihydro-[1,4]dioxino[2,3-b]pyridin-7-ol

The title product was prepared according to the preparation procedure in steps (B)-(E) of intermediate 52 using corresponding starting materials and reagents. MS (m/z): 271.2 $[M+H]^+$

The following intermediate was prepared according to the preparation procedure in steps (C)-(E) of intermediate 53 using corresponding starting materials and reagents under appropriate conditions that will be recognized by one skilled in the art.

| Intermediate | Structural formula | MS $(M+H)^+$ |
|---|---|---|
| **54** | 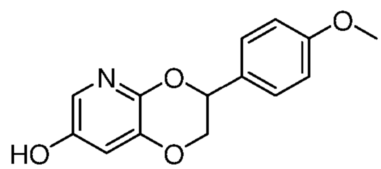 | **260.1** |

**Intermediate 55: 3-(6-methoxypyridin-3-yl)-2,3-dihydro-[1,4]dioxino[2,3-b]pyridin-7-ol**

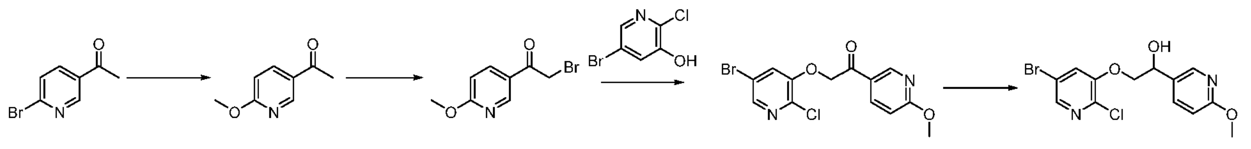

A B C

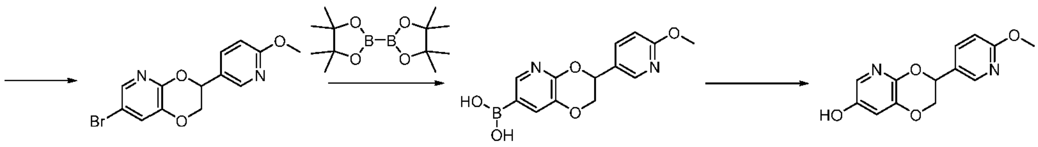

D E

(A) 1-(6-methoxypyridin-3-yl)ethan-1-one

To a reaction flask, 1-(6-bromopyridin-3-yl)ethan-1-one (3.20 g, 16.0 mmol), methanol (30 ml) and sodium methoxide (1.70 g, 32.0 mmol) were added. Under nitrogen atmosphere, the mixture was heated to reflux and stirred for 3 hours, and then cooled to room temperature. The reaction solution was concentrated, added water (30 ml) and then extracted with ethyl acetate (50 ml) twice. The organic phases were combined and washed with saturated brine (30 ml). The resulting organic phase was dried, filtered and concentrated to obtain 2.4 g of a light yellow solid. MS (m/z): 152.1 $[M+H]^+$.

(B) 2-((5-bromo-2-chloropyridin-3-yl)oxy)-1-(6-methoxypyridin-3-yl)ethan-1-one

The title product was prepared according to the preparation procedure in steps (B) and (C) of intermediate 53 using corresponding starting materials and reagents. MS (m/z): 357.0 $[M+H]^+$.

(C) 2-((5-bromo-2-chloropyridin-3-yl)oxy)-1-(6-methoxypyridin-3-yl)ethan-1-ol

2-((5-bromo-2-chloropyridin-3-yl)oxy)-1-(6-methoxypyridin-3-yl)ethan-1-one (3.0 g, 8.4 mmol) and anhydrous tetrahydrofuran (45 ml) were added to a reaction flask. Under nitrogen atmosphere, the mixture was cooled to -10°C and sodium borohydride (159 mg, 4.2 mmol) was added. The resulting mixture was stirred for 2 hours at -10°C. The reaction solution was quenched by adding 5 ml of water dropwise and concentrated. The residue was purified with flash column chromatography (eluted with a gradient of water:acetonitrile = 100 : 0-0 : 100) and flash column chromatography (eluted with a gradient of dichloromethane:methanol = 100 : 0-90 : 10) to obtain 1.8 g of a white solid. MS (m/z): 359.0 $[M+H]^+$.

(D) 7-bromo-3-(6-methoxypyridin-3-yl)-2,3-dihydro-[1,4]dioxino[2,3-b]pyridine

2-((5-bromo-2-chloropyridin-3-yl)oxy)-1-(6-methoxypyridin-3-yl)ethan-1-ol (1.80 g, 7.5 mmol) and anhydrous tetrahydrofuran (60 ml) were added to a reaction flask. Under nitrogen atmosphere, the mixture was cooled to -70°C, and KHMDS (7.5 ml, 1 M in tetrahydrofuran solution) was added dropwise. The resulting mixture was warmed to - 20°C slowly and then stirred for 3 hours at a temperature of -20°C to 0°C. The reaction solution was quenched by adding saturated ammonium chloride aqueous solution (50 ml), and extracted with ethyl acetate (60 ml). After the organic phase was concentrated, the resulting residue was purified with flash column chromatography (eluted with a gradient of water (0.1% formic acid): acetonitrile = 100 : 0-0 : 100) and flash column chromatography (eluted with a gradient of dichloromethane:methanol = 100 : 0-90 : 10) to obtain 870 mg of a white solid. MS (m/z): 323.0 $[M+H]^+$.

(E) 3-(6-methoxypyridin-3-yl)-2,3-dihydro-[1,4]dioxino[2,3-b]pyridin-7-ol

The title product was prepared according to the preparation procedure in steps (D) and (E) of intermediate 52 using corresponding starting materials and reagents. MS (m/z): 261.2 $[M+H]^+$.

**Intermediate 56: (S)-3-(6-methoxypyridin-3-yl)-2,3-dihydro-[1,4]dioxino[2,3-b]pyridin-7-ol**

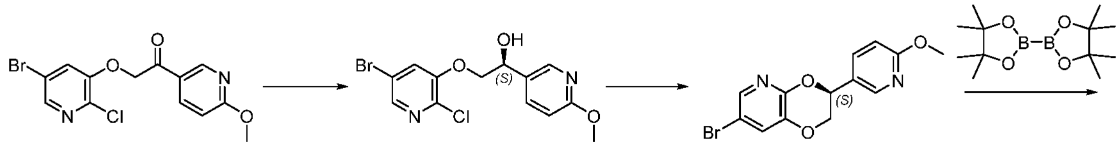

A B

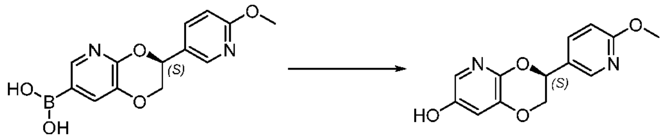

C D

(A) (S)-2-((5-bromo-2-chloropyridin-3-yl)oxy)-1-(6-methoxypyridin-3-yl)ethan-1-ol

Under nitrogen atmosphere, triethylamine (1.1 ml, 8.0 mmol) was added dropwise to formic acid (0.75 ml, 20 mmol) at a temperature below 25°C. 2-((5-bromo-2-chloropyridin-3-yl)oxy)-1-(6-methoxypyridin-3-yl)ethan-1-one (1.50 g, 4.2 mmol), ethyl acetate (100 ml) and RuCl(p-cymene) [(R,R)-Ts-DPEN] (320 mg, 0.5 mmol, CAS: 192139-92-7) were added to a reaction flask, and then a mixture of triethylamine and formic acid mentioned above was added dropwise. Under nitrogen atmosphere, the resulting mixture was stirred at room temperature overnight. The reaction solution was successively washed with saturated ammonium chloride (60 ml), saturated sodium bicarbonate (60 ml) and saturated brine (60 ml). After the organic phase was concentrated, the resulting residue was purified with flash column chromatography (eluted with a gradient of water:acetonitrile = 100 : 0-0 : 100) to obtain 1.2 g of a brown solid. MS (m/z): 359.0 $[M+H]^+$.

(B) (S)-7-bromo-3-(6-methoxypyridin-3-yl)-2,3-dihydro-[1,4]dioxino[2,3-b]pyridine

(S)-2-((5-bromo-2-chloropyridin-3-yl)oxy)-1-(6-methoxypyridin-3-yl)ethan-1-ol (1.20 g, 3.3 mmol) and anhydrous tetrahydrofuran (40 ml) were added to a reaction flask. Under nitrogen atmosphere, the mixture was cooled to -70°C and KHMDS (5 ml, 1 M in tetrahydrofuran solution) was added dropwise. The resulting mixture was warmed to - 20°C slowly and then stirred for 3 hours at a temperature of -20°C to 0°C. The reaction solution was quenched by adding saturated ammonium chloride aqueous solution (40 ml), and extracted with ethyl acetate (40 ml). The organic phase was washed with saturated brine (40 ml) and concentrated, and the resulting residue was purified with flash column chromatography (eluted with a gradient of water (0.1% formic acid):acetonitrile = 100 : 0-0 : 100) and chiral preparative HPLC to obtain 280 mg of a white solid. MS (m/z): 323.0 $[M+H]^+$. Chiral preparative HPLC conditions: column: ODH ($2 \times 25$ cm); mobile phase: acetonitrile/ethanol = 10 : 90 (0.1% ammonia water); flow rate: 15 ml/minute; detector: UV 280 nm.

(C) (S)-(3-(6-methoxypyridin-3-yl)-2,3-dihydro-[1,4]dioxino[2,3-b]pyridin-7-yl)boronic acid

(S)-7-bromo-3-(6-methoxypyridin-3-yl)-2,3-dihydro-[1,4]dioxino[2,3-b]pyridine (280 mg, 0.87 mmol), $Pin_2B_2$ (442 mg, 1.74 mmol), Pd(dppf)$Cl_2$ (64 mg, 0.09 mmol), potassium acetate (213 mg, 2.2 mmol) and dioxane (12 ml) were added to a reaction flask. The mixture was heated to reflux and stirred overnight under nitrogen atmosphere. After cooled to room temperature, the reaction solution was concentrated, and the resulting residue was purified with flash column chromatography (eluted with a gradient of water (0.1% formic acid):acetonitrile = 100 : 0-0 : 100) to obtain 250 mg of the product as a brown solid. MS (m/z): 289.2 $[M+H]^+$.

(D) (S)-3-(6-methoxypyridin-3-yl)-2,3-dihydro-[1,4]dioxino[2,3-b]pyridin-7-ol

(S)-(3-(6-methoxypyridin-3-yl)-2,3-dihydro-[1,4]dioxino[2,3-b]pyridin-7-yl)boronic acid (250 mg, 0.87 mmol) and methanol (12 ml) were added to a reaction flask. In an ice bath, an aqueous solution (3 ml) of sodium hydroxide (176 mg, 4.4 mmol) was added dropwise, and then hydrogen peroxide (0.46 ml, 4.4 mmol) was added dropwise. The resulting mixture was warmed to room temperature and stirred for 4 hours. The reaction solution was added dropwise saturated sodium hydrogen sulfite aqueous solution (0.5 ml) and concentrated, and the resulting residue was purified with flash column chromatography (eluted with a gradient of water: acetonitrile = 100 : 0-0 : 100) to obtain 200 mg of a gray solid. MS (m/z): 261.1 $[M+H]^+$.

The following intermediate was prepared according to the preparation procedure of intermediate 56 using

corresponding starting materials and reagents under appropriate conditions that will be recognized by one skilled in the art.

| Intermediate | Structural formula | MS (M+H)+ |
|---|---|---|
| **57** | 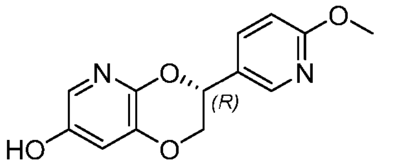 | **261.1** |

**Intermediate 58: 3-(5-cyclopropylpyridin-2-yl)-2,3-dihydro-[1,4]dioxino[2,3-*b*]pyridin-7-ol**

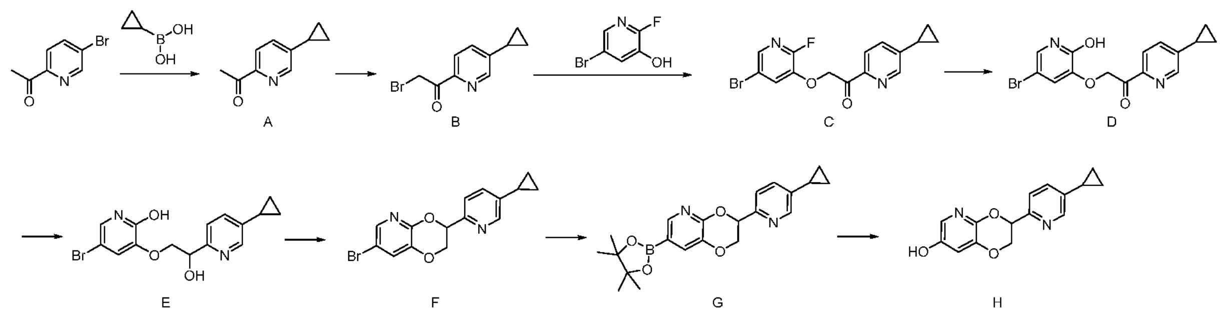


(A) 1-(5-cyclopropylpyridin-2-yl)ethan-1-one

Under nitrogen atmosphere, 1-(5-bromopyridin-2-yl)ethan-1-one (3 g, 11.3 mmol), cyclopropylboronic acid (1.94 g, 22.6 mmol), $Pd(OAc)_2$ (253 mg, 1.13 mmol), tricyclohexyl phosphine (633 mg, 2.26 mmol), potassium phosphate (7.2 g, 33.9 mmol), toluene (60 ml) and water (6 ml) were successively added to a reaction flask, and the mixture was heated to 110°C and stirred for 16 hours. After the reaction solution was concentrated, the resulting residue was purified with flash column chromatography (petroleum ether:ethyl acetate = 100 : 0-50 : 50) to obtain 1.3 g of the product. MS (m/z): 162.1 $[M+H]^+$

(B) 2-bromo-1-(5-cyclopropylpyridin-2-yl)ethan-1-one

In a reaction flask, 1-(5-cyclopropylpyridin-2-yl)ethan-1-one (1.3 g, 8.1 mmol) was dissolved in a solution of hydrobromic acid in acetic acid (15 ml, 33%wt), and then liquid bromine (1.78 g, 8.1 mmol) was added. The mixture was stirred at room temperature for 1 hour. The reaction solution was concentrated, added ethyl acetate, neutralized with sodium bicarbonate aqueous solution, extracted and concentrated. The residue was purified with flash column chromatography (petroleum ether:ethyl acetate = 100 : 0-50 : 50) to obtain 1.3 g of the product. MS (m/z): 240.0 $[M+H]^+$

(C) 2-((5-bromo-2-fluoropyridin-3-yl)oxy)-1-(5-cyclopropylpyridin-2-yl)ethan-1-one

In a reaction flask, 2-bromo-1-(5-cyclopropylpyridin-2-yl)ethan-1-one (1.24 g, 5.2 mmol) and 5-bromo-2-fluoropyridin-3-ol (1 g, 5.2 mmol) were dissolved in 15 ml of DMF, and then sodium hydride (208 mg, 5.2 mmol) was added. The resulting mixture was stirred at room temperature for 2 hours. The reaction solution was quenched with saturated ammonium chloride solution. The mixture was extracted with EA, washed with brine and concentrated, and the residue was purified with flash column chromatography (petroleum ether:ethyl acetate = 100 : 0-50 : 50) to obtain 1.4 g of the product. MS (m/z): 351.0 $[M+H]^+$

(D) 2-((5-bromo-2-hydroxypyridin-3-yl)oxy)-1-(5-cyclopropylpyridin-2-yl)ethan-1-one

2-((5-bromo-2-fluoropyridin-3-yl)oxy)-1-(5-cyclopropylpyridin-2-yl)ethan-1-one (1.4 g, 4 mmol), sodium acetate (3.28 g, 40 mmol) and acetic acid (10 ml) were successively added to a sealed tube, and the mixture was stirred at 140°C for 16 hours. The reaction solution was concentrated, neutralized with sodium bicarbonate aqueous solution, extracted with ethyl acetate and concentrated. The residue was purified with flash column chromatography (eluted with a gradient of

water:methanol = 100 : 0-0 : 100) to obtain 1.2 g of the product. MS (m/z): 349.0 $[M+H]^+$

(E) 5-bromo-3-(2-(5-cyclopropylpyridin-2-yl)-2-hydroxyethoxy)pyridin-2-ol

2-((5-bromo-2-hydroxypyridin-3-yl)oxy)-1-(5-cyclopropylpyridin-2-yl)ethan-1-one (1.2 g, 3.45 mmol), RuCl(p-cymene) [(R,R)-Ts-DPEN] (109 mg, 0.17 mmol, CAS: 192139-92-7) and methanol (15 ml) were added to a reaction flask. Under nitrogen atmosphere, 1 ml of triethylamine/formic acid (molar ratio, 2 : 5) solution was added. After the addition was completed, the mixture was stirred at 50°C overnight and then concentrated, and the residue was purified with flash column chromatography (eluted with a gradient of water:methanol = 100 : 0-0 : 100) to obtain 800 mg of the product. MS (m/z): 351.0 $[M+H]^+$.

(F) 7-bromo-3-(5-cyclopropylpyridin-2-yl)-2,3-dihydro-[1,4]dioxino[2,3-b]pyridine

Under nitrogen atmosphere, 5-bromo-3-(2-(5-cyclopropylpyridin-2-yl)-2-hydroxyethoxy)pyridin-2-ol (850 mg, 2.43 mmol), tetrahydrofuran (15 ml), $PPh_3$ (1.27 g, 4.86 mmol) and DIAD (981 mg, 4.86 mmol) were successively added to a reaction flask. The mixture was stirred at room temperature for 3 hours. After the reaction solution was concentrated, the resulting residue was purified with flash column chromatography (petroleum ether:ethyl acetate = 100 : 0-50 : 50) to obtain 700 mg of the product. MS (m/z): 333.0 $[M+H]^+$

(G) 3-(5-cyclopropylpyridin-2-yl)-7-(4,4,5,5-tetramethyl-1,3,2-dioxaborolan-2-yl)-2,3-dihydro-[1,4]dioxino[2,3-b]pyridine

Under nitrogen atmosphere, 7-bromo-3-(5-cyclopropylpyridin-2-yl)-2,3-dihydro-[1,4]dioxino[2,3-*b*]pyridine (700 mg, 2.11 mmol), $Pin_2B_2$ (803 mg, 3.16 mmol), Pd(dppf)$Cl_2$ (154 mg, 0.21 mmol), potassium acetate (620 mg, 6.33 mmol) and dioxane (10 ml) were successively added to a reaction flask, and the mixture was heated to 90°C and stirred for 16 hours. The reaction solution was concentrated, and the residue was purified with flash column chromatography (petroleum ether:ethyl acetate = 100 : 0-50 : 50) to obtain 600 mg of the product as a white solid. MS (m/z): 381.2$[M+1]^+$ (H) 3-(5-cyclopropylpyridin-2-yl)-2,3-dihydro-[1,4]dioxino[2,3-*b*]pyridin-7-ol

To a reaction flask, 3-(5-cyclopropylpyridin-2-yl)-7-(4,4,5,5-tetramethyl-1,3,2-dioxaborolan-2-yl)-2,3-dihydro-[1,4]dioxino[2,3-*b*]pyridine (600 mg, 1.58 mmol), sodium hydroxide (126 mg, 3.16 mmol), tetrahydrofuran (10 ml) and water (2 ml) were successively added, and then $H_2O_2$ (0.5 ml) was added. The mixture was stirred at room temperature for 3 hours. The reaction solution was added $Na_2S_2O_3$ aqueous solution and extracted with ethyl acetate. The organic layer was washed with saturated brine, dried over anhydrous sodium sulfate and filtered. The filtrate was concentrated, and the residue was purified with flash column chromatography (petroleum ether:ethyl acetate = 100 : 0-0 : 100) to obtain 400 mg of the product as a white solid. MS (m/z): 271.1 $[M+1]^+$

The following intermediate was prepared according to the preparation procedure of intermediate 58 using corresponding starting materials and reagents under appropriate conditions that will be recognized by one skilled in the art.

| Intermediate | Structural formula | MS $(M+H)^+$ |
|---|---|---|
| 59 | 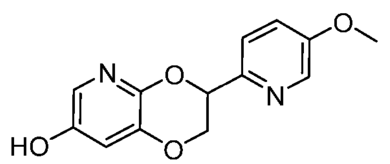 | 261.1 |

**Example 2**

**Preparation of compounds 1-86**

**Compound 1**

**7-((6-((4-methoxybenzyl)oxy)pyridin-3-yl)oxy)-3-methyl-4*H*-pyrido[1,2-a]pyrimidin-4-one**

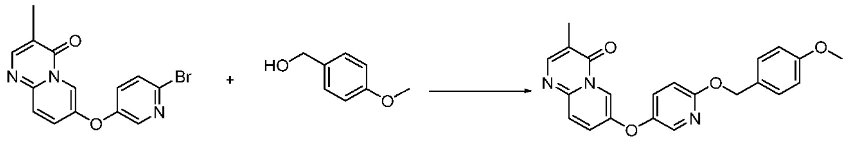

7-((6-bromopyridin-3-yl)oxy)-3-methyl-4*H*-pyrido[1,2-a]pyrimidin-4-one (332 mg, 1.0 mmol), 4-methoxybenzyl alcohol (276 mg, 2.0 mmol), cesium carbonate (652 mg, 2.0 mmol), potassium iodide (332 mg, 2.0 mmol) and DMF (10 ml) were added to a reaction flask, and the reaction solution was heated to reflux for 24 hours. After the reaction was completed, the reaction was quenched by adding water (2 ml). The reaction solution was concentrated, and the residue was purified with flash column chromatography (eluted with a gradient of water:methanol = 100 : 0-0 : 100) and preparative thin-layer chromatography (dichloromethane:methanol = 20 : 1) to obtain 13 mg of the title product as a white solid. MS (m/z): 390.0 $[M+H]^+$

$^1H$ NMR (400 MHz, DMSO-d6) δ 8.30 (d, J = 2.8 Hz, 1H), 8.24 (d, J = 0.9 Hz, 1H), 8.17 - 8.14 (m, 1H), 7.84 (dd, J = 9.7, 2.8 Hz, 1H), 7.73 - 7.66 (m, 2H), 7.38 (dd, J = 9.1, 2.5 Hz, 2H), 6.94 (d, J = 9.0 Hz, 1H), 6.92 (d, J = 2.0 Hz, 1H), 6.91 (d, J = 2.1 Hz, 1H), 5.24 (s, 2H), 3.73 (s, 3H), 2.08 (s, 3H).

The following compounds were prepared according to the preparation procedure of compound 1 using corresponding intermediates and reagents under appropriate conditions that will be recognized by one skilled in the art.

| Compo und | Structural formula | MS $(M+H)^+$ | $^1$HNMR |
|---|---|---|---|
| 2 | 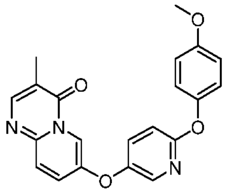 | 376.0 | $^1H$ NMR (400 MHz, DMSO-d6) δ 8.37 (d, J = 2.3 Hz, 1H), 8.25 (d, J = 0.9 Hz, 1H), 8.11 - 8.07 (m, 1H), 7.84 (dd, J = 9.7, 2.8 Hz, 1H), 7.78 (dd, J = 8.9, 3.1 Hz, 1H), 7.70 (dd, J = 9.6, 0.5 Hz, 1H), 7.06 (dt, J = 3.6, 2.5 Hz, 3H), 6.96 - 6.93 (m, 2H), 3.73 (s, 3H), 2.09 (d, J = 0.6 Hz, 3H). |
| 3 | 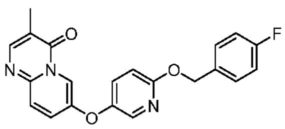 | 378.2 | $^1H$ NMR (400 MHz, DMSO-d6) δ 8.34 (d, J = 2.7 Hz, 1H), 8.28 (s, J = 0.6 Hz, 1H), 8.19 (d, J = 3.0 Hz, 1H), 7.87 (dd, J = 9.7, 2.8 Hz, 1H), 7.78 - 7.69 (m, 2H), 7.57 - 7.49 (m, 2H), 7.26 - 7.18 (m, 2H), 7.01 (d, J = 9.0 Hz, 1H), 5.34 (s, 2H), 2.11 (s, 3H). |
| 4 | 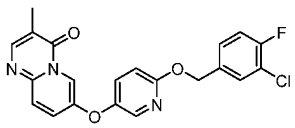 | 412.1 | $^1H$ NMR (400 MHz, DMSO-d6) δ 8.35 (d, J = 2.8 Hz, 1H), 8.28 (s, 1H), 8.19 (d, J = 3.0 Hz, 1H), 7.87 (dd, J = 9.7, 2.7 Hz, 1H), 7.76 (dd, J = 9.0, 3.0 Hz, 1H), 7.75 - 7.70 (m, 2H), 7.54 - 7.49 (m, 1H), 7.47 - 7.42 (m, 1H), 7.04 (d, J = 9.0 Hz, 1H), 5.34 (s, 2H), 2.12 (s, 3H). |

**Compound 5**

**7-((6-((4-cyclopropylbenzyl)oxy)-5-ethoxypyridin-3-yl)oxy)-3-methyl-4H-pyrido[1,2-a]pyrimidin-4-one**

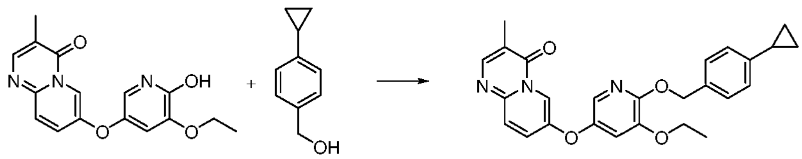

Under nitrogen atmosphere, 7-((5-ethoxy-6-hydroxypyridin-3-yl)oxy)-3-methyl-4*H*-pyrido[1,2-a]pyrimidin-4-one (100 mg, 0.32 mmol), (4-cyclopropylphenyl)methanol (74 mg, 0.48 mmol), triphenylphosphine (131 mg, 0.48 mmol), THF (10 ml) and DIAD (100 mg, 0.48 mmol) were successively added to a reaction flask, and the mixture was stirred at room temperature overnight. After the reaction was quenched by adding water (2 ml), the reaction solution was concentrated, and the residue was separated with flash column chromatography (eluted with a gradient of water:methanol = 100 : 0-0 : 100). The resulting product was then purified with preparative thin-layer chromatography (dichloromethane:methanol = 20 : 1) to obtain 45 mg of the title product as a white solid. MS (m/z): 444.1 [M+H]+

$^1$H NMR (400 MHz, DMSO-d6) δ 8.36 (d, J = 2.6 Hz, 1H), 8.27 (s, 1H), 7.86 (dd, J = 9.7, 2.7 Hz, 1H), 7.72 (d, J = 9.7 Hz, 1H), 7.67 (d, J = 2.4 Hz, 1H), 7.37 (d, J = 2.4 Hz, 1H), 7.33 (d, J = 8.0 Hz, 2H), 7.08 (d, J = 8.1 Hz, 2H), 5.31 (s, 2H), 4.08 - 3.96 (m, 2H), 2.11 (s, 3H), 1.98 - 1.85 (m, 1H), 1.29 (t, J = 6.9 Hz, 3H), 0.99 - 0.89 (m, 2H), 0.71 - 0.61 (m, 2H).

The following compounds were prepared according to the preparation procedure of compound 5 using corresponding intermediates and reagents under appropriate conditions that will be recognized by one skilled in the art.

| Compound | Structural formula | MS (M+H)+ | $^1$HNMR | Intermediates used |
|---|---|---|---|---|
| 6 | 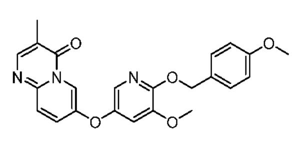 | 420.1 | $^1$H NMR (400 MHz, DMSO-d6) δ 8.35 (d, J = 2.7 Hz, 1H), 8.25 (s, 1H), 7.84 (dd, J = 9.7, 2.7 Hz, 1H), 7.69 (d, J = 9.7 Hz, 1H), 7.67 (d, J = 2.3 Hz, 1H), 7.40-7.36 (m, 2H), 7.35 (d, J = 2.4 Hz, 1H), 6.95-6.89 (m, 2H), 5.25 (s, 2H), 3.74 (s, 6H), 2.13 - 2.06 (m, 3H). | **33** |
| 7 | 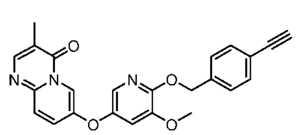 | 414.1 | $^1$H NMR (400 MHz, DMSO-d6) δ 8.39 (d, J = 2.7 Hz, 1H), 8.28 (s, 1H), 7.87 (dd, J = 9.7, 2.7 Hz, 1H), 7.73 (d, J = 9.7 Hz, 1H), 7.68 (d, J = 2.4 Hz, 1H), 7.55 - 7.44 (m, 4H), 7.41 (d, J = 2.4 Hz, 1H), 5.39 (s, 2H), 4.21 (s, 1H), 3.79 (s, 3H), 2.12 (s, 3H). | **33** |
| 8 | 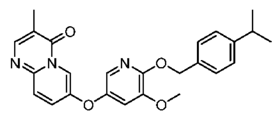 | 432.2 | $^1$H NMR (400 MHz, DMSO-d6) δ 8.37 (d, J = 2.7 Hz, 1H), 8.25 (s, 1H), 7.84 (dd, J = 9.7, 2.8 Hz, 1H), 7.73-7.64 (m, 2H), 7.40 - 7.32 (m, 3H), 7.24 (d, J = 8.1 Hz, 2H), 5.29 (s, 2H), 3.75 (s, 3H), 2.87 (hept, J = 7.0 Hz, 1H), 2.10 (s, 3H), 1.18 (d, J = 6.9 Hz, 6H). | **33 and 36** |
| 9 | 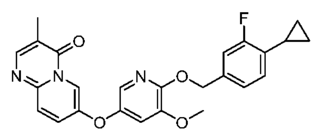 | 448.2 | $^1$H NMR (400 MHz, DMSO-d6) δ 8.37 (d, J = 2.7 Hz, 1H), 8.25 (s, 1H), 7.84 (dd, J = 9.7, 2.8 Hz, 1H), 7.70 (d, J = 9.7 Hz, 1H), 7.66 (d, J = 2.4 Hz, 1H), 7.37 (d, J = 2.4 Hz, 1H), 7.23 - 7.14 (m, 2H), 7.01-6.95 (m, 1H), 5.30 (s, 2H), 3.76 (s, 3H), 2.10 (s, 3H), 2.06 - 1.95 (m, 1H), 1.00 - 0.89 (m, 2H), 0.75 - 0.66 (m, 2H). | **3 and 33** |
| 10 | 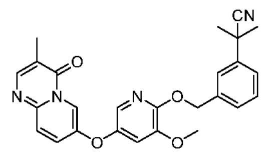 | 457.2 | $^1$H NMR (400 MHz, DMSO-d6) δ 8.37 (d, J = 2.9 Hz, 1H), 8.25 (d, J = 0.5 Hz, 1H), 7.84 (dd, J = 9.7, 2.8 Hz, 1H), 7.72 - 7.67 (m, 2H), 7.62 (s, 1H), 7.50 - 7.46 (m, 1H), 7.45 - 7.41 (m, 2H), 7.39 (d, J = 2.4 Hz, 1H), 5.38 (s, 2H), 3.77 (s, 3H), 2.10 (s, 3H), 1.68 (s, 6H). | **4 and 33** |
| 11 | 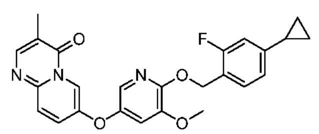 | 448.2 | $^1$H NMR (400 MHz, DMSO-d6) δ 8.38 (d, J = 2.7 Hz, 1H), 8.26 (d, J = 0.8 Hz, 1H), 7.84 (dd, J = 9.7, 2.8 Hz, 1H), 7.72 - 7.65 (m, 2H), 7.38 (dd, J = 10.8, 5.3 Hz, 2H), 6.97 - 6.89 (m, 2H), 5.31 (s, 2H), 3.74 (s, 3H), 2.10 (s, 3H), 2.00 - 1.89 (m, 1H), 1.00 - 0.92 (m, 2H), 0.73 - 0.66 (m, 2H). | **5 and 33** |
| 12 | 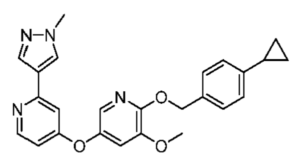 | 429.2 | $^1$H NMR (400 MHz, DMSO-d6) δ 8.33 (d, J = 5.7 Hz, 1H), 8.25 (s, 1H), 7.95 (s, 1H), 7.62 (dd, J = 2.4, 0.7 Hz, 1H), 7.33-7.28 (m, 3H), 7.20 (d, J = 2.4 Hz, 1H), 7.07 (d, J = 8.0 Hz, 2H), 6.66 (dd, J = 5.7, 1.8 Hz, 1H), 5.27 (s, 2H), 3.83 (s, 3H), 3.75 (s, 3H), 1.93-1.84 (m, 1H), 0.96 - 0.87 (m, 2H), 0.69 - 0.60 (m, 2H). | **42** |

(continued)

| Compound | Structural formula | MS (M+H)$^+$ | $^1$HNMR | Intermediates used |
|---|---|---|---|---|
| 13 | 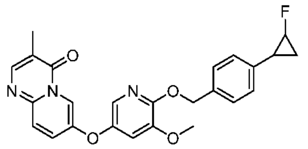 | 448.2 | $^1$H NMR (400 MHz, DMSO-d6) δ 8.36 (dd, J = 4.8, 2.7 Hz, 1H), 8.26 (s, 1H), 7.85 (dd, J = 9.6, 2.7 Hz, 1H), 7.70 (d, J = 9.7 Hz, 1H), 7.68-7.65(m, 1H), 7.40-7.32 (m, 3H), 7.25 (d, J = 8.1 Hz, 1H), 7.09 (d, J = 8.1 Hz, 1H), 5.30 (d, J = 6.3 Hz, 2H), 5.03 - 4.72 (m, 1H), 3.75 (d, J = 1.4 Hz, 3H), 2.10 (s, 3H), 1.59 - 1.07 (m, 3H). | **33** |
| 14 | 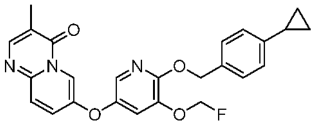 | 448.1 | $^1$H NMR (400 MHz, DMSO-d6) δ 8.38 (d, J = 2.8 Hz, 1H), 8.25 (d, J = 0.7 Hz, 1H), 7.89 (dd, J = 2.5, 0.6 Hz, 1H), 7.84 (dd, J = 9.7, 2.7 Hz, 1H), 7.70 (d, J = 9.7 Hz, 1H), 7.58 (d, J = 2.5 Hz, 1H), 7.31 (d, J = 8.0 Hz, 2H), 7.06 (d, J = 8.1 Hz, 2H), 5.93 (s, 1H), 5.80 (s, 1H), 5.30 (s, 2H), 2.09 (s, 3H), 1.95 - 1.82 (m, 1H), 0.95 - 0.88 (m, 2H), 0.66 - 0.60 (m, 2H). | **43** |
| 15 | 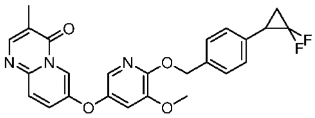 | 466.1 | $^1$H NMR (400 MHz, DMSO-d6) δ 8.35 (d, J = 2.6 Hz, 1H), 8.25 (s, 1H), 7.84 (dd, J = 9.7, 2.7 Hz, 1H), 7.69 (d, J = 9.7 Hz, 1H), 7.66 (d, J = 2.4 Hz, 1H), 7.40 (d, J = 8.0 Hz, 2H), 7.37 (d, J = 2.4 Hz, 1H), 7.27 (d, J = 8.0 Hz, 2H), 5.32 (s, 2H), 3.74 (s, 3H), 3.05 - 2.93 (m, 1H), 2.09 (s, 3H), 2.00-1.89 (m, 2H). | **33** |
| 16 | 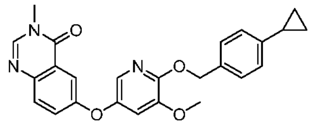 | 430.1 | $^1$H NMR (400 MHz, DMSO-d6) δ 8.29 (s, 1H), 7.69 (d, J = 8.9 Hz, 1H), 7.59 (d, J = 2.3 Hz, 1H), 7.54 (dd, J = 8.9, 2.9 Hz, 1H), 7.39 (d, J = 2.9 Hz, 1H), 7.31 (d, J = 8.1 Hz, 2H), 7.26 (d, J = 2.4 Hz, 1H), 7.06 (d, J = 8.2 Hz, 2H), 5.27 (s, 2H), 3.73 (s, 3H), 3.44 (s, 3H), 1.93 - 1.83 (m, 1H), 0.98 - 0.86 (m, 2H), 0.68 - 0.59 (m, 2H). | **44** |
| 17 | 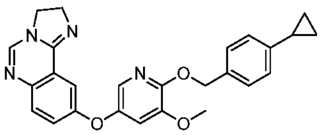 | 441.2 | $^1$H NMR (400 MHz, DMSO-d6) δ 7.89 (s, 1H), 7.55 (d, J = 2.0 Hz, 1H), 7.42 (d, J = 8.8 Hz, 1H), 7.33-7.28 (m, 3H), 7.23 (d, J = 1.9 Hz, 1H), 7.15 (d, J = 2.8 Hz, 1H), 7.06 (d, J = 7.9 Hz, 2H), 5.25 (s, 2H), 4.06 (t, J = 9.8 Hz, 2H), 3.86 (t, J = 9.8 Hz, 2H), 3.73 (s, 3H), 1.93 - 1.85 (m, 1H), 0.97 - 0.87 (m, 2H), 0.68 - 0.58 (m, 2H). | **45** |
| 18 | 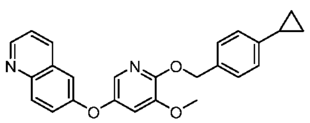 | 399.1 | $^1$H NMR (400 MHz, DMSO-d6) δ 8.77 (dd, J = 4.1, 1.5 Hz, 1H), 8.24 (d, J = 7.0 Hz, 1H), 8.01 (d, J = 9.2 Hz, 1H), 7.60 (dd, J = 2.4, 0.4 Hz, 1H), 7.55 (dd, J = 9.1, 2.8 Hz, 1H), 7.45 (dd, J = 8.3, 4.2 Hz, 1H), 7.32 (s, 1H), 7.30 (s, 2H), 7.29-7.27 (m, 1H), 7.07 (s, 1H), 7.05 (s, 1H), 5.27 (s, 2H), 3.74 (s, 3H), 1.93-1.84 (m, 1H), 0.96-0.85 (m, 2H), 0.68-0.59 (m, 2H). | **46** |

**Compound 19**

**7-((6-((4-chlorobenzyl)oxy)-5-methoxypyridin-3-yl)oxy)-3-methyl-4*H*-pyrido[1,2-a]pyrimidin-4-one**

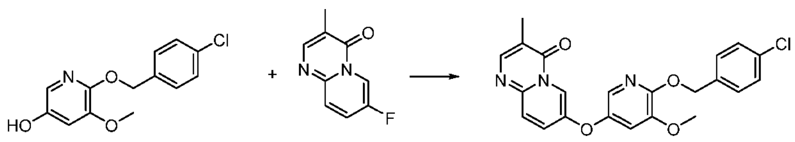

7-fluoro-3-methyl-4*H*-pyrido[1,2-a]pyrimidin-4-one (112 mg, 0.63 mmol), 6-((4-chlorobenzyl)oxy)-5-methoxypyridin-3-ol (168 mg, 0.63 mmol), cesium carbonate (205 mg, 0.63 mmol) and DMF (3 ml) were added to a reaction flask. The mixture was heated at 120°C for 2 hours. The reaction solution was cooled and then concentrated. The residue was purified with flash column chromatography (eluted with a gradient of water:methanol = 100 : 0-0 : 100) to obtain 38 mg of the title product as a white solid. MS (m/z): 424.1 $[M+H]^+$

$^1H$ NMR (400 MHz, DMSO-d6) δ 8.35 (d, J = 2.7 Hz, 1H), 8.24 (s, 1H), 7.83 (dd, J = 9.7, 2.7 Hz, 1H), 7.69 (d, J = 9.7 Hz, 1H), 7.66 (d, J = 2.4 Hz, 1H), 7.49 - 7.40 (m, 4H), 7.38 (d, J = 2.4 Hz, 1H), 5.33 (s, 2H), 3.75 (s, 3H), 2.08 (s, 3H).

The following compounds were prepared according to the preparation procedure of compound 19 using corresponding intermediates and reagents under appropriate conditions that will be recognized by one skilled in the art.

| Compound | Structural formula | MS $(M+H)^+$ | $^1$HNMR | Intermediates used |
|---|---|---|---|---|
| 20 | 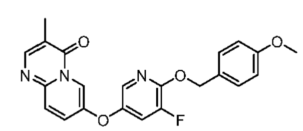 | 408.1 | $^1H$ NMR (400 MHz, DMSO-d6) δ 8.47 (d, J = 2.5 Hz, 1H), 8.29 (d, J = 0.9 Hz, 1H), 8.05 (d, J = 2.5 Hz, 1H), 7.95 - 7.83 (m, 2H), 7.73 (d, J = 9.7 Hz, 1H), 7.48 - 7.37 (m, 2H), 7.00 - 6.91 (m, 2H), 5.36 (s, 2H), 3.76 (s, 3H), 2.13 (s, 3H). | 34 and 37 |
| 21 | 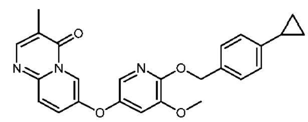 | 430.2 | $^1H$ NMR (400 MHz, DMSO-d6) δ 8.35 (d, J = 2.8 Hz, 1H), 8.26 - 8.22 (m, 1H), 7.83 (dd, J = 9.7, 2.8 Hz, 1H), 7.69 (d, J = 9.7 Hz, 1H), 7.66 (d, J = 2.5 Hz, 1H), 7.35 (d, J = 2.5 Hz, 1H), 7.30 (d, J = 8.1Hz, 2H), 7.08-7.03 (m, 2H), 5.26 (s, 2H), 3.73 (s, 3H), 2.09 (s, 3H), 1.95 - 1.82 (m, 1H), 0.95 - 0.87 (m, 2H), 0.68 - 0.58 (m, 2H). | 26 and 34 |
| 22 | 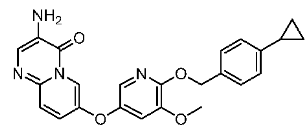 | 431.2 | $^1H$ NMR (400 MHz, $CDCl_3$) δ 8.40 (d, J = 2.6 Hz, 1H), 7.96 (s, 1H), 7.55 (dd, J = 2.4, 0.6 Hz, 1H), 7.52 (d, J = 9.7 Hz, 1H), 7.37 (d, J = 8.0 Hz, 2H), 7.32 - 7.26 (m, 1H), 7.06 (d, J = 8.1 Hz,2H), 6.87 (d, J = 2.4 Hz, 1H), 5.40 (s, 2H), 4.06 (s, 2H), 3.81 (s, 3H), 1.92 - 1.83 (m, 1H), 0.97 - 0.91 (m, 2H), 0.70 - 0.64 (m, 2H). | 12 and 26 |
| 23 | 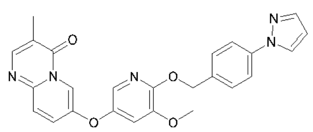 | 456.2 | $^1H$ NMR (400 MHz, DMSO-d6) δ 8.49 (d, J = 2.5 Hz, 1H), 8.36 (d, J = 2.7 Hz, 1H), 8.25 (d, J = 0.7 Hz, 1H), 7.87 - 7.81 (m, 3H), 7.75 - 7.66 (m, 3H), 7.56 (d, J = 8.4 Hz, 2H), 7.38 (d, J = 2.4 Hz, 1H), 6.56 - 6.48 (m, 1H), 5.37 (s, 2H), 3.76 (s, 3H), 2.09 (s, 3H). | 34 and 41 |
| 24 | 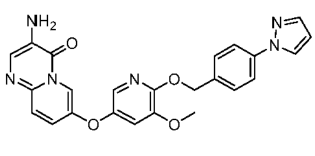 | 457.2 | $^1H$ NMR (400 MHz, DMSO-d6) δ 8.49 (d, J = 2.5 Hz, 1H), 8.15 (d, J = 2.6 Hz, 1H), 7.84 (dd, J = 7.7, 4.5 Hz, 3H), 7.72 (d, J = 1.7 Hz, 1H), 7.67 - 7.64 (m, 1H), 7.56 (d, J = 8.4 Hz, 2H), 7.52 (d, J = 9.7 Hz, 1H), 7.40 (dd, J = 9.7, 2.7 Hz, 1H), 7.37 (d, J = 2.3 Hz, 1H), 6.53-6.51 (m, 1H), 5.37 (s, 2H), 5.27 (s, 2H), 3.76 (s, 3H). | 12 and 41 |

(continued)

| Compound | Structural formula | MS (M+H)+ | 1HNMR | Intermediates used |
|---|---|---|---|---|
| 25 | 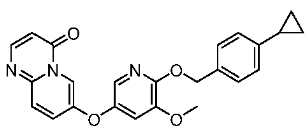 | 416.1 | 1H NMR (400 MHz, DMSO-d6) δ 8.42 (d, J = 2.7 Hz, 1H), 8.33 - 8.27 (m, 1H), 7.98 (dd, J = 9.6, 2.8 Hz, 1H), 7.79 (d, J = 9.7 Hz, 1H), 7.70 (dd, J = 2.5, 0.5 Hz, 1H), 7.39 (d, J = 2.4 Hz, 1H), 7.33 (d, J = 8.1 Hz, 2H), 7.08 (d, J = 8.1 Hz, 2H), 6.39 (d, J = 6.3 Hz, 1H), 5.29 (s, 2H), 3.76 (s, 3H), 1.98 - 1.86 (m, 1H), 0.97 - 0.92 (m, 2H), 0.68 - 0.64 (m, 2H). | 15 and 26 |
| 26 | 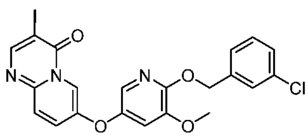 | 424.1 | 1H NMR (400 MHz, DMSO-d6) δ 8.36 (d, J = 2.7 Hz, 1H), 8.27 - 8.22 (m, 1H), 7.84 (dd, J = 9.7, 2.8 Hz, 1H), 7.69 (d, J = 9.7 Hz, 1H), 7.66 (d, J = 2.4 Hz, 1H), 7.50 (s, 1H), 7.42 - 7.32 (m, 4H), 5.34 (s, 2H), 3.76 (s, 3H), 2.09 (s, 3H). | 34 and 38 |
| 27 | 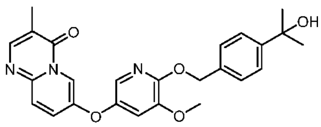 | 448.2 | 1H NMR (400 MHz, DMSO-d6) δ 8.35 (d, J = 2.7 Hz, 1H), 8.25 (d, J = 0.8 Hz, 1H), 7.84 (dd, J = 9.7, 2.8 Hz, 1H), 7.69 (d, J = 9.7 Hz, 1H), 7.66 (d, J = 2.4 Hz, 1H), 7.47 - 7.41 (m, 2H), 7.38 - 7.33 (m, 3H), 5.29 (s, 2H), 5.00 (s, 1H), 3.74 (s, 3H), 2.09 (s, 3H), 1.39 (s, 6H). | 34 and 39 |
| 28 | 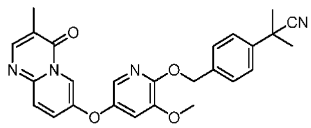 | 457.2 | 1H NMR (400 MHz, DMSO-d6) δ 8.35 (d, J = 2.7 Hz, 1H), 8.25 - 8.23 (m, 1H), 7.84 (dd, J = 9.7, 2.8 Hz, 1H), 7.69 (d, J = 9.7 Hz, 1H), 7.66 (d, J = 2.5 Hz, 1H), 7.54 - 7.45 (m, 4H), 7.38-7.36 (m, 1H), 5.34 (s, 2H), 3.75 (s, 3H), 2.09 (s, 3H), 1.66 (s, 6H). | 34 and 40 |
| 29 | 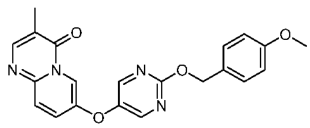 | 391.1 | 1H NMR (400 MHz, DMSO-d6) δ 8.66 (d, J = 1.7 Hz, 2H), 8.46 (d, J = 2.8 Hz, 1H), 8.26 (d, J = 0.8 Hz, 1H), 7.89 (dd, J = 9.7, 2.8 Hz, 1H), 7.71 (d, J = 9.7 Hz, 1H), 7.39 (dd, J = 9.1, 2.6 Hz, 2H), 6.95 - 6.90 (m, 2H), 5.29 (s, 2H), 3.73 (s, 3H), 2.10 (s, 3H). | 31 and 34 |
| 30 | 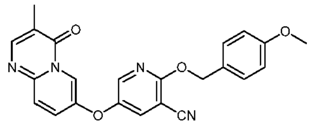 | 415.1 | 1H NMR (400 MHz, DMSO-d6) δ 8.52 (d, J = 2.7 Hz, 1H), 8.50 (dd, J = 3.0, 0.4 Hz, 1H), 8.33 (dd, J = 3.0, 0.4 Hz, 1H), 8.27 (d, J = 0.8 Hz, 1H), 7.85 (dd, J = 9.7, 2.7 Hz, 1H), 7.71 (d, J = 9.7 Hz, 1H), 7.41 (d, J = 8.5 Hz, 2H), 6.95 (dd, J = 9.1, 2.5 Hz, 2H), 5.38 (s, 2H), 3.74 (s, 3H), 2.10 (s, 3H). | 28 and 34 |
| 31 | 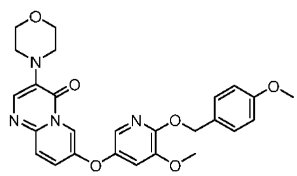 | 491.2 | 1H NMR (400 MHz, DMSO-d6) δ 8.26 (dd, J = 2.5, 0.9 Hz, 1H), 7.97 (s, 1H), 7.69 - 7.63 (m, 3H), 7.39 - 7.36 (m, 2H), 7.34 (d, J = 2.5 Hz, 1H), 6.93 - 6.90 (m, 2H), 5.25 (s, 2H), 3.73 (s, 6H), 3.71 - 3.69 (m, 4H), 3.13 - 3.10 (m, 4H). | 17 and 20 |
| 32 | 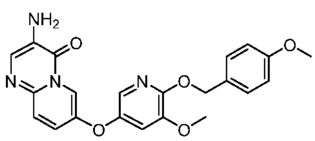 | 421.2 | 1H NMR (400 MHz, DMSO-d6) δ 8.15 (dd, J = 2.7, 0.7 Hz, 1H), 7.85 (d, J = 1.5 Hz, 1H), 7.65 (d, J = 2.5 Hz, 1H), 7.54 - 7.50 (m, 1H), 7.41 - 7.38 (m, 2H), 7.37 - 7.33 (m, 2H), 6.93 - 6.90 (m, 2H), 5.26 (s, 2H), 5.25 (s, 2H), 3.73 (d, J = 0.8 Hz, 6H). | 12 and 20 |

(continued)

| Compound | Structural formula | MS (M+H)+ | 1HNMR | Intermediates used |
|---|---|---|---|---|
| 33 | 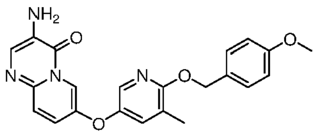 | 405.0 | $^1$H NMR (400 MHz, DMSO-d6) δ 8.12 - 8.09 (m, 1H), 7.96 (dd, J = 2.9, 0.7 Hz, 1H), 7.85 (d, J = 1.5 Hz, 1H), 7.57 (dd, J = 2.9, 0.9 Hz, 1H), 7.54 - 7.48 (m, 1H), 7.38 (dd, J = 5.9, 3.9 Hz, 3H), 6.93 - 6.90 (m, 2H), 5.27 (s, 2H), 5.26 - 5.25 (m, 2H), 3.73 (s, 3H), 2.14 (s, 3H). | 12 and 27 |
| 34 | 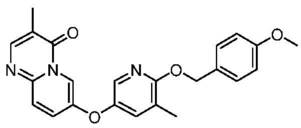 | 404.1 | $^1$H NMR (400 MHz, DMSO-d6) δ 8.31 (dd, J = 3.5, 1.3 Hz, 1H), 8.25 (d, J = 0.9 Hz, 1H), 7.98 (dd, J = 2.9, 0.6 Hz, 1H), 7.86 - 7.81 (m, 1H), 7.70 (dt, J = 9.7, 1.1 Hz, 1H), 7.59 (dd, J = 2.9, 0.9 Hz, 1H), 7.40 - 7.37 (m, 2H), 6.93 - 6.91 (m, 2H), 5.27 (s, 2H), 3.73 (s, 3H), 2.14 (s, 3H), 2.08 (d, J = 0.7 Hz, 3H). | 27 and 34 |
| 35 | 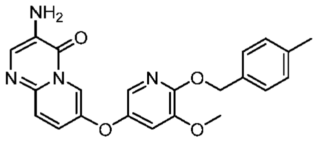 | 405.0 | $^1$H NMR (400 MHz, DMSO-d6) δ 8.15 (dd, J = 2.7, 0.5 Hz, 1H), 7.85 (s, 1H), 7.64 (d, J = 2.4 Hz, 1H), 7.53 - 7.49 (m, 1H), 7.39 (dd, J = 9.7, 2.6 Hz, 1H), 7.34 (d, J = 2.5 Hz, 1H), 7.32 (d, J = 8.0 Hz, 2H), 7.16 (d, J = 7.8 Hz, 2H), 5.28 (s, 2H), 5.25 (s, 2H), 3.74 (s, 3H), 2.28 (s, 3H). | 12 and 24 |
| 36 | 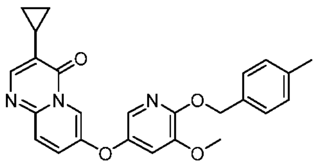 | 430.1 | $^1$H NMR (400 MHz, DMSO-d6) δ 8.34 (d, J = 2.7 Hz, 1H), 8.05 (d, J = 2.1 Hz, 1H), 7.82 (dd, J = 9.7, 2.8 Hz, 1H), 7.67 (dd, J = 8.5, 6.0 Hz, 2H), 7.35 (d, J = 2.4 Hz, 1H), 7.32 (d, J = 8.0 Hz, 2H), 7.17 (d, J = 7.8 Hz, 2H), 5.28 (s, 2H), 3.74 (s, 3H), 2.28 (s, 3H), 1.93 (dq, J = 8.3, 5.4 Hz, 1H), 0.87 - 0.83 (m, 2H), 0.81 - 0.77 (m, 2H). | 18 and 24 |
| 37 | 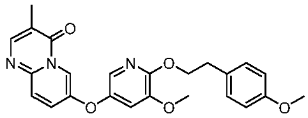 | 434.2 | $^1$H NMR (400 MHz, DMSO-d6) δ 8.34 (d, J = 2.8 Hz, 1H), 8.25 (d, J = 0.8 Hz, 1H), 7.83 (dd, J = 9.7, 2.8 Hz, 1H), 7.69 (d, J = 9.7 Hz, 1H), 7.64 (d, J = 2.5 Hz, 1H), 7.34 (d, J = 2.5 Hz, 1H), 7.20 (d, J = 8.6 Hz, 2H), 6.85 - 6.83 (m, 2H), 4.41 (t, J = 7.2 Hz, 2H), 3.74 (s, 3H), 3.69 (s, 3H), 2.96 (t, J = 7.1 Hz, 2H), 2.09 (s, 3H). | 22 and 34 |
| 38 | 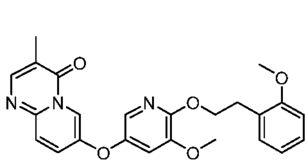 | 434.1 | $^1$H NMR (400 MHz, DMSO-d6) δ 8.36 - 8.31 (m, 1H), 8.25 (d, J = 0.9 Hz, 1H), 7.83 (dd, J = 9.7, 2.8 Hz, 1H), 7.69 (dt, J = 9.7, 1.1 Hz, 1H), 7.63 (t, J = 2.0 Hz, 1H), 7.33 (d, J = 2.5 Hz, 1H), 7.19 (t, J = 4.8 Hz, 2H), 6.95 (d, J = 7.8 Hz, 1H), 6.88 - 6.84 (m, 1H), 4.40 (dd, J = 8.9, 5.6 Hz, 2H), 3.76 (s, 3H), 3.73 (s, 3H), 3.00 (t, J = 7.2 Hz, 2H), 2.09 (d, J = 0.7 Hz, 3H). | 25 and 34 |
| 39 | 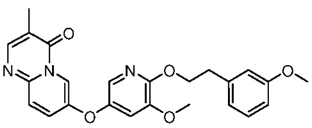 | 434.0 | $^1$H NMR (400 MHz, DMSO-d6) δ 8.34 (dd, J = 3.6, 1.2 Hz, 1H), 8.25 (d, J = 0.9 Hz, 1H), 7.83 (dd, J = 9.7, 2.8 Hz, 1H), 7.71 - 7.67 (m, 1H), 7.64 (t, J = 2.1 Hz, 1H), 7.34 (d, J = 2.5 Hz, 1H), 7.19 (dd, J = 8.8, 6.9 Hz, 1H), 6.88 - 6.87 (m, 1H), 6.85 (d, J = 7.6 Hz, 1H), 6.76 (ddd, J = 8.2, 2.6, 0.8 Hz, 1H), 4.45 (t, J = 7.1 Hz, 2H), 3.74 (s, 3H), 3.71 (s, 3H), 3.00 (t, J = 7.0 Hz, 2H), 2.09 (d, J = 0.6 Hz, 3H). | 23 and 34 |

(continued)

| Compound | Structural formula | MS (M+H)+ | 1HNMR | Intermediates used |
|---|---|---|---|---|
| 40 | 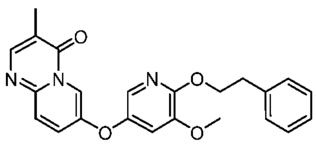 | 404.0 | 1H NMR (400 MHz, DMSO-d6) δ 8.35 (d, J = 2.7 Hz, 1H), 8.25 (d, J = 1.0 Hz, 1H), 7.83 (dd, J = 9.7, 2.8 Hz, 1H), 7.70 (t, J = 5.8 Hz, 1H), 7.64 (t, J = 2.0 Hz, 1H), 7.34 (d, J = 2.5 Hz, 1H), 7.28 (d, J = 4.8 Hz, 4H), 7.23 - 7.16 (m, 1H), 4.46 (t, J = 7.1 Hz, 2H), 3.74 (s, 3H), 3.03 (t, J = 7.1 Hz, 2H), 2.09 (s, 3H). | 21 and 34 |
| 41 | 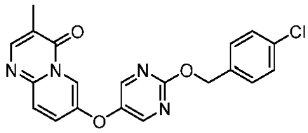 | 395.1 | 1H NMR (400 MHz, DMSO-d6) δ 8.68 - 8.64 (m, 2H), 8.47 (d, J = 2.7 Hz, 1H), 8.26 (s, 1H), 7.88 (dd, J = 9.7, 2.8 Hz, 1H), 7.71 (d, J = 9.7 Hz, 1H), 7.49 - 7.41 (m, 4H), 5.37 (s, 2H), 2.10 (s, 3H). | 30 and 34 |
| 42 | 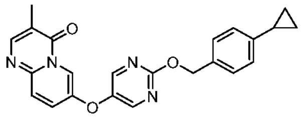 | 401.1 | 1H NMR (400 MHz, DMSO-d6) δ 8.66 (s, 2H), 8.47 (d, J = 2.8 Hz, 1H), 8.27 (d, J = 0.8 Hz, 1H), 7.89 (dd, J = 9.7, 2.8 Hz, 1H), 7.71 (d, J = 9.7 Hz, 1H), 7.32 (d, J = 8.0 Hz, 2H), 7.07 (d, J = 8.1 Hz, 2H), 5.31 (s, 2H), 2.11 (s, 3H), 1.93 - 1.86 (m, 1H), 0.95 - 0.90 (m, 2H), 0.66 - 0.62 (m, 2H). | 29 and 34 |
| 43 | 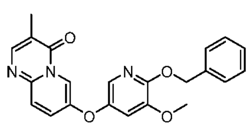 | 390.1 | 1H NMR (400 MHz, DMSO-d6) δ 8.35 (dd, J = 2.8, 0.6 Hz, 1H), 8.25 (d, J = 0.9 Hz, 1H), 7.84 (dd, J = 9.7, 2.8 Hz, 1H), 7.73 - 7.64 (m, 2H), 7.48 - 7.41 (m, 2H), 7.40 - 7.27 (m, 4H), 5.33 (s, 2H), 3.75 (s, 3H), 2.09 (d, J = 0.7 Hz, 3H). | 34 and 35 |
| 44 | 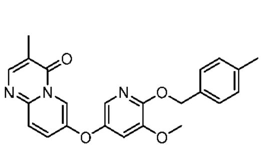 | 404.1 | 1H NMR (400 MHz, DMSO-d6) δ 8.35 (d, J = 2.8 Hz, 1H), 8.25 (d, J = 0.9 Hz, 1H), 7.84 (dd, J = 9.7, 2.8 Hz, 1H), 7.72 - 7.67 (m, 1H), 7.66 (d, J = 2.4 Hz, 1H), 7.36 (d, J = 2.5 Hz, 1H), 7.32 (d, J = 8.0 Hz, 2H), 7.17 (d, J = 7.8 Hz, 2H), 5.28 (s, 2H), 3.74 (s, 3H), 2.28 (s, 3H), 2.09 (d, J = 0.6 Hz, 3H). | 24 and 34 |

**Compound 45**

**7-((3-(4-cyclopropylphenyl)-2,3-dihydro-[1,4]dioxino[2,3-b]pyridin-7-yl)oxy)-3-methyl-4*H*-pyrido[1,2-a]pyrimidin-4-one**

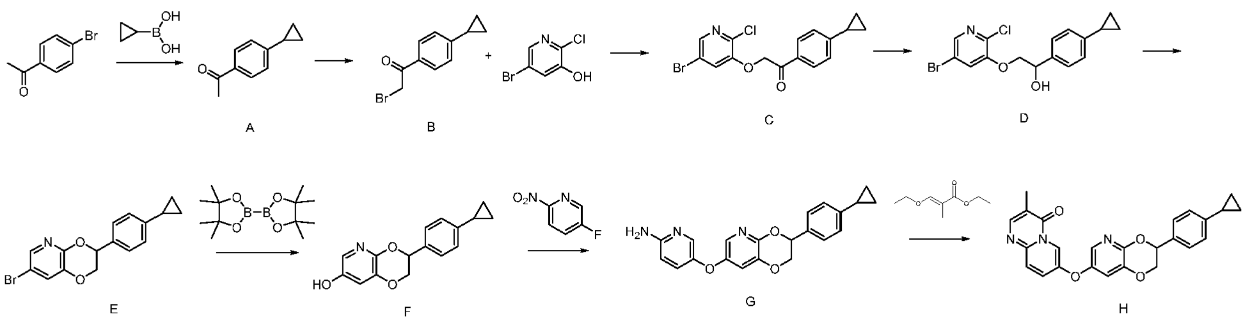
A B C D E F G H

(A) 1-(4-cyclopropylphenyl)ethan-1-one

1-(4-bromophenyl)ethan-1-one (1990 mg, 10.0 mmol), cyclopropylboronic acid (2577 mg, 30.0 mmol), palladium acetate (225 mg, 1.0 mmol), tricyclohexyl phosphine (280 mg, 1.0 mmol), potassium phosphate (4243 mg, 20.0 mmol), toluene (40 ml) and water (5 ml) were added to a reaction flask. Under nitrogen atmosphere, the reaction solution was heated to reflux for 15 hours and concentrated. The residue was purified with flash column chromatography (eluted with a gradient of water:methanol = 100 : 0-0 : 100) to obtain 1.3 g of the title product as a white solid. MS (m/z): 161.1 $[M+H]^+$

(B) 2-bromo-1-(4-cyclopropylphenyl)ethan-1-one

1-(4-cyclopropylphenyl)ethan-1-one (800 mg, 5.0 mmol), N-bromosuccinimide (934 mg, 5.25 mmol), p-toluenesulfonic acid (172 mg, 1.0 mmol) and acetonitrile (30 ml) were added to the reaction flask. The reaction solution was heated to reflux for 5 hours and concentrated, and the residue was purified with flash column chromatography (eluted with a gradient of water:methanol = 100 : 0-0 : 100) to obtain 810 mg of the title product as a pale yellow solid. MS (m/z): 239.0, 241.0 $[M+H]^+$

(C) 2-((5-bromo-2-chloropyridin-3-yl)oxy)-1-(4-cyclopropylphenyl)ethan-1-one

In a reaction flask, 5-bromo-2-chloropyridin-3-ol (505 mg, 2.43 mmol) was dissolved in DMF (10 ml), and then sodium hydride (107 mg, 2.67 mmol) was added in portions to the reaction solution. After the addition was completed, the reaction solution was stirred at room temperature for half an hour. Subsequently, 2-bromo-1-(4-cyclopropylphenyl) ethan-1-one (580 mg, 2.43 mmol) was dissolved in 3 ml of DMF and then added dropwise to the reaction solution. After the addition was completed, the reaction solution was stirred at room temperature for 2 hours. After the reaction was completed, water (50 ml) was added, and the mixture was extracted with ethyl acetate (50 ml) twice. The organic phases were combined, washed with saturated brine (30 ml) twice and then concentrated. The residue was purified with flash column chromatography (eluted with a gradient of water:methanol = 100 : 0-0 : 100) to obtain 780 mg of the title product as a pale yellow solid. MS (m/z): 366.0, 368.0 $[M+H]^+$

(D) 2-((5-bromo-2-chloropyridin-3-yl)oxy)-1-(4-cyclopropylphenyl)ethan-1-ol

In a reaction flask, 2-((5-bromo-2-chloropyridin-3-yl)oxy)-1-(4-cyclopropylphenyl)ethan-1-one (780 mg, 2.13 mmol) was dissolved in methanol (30 ml), and sodium borohydride (161 mg, 4.26 mmol) was added in portions to the mixture in an ice bath. After the addition was completed, the reaction solution was stirred at room temperature for 2 hours, adjusted with dilute hydrochloric acid to a pH value of about 5-6 and concentrated. The residue was purified with flash column chromatography (eluted with a gradient of water:methanol = 100 : 0-0 : 100) to obtain 680 mg of the title product as a white solid. MS (m/z): 268.0, 270.0 $[M+H]^+$

(E) 7-bromo-3-(4-cyclopropylphenyl)-2,3-dihydro-[1,4]dioxino[2,3-b]pyridine

In a reaction flask, 2-((5-bromo-2-chloropyridin-3-yl)oxy)-1-(4-cyclopropylphenyl)ethan-1-ol (620 mg, 1.68 mmol) was dissolved in DMF (3 ml), and then sodium hydride (74 mg, 1.85 mmol) was added in portions to the reaction solution. After the addition was completed, the reaction solution was stirred at room temperature for 10 minutes and stirred at 60°C for 2 hours. After the reaction was completed, the reaction solution was cooled to room temperature, then added water (50 ml) and extracted with ethyl acetate (50 ml) twice. The organic phases were combined, washed with saturated brine (30 ml) twice and then concentrated. The residue was purified with flash column chromatography (eluted with a gradient of water:methanol = 100 : 0-0 : 100) to obtain 230 mg of the title product as a pale yellow solid. MS (m/z): 332.0, 334.0 $[M+H]^+$

(F) 3-(4-cyclopropylphenyl)-2,3-dihydro-[1,4]dioxino[2,3-b]pyridin-7-ol

Under nitrogen atmosphere, 7-bromo-3-(4-cyclopropylphenyl)-2,3-dihydro-[1,4]dioxino[2,3-b]pyridine (230 mg, 0.692 mmol), $Pin_2B_2$ (352 mg, 1.38 mmol), potassium acetate (135 mg, 1.38 mmol), $Pd(dppf)Cl_2$ (50 mg, 0.07 mmol), dioxane (20 ml) and water (4 ml) were successively added to a reaction flask. The reaction solution was heated to reflux and stirred for 6 hours. After cooled to room temperature, the reaction solution was concentrated, and ethyl acetate (100 ml) was added. The mixture was stirred for 1 hour and filtered. The solid was washed with ethyl acetate. The filtrate was concentrated, and the residue was dissolved in THF (20 ml) and sodium hydroxide aqueous solution (56 mg of sodium hydroxide dissolved in 2 ml of water); and then hydrogen peroxide (1 ml, 30% (by weight)) was added dropwise in an ice

bath. After warmed to room temperature, the mixture was stirred for 1 hour. After cooling in an ice bath, saturated sodium thiosulfate aqueous solution was added dropwise. The mixture was stirred for 5 minutes, and concentrated after no peroxide was detected with a potassium iodide-starch test paper. The residue was purified with flash column chromatography (eluted with a gradient of petroleum ether:ethyl acetate=100 : 0-0 : 100) to obtain 80 mg of the title product as a white solid. MS (m/z): 270.0 [M+H]$^+$ (G) 5-((3-(4-cyclopropylphenyl)-2,3-dihydro-[1,4]dioxino[2,3-b]pyridin-7-yl)oxy)pyridin-2-amine

3-(4-cyclopropylphenyl)-2,3-dihydro-[1,4]dioxino[2,3-b]pyridin-7-ol (50 mg, 0.185 mmol), 5-fluoro-2-nitropyridine (27 mg, 0.185 mmol), potassium carbonate (26 mg, 0.185 mmol) and DMF (2 ml) were added to a reaction flask. The reaction solution was heated at 50°C for 2 hours. The reaction solution was concentrated, and the residue was dissolved in 20 ml of 95% ethanol, and then iron powder (42 mg, 0.742 mmol) and ammonium chloride (50 mg, 0.925 mmol) were added. The reaction solution was heated to reflux for 2 hours. After the reaction was completed, the reaction solution was concentrated, and the residue was purified with flash column chromatography (eluted with a gradient of water:methanol = 100 : 0-0 : 100) to obtain 43 mg of the title product as a solid. MS (m/z): 362.1 [M+H]$^+$

(H) 7-((3-(4-cyclopropylphenyl)-2,3-dihydro-[1,4]dioxino[2,3-b]pyridin-7-yl)oxy)-3-methyl-4*H*-pyrido[1,2-a]pyrimidin-4-one

5-((3-(4-cyclopropylphenyl)-2,3-dihydro-[1,4]dioxino[2,3-b]pyridin-7-yl)oxy)pyridin-2-amine (43 mg, 0.119 mmol), ethyl (E)-3-ethoxy-2-methylacrylate (38 mg, 0.238 mmol) and acetic acid (3 ml) were added to a microwave tube. The reaction was heated at 110°C for 2 hours. After the reaction was completed, the reaction solution was concentrated, and the residue was purified with flash column chromatography (eluted with a gradient of water:methanol = 100 : 0-0 : 100) and preparative thin-layer chromatography (dichloromethane:methanol = 20 : 1) to obtain 13 mg of the title product as a white solid. MS (m/z): 428.1 [M+H]$^+$

$^1$H NMR (400 MHz, DMSO-d6) δ 8.39 (s, 1H), 8.25 (s, 1H), 7.86-7.81 (m, 1H), 7.80-7.76(m, 1H), 7.70 (d, J = 9.6 Hz, 1H), 7.48-7.43 (m, 1H), 7.39 - 7.29 (m, 2H), 7.15 - 7.07 (m, 2H), 5.44-5.25 (m, 1H), 4.57-4.40 (m, 1H), 4.38-4.12 (m, 1H), 2.10 (s, 3H), 1.94-1.87 (m, 1H), 0.97-0.89 (m, 2H), 0.69-0.62 (m, 2H).

**Compound 46**

**2-((4-cyclopropylbenzyl)oxy)-3-methoxy-5-((2-(1-methyl-1*H*-pyrazol-4-yl)pyridin-4-yl)methyl)pyridine**

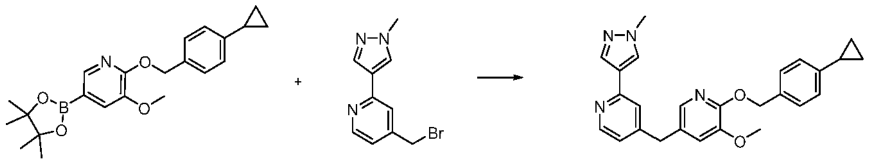

Under nitrogen atmosphere, 2-((4-cyclopropylbenzyl)oxy)-3-methoxy-5-(4,4,5,5-tetramethyl-1,3,2-dioxaborolan-2-yl)pyridine (prepared according to the preparation procedure in step (B) of intermediate 20, using corresponding starting materials and reagents) (2500 mg, 6.55 mmol), 4-(bromomethyl)-2-(1-methyl-1*H*-pyrazol-4-yl)pyridine (1512 mg, 6.55 mmol), potassium phosphate (2780 mg, 13.1 mmol), Pd(dppf)$Cl_2$ (240 mg, 0.327 mmol), dioxane (40 ml) and water (5 ml) were added to a reaction flask. The reaction solution was heated to reflux and stirred for 15 hours, and then concentrated. The residue was purified with flash column chromatography (eluted with a gradient of water:methanol = 100 : 0-0 : 100) and preparative thin-layer chromatography (dichloromethane:methanol = 20 : 1) to obtain 185 mg of the title product as a white solid. MS (m/z): 427.2 [M+H]$^+$

$^1$H NMR (400 MHz, DMSO-d6) δ 8.34 (d, J = 5.1 Hz, 1H), 8.20 (s, 1H), 7.93 (s, 1H), 7.61 (d, J = 1.3 Hz, 1H), 7.53 (s, 1H), 7.26 (s, 1H), 7.24 (s, 1H), 7.20 (d, J = 1.7 Hz, 1H), 7.04-6.98 (m, 3H), 5.21 (s, 2H), 3.85 (s, 2H), 3.84 (s, 3H), 3.70 (s, 3H), 1.91 - 1.79 (m, 1H), 0.92 - 0.85 (m, 2H), 0.64 - 0.57 (m, 2H).

**Compound 47**

**7-((5-methoxy-6-((4-methoxybenzyl)oxy)pyridin-3-yl)methyl)-3-methyl-4*H*-pyrido[1,2-a]pyrimidin-4-one**

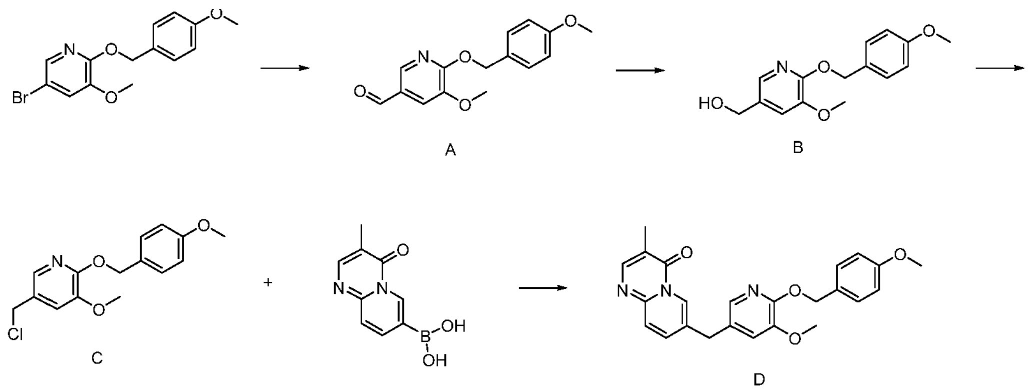

A B C D

(A) 5-methoxy-6-((4-methoxybenzyl)oxy)nicotinaldehyde

To a reaction flask, 5-bromo-3-methoxy-2-((4-methoxybenzyl)oxy)pyridine (800 mg, 2.47 mmol) and anhydrous THF (30 ml) were added, and the reaction solution was cooled to -78°C. Under nitrogen atmosphere, 2.4 M of n-BuLi (1.13 ml, 2.71 mmol) was added dropwise; and after the mixture was stirred for 20 minutes, DMF (360 mg, 4.94 mmol) was added dropwise. The resulting mixture was stirred at -78°C for another 1 hour and then warmed to 0°C slowly. The reaction was quenched by adding saturated ammonium chloride aqueous solution. The reaction solution was extracted with EA, and the organic phase was dried and concentrated. The residue was purified with flash column chromatography (eluted with a gradient of PE/EA= 100 : 0-0 : 100) to obtain 280 mg of the product as a white solid.MS (m/z): 296.1 $[M+Na]^+$

(B) (5-methoxy-6-((4-methoxybenzyl)oxy)pyridin-3-yl)methanol

To a reaction flask, 5-methoxy-6-((4-methoxybenzyl)oxy)nicotinaldehyde (280 mg, 1.025 mmol), THF (10 ml) and methanol (10 ml) were added, and then sodium borohydride (39 mg, 1.025 mmol) was added. After stirred at room temperature for 30 minutes, the reaction solution was concentrated, added water, and extracted with EA. The organic phase was dried and concentrated, and the residue was purified with flash column chromatography (eluted with a gradient of PE/EA= 100 : 0-0 : 100) to obtain 268 mg of the product as a white solid. MS (m/z): 298.1 $[M+Na]^+$

(C) 5-(chloromethyl)-3-methoxy-2-((4-methoxybenzyl)oxy)pyridine

To a reaction flask, (5-methoxy-6-((4-methoxybenzyl)oxy)pyridin-3-yl)methanol (90 mg, 0.327 mmol), DCM (20 ml) and TEA (0.136 ml, 0.981 mmol) were added, and MsCl (45 mg, 0.393 mmol) was then added dropwise. After stirred at room temperature for 30 minutes, the resulting mixture was washed with water, and the organic phase was dried and concentrated to obtain 110 mg of the product as a colorless oil.

(D) 7-((5-methoxy-6-((4-methoxybenzyl)oxy)pyridin-3-yl)methyl)-3-methyl-4H-pyrido[1,2-a]pyrimidin-4-one

5-(chloromethyl)-3-methoxy-2-((4-methoxybenzyl)oxy)pyridine (110 mg, 0.327 mmol), (3-methyl-4-oxo-4*H*-pyrido[1,2-a]pyrimidin-7-yl)boronic acid (67 mg, 0.327 mmol), $Na_2CO_3$ (104 mg, 0.981 mmol), $Pd(dppf)Cl_2 \cdot CH_2Cl_2$ (27 mg, 0.0327 mmol), dioxane (10.0 ml) and water (2.0 ml) were added to a reaction flask. Under nitrogen atmosphere, the mixture was heated to 60°C-80°C and stirred for 30 minutes. After cooling, the reaction solution was concentrated and purified with flash column chromatography (eluted with a gradient of $H_2O$/MeOH = 100 : 0-0 : 100) to obtain 49 mg of the product as a white solid. MS (m/z): 418.2 $[M+H]^+$

1H NMR (400 MHz, DMSO-d6) δ 8.82 (s, 1H), 8.22 (s, 1H), 7.73 (dd, J = 9.2, 2.0 Hz, 1H), 7.67 (d, J = 1.8 Hz, 1H), 7.55 (d, J = 9.2 Hz, 1H), 7.33 (d, J = 8.6 Hz, 2H), 7.24 (d, J = 1.9 Hz, 1H), 6.97 - 6.82 (m, 2H), 5.21 (s, 2H), 3.99 (s, 2H), 3.71 (s, 3H), 3.69 (s, 3H), 2.10 (s, 3H).

**Compounds 48-51**

**(R/S)-3-(4-cyclopropylphenyl)-7-((2-(1-methyl-1*H*-pyrazol-4-yl)pyridin-4-yl)oxy)-2,3-dihydro-[1,4]dioxino[2,3-b] pyridine and (R/S)-2-(4-cyclopropylphenyl)-7-((2-(1-methyl-1*H*-pyrazol-4-yl)pyridin-4-yl)oxy)-2,3-dihydro-[1,4] dioxino[2,3-b]pyridine**

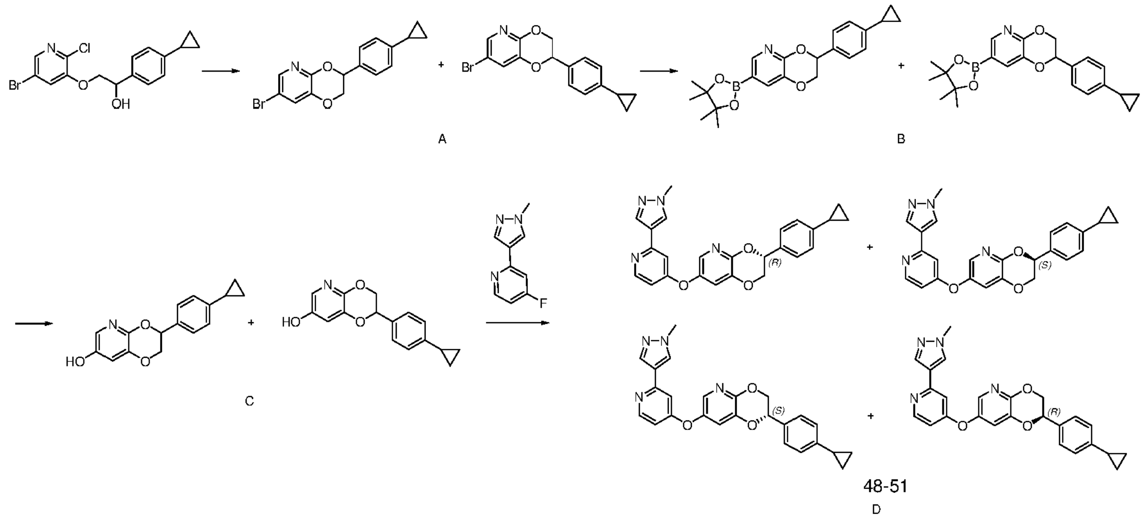

A B C 48-51 D

(A) 7-bromo-3-(4-cyclopropylphenyl)-2,3-dihydro-[1,4]dioxino[2,3-b]pyridine and 7-bromo-2-(4-cyclopropylphenyl)-2,3-dihydro-[1,4]dioxino[2,3-b]pyridine

In a reaction flask, 2-((5-bromo-2-chloropyridin-3-yl)oxy)-1-(4-cyclopropylphenyl)ethan-1-ol (1.8 g, 4.88 mmol) and potassium bis(trimethylsilyl)amide (1.07 g, 5.37 mmol) were dissolved in anhydrous tetrahydrofuran (10 ml). After the addition was completed, the reaction solution was stirred at 60°C for 4 hours. After the reaction was completed, the reaction solution was concentrated. The residue was purified with flash column chromatography (eluted with a gradient of water:methanol = 100 : 0-0 : 100) to obtain 900 mg of a mixture of the title products as a pale yellow solid. MS (m/z): 332.0, 334.0 $[M+H]^+$

(B) 3-(4-cyclopropylphenyl)-7-(4,4,5,5-tetramethyl-1,3,2-dioxaborolan-2-yl)-2,3-dihydro-[1,4]dioxino[2,3-b]pyridine and 2-(4-cyclopropylphenyl)-7-(4,4,5,5-tetramethyl-1,3,2-dioxaborolan-2-yl)-2,3-dihydro-[1,4]dioxino[2,3-b]pyridine

Under nitrogen atmosphere, a mixture of 7-bromo-3-(4-cyclopropylphenyl)-2,3-dihydro-[1,4]dioxino[2,3-b]pyridine and 7-bromo-2-(4-cyclopropylphenyl)-2,3-dihydro-[1,4]dioxino[2,3-b]pyridine (900 mg, 2.71 mmol), $Pin_2B_2$ (1376 mg, 5.42 mmol), potassium acetate (532 mg, 5.42 mmol), $Pd(dppf)Cl_2$ (99 mg, 0.135 mmol) and dioxane (30 ml) were successively added to a reaction flask, and the mixture was heated to reflux and stirred for 6 hours. After cooled to room temperature, the reaction solution was concentrated, and ethyl acetate (100 ml) was added. The mixture was stirred for 1 hour and filtered. The solid was washed with ethyl acetate. The filtrate was concentrated to obtain a crude product, and the crude product was used in the reaction of the next step directly. MS (m/z): 380.0 $[M+H]^+$

(C) 3-(4-cyclopropylphenyl)-2,3-dihydro-[1,4]dioxino[2,3-b]pyridin-7-ol and 2-(4-cyclopropylphenyl)-2,3-dihydro-[1,4]dioxino[2,3-b]pyridin-7-ol

The crude (1068 mg, 2.82 mmol) prepared in the previous step, THF (30 ml) and sodium hydroxide aqueous solution (226 mg, dissolved in 3 ml of water) were added to a reaction flask, and then hydrogen peroxide (3 ml, 30%wt) was added dropwise in an ice bath. After warmed to room temperature, the mixture was stirred for 1 hour. After cooling in an ice bath, saturated sodium thiosulfate aqueous solution was added dropwise. The mixture was stirred for 5 minutes, and concentrated after no peroxide was detected with a potassium iodide-starch test paper. The residue was purified with flash column chromatography (eluted with a gradient of petroleum ether:ethyl acetate = 100 : 0-0 : 100) to obtain 480 mg of a mixture of the title products as a white solid. MS (m/z): 270.0 $[M+H]^+$

(D) (R/S)-3-(4-cyclopropylphenyl)-7-((2-(1-methyl-1*H*-pyrazol-4-yl)pyridin-4-yl)oxy)-2,3-dihydro-[1,4]dioxino[2,3-b]pyridine and (R/S)-2-(4-cyclopropylphenyl)-7-((2-(1-methyl-1*H*-pyrazol-4-yl)pyridin-4-yl)oxy)-2,3-dihydro-[1,4]dioxino[2,3-b]pyridine

In a reaction flask, a mixture (300 mg, 1.11 mmol) of 3-(4-cyclopropylphenyl)-2,3-dihydro-[1,4]dioxino[2,3-b]pyridin-7-ol and 2-(4-cyclopropylphenyl)-2,3-dihydro-[1,4]dioxino[2,3-b]pyridin-7-ol prepared in the previous step, 4-fluoro-2-(1-methyl-1H-pyrazol-4-yl)pyridine (196 mg, 1.11 mmol) and cesium carbonate (362 mg, 1.11 mmol) were dissolved in DMF (3 ml), and the resulting mixture was heated at 90°C for 2 hours. The reaction solution was concentrated

to dryness, and the residue was purified with flash column chromatography (eluted with a gradient of water:methanol = 100 : 0-0 : 100) to obtain 115 mg of a white solid mixture. The mixture was resolved by chiral HPLC to obtain two pairs of enantiomers. Chiral HPLC conditions: column: IC (2 × 25 cm); mobile phase: acetonitrile/ethanol = 10 : 90 (0.1% ammonia water); flow rate: 15 ml/minute; detector: UV 254 nm.

The first eluent (compound 48, 10 mg, RT = 8.693 minutes), ee% = 100%, $[M+H]^+$ 427.4. $^1H$ NMR (400 MHz, DMSO-d6) δ 8.35 (d, J = 5.7 Hz, 1H), 8.25 (s, 1H), 7.95 (s, 1H), 7.73 (dd, J = 2.6, 0.8 Hz, 1H), 7.39 (d, J = 2.6 Hz, 1H), 7.35 (d, J = 8.2 Hz, 2H), 7.21 (d, J = 2.4 Hz, 1H), 7.13 (d, J = 8.1 Hz, 2H), 6.68 - 6.62 (m, 1H), 5.39 (dd, J = 8.5, 2.2 Hz, 1H), 4.44 (dd, J = 11.6, 2.3 Hz, 1H), 4.12 (dd, J = 11.5, 8.7 Hz, 1H), 3.83 (s, 3H), 1.96 - 1.85 (m, 1H), 0.98 - 0.89 (m, 2H), 0.69 - 0.63 (m, 2H).

The second eluent (compound 49, 12 mg, RT = 9.317 minutes), ee% = 90%, $[M+H]^+$427.4. $^1H$ NMR (400 MHz, DMSO-d6) δ 8.34 (d, J = 5.7 Hz, 1H), 8.25 (s, 1H), 7.95 (s, 1H), 7.72 (d, J = 2.6 Hz, 1H), 7.41 (d, J = 2.6 Hz, 1H), 7.33 (d, J = 8.2 Hz, 2H), 7.22 (d, J = 2.4 Hz, 1H), 7.10 (d, J = 8.2 Hz, 2H), 6.65 (dd, J = 5.7, 2.4 Hz, 1H), 5.27 (dd, J = 8.5, 2.2 Hz, 1H), 4.55 (dd, J = 11.8, 2.3 Hz, 1H), 4.31 (dd, J = 11.8, 8.6 Hz, 1H), 3.83 (s, 3H), 1.94 - 1.86 (m, 1H), 0.96 - 0.89 (m, 2H), 0.70 - 0.61 (m, 2H).

The third eluent (compound 50, 18 mg, RT = 10.392 minutes), ee% = 95%, $[M+H]^+$ 427.4. $^1H$ NMR (400 MHz, DMSO-d6) δ 8.34 (d, J = 5.7 Hz, 1H), 8.25 (s, 1H), 7.95 (s, 1H), 7.72 (dd, J = 2.6, 0.4 Hz, 1H), 7.42 - 7.39 (m, 1H), 7.33 (d, J = 8.2 Hz, 2H), 7.22 (d, J = 2.4 Hz, 1H), 7.10 (d, J = 8.2 Hz, 2H), 6.65 (dd, J = 5.7, 2.4 Hz, 1H), 5.27 (dd, J = 8.5, 2.2 Hz, 1H), 4.55 (dd, J = 11.8, 2.4 Hz, 1H), 4.31 (dd, J = 11.8, 8.6 Hz, 1H), 3.83 (s, 3H), 1.89 (td, J = 8.4, 4.2 Hz, 1H), 0.96 - 0.88 (m, 2H), 0.70 - 0.61 (m, 2H).

The fourth eluent (compound 51, 18 mg, RT = 11.410 minutes), ee% = 90%, $[M+H]^+$ 427.4. $^1H$ NMR (400 MHz, DMSO-d6) δ 8.35 (d, J = 5.7 Hz, 1H), 8.25 (s, 1H), 7.95 (s, 1H), 7.73 (dd, J = 2.5, 0.5 Hz, 1H), 7.39 (dd, J = 2.5, 0.4 Hz, 1H), 7.35 (d, J = 8.2 Hz, 2H), 7.21 (d, J = 2.4 Hz, 1H), 7.13 (d, J = 8.2 Hz, 2H), 6.66 (dd, J = 5.5, 2.2 Hz, 1H), 5.39 (dd, J = 8.5, 2.2 Hz, 1H), 4.44 (dd, J = 11.6, 2.4 Hz, 1H), 4.12 (dd, J = 11.5, 8.6 Hz, 1H), 3.83 (s, 3H), 1.96 - 1.85 (m, 1H), 0.95 - 0.88 (m, 2H), 0.70 - 0.61 (m, 2H).

## Compound 52

**2-(4-(4-((3-(4-cyclopropylphenyl)-2,3-dihydro-[1,4]dioxino[2,3-b]pyridin-7-yl)oxy)pyridin-2-yl)-1*H*-pyrazol-1-yl)ethan-1-ol**

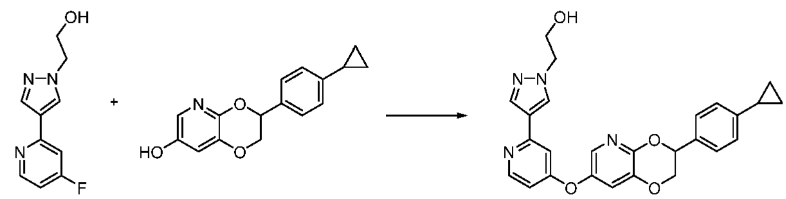

In a reaction flask, 3-(4-cyclopropylphenyl)-2,3-dihydro-[1,4]dioxino[2,3-b]pyridin-7-ol (150 mg, 0.57 mmol), 2-(4-(4-fluoropyridin-2-yl)-1*H*-pyrazol-1-yl)ethan-1-ol (118 mg, 0.57 mmol) and cesium carbonate (186 mg, 0.57 mmol) were dissolved in DMF (3 ml), and the mixture was heated at 90°C for 2 hours. The reaction solution was concentrated to dryness, and the residue was purified with flash column chromatography (eluted with a gradient of water:methanol = 100 : 0-0 : 100) to obtain 6 mg of the title compound as a white solid. MS (m/z): 457.2 $[M+H]^+$

$^1H$ NMR (400 MHz, DMSO-d6) δ 8.35 (d, J = 5.5 Hz, 1H), 8.25 (s, 1H), 7.97 (s, 1H), 7.72 (dd, J = 4.3, 2.5 Hz, 1H), 7.40 (dd, J = 7.0, 2.4 Hz, 1H), 7.34 (t, J = 7.8 Hz, 2H), 7.26 - 7.21 (m, 1H), 7.12 (t, J = 8.9 Hz, 2H), 6.69 - 6.62 (m, 1H), 5.33 (dd, J = 45.8, 7.6 Hz, 1H), 4.90 (s, 1H), 4.49 (dd, J = 45.8, 9.7 Hz, 1H), 4.36 - 4.14 (m, 1H), 4.14 - 4.09 (m, 2H), 3.72 (d, J = 5.3 Hz, 2H), 2.85 (s, 1H), 2.69 (s, 1H), 1.95 - 1.88 (m, 1H), 0.96 - 0.91 (m, 2H), 0.66 (t, J = 4.7 Hz, 2H).

## Compounds 53-54

**(R/S)-3-(6-cyclopropylpyridin-3-yl)-7-((2-(1-methyl-1*H*-pyrazol-4-yl)pyridin-4-yl)oxy)-2,3-dihydro-[1,4]dioxino[2,3-b]pyridine**

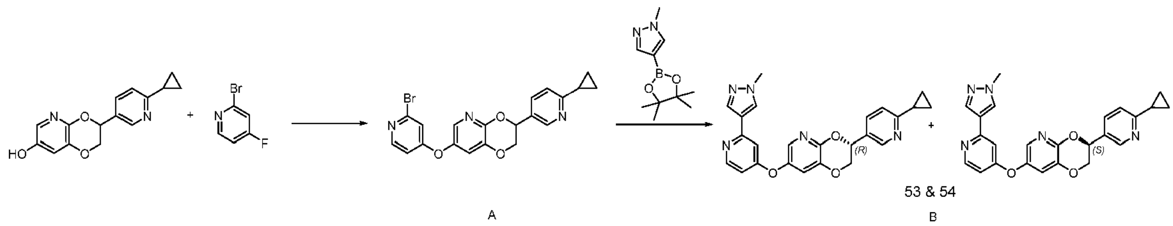

A

53 & 54

B

(A) 7-((2-bromopyridin-4-yl)oxy)-3-(6-cyclopropylpyridin-3-yl)-2,3-dihydro-[1,4]dioxino[2,3-b]pyridine

In a reaction flask, 3-(6-cyclopropylpyridin-3-yl)-2,3-dihydro-[1,4]dioxino[2,3-b]pyridin-7-ol (210 mg, 0.777 mmol), 2-bromo-4-fluoropyridine (205 mg, 1.165 mmol) and cesium carbonate (379 mg, 1.165 mmol) were dissolved in DMF (3 ml), and the mixture was heated at 90°C for 1 hour. The reaction solution was concentrated to dryness, and the residue was purified with flash column chromatography (eluted with a gradient of water:methanol = 100 : 0-0 : 100) to obtain 180 mg of the title product as a white solid. MS (m/z): 426.2, 428.2 $[M+H]^+$

(B) (R/S)-3-(6-cyclopropylpyridin-3-yl)-7-((2-(1-methyl-1*H*-pyrazol-4-yl)pyridin-4-yl)oxy)-2,3 -dihydro-[1,4]dioxino[2,3-b]pyridine

7-((2-bromopyridin-4-yl)oxy)-3-(6-cyclopropylpyridin-3-yl)-2,3-dihydro-[1,4]dioxino[2,3-b]pyridine (130 mg, 0.305 mmol), 1-methyl-4-(4,4,5,5-tetramethyl-1,3,2-dioxaborolan-2-yl)-1*H*-pyrazole (127 mg, 0.610 mmol), potassium carbonate (85 mg, 0.610 mmol), $Pd(dppf)Cl_2$ (22 mg, 0.03 mmol), dioxane (20 ml) and water (4 ml) were added to a reaction flask; and under nitrogen atmosphere, the reaction solution was heated to reflux and stirred for 5 hours. After the reaction was completed, the reaction solution was concentrated to dryness, and the residue was purified with flash column chromatography (eluted with a gradient of water:methanol = 100 : 0-0 : 100) to obtain 130 mg of a white solid mixture. The mixture was resolved by chiral HPLC to obtain one pair of enantiomers. Chiral HPLC conditions: column: IC (2 × 25 cm); mobile phase: acetonitrile/ethanol = 5 : 95 (0.1% ammonia water); flow rate: 18 ml/minute; detector: UV 254 nm.

The first eluent (compound 53, 52 mg, RT = 10.192 minutes), ee% = 95%, $[M+H]^+$ 428.2. $^1H$ NMR (400 MHz, DMSO-d6) δ 8.51 (d, J = 2.0 Hz, 1H), 8.35 (d, J = 5.7 Hz, 1H), 8.25 (s, 1H), 7.96 (s, 1H), 7.75 (dt, J = 4.2, 3.4 Hz, 2H), 7.42 (dd, J = 2.5, 0.6 Hz, 1H), 7.36 (d, J = 8.1 Hz, 1H), 7.22 (d, J = 2.4 Hz, 1H), 6.68 - 6.63 (m, 1H), 5.47 (dd, J = 8.5, 2.2 Hz, 1H), 4.49 (dd, J = 11.7, 2.3 Hz, 1H), 4.22 (dd, J = 11.5, 8.6 Hz, 1H), 3.83 (s, 3H), 2.16 - 2.06 (m, 1H), 0.98 - 0.88 (m, 4H).

The second eluent (compound 54, 50 mg, RT = 11.170 minutes), ee% = 100%, $[M+H]^+$ 428.2. $^1H$ NMR (400 MHz, DMSO-d6) δ 8.51 (d, J = 2.1 Hz, 1H), 8.35 (d, J = 5.6 Hz, 1H), 8.25 (s, 1H), 7.96 (s, 1H), 7.78 - 7.69 (m, 2H), 7.42 (dd, J = 2.6, 0.8 Hz, 1H), 7.36 (d, J = 8.1 Hz, 1H), 7.22 (d, J = 2.3 Hz, 1H), 6.66 (ddd, J = 5.7, 2.4, 0.7 Hz, 1H), 5.47 (dd, J = 8.5, 2.2 Hz, 1H), 4.49 (dd, J = 11.6, 2.2 Hz, 1H), 4.21 (dd, J = 11.5, 8.7 Hz, 1H), 3.83 (s, 3H), 2.15 - 2.05 (m, 1H), 0.98 - 0.87 (m, 4H).

**Compounds 55-56**

**(R/S)-7-((3-(4-cyclopropylphenyl)-2,3-dihydro-[1,4]dioxino[2,3-b]pyridin-7-yl)oxy)-4H-pyrido[1,2-a]pyrimidin-4-one**

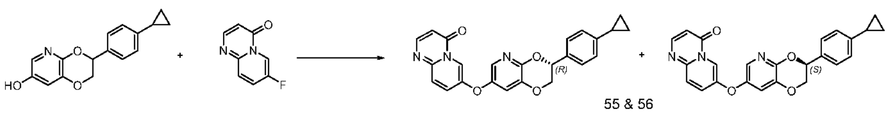

55 & 56

In a reaction flask, 3-(4-cyclopropylphenyl)-2,3-dihydro-[1,4]dioxino[2,3-b]pyridin-7-ol (80 mg, 0.297 mmol), 7-fluoro-4*H*-pyrido[1,2-a]pyrimidin-4-one (49 mg, 0.297 mmol) and cesium carbonate (97 mg, 0.297 mmol) were dissolved in DMF (2 ml), and the mixture was heated at 90°C for 2 hours. The reaction solution was concentrated to dryness, and the residue was purified with flash column chromatography (eluted with a gradient of water:methanol = 100 : 0-0 : 100) to obtain 120 mg of a white solid mixture. The mixture was resolved by chiral HPLC to obtain one pair of enantiomers. Chiral HPLC conditions: column: IC (2×25 cm); mobile phase: acetonitrile/ethanol = 5 : 95 (0.1% ammonia water); flow rate: 18 ml/minute; detector: UV 254 nm.

The first eluent (compound 55, 52 mg, RT = 16.519 minutes), ee% = 100%, $[M+H]^+$ 414.2. $^1H$ NMR (400 MHz, DMSO-d6) δ 8.42 (d, J = 2.7 Hz, 1H), 8.28 (d, J = 6.3 Hz, 1H), 7.95 (dd, J = 9.6, 2.8 Hz, 1H), 7.80 (d, J = 2.6 Hz, 1H), 7.77 (d, J = 9.6 Hz, 1H), 7.47 (d, J = 2.6 Hz, 1H), 7.35 (d, J = 8.2 Hz, 2H), 7.12 (d, J = 8.3 Hz, 2H), 6.37 (d, J = 6.3 Hz, 1H), 5.40 (dd, J =

8.6, 2.3 Hz, 1H), 4.42 (dd, J = 11.6, 2.5 Hz, 1H), 4.16 (dd, J = 11.6, 8.6 Hz, 1H), 1.95 - 1.85 (m, 1H), 0.98 - 0.89 (m, 2H), 0.69 - 0.62 (m, 2H).

The second eluent (compound 56, 50 mg, RT = 18.566 minutes), ee% = 90%, $[M+H]^+$ 414.2. $^1H$ NMR (400 MHz, DMSO-d6) δ 8.42 (d, J = 2.7 Hz, 1H), 8.28 (d, J = 6.3 Hz, 1H), 7.95 (dd, J = 9.6, 2.8 Hz, 1H), 7.80 (d, J = 2.6 Hz, 1H), 7.77 (d, J = 9.6 Hz, 1H), 7.47 (d, J = 2.6 Hz, 1H), 7.35 (d, J = 8.2 Hz, 2H), 7.12 (d, J = 8.3 Hz, 2H), 6.37 (d, J = 6.3 Hz, 1H), 5.41 (dd, J = 8.6, 2.3 Hz, 1H), 4.42 (dd, J = 11.6, 2.5 Hz, 1H), 4.16 (dd, J = 11.6, 8.6 Hz, 1H), 1.96 - 1.83 (m, 1H), 0.96 - 0.90 (m, 2H), 0.68 - 0.62 (m, 2H).

The following compounds were prepared according to the preparation procedure of compounds 55 and 56 (using corresponding intermediates and reagents) and according to the chiral resolution conditions thereof under appropriate conditions that will be recognized by one skilled in the art.

| Compound | Structural formula | MS $(M+H)^+$ | $^1$HNMR |
|---|---|---|---|
| 57 | 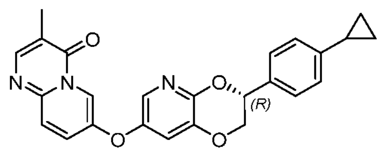 | 428.2 | 1H NMR (400 MHz, DMSO-d6) δ 8.38 (d, J = 2.7 Hz, 1H), 8.25 (d, J = 0.7 Hz, 1H), 7.83 (dd, J = 9.7, 2.8 Hz, 1H), 7.79 (d, J = 2.7 Hz, 1H), 7.70 (d, J = 9.7 Hz, 1H), 7.45 (d, J = 2.6 Hz, 1H), 7.35 (d, J = 8.3 Hz, 2H), 7.12 (d, J = 8.3 Hz, 2H), 5.40 (dd, J = 8.5, 2.3 Hz, 1H), 4.42 (dd, J = 11.6, 2.5 Hz, 1H), 4.15 (dd, J = 11.6, 8.6 Hz, 1H), 2.09 (s, 3H), 1.95 - 1.87 (m, 1H), 0.96 - 0.90 (m, 2H), 0.69 - 0.63 (m, 2H). |
| 58 | 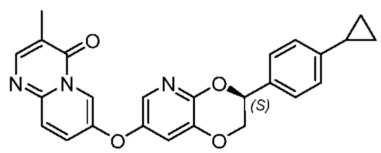 | 428.2 | 1H NMR (400 MHz, DMSO-d6) δ 8.38 (d, J = 2.7 Hz, 1H), 8.25 (s, 1H), 7.83 (dd, J = 9.7, 2.7 Hz, 1H), 7.79 (dd, J = 2.6, 0.5 Hz, 1H), 7.70 (d, J = 9.7 Hz, 1H), 7.46 - 7.42 (m, 1H), 7.35 (d, J = 8.2 Hz, 2H), 7.12 (d, J = 8.2 Hz, 2H), 5.40 (dd, J = 8.4, 2.1 Hz, 1H), 4.42 (dd, J = 11.6, 2.3 Hz, 1H), 4.15 (dd, J = 11.6, 8.6 Hz, 1H), 2.09 (s, 3H), 1.96 - 1.86 (m, 1H), 0.97 - 0.90 (m, 2H), 0.70 - 0.62 (m, 2H). |
| 59 | 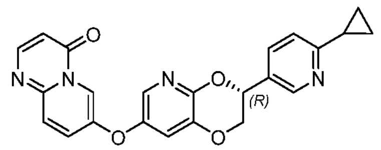 | 415.2 | 1H NMR (400 MHz, DMSO-d6) δ 8.50 (d, J = 2.0 Hz, 1H), 8.42 (d, J = 2.8 Hz, 1H), 8.28 (dd, J = 6.3, 0.8 Hz, 1H), 7.98 - 7.92 (m, 1H), 7.81 (dd, J = 2.6, 0.8 Hz, 1H), 7.79 - 7.71 (m, 2H), 7.49 (dd, J = 2.6, 0.8 Hz, 1H), 7.35 (d, J = 8.1 Hz, 1H), 6.37 (d, J = 6.3 Hz, 1H), 5.48 (dd, J = 8.5, 2.2 Hz, 1H), 4.47 (dd, J = 11.7, 2.2 Hz, 1H), 4.25 (dd, J = 11.5, 8.7 Hz, 1H), 2.18 - 2.00 (m, 1H), 0.99 - 0.86 (m, 4H). |
| 60 | 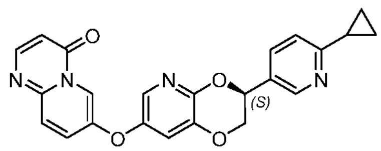 | 415.2 | 1H NMR (400 MHz, DMSO-d6) δ 8.50 (d, J = 2.0 Hz, 1H), 8.42 (d, J = 2.8 Hz, 1H), 8.28 (dd, J = 6.3, 0.8 Hz, 1H), 7.98 - 7.92 (m, 1H), 7.81 (dd, J = 2.6, 0.8 Hz, 1H), 7.79 - 7.71 (m, 2H), 7.49 (dd, J = 2.6, 0.8 Hz, 1H), 7.35 (d, J = 8.1 Hz, 1H), 6.37 (d, J = 6.2 Hz, 1H), 5.48 (dd, J = 8.5, 2.2 Hz, 1H), 4.47 (dd, J = 11.6, 2.2 Hz, 1H), 4.25 (dd, J = 11.5, 8.6 Hz, 1H), 2.14 - 2.04 (m, 1H), 0.96 - 0.88 (m, 4H). |
| 61 | 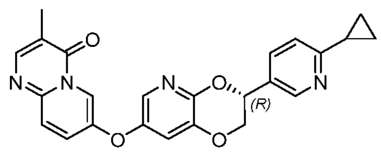 | 429.2 | 1H NMR (400 MHz, DMSO-d6) δ 8.50 (d, J = 2.1 Hz, 1H), 8.39 (d, J = 2.7 Hz, 1H), 8.25 (d, J = 0.7 Hz, 1H), 7.83 (dd, J = 9.7, 2.7 Hz, 1H), 7.80 (dd, J = 2.6, 0.4 Hz, 1H), 7.74 (dd, J = 8.1, 2.3 Hz, 1H), 7.70 (d, J = 9.7 Hz, 1H), 7.48 - 7.45 (m, 1H), 7.35 (d, J = 8.1 Hz, 1H), 5.48 (dd, J = 8.5, 2.3 Hz, 1H), 4.47 (dd, J = 11.6, 2.4 Hz, 1H), 4.24 (dd, J = 11.6, 8.6 Hz, 1H), 2.12 (dd, J = 8.8, 5.7 Hz, 1H), 2.09 (s, 3H), 0.96 - 0.87 (m, 4H). |

(continued)

| Compound | Structural formula | MS $(M+H)^+$ | $^1$HNMR |
|---|---|---|---|
| 62 | 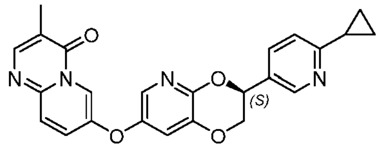 | 429.2 | 1H NMR (400 MHz, DMSO-d6) δ 8.50 (s, 1H), 8.39 (d, J = 2.7 Hz, 1H), 8.25 (s, 1H), 7.84 (dd, J = 9.7, 2.7 Hz, 1H), 7.80 (dd, J = 2.6, 1.0 Hz, 1H), 7.74 (dd, J = 8.0, 1.9 Hz, 1H), 7.70 (d, J = 9.7 Hz, 1H), 7.47 (dd, J = 2.6, 1.0 Hz, 1H), 7.35 (d, J = 8.1 Hz, 1H), 5.48 (d, J = 6.4 Hz, 1H), 4.47 (dd, J = 11.6, 2.0 Hz, 1H), 4.24 (dd, J = 11.5, 8.6 Hz, 1H), 2.12 (dd, J = 8.9, 5.3 Hz, 1H), 2.09 (s, 3H), 0.96 - 0.88 (m, 4H). |
| 63 | 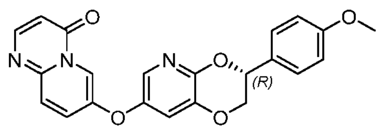 | 404.2 | 1H NMR (400 MHz, DMSO-d6) δ 8.42 (s, 1H), 8.28 (d, J = 6.2 Hz, 1H), 8.04 - 7.89 (m, 1H), 7.83 - 7.70 (m, 2H), 7.47 (d, J = 1.9 Hz, 1H), 7.42 (d, J = 8.5 Hz, 2H), 6.98 (d, J = 8.4 Hz, 2H), 6.37 (d, J = 6.2 Hz, 1H), 5.39 (d, J = 7.4 Hz, 1H), 4.41 (d, J = 9.7 Hz, 1H), 4.27 - 4.12 (m, 1H), 3.75 (s, 3H). |
| 64 | 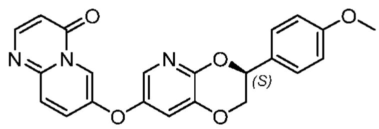 | 404.2 | 1H NMR (400 MHz, DMSO-d6) δ 8.42 (d, J = 2.7 Hz, 1H), 8.28 (d, J = 6.3 Hz, 1H), 7.95 (dd, J = 9.6, 2.8 Hz, 1H), 7.80 (d, J = 2.6 Hz, 1H), 7.77 (d, J = 9.7 Hz, 1H), 7.47 (d, J = 2.6 Hz, 1H), 7.42 (d, J = 8.7 Hz, 2H), 6.98 (d, J = 8.8 Hz, 2H), 6.37 (d, J = 6.3 Hz, 1H), 5.39 (dd, J = 8.7, 2.3 Hz, 1H), 4.41 (dd, J = 11.6, 2.5 Hz, 1H), 4.18 (dd, J = 11.6, 8.8 Hz, 1H), 3.75 (s, 3H). |
| 65 | 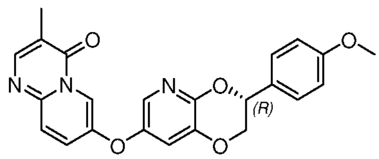 | 418.2 | 1H NMR (400 MHz, DMSO-d6) δ 8.37 (d, J = 1.9 Hz, 1H), 8.25 (d, J = 0.9 Hz, 1H), 7.89 - 7.80 (m, 1H), 7.79 (dd, J = 2.5, 1.7 Hz, 1H), 7.70 (d, J = 9.6 Hz, 1H), 7.45 (dd, J = 2.5, 1.7 Hz, 1H), 7.44 - 7.37 (m, 2H), 7.03 - 6.90 (m, 2H), 5.38 (d, J = 8.5 Hz, 1H), 4.41 (d, J = 11.4 Hz, 1H), 4.28 - 4.07 (m, 1H), 3.75 (d, J = 1.5 Hz, 3H), 2.09 (s, 3H). |
| 66 | 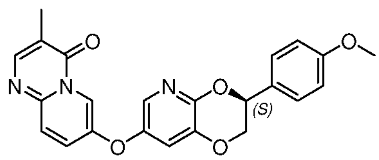 | 418.1 | 1H NMR (400 MHz, DMSO-d6) δ 8.38 (d, J = 2.7 Hz, 1H), 8.25 (s, 1H), 7.84 (dd, J = 9.7, 2.7 Hz, 1H), 7.81 - 7.76 (m, 1H), 7.71 (d, J = 9.7 Hz, 1H), 7.46 (d, J = 2.6 Hz, 1H), 7.42 (d, J = 8.6 Hz, 2H), 6.98 (d, J = 8.5 Hz, 2H), 5.39 (dd, J = 8.7, 2.1 Hz, 1H), 4.41 (dd, J = 11.6, 2.3 Hz, 1H), 4.17 (dd, J = 11.4, 8.9 Hz, 1H), 3.75 (s, 3H), 2.09 (s, 3H). |
| 67 | 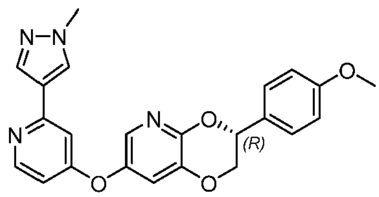 | 417.2 | 1H NMR (400 MHz, DMSO-d6) δ 8.35 (d, J = 5.7 Hz, 1H), 8.25 (s, 1H), 7.96 (s, 1H), 7.73 (dd, J = 2.3, 1.5 Hz, 1H), 7.41 (dd, J = 6.2, 5.1 Hz, 3H), 7.22 (d, J = 1.7 Hz, 1H), 7.05 - 6.89 (m, 2H), 6.71 - 6.60 (m, 1H), 5.37 (d, J = 8.4 Hz, 1H), 4.43 (d, J = 11.3 Hz, 1H), 4.24 - 4.09 (m, 1H), 3.83 (s, 3H), 3.75 (d, J = 1.3 Hz, 3H). |
| 68 | 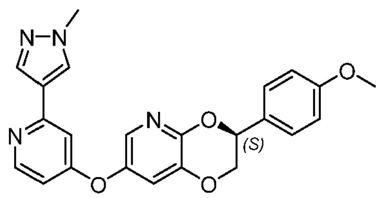 | 417.2 | 1H NMR (400 MHz, DMSO-d6) δ 8.41 - 8.29 (m, 1H), 8.25 (d, J = 7.4 Hz, 1H), 8.04 - 7.90 (m, 1H), 7.73 (dd, J = 7.5, 2.4 Hz, 1H), 7.41 (dd, J = 9.5, 6.8 Hz, 3H), 7.22 (dd, J = 7.3, 2.1 Hz, 1H), 6.99 (t, J = 7.9 Hz, 2H), 6.66 (dd, J = 9.3, 3.7 Hz, 1H), 5.37 (t, J = 6.8 Hz, 1H), 4.42 (dd, J = 9.5, 7.2 Hz, 1H), 4.14 (dt, J = 11.4, 8.1 Hz, 1H), 3.83 (d, J = 7.5 Hz, 3H), 3.79 - 3.66 (m, 3H). |

(continued)

| Compound | Structural formula | MS (M+H)+ | 1HNMR |
|---|---|---|---|
| 69 | 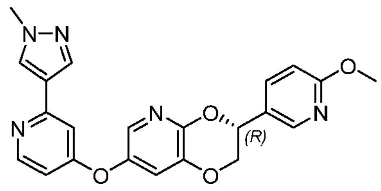 | 418.2 | 1H NMR (400 MHz, $CDCl_3$) δ 8.42 (d, J = 5.7 Hz, 1H), 8.26 (d, J = 2.0 Hz, 1H), 7.90 (s, 1H), 7.88 (s, 1H), 7.78 (d, J = 2.5 Hz, 1H), 7.70 (dd, J = 8.6, 2.4 Hz, 1H), 7.10 (d, J = 2.5 Hz, 1H), 7.00 (d, J = 2.3 Hz, 1H), 6.83 (d, J = 8.6 Hz, 1H), 6.66 (dd, J = 5.7, 2.4 Hz, 1H), 5.33 (dd, J = 8.8, 2.2 Hz, 1H), 4.39 (dd, J = 11.9, 2.2 Hz, 1H), 4.11 (dd, J = 11.7, 9.0 Hz, 1H), 3.97 (s, J = 0.7 Hz, 3H), 3.95 (s, 3H). |
| 70 | 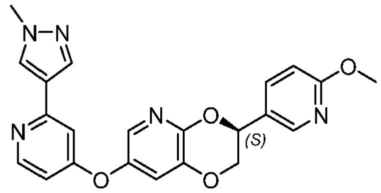 | 418.2 | 1H NMR (400 MHz, $CDCl_3$) δ 8.41 (d, J = 5.7 Hz, 1H), 8.25 (d, J = 2.1 Hz, 1H), 7.89 (s, 1H), 7.88 (s, 1H), 7.77 (d, J = 2.4 Hz, 1H), 7.69 (dd, J = 8.6, 2.3 Hz, 1H), 7.10 (d, J = 1.8 Hz, 1H), 7.00 (d, J = 2.2 Hz, 1H), 6.82 (d, J = 8.6 Hz, 1H), 6.66 (dd, J = 5.7, 1.8 Hz, 1H), 5.33 (dd, J = 8.9, 2.1 Hz, 1H), 4.38 (dd, J = 11.7, 2.1 Hz, 1H), 4.10 (dd, J = 11.5, 9.1 Hz, 1H), 3.96 (s, 3H), 3.94 (s, 3H). |
| 71 | 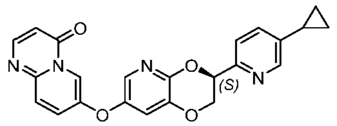 | 415.2 | 1H NMR (400 MHz, DMSO-d6) δ 8.42 (dd, J = 6.1, 2.4 Hz, 2H), 8.27 (dd, J = 6.3, 0.9 Hz, 1H), 7.98 - 7.90 (m, 1H), 7.81 (dd, J = 2.6, 0.9 Hz, 1H), 7.76 (d, J = 9.6 Hz, 1H), 7.50 (dd, J = 8.2, 1.6 Hz, 1H), 7.46 - 7.38 (m, 2H), 6.37 (d, J = 6.2 Hz, 1H), 5.53 (dd, J = 7.1, 2.2 Hz, 1H), 4.56 (dd, J = 11.6, 1.9 Hz, 1H), 4.35 (dd, J = 11.5, 7.3 Hz, 1H), 2.02 - 1.90 (m, 1H), 1.02 - 0.96 (m, 2H), 0.76 - 0.71 (m, 2H). |
| 72 | 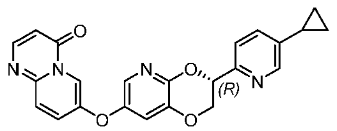 | 415.1 | 1H NMR (400 MHz, DMSO-d6) δ 8.42 (dt, J = 5.6, 2.9 Hz, 2H), 8.27 (dd, J = 6.3, 3.9 Hz, 1H), 7.98 - 7.89 (m, 1H), 7.83 - 7.80 (m, 1H), 7.79 - 7.73 (m, 1H), 7.53 - 7.47 (m, 1H), 7.42 (dt, J = 8.1, 3.7 Hz, 2H), 6.36 (dt, J = 11.6, 5.8 Hz, 1H), 5.57 - 5.45 (m, 1H), 4.62 - 4.49 (m, 1H), 4.41 - 4.29 (m, 1H), 2.02 - 1.86 (m, 1H), 0.99 (ddd, J = 8.1, 6.2, 3.9 Hz, 2H), 0.76 - 0.71 (m, 2H). |
| 73 | 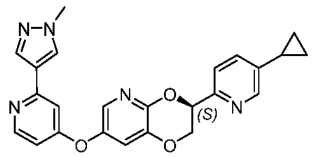 | 428.2 | 1H NMR (400 MHz, DMSO-d6) δ 8.42 (d, J = 2.4 Hz, 1H), 8.34 (dd, J = 5.7, 3.7 Hz, 1H), 8.25 (d, J = 3.6 Hz, 1H), 7.98 - 7.89 (m, 1H), 7.78 - 7.71 (m, 1H), 7.55 - 7.45 (m, 1H), 7.42 (dd, J = 8.1, 3.7 Hz, 1H), 7.37 (d, J = 2.6 Hz, 1H), 7.22 - 7.19 (m, 1H), 6.70 - 6.61 (m, 1H), 5.51 (dd, J = 7.1, 2.9 Hz, 1H), 4.61 - 4.49 (m, 1H), 4.34 (ddd, J = 11.5, 7.5, 3.9 Hz, 1H), 3.83 (d, J = 3.8 Hz, 3H), 2.02 - 1.88 (m, 1H), 1.01 - 0.96 (m, 2H), 0.76 - 0.73 (m, 2H). |
| 74 | 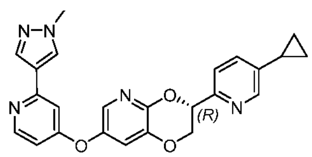 | 428.2 | 1H NMR (400 MHz, DMSO-d6) δ 8.42 (s, 1H), 8.34 (d, J = 5.7 Hz, 1H), 8.25 (s, 1H), 7.95 (s, 1H), 7.75 (dd, J = 2.5, 1.0 Hz, 1H), 7.51 (dd, J = 8.1, 1.8 Hz, 1H), 7.42 (d, J = 8.2 Hz, 1H), 7.38 (dd, J = 2.5, 1.0 Hz, 1H), 7.21 (d, J = 2.1 Hz, 1H), 6.66 (ddd, J = 5.7, 2.4, 0.9 Hz, 1H), 5.51 (dd, J = 7.3, 2.3 Hz, 1H), 4.56 (dd, J = 11.5, 1.9 Hz, 1H), 4.34 (dd, J = 11.5, 7.6 Hz, 1H), 3.81 (d, J = 18.7 Hz, 3H), 1.99 - 1.93 (m, 1H), 0.99 (dd, J = 6.7, 5.3 Hz, 2H), 0.74 (dd, J = 8.0, 2.7 Hz, 2H). |

(continued)

| Compound | Structural formula | MS (M+H)+ | 1HNMR |
|---|---|---|---|
| 75 | 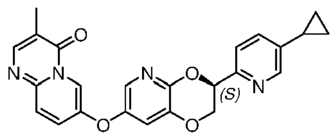 | 429.2 | 1H NMR (400 MHz, DMSO-d6) δ 8.41 (s, 1H), 8.38 (s, 1H), 8.25 (d, J = 0.8 Hz, 1H), 7.81 (ddd, J = 7.3, 5.4, 3.7 Hz, 2H), 7.69 (dd, J = 9.7, 0.8 Hz, 1H), 7.50 (d, J = 8.2 Hz, 1H), 7.41 (dd, J = 5.4, 2.9 Hz, 2H), 5.53 (d, J = 7.0 Hz, 1H), 4.55 (d, J = 11.5 Hz, 1H), 4.35 (dd, J = 11.5, 7.3 Hz, 1H), 2.09 (s, 3H), 2.02 - 1.90 (m, 1H), 1.04 - 0.94 (m, 2H), 0.86 - 0.70 (m, 2H). |
| 76 | 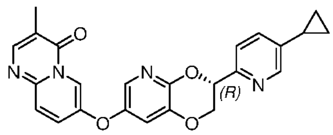 | 429.1 | 1H NMR (400 MHz, DMSO-d6) δ 8.42 (d, J = 2.2 Hz, 1H), 8.39 (d, J = 2.7 Hz, 1H), 8.25 (s, 1H), 7.81 (ddd, J = 6.1, 3.6, 1.5 Hz, 2H), 7.70 (d, J = 9.7 Hz, 1H), 7.50 (dd, J = 8.1, 1.8 Hz, 1H), 7.44 - 7.37 (m, 2H), 5.53 (dd, J = 7.2, 2.3 Hz, 1H), 4.55 (dd, J = 11.6, 2.0 Hz, 1H), 4.42 - 4.31 (m, 1H), 2.09 (s, 3H), 2.02 - 1.90 (m, 1H), 1.01 - 0.96 (m, 2H), 0.75 (dd, J = 5.8, 4.8 Hz, 2H). |
| 77 | 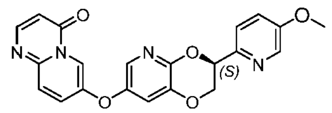 | 405.1 | 1H NMR (400 MHz, DMSO-d6) δ 8.43 (d, J = 2.7 Hz, 1H), 8.30 (d, J = 2.5 Hz, 1H), 8.28 (d, J = 6.3 Hz, 1H), 7.95 (dd, J = 9.6, 2.8 Hz, 1H), 7.82 (d, J = 2.6 Hz, 1H), 7.77 (d, J = 9.6 Hz, 1H), 7.53 - 7.47 (m, 2H), 7.45 (t, J = 4.1 Hz, 1H), 6.37 (d, J = 6.3 Hz, 1H), 5.51 (dd, J = 7.5, 2.4 Hz, 1H), 4.55 (dd, J = 11.6, 2.5 Hz, 1H), 4.35 (dd, J = 11.6, 7.6 Hz, 1H), 3.83 (s, 3H). |
| 78 | 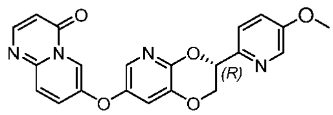 | 405.1 | 1H NMR (400 MHz, DMSO-d6) δ 8.43 (d, J = 2.7 Hz, 1H), 8.30 (d, J = 2.7 Hz, 1H), 8.28 (dd, J = 6.3, 0.8 Hz, 1H), 7.99 - 7.91 (m, 1H), 7.82 (dd, J = 2.6, 0.8 Hz, 1H), 7.77 (d, J = 9.6 Hz, 1H), 7.53 - 7.47 (m, 2H), 7.45 (dd, J = 2.6, 0.8 Hz, 1H), 6.37 (d, J = 6.2 Hz, 1H), 5.51 (dd, J = 7.5, 2.3 Hz, 1H), 4.55 (dd, J = 11.8, 2.3 Hz, 1H), 4.35 (dd, J = 11.5, 7.6 Hz, 1H), 3.83 (d, J = 0.7 Hz, 3H). |
| 79 | 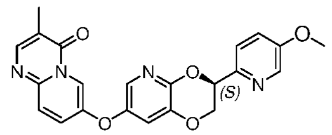 | 419.1 | 1H NMR (400 MHz, DMSO-d6) δ 8.39 (d, J = 2.7 Hz, 1H), 8.30 (d, J = 2.6 Hz, 1H), 8.26 (d, J = 0.7 Hz, 1H), 7.84 (dd, J = 9.8, 2.5 Hz, 1H), 7.80 (dd, J = 2.6, 0.8 Hz, 1H), 7.70 (d, J = 9.7 Hz, 1H), 7.49 (dd, J = 6.7, 4.0 Hz, 2H), 7.43 (dd, J = 2.6, 0.8 Hz, 1H), 5.51 (dd, J = 7.5, 2.4 Hz, 1H), 4.55 (dd, J = 11.6, 2.3 Hz, 1H), 4.35 (dd, J = 11.5, 7.5 Hz, 1H), 3.83 (d, J = 0.7 Hz, 3H), 2.09 (s, 3H). |
| 80 | 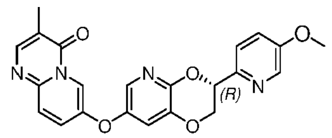 | 419.1 | 1H NMR (400 MHz, DMSO-d6) δ 8.39 (d, J = 2.7 Hz, 1H), 8.32 - 8.30 (m, 1H), 8.27 - 8.25 (m, 1H), 7.84 (dd, J = 9.6, 2.7 Hz, 1H), 7.80 (dd, J = 2.6, 0.7 Hz, 1H), 7.70 (d, J = 9.7 Hz, 1H), 7.49 (dd, J = 7.2, 4.0 Hz, 2H), 7.43 (dd, J = 2.6, 0.7 Hz, 1H), 5.51 (dd, J = 7.5, 2.4 Hz, 1H), 4.55 (dd, J = 11.6, 2.4 Hz, 1H), 4.35 (dd, J = 11.5, 7.6 Hz, 1H), 3.83 (d, J = 0.6 Hz, 3H), 2.09 (s, 3H). |
| 81 | 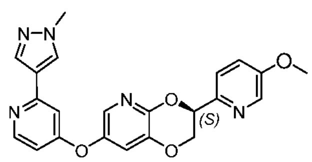 | 418.1 | 1H NMR (400 MHz, DMSO-d6) δ 8.35 (d, J = 5.7 Hz, 1H), 8.32 (d, J = 2.7 Hz, 1H), 8.25 (s, 1H), 7.96 (s, 1H), 7.75 (dd, J = 2.6, 0.6 Hz, 1H), 7.53 - 7.47 (m, 2H), 7.39 (dd, J = 2.5, 0.5 Hz, 1H), 7.21 (d, J = 2.4 Hz, 1H), 6.66 (dd, J = 5.7, 2.4 Hz, 1H), 5.50 (dd, J = 7.6, 2.5 Hz, 1H), 4.56 (dd, J = 11.6, 2.5 Hz, 1H), 4.34 (dd, J = 11.5, 7.7 Hz, 1H), 3.83 (d, J = 1.1 Hz, 6H). |

(continued)

| Compound | Structural formula | MS (M+H)+ | 1HNMR |
|---|---|---|---|
| 82 | 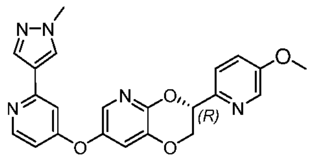 | 418.1 | 1H NMR (400 MHz, DMSO-d6) δ 8.35 (d, J = 5.7 Hz, 1H), 8.32 (d, J = 2.7 Hz, 1H), 8.25 (s, 1H), 7.96 (s, 1H), 7.75 (dd, J = 2.6, 0.7 Hz, 1H), 7.53 - 7.47 (m, 2H), 7.39 (dd, J = 2.6, 0.7 Hz, 1H), 7.22 (d, J = 2.4 Hz, 1H), 6.70 - 6.63 (m, 1H), 5.50 (dd, J = 7.6, 2.5 Hz, 1H), 4.56 (dd, J = 11.5, 2.4 Hz, 1H), 4.34 (dd, J = 11.6, 7.7 Hz, 1H), 3.84 - 3.83 (m, 6H). |

The enantiomers in the table are subjected to chiral HPLC under the following conditions (flow rate: 18 mL/minute; detector: UV 254 nm):

| Compound | Column | Mobile phase (0.1% ammonia water) | RT/minute | ee% |
|---|---|---|---|---|
| 57 | IC (2 × 25 cm) | Acetonitrile/ethanol = 5 : 95 | 15.859 | 98% |
| 58 | | | 18.723 | 95% |
| 59 | IC (2 × 25 cm) | Acetonitrile/ethanol = 20 : 80 | 17.615 | 100% |
| 60 | | | 21.996 | 97% |
| 61 | IC (2 × 25 cm) | Acetonitrile/ethanol = 20 : 80 | 18.204 | 100% |
| 62 | | | 21.943 | 95% |
| 63 | IC (2 × 25 cm) | Acetonitrile/ethanol = 20 : 80 | 9.330 | 100% |
| 64 | | | 17.490 | 96% |
| 65 | IC (2 × 25 cm) | Acetonitrile/ethanol = 20 : 80 | 15.850 | 100% |
| 66 | | | 16.710 | 100% |
| 67 | IC (2 × 25 cm) | Acetonitrile/ethanol = 20 : 80 | 8.390 | 100% |
| 68 | | | 9.030 | 99% |
| 69 | IC (2 × 25 cm) | Acetonitrile/ethanol = 10 : 90 | 11.800 | 100% |
| 70 | ODH (2 × 25 cm) | Acetonitrile/ethanol = 10 : 90 | 8.700 | 89% |
| 71 | IC (2 × 25 cm) | Acetonitrile/ethanol = 10 : 90 | 20.463 | 100% |
| 72 | | | 10.654 | 100% |
| 73 | IC (2 × 25 cm) | Acetonitrile/ethanol = 10 : 90 | 7.733 | 100% |
| 74 | | | 6.241 | 100% |
| 75 | IC (2 × 25 cm) | Acetonitrile/ethanol = 10 : 90 | 26.062 | 100% |
| 76 | | | 9.441 | 100% |
| 77 | IC (2 × 25 cm) | Acetonitrile/ethanol = 10 : 90 | 22.844 | 100% |
| 78 | | | 11.352 | 100% |
| 79 | IC (2 × 25 cm) | Acetonitrile/ethanol = 10 : 90 | 22.830 | 100% |
| 80 | | | 8.636 | 100% |
| 81 | IC (2 × 25 cm) | Acetonitrile/ethanol = 10 : 90 | 14.035 | 100% |
| 82 | | | 10.213 | 100% |

**Compound 83**

**(S)-7-((3-(6-methoxypyridin-3-yl)-2,3-dihydro-[1,4]dioxino[2,3-b]pyridin-7-yl)oxy)-4*H*-pyrido[1,2-a]pyrimidin-4-one**

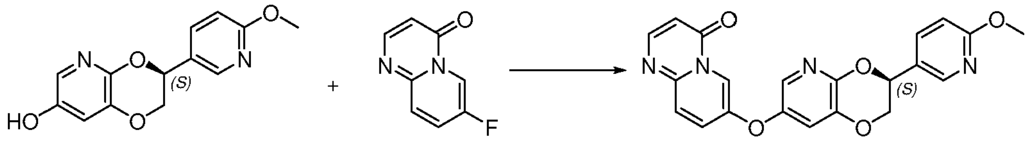

(S)-3-(6-methoxypyridin-3-yl)-2,3-dihydro-[1,4]dioxino[2,3-b]pyridin-7-ol (60 mg, 0.23 mmol), 7-fluoro-4*H*-pyrido[1,2-a]pyrimidin-4-one (45 mg, 0.28 mmol), cesium carbonate (112 mg, 0.35 mmol) and anhydrous DMF (5 ml) were added to a reaction flask; and under nitrogen atmosphere, the mixture was heated to 90°C and stirred for 4 hours. After cooled to room temperature, the reaction solution was purified with flash column chromatography (eluted with a gradient of water (0.1% formic acid):acetonitrile = 100 : 0-0 : 100) and then with flash column chromatography (eluted with a gradient of dichloromethane:methanol = 100:0-90:10), and then subjected to a recrystallization with ethanol (30 ml) to obtain 50 mg of the product as a white solid. MS (m/z): 405.1 $[M+H]^+$.

$^1$H NMR (400 MHz, $CDCl_3$) δ 8.63 (s, J = 2.5 Hz, 1H), 8.28 (d, J = 5.4 Hz, 1H), 8.26 (d, J = 2.2 Hz, 1H), 7.77 (d, J = 1.6 Hz, 1H), 7.73 - 7.65 (m, 3H), 7.10 (d, J = 1.6 Hz, 1H), 6.83 (d, J = 8.6 Hz, 1H), 6.44 (d, J = 5.9 Hz, 1H), 5.32 (dd, J = 9.2, 7.1 Hz, 1H), 4.38 (dd, J = 11.7, 1.7 Hz, 1H), 4.11 (dd, J = 10.9, 9.0 Hz, 1H), 3.96 (s, J = 0.9 Hz, 3H).

The following compounds were prepared according to the preparation procedure of compound 83 using corresponding intermediates and reagents under appropriate conditions that will be recognized by one skilled in the art.

| Compound | Structural formula | MS $(M+H)^+$ | $^1$HNMR |
|---|---|---|---|
| 84 | 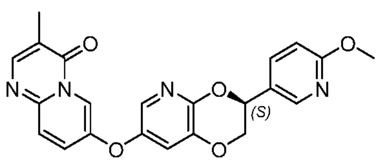 | 419.1 | 1H NMR (400 MHz, $CDCl_3$) δ 8.60 (s, J = 2.4 Hz, 1H), 8.25 (s, J = 2.3 Hz, 1H), 8.22 (s, 1H), 7.76 (d, J = 1.4 Hz, 1H), 7.69 (dd, J = 8.6, 1.7 Hz, 1H), 7.65 (d, J = 9.7 Hz, 1H), 7.57 (dd, J = 9.7, 1.7 Hz, 1H), 7.09 (d, J = 1.4 Hz, 1H), 6.83 (d, J = 8.6 Hz, 1H), 5.32 (dd, J = 8.9, 1.8 Hz, 1H), 4.37 (dd, J = 11.7, 1.4 Hz, 1H), 4.10 (dd, J = 11.1, 9.5 Hz, 1H), 3.96 (s, J = 1.2 Hz, 3H), 2.25 (s, 3H). |
| 85 | 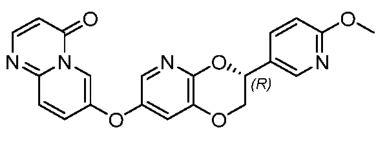 | 405.1 | 1H NMR (400 MHz, $CDCl_3$) δ 8.63 (d, J = 2.5 Hz, 1H), 8.28 (d, J = 6.3 Hz, 1H), 8.26 (d, J = 2.1 Hz, 1H), 7.77 (d, J = 2.6 Hz, 1H), 7.74 - 7.65 (m, 3H), 7.10 (d, J = 2.6 Hz, 1H), 6.83 (d, J = 8.6 Hz, 1H), 6.44 (d, J = 6.3 Hz, 1H), 5.33 (dd, J = 8.9, 2.3 Hz, 1H), 4.38 (dd, J = 11.7, 1.8 Hz, 1H), 4.11 (dd, J = 11.1, 9.0 Hz, 1H), 3.96 (s, J = 0.8 Hz, 3H). |
| 86 | 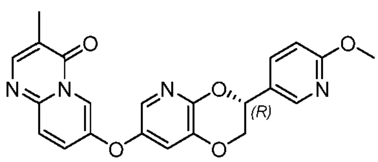 | 419.1 | 1H NMR (400 MHz, $CDCl_3$) δ 8.60 (d, J = 2.6 Hz, 1H), 8.25 (d, J = 2.3 Hz, 1H), 8.22 (s, 1H), 7.76 (d, J = 2.6 Hz, 1H), 7.69 (dd, J = 8.6, 2.5 Hz, 1H), 7.65 (d, J = 9.6 Hz, 1H), 7.57 (dd, J = 9.7, 2.6 Hz, 1H), 7.09 (d, J = 2.6 Hz, 1H), 6.83 (d, J = 8.6 Hz, 1H), 5.32 (dd, J = 9.0, 2.3 Hz, 1H), 4.37 (dd, J = 11.7, 2.5 Hz, 1H), 4.10 (dd, J = 11.7, 9.0 Hz, 1H), 3.96 (s, 3H), 2.25 (s, 3H). |

**Example 3**

**Measurement of CSF1R kinase activity at molecular level**

**1. Reagents and materials:**

Z-LYTE™ Tyr 1 peptide substrate: Invitrogen, PV3190;

Z-LYTE™ Tyr 1 phosphorylated peptide substrate: Invitrogen, PV3258;

5X kinase buffer: Invitrogen, PV3189;

10 mM ATP: Invitrogen, PV3227;

Development reagent B: Invitrogen, PV3295;
Development buffer: Invitrogen, P3127;
Stopping solution: Invitrogen, P3094;
Recombinant human CSF1R kinase: Invitrogen, PR4598A;
384 well plate, black: Corning, 3575;
Envision: Perkin Elmer.

**2. Preparation of reaction solutions**

1) 1.33X kinase buffer: 5X kinase buffer was diluted to a 1.33X kinase buffer with $ddH_2O$.
2) Dilution of a 4X test compound: The test compound was diluted in gradient to 4-fold the reaction concentration and the concentration of DMSO was kept at 8%. The final concentration of the compound in reaction was: 1, 0.33, 0.11, 0.037, 0.012, 0.004, 0.0014, 0.00046 μM, and the final concentration of DMSO was 2%.
3) A mixture of kinase/peptide substrate: In 1.33X kinase buffer, the kinase and Z-LYTE™ Tyr 1 peptide substrate were diluted to 0.12 μg/mL and 4 μM, respectively, to prepare a mixture of kinase/peptide substrate. The mixture was mixed gently with a pipette.
4) A phosphorylated peptide substrate solution (PP solution): 0.4 μL of Z-LYTE™ Tyr1 phosphorylated peptide substrate was added to 99.6 μL of 1.33X kinase buffer.
5) ATP solution: 10 mM of ATP was diluted to 760 μM with 1.33X kinase buffer to prepare ATP solution.
6) Development solution: Development reagent B was diluted with Development buffer at a ratio of 1 : 200.

**3. Methods**

1) Kinase reaction (10 μL system)
2.5 μL of 4X test compound was added to each reaction well of the 384 plate, and the corresponding volume of 8% DMSO was added to the control well. The plate was placed on ice. 5 μL of a mixture of kinase/peptide substrate, 2.5 μL of kinase buffer and ATP solution were successively added to each well. Three control groups were set up: Group C1 contains only kinase buffer, group C2 contains the mixture of kinase/peptide substrate, kinase buffer and ATP, and group C3 contains 5 μL of PP solution. After adding the reaction components, the 384-well plate was sealed and incubated at 25-30 °C for 1 h in dark.
2) Development reaction
5 μL of Development solution was added to each well, sealed and incubated at 25-30°C for another 1 h in dark.
3) Reaction stopping and plate reading
5 μL of stopping solution was added to each well. The Coumarin value (excitation at 400 nm, emission at 445 nm) and Fluorescein value (excitation at 400 nm, emission at 520 nm) were measured, respectively.

**4. Data Analysis**

% phosphorylation rate = 100%-100% × [ER × C3 520 nm - C3 445 nm]/[(C1 445 nm - C3 445 nm) + ER × (C3 520 nm - C1 520 nm)]

wherein

ER (emission ratio): Coumarin emission read-out (445 nm)/Fluorescein emission read-out (520 nm);
C3 445nm: 100% phosphorylated Coumarin emission read-out;
C3 520nm: 100% phosphorylated Fluorescein emission read-out;
C1 445nm: 0% phosphorylated Coumarin emission read-out;
C1 520nm: 0% phosphorylated Fluorescein emission read-out.

Inhibition rate %(IR) = [1- % phosphorylation $rate_{test\ sample}$/100% phosphorylation $rate_{control}$] × 100%

wherein

% phosphorylation $rate_{test\ sample}$: phosphorylation rate of the test compound;
100% phosphorylation $rate_{control}$: phosphorylation rate of C3 control group.

**5. $IC_{50}$ values:** calculated by using software XL-Fit™ (version 5.3) supplied by ID Business Solutions (Guildford, UK), which is an additional software to Microsoft Excel.

**6. Test results**

| Compound No. | $IC_{50}$ (μM) | Compound No. | $IC_{50}$ (μM) | Compound No. | $IC_{50}$ (μM) |
|---|---|---|---|---|---|
| 1 | 0.01 | 17 | 0.015 | 33 | 0.150 |
| 2 | 0.11 | 18 | 0.033 | 34 | 0.499 |
| 3 | 0.081 | 19 | 0.005 | 35 | 0.006 |
| 4 | 0.129 | 20 | 0.024 | 36 | 0.021 |
| 5 | 0.134 | 21 | 0.013 | 37 | 0.033 |
| 6 | 0.007 | 22 | 0.012 | 38 | 0.024 |
| 7 | 0.015 | 23 | 0.005 | 39 | 0.015 |
| 8 | 0.067 | 24 | 0.003 | 40 | 0.033 |
| 9 | 0.063 | 25 | 0.008 | 41 | 0.023 |
| 10 | 0.044 | 26 | 0.006 | 42 | 0.013 |
| 11 | 0.061 | 27 | 0.016 | 43 | 0.004 |
| 12 | 0.013 | 28 | 0.070 | 44 | 0.004 |
| 13 | 0.008 | 29 | 0.006 | 45 | 0.036 |
| 14 | 0.028 | 30 | 0.460 | 46 | 0.028 |
| 15 | 0.010 | 31 | 0.006 | 47 | 0.013 |
| 16 | 0.022 | 32 | 0.003 | 48 | 0.005 |
| 49 | 0.023 | 62 | 0.004 | 75 | 0.015 |
| 50 | 0.073 | 63 | 0.005 | 76 | 0.008 |
| 51 | 0.008 | 64 | 0.010 | 77 | 0.080 |
| 52 | 0.019 | 65 | 0.005 | 78 | 0.026 |
| 53 | 0.003 | 66 | 0.010 | 79 | 0.030 |
| 54 | 0.002 | 67 | 0.005 | 80 | 0.013 |
| 55 | 0.008 | 68 | 0.006 | 81 | 0.010 |

(continued)

| Compound No. | $IC_{50}$ (μM) | Compound No. | $IC_{50}$ (μM) | Compound No. | $IC_{50}$ (μM) |
|---|---|---|---|---|---|
| 56 | 0.012 | 69 | 0.003 | 82 | 0.011 |
| 57 | 0.015 | 70 | 0.008 | 83 | 0.007 |
| 58 | 0.012 | 71 | 0.030 | 84 | 0.008 |
| 59 | 0.005 | 72 | 0.014 | 85 | 0.017 |
| 60 | 0.006 | 73 | 0.006 | 86 | 0.008 |
| 61 | 0.003 | 74 | 0.005 | | |

**Example 4**

**Detection of CSF1R phosphorylation activity at cell level**

**1. Cell line**

THP-1 (ATCC), human acute monocytic leukemia cells. The cells were cultured in an RPMI 1640 medium containing 10% fetal bovine serum (FBS).

**2. Reagents and instruments**

- Human phosphorylation-CSF1R ELISA kit: R&D, #DYC3268-2;
- RPMI 1640 culture solution: GIBCO, #10491;
- Human M-CSF recombinant cytokine: R&D, #216-MC-500;
- Cell lysis buffer: Cell Signal, #9803S;
- 1XPBS buffer (1L): 8.0 g of NaCl, 0.2 g of KCl, 3.58 g of $Na_2HPO_4$-$12H_2O$, 0.24 g of $KH_2PO_4$ were dissolved in 1L of dd $H_2O$, and the pH was adjusted to 7.4;
- Blocking solution: PBS buffer containing 1% bovine serum albumin (BSA);
- PBST washing liquid: PBS buffer containing 0.05% Tween-20;
- Chromogenic substrate: R&D, #DY999;
- 2N $H_2SO_4$;
- Microwell plate reader: Labsystems Multiskan K3: Thermo; Envision: Perkin Elmer;
- ELISA plate: Corning, #9018;
- Cell culture plate: Facol, #353027.

**3. Treatment of cells and preparation of lysis buffer**

THP-1 cells were resuspended in RPMI-1640 culture solution containing 2% FBS, and the culture was added to a 96-well plate at a density of $5 \times 10^4$/well, 50 μL/well, and cultured in a cell incubator at 5% $CO_2$ and 37°C overnight; The test compound was diluted with a serum-free RPMI-1640 medium to 3, 1.1, 0.37, 0.12, 0.04, 0.014, 0.005 and 0.002 μM, and the concentration of DMSO was 5%. 5 μL of the diluted compound was added to the 50 μL cell culture system, the culture was cultured in a 5% $CO_2$, 37°C cell incubator for 60 min, 300 ng/mL of M-CSF was added to the cells, and the mixture was stimulated in the 37°C cell incubator for 1 min, added 50 μL of cell lysis buffer, and stored in a refrigerator at -80°C.

**4. ELISA detection steps**

100 μL/well of the p-CSF1R capture antibody diluted to 0.8 μg/mL with PBS was added to the ELISA plate, and the plate was coated on a shaker at room temperature overnight. The culture was washed with PBST, and then added blocking solution and incubated at room temperature for 2 h. The culture was washed with PBST, added 90 μL of cell lysis buffer, and incubated on a shaker at 25°C for 2 h. The plate was washed with PBST three times, added 100 μL of anti-p-tyrosine-HRP detection antibody diluted with 0.1% PBS-BSA diluent, and incubated on a shaker at 25°C for 2 h. The plate was washed with PBST washing liquid, added 100 μL of chromogenic substrate and incubated at room temperature for 10-20 min. The reaction was stopped by adding 50 μL of 2N $H_2SO_4$. The optical density signal (450/570 nm) of each well

was detected in Labsystems Multiskan K3 or Envision.

**5. Data Analysis**

$$\text{Inhibition rate (\%)} = 100\ \% - \frac{\text{Drug-treated well read-out - background read-out}}{\text{Cell well read-out - background read-out}} \times 100\%$$

wherein

- Drug-treated well read-out: indicating the optical density signal of a cell well with the test compound treatment.
- Background read-out: indicating the optical density signal of a well without cells but with cell lysis buffer.
- Cell well read-out: indicating the optical density signal of a cell well not treated with the compound.

**6. Calculation of $IC_{50}$:** obtained by using XL-Fit 5.3 software.

**7. Test results**

| Compound No. | $IC_{50}$ (μM) | Compound No. | $IC_{50}$ (μM) | Compound No. | $IC_{50}$ (μM) |
|---|---|---|---|---|---|
| 1 | 0.052 | 20 | 0.06 | 40 | 0.19 |
| 2 | 0.626 | 21 | 0.008 | 41 | 0.551 |
| 6 | 0.004 | 22 | 0.009 | 42 | 0.078 |
| 7 | 0.023 | 23 | 0.129 | 43 | 0.054 |
| 8 | 0.029 | 24 | 0.034 | 44 | 0.019 |
| 9 | 0.006 | 25 | 0.004 | 45 | 0.061 |
| 10 | 0.080 | 26 | 0.108 | 46 | 0.005 |
| 11 | 0.164 | 27 | 0.045 | 47 | 0.012 |
| 12 | 0.005 | 29 | 0.023 | 48 | 0.005 |
| 13 | 0.017 | 31 | 0.010 | 49 | 0.060 |
| 14 | 0.048 | 32 | 0.004 | 50 | 0.197 |
| 15 | 0.015 | 35 | 0.012 | 51 | 0.006 |
| 16 | 0.005 | 36 | 0.056 | 52 | 0.034 |
| 17 | 0.018 | 37 | 0.140 | 53 | 0.019 |
| 18 | 0.005 | 38 | 0.381 | 54 | 0.022 |
| 19 | 0.031 | 39 | 0.300 | 55 | 0.007 |
| 56 | 0.014 | 66 | 0.056 | 76 | 0.018 |
| 57 | 0.016 | 67 | 0.016 | 78 | 0.064 |
| 58 | 0.026 | 68 | 0.021 | 79 | 0.182 |
| 59 | 0.050 | 69 | 0.017 | 80 | 0.022 |
| 60 | 0.052 | 70 | 0.015 | 81 | 0.032 |
| 61 | 0.029 | 71 | 0.114 | 82 | 0.013 |
| 62 | 0.047 | 72 | 0.032 | 83 | 0.138 |

(continued)

| Compound No. | $IC_{50}$ (μM) | Compound No. | $IC_{50}$ (μM) | Compound No. | $IC_{50}$ (μM) |
|---|---|---|---|---|---|
| 63 | 0.018 | 73 | 0.017 | 84 | 0.084 |
| 64 | 0.042 | 74 | 0.009 | 85 | 0.154 |
| 65 | 0.010 | 75 | 0.117 | 86 | 0.056 |

**Example 5**

**Macrophage Cell Viability Assay**

**1. Reagents and instruments**

Ficoll-Paque PLUS: GE Healthcare, #17-1440-02;
Monocyte Isolation Kit II, human: Miltenyi, #130-091-153;
RoboSep buffer: Stemcell technologies, #20104;
LS Columns: Miltenyi Biotec, #130-042-401;
rh M-CSF: R&D systems, #216-MC-025;
rm M-CSF: R&D systems, #416-ML-050;
0.25% Trypsin-EDTA: GIBCO, #25200-072;
penicillin/streptomycin: GIBCO, #15140122;
CellTiter-Glo® 2.0: promega, #G9243;
Biocoat Poly-D-Lysine 96 Well plate: Corning, #356692;
Envision: PerkinElmer.

**2. Assay procedure**

1) Human monocyte derived macrophage (hMDM) cell isolation

- Peripheral blood was collected from healthy volunteers and diluted with PBS of equal volume.
- 15 mL of ficoll was added to a 50 mL centrifuge tube followed by addition of 20 mL diluted blood, centrifuged for 30 min at 400 g.
- The mononuclear cells at the interface were collected and diluted with 4-fold volume of PBS, centrifuged for 10 min at 400 g.
- Cells were washed with 50 mL PBS and centrifuged for 10 min at 200 g twice to remove platelets.
- Monocytes were isolated with Monocyte Isolation Kit II, human according to manufacturer's instructions.
- Monocytes were washed once with PBS (centrifuged for 5 min at 400 g), and resuspended in RPMI 1640, supplemented with 10% fetal bovine serum (FBS), penicillin/streptomycin.
- 100 ng/mL rh M-CSF (final concentration) was added to cells and incubated at 37°C, 5%$CO_2$ for 6 days; medium was changed every two days.

2) Mouse bone marrow derived macrophage (mBMDM) cell isolation

- C57BL/6j mice were cleaned using 75% ethanol.
- Femur and tibia were harvested and removed all muscle tissue from the bones.
- Bone marrow cells were flushed into the 1.5 mL tube with 1 mL medium using a 5 mL syringe.
- Fresh cells were resuspended and washed with PBS.
- 10 ng/mL rm M-CSF (final concentration) was added to cells and incubated at 37°C, 5%$CO_2$ for 6 days; medium was changed every two days.

3) CellTiter-Glo

- hMDM or mBMDM cells were harvested with 0.25% Trypsin-EDTA.

- 100 μL hMDM or mBMDM cell suspension were seeded in the 96 well plate at 5000 cell/well supplemented with rh M-CSF (100 ng/mL) or rm M-CSF (10 ng/mL) respectively. Cells without rh M-CSF or rm M-CSF as background control.
- 10 μL serial diluted compounds or 0.1% DMSO medium (as control) were added in the plate, incubated at 37°C, 5%$CO_2$ for 4 days.
- Cells were treated with 50 μL CellTiter-Glo reagent on an orbital shaker for 10 min.
- Read the luminescence signal of the plates on Envision.

**3. Data Analysis**

$$\text{Inhibition rate (\%)} = 100\% - \frac{\text{Compound}_{\text{lum}} - \text{Background}_{\text{lum}}}{\text{Cell}_{\text{lum}} - \text{Background}_{\text{lum}}} \times 100\%$$

- Compound $_{lum}$: luminescence signal of compound treatment;
- Cell $_{lum}$: luminescence signal of 0.1% DMSO treatment;
- Background $_{lum}$: luminescence signal of 0.1% DMSO treatment without rh M-CSF/ rm M-CSF.

**4. $IC_{50}$ calculation**

The $IC_{50}$ values were calculated by XL-Fit5.3, using the fitting model #205.

**5. Results**

According to the assay described above, the compounds of the present invention showed good potency in inhibiting human monocyte derived macrophage cell or mouse bone marrow derived macrophage cell.

## Claims

**1.** A compound of formula (I):

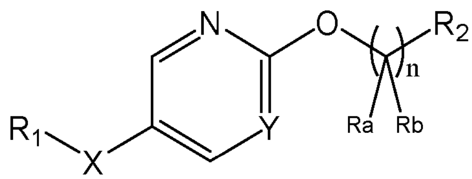

(I)

or a pharmaceutically acceptable salt thereof, or a solvate, a racemic mixture, an enantiomer, a diastereomer or a tautomer thereof, wherein

$R_1$ is chosen from:

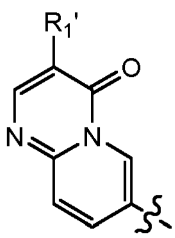,
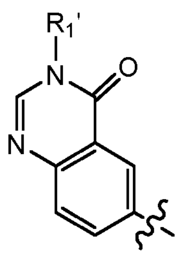,
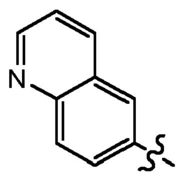,
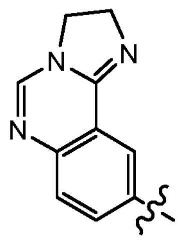

and

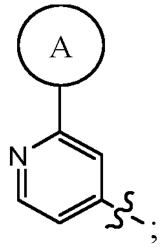

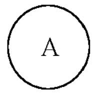

is chosen from phenyl and 5-6 membered heteroaryl, each of which is optionally substituted with one or more groups independently chosen from: -CN, halogen, $C_{1-6}$ alkyl, -O($C_{1-6}$ alkyl), $C_{1-6}$ haloalkyl, -O($C_{1-6}$ haloalkyl), -$C_{1-6}$ alkylene-CN, and -$C_{1-6}$ alkylene-OH;

$R_1$' is chosen from H, halogen, -CN, $C_{1-6}$ alkyl, $C_{1-6}$ haloalkyl, -$C_{1-6}$ alkylene-CN, - $C_{1-6}$ alkylene-OH, -O($C_{1-6}$ alkyl), -O($C_{1-6}$ haloalkyl), $C_{3-8}$ cycloalkyl, 4-8 membered heterocyclyl, and $NR_4R_5$; $R_4$ and $R_5$ are each independently chosen from H, halogen, - CN, $C_{1-6}$ alkyl, $C_{1-6}$ haloalkyl, -$C_{1-6}$ alkylene-CN, -$C_{1-6}$ alkylene-OH, and -O($C_{1-6}$ alkyl); or $R_4$ and $R_5$ together with the N atom to which they are attached form a 4-8 membered heterocyclic ring;

X is O or $CR_6R_7$; $R_6$ and $R_7$ are each independently chosen from H, halogen, $C_{1-6}$ alkyl, $C_{1-6}$ haloalkyl, -$C_{1-6}$ alkylene-CN, -$C_{1-6}$ alkylene-OH, -O($C_{1-6}$ alkyl) and -O($C_{1-6}$ haloalkyl);

Y is N or $CR_3$; $R_3$ is chosen from H, -CN, halogen, $C_{1-6}$ alkyl, -O($C_{1-6}$ alkyl), -$C_{1-6}$ alkylene-CN, -$C_{1-6}$ alkylene-OH, $C_{1-6}$ haloalkyl, and -O($C_{1-6}$ haloalkyl);

$R_a$ and $R_b$ are each independently chosen from H, halogen, -CN, $C_{1-6}$ alkyl, $C_{1-6}$ haloalkyl, -$C_{1-6}$ alkylene-CN, -$C_{1-6}$ alkylene-OH, -O($C_{1-6}$ alkyl) and -O($C_{1-6}$ haloalkyl);

n is 0, 1, 2, 3, or 4;

$R_2$ is phenyl or 5-10 membered heteroaryl, each of which is optionally substituted with one or more groups independently chosen from: -CN, halogen, $C_{1-6}$ alkyl, $C_{2-6}$ alkenyl, $C_{2-6}$ alkynyl, -O($C_{1-6}$ alkyl), $C_{1-6}$ haloalkyl, -O($C_{1-6}$ haloalkyl), -$C_{1-6}$ alkylene-CN, -$C_{1-6}$ alkylene-OH, $C_{3-8}$ cycloalkyl, 4-8 membered heterocyclyl, and 5-6 membered heteroaryl, wherein the $C_{3-8}$ cycloalkyl, 4-8 membered heterocyclyl, or 5-6 membered heteroaryl, as a substituent of $R_2$, is each optionally substituted with one or more groups independently chosen from: -CN, halogen, $C_{1-6}$ alkyl, -O($C_{1-6}$ alkyl), $C_{1-6}$ haloalkyl, -O($C_{1-6}$ haloalkyl), -$C_{1-6}$ alkylene-CN, and -$C_{1-6}$ alkylene-OH; or, when Y is $CR_3$ and n is not 0, $R_3$ and one $R_a$ together with the carbon atoms to which they are attached and the atom(s) among the carbon atoms form a 4-8 membered heterocyclic ring.

**2.** The compound, or the pharmaceutically acceptable salt thereof, or the solvate, the racemic mixture, the enantiomer, the diastereomer or the tautomer thereof according to claim 1, wherein $R_1$ is chosen from:

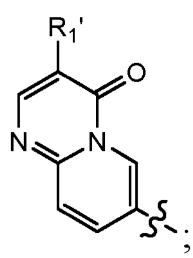

$R_1$' is chosen from H, $C_{1-6}$ alkyl, $C_{3-8}$ cycloalkyl, and $NR_4R_5$; $R_4$ and $R_5$ are both H; or $R_4$ and $R_5$ together with the N atom to which they are attached form a 4-8 membered heterocyclic ring; preferably, $R_1$' is chosen from H and $C_{1-6}$ alkyl;

or

$R_1$ is chosen from

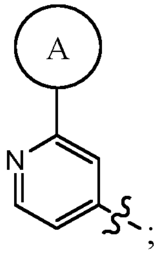

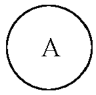

is 5-6 membered heteroaryl, which is optionally substituted with one or more groups independently chosen from: $C_{1-6}$ alkyl and -$C_{1-6}$ alkylene-OH; preferably,

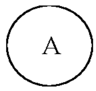

is pyrazolyl, which is optionally substituted with one or more groups independently chosen from: $C_{1-6}$ alkyl and -$C_{1-6}$ alkylene-OH; more preferably,

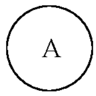

is pyrazolyl, which is optionally substituted with one or more groups independently chosen from: $C_{1-6}$ alkyl.

3. The compound, or the pharmaceutically acceptable salt thereof, or the solvate, the racemic mixture, the enantiomer, the diastereomer or the tautomer thereof according to any one of claims 1-2, wherein X is O or $CH_2$; and preferably, X is O.

4. The compound, or the pharmaceutically acceptable salt thereof, or the solvate, the racemic mixture, the enantiomer, the diastereomer or the tautomer thereof according to any one of claims 1-3, wherein Y is N or $CR_3$; and $R_3$ is chosen from H, -CN, halogen, $C_{1-6}$ alkyl, -O($C_{1-6}$ alkyl), and -O($C_{1-6}$ haloalkyl); e.g., Y is $CR_3$, and $R_3$ is chosen from H, -CN, halogen, $C_{1-6}$ alkyl, -O($C_{1-6}$ alkyl), or -O($C_{1-6}$ haloalkyl); preferably, $R_3$ is -O($C_{1-6}$ alkyl); and more preferably, $R_3$ is -O($C_{1-3}$ alkyl).

5. The compound, or the pharmaceutically acceptable salt thereof, or the solvate, the racemic mixture, the enantiomer, the diastereomer or the tautomer thereof according to any one of claims 1-4, wherein $R_a$ and $R_b$ are both H, and n is 0, 1 or 2; and preferably, $R_a$ and $R_b$ are both H, and n is 1.

6. The compound, or the pharmaceutically acceptable salt thereof, or the solvate, the racemic mixture, the enantiomer, the diastereomer or the tautomer thereof according to any one of claims 1-5, wherein $R_2$ is phenyl or 5-6 membered heteroaryl, each of which is optionally substituted with one or more groups independently chosen from: halogen, $C_{1-6}$ alkyl, $C_{2-6}$ alkynyl, -O($C_{1-6}$ alkyl), -$C_{1-6}$ alkylene-CN, -$C_{1-6}$ alkylene-OH, $C_{3-8}$ cycloalkyl, and 5-6 membered heteroaryl, wherein the $C_{3-8}$ cycloalkyl or 5-6 membered heteroaryl, as a substituent of $R_2$, is each optionally substituted with one or more halogen; and preferably, $R_2$ is phenyl or pyridyl, each of which is optionally substituted with one or more groups independently chosen from: halogen, $C_{1-6}$ alkyl, -O($C_{1-6}$ alkyl), and $C_{3-8}$ cycloalkyl, wherein the $C_{3-8}$ cycloalkyl, as a substituent of $R_2$, is optionally substituted with one or more halogen.

7. The compound, or the pharmaceutically acceptable salt thereof, or the solvate, the racemic mixture, the enantiomer, the diastereomer or the tautomer thereof according to claim 1, wherein the compound has a structure of formula (I-1a):

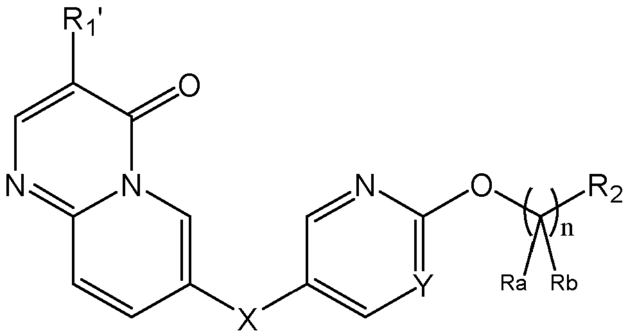

(I-1a)

wherein

$R_1$' is chosen from H, $C_{1-6}$ alkyl, $C_{3-8}$ cycloalkyl, and $NR_4R_5$; $R_4$ and $R_5$ are both H; or $R_4$ and $R_5$ together with the N atom to which they are attached form a 4-8 membered heterocyclic ring; preferably form a 5 or 6 membered heterocyclic ring which, in addition to the N atom to which $R_4$ and $R_5$ are attached, contains 0, 1, or 2 heteroatoms independently chosen from N, O or S, and more preferably form a morpholine ring;
X is O or $CH_2$;
Y is N or $CR_3$; $R_3$ is chosen from H, -CN, halogen, $C_{1-6}$ alkyl, -O($C_{1-6}$ alkyl), and - O($C_{1-6}$ haloalkyl);
$R_a$ and $R_b$ are both H;
n is 0, 1 or 2; and
$R_2$ is phenyl, which is optionally substituted with one or more groups independently chosen from: halogen, $C_{1-6}$ alkyl, $C_{2-6}$ alkynyl, -O($C_{1-6}$ alkyl), -$C_{1-6}$ alkylene-CN, -$C_{1-6}$ alkylene-OH, $C_{3-8}$ cycloalkyl, and 5-6 membered heteroaryl, wherein the $C_{3-8}$ cycloalkyl or 5-6 membered heteroaryl, as a substituent of $R_2$, is each optionally substituted with one or more halogen.

**8.** The compound, or the pharmaceutically acceptable salt thereof, or the solvate, the racemic mixture, the enantiomer, the diastereomer or the tautomer thereof according to claim 7, wherein

$R_1$' is chosen from H and $C_{1-6}$ alkyl;
X is O;
Y is $CR_3$; $R_3$ is chosen from H, -CN, halogen, $C_{1-6}$ alkyl, -O($C_{1-6}$ alkyl), and -O($C_{1-6}$ haloalkyl);
$R_a$ and $R_b$ are both H;
n is 1; and
$R_2$ is phenyl, which is optionally substituted with one or more groups independently chosen from: halogen, $C_{1-6}$ alkyl, -O($C_{1-6}$ alkyl), and $C_{3-8}$ cycloalkyl, wherein the $C_{3-8}$ cycloalkyl, as a substituent of $R_2$, is optionally substituted with one or more halogen.

**9.** The compound, or the pharmaceutically acceptable salt thereof, or the solvate, the racemic mixture, the enantiomer, the diastereomer or the tautomer thereof according to claim 1, wherein the compound has a structure of formula (I-1e):

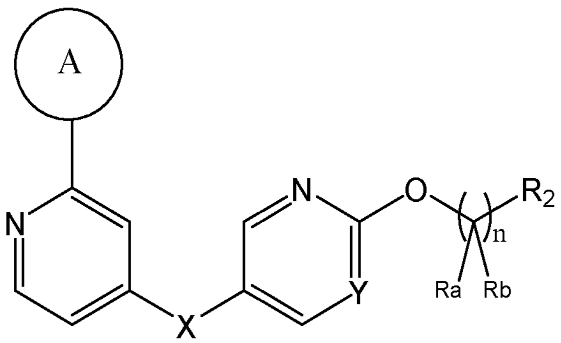

(I-1e)

wherein

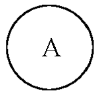

is 5-6 membered heteroaryl, which is optionally substituted with one or more groups independently chosen from: $C_{1-6}$ alkyl; preferably,

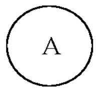

is pyrazolyl, which is optionally substituted with one or more groups independently chosen from: $C_{1-6}$ alkyl;
X is O or $CH_2$;
Y is $CR_3$; $R_3$ is chosen from H, $C_{1-6}$ alkyl, -O($C_{1-6}$ alkyl), and -O($C_{1-6}$ haloalkyl);
$R_a$ and $R_b$ are both H;
n is 1 or 2; and
$R_2$ is phenyl, which is optionally substituted with one or more groups independently chosen from: halogen, $C_{1-6}$ alkyl, -O($C_{1-6}$ alkyl), and $C_{3-8}$ cycloalkyl, wherein the $C_{3-8}$ cycloalkyl, as a substituent of $R_2$, is optionally substituted with one or more halogen.

**10.** The compound, or the pharmaceutically acceptable salt thereof, or the solvate, the racemic mixture, the enantiomer, the diastereomer or the tautomer thereof according to claim 1, wherein the compound has a structure of formula (I-2) or formula (I-3); and preferably, the compound has a structure of formula (I-2):

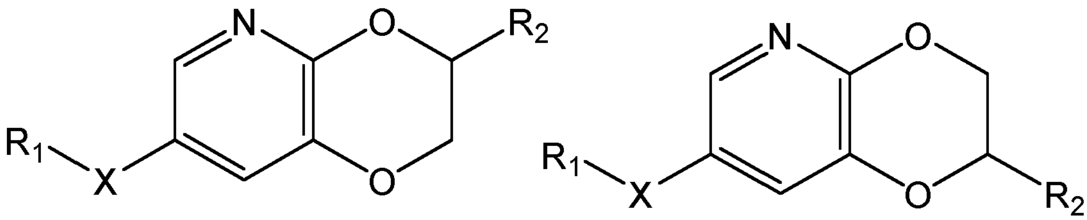

(I-2) (I-3)

wherein $R_1$, $R_2$ and X are as defined in claim 1.

**11.** The compound, or the pharmaceutically acceptable salt thereof, or the solvate, the racemic mixture, the enantiomer, the diastereomer or the tautomer thereof according to claim 10, wherein the compound has a structure of formula (I-2a):

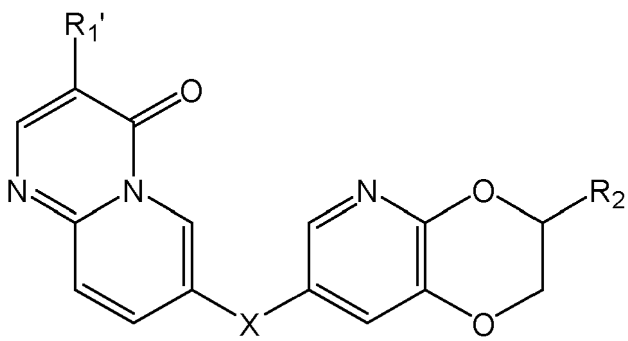

(I-2a)

wherein

$R_1$' is chosen from H and $C_{1-6}$ alkyl;
X is O;
$R_2$ is phenyl or 5-6 membered heteroaryl, each of which is optionally substituted with one or more groups independently chosen from: halogen, $C_{1-6}$ alkyl, -O($C_{1-6}$ alkyl), and $C_{3-8}$ cycloalkyl, wherein the $C_{3-8}$ cycloalkyl, as a substituent of $R_2$, is optionally substituted with one or more halogen; and preferably, $R_2$ is phenyl or pyridyl, each of which is optionally substituted with one or more groups independently chosen from: - O($C_{1-6}$ alkyl) and $C_{3-8}$ cycloalkyl;
or
the compound has a structure of formula (I-2b):

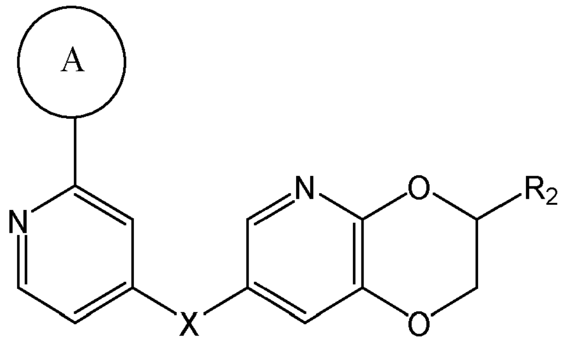

(I-2b)

wherein

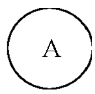

is 5-6 membered heteroaryl, which is optionally substituted with one or more groups independently chosen from: $C_{1-6}$ alkyl and -$C_{1-6}$ alkylene-OH; preferably,

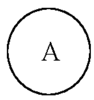

is pyrazolyl, which is optionally substituted with one or more groups independently chosen from: $C_{1-6}$ alkyl and -$C_{1-6}$ alkylene-OH; more preferably,

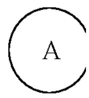

is pyrazolyl, which is optionally substituted with one or more groups independently chosen from: $C_{1-6}$ alkyl;
X is O;
$R_2$ is phenyl or 5-6 membered heteroaryl, each of which is optionally substituted with one or more groups independently chosen from: halogen, $C_{1-6}$ alkyl, -O($C_{1-6}$ alkyl), and $C_{3-8}$ cycloalkyl, wherein the $C_{3-8}$ cycloalkyl, as a substituent of $R_2$, is optionally substituted with one or more halogen; preferably, $R_2$ is phenyl or pyridyl, each of which is optionally substituted with one or more groups independently chosen from: -O($C_{1-6}$ alkyl) and $C_{3-8}$ cycloalkyl; and more preferably, $R_2$ is pyridyl, which is optionally substituted with one or more groups independently chosen from: -O($C_{1-6}$ alkyl) and $C_{3-8}$ cycloalkyl.

**12.** The compound of formula (I) according to claim 1, which is chosen from the following compounds:

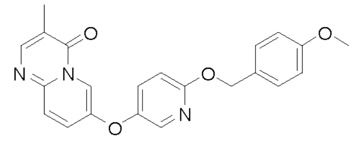

1

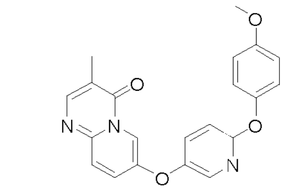

2

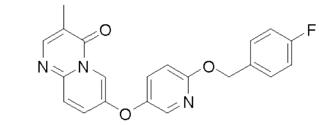

3

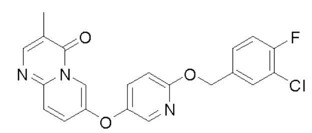

4

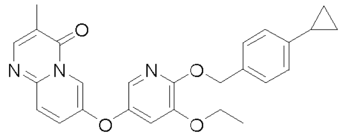

5

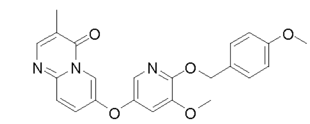

6

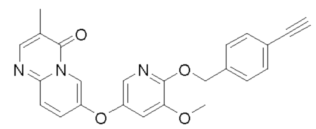

7

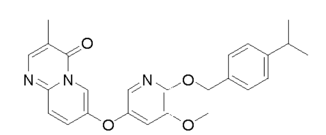

8

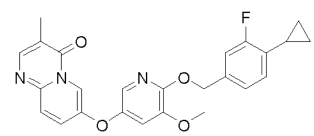

9

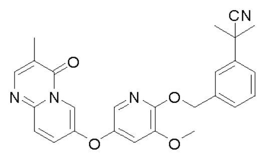

10

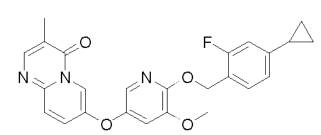

11

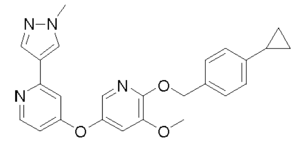

12

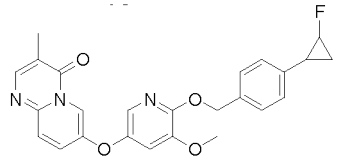

13

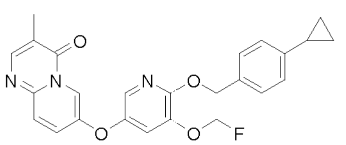

14

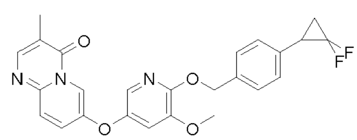

15

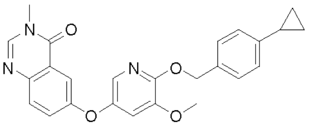

16

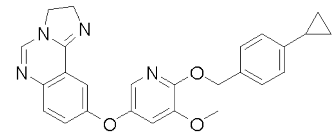

17

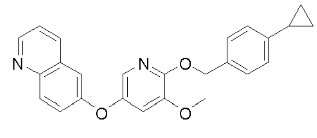

18

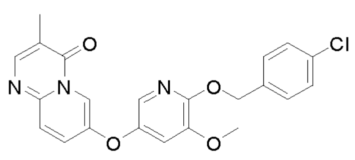

19

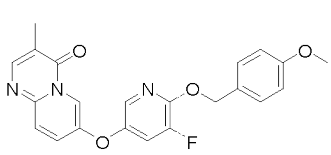

20

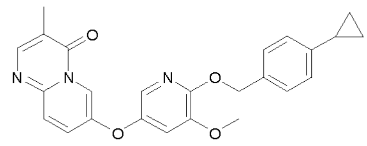

21

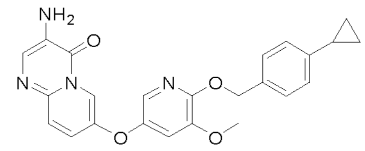

22

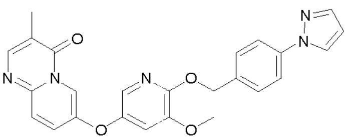

23

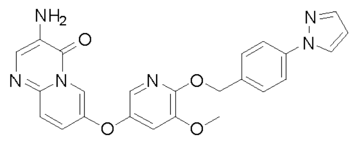

24

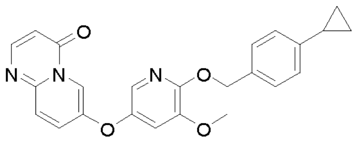

25

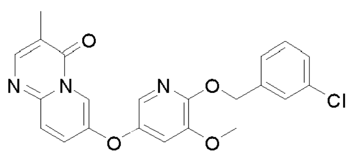

26

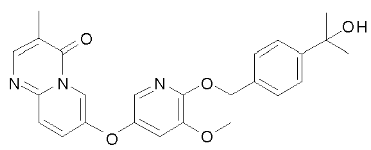

27

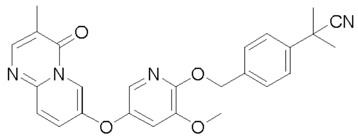

28

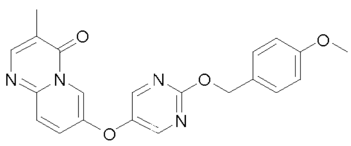

29

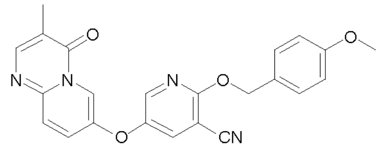

30

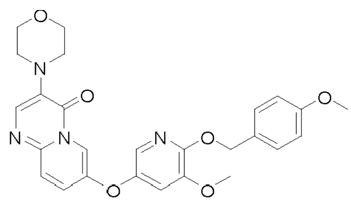

31

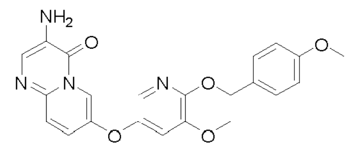

32

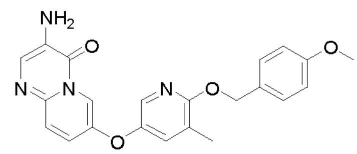

33

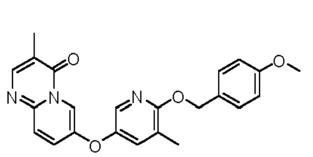

34

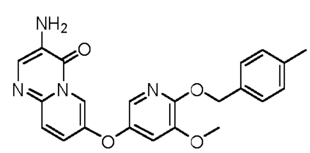

35

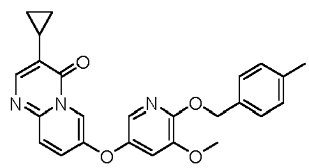

36

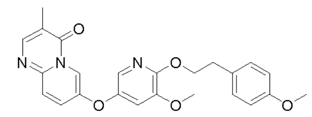

37

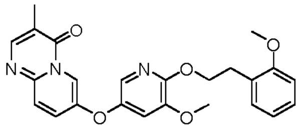

38

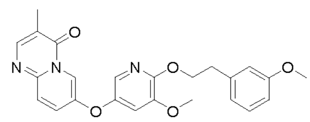

39

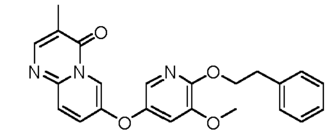

40

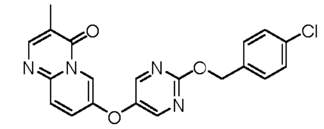

41

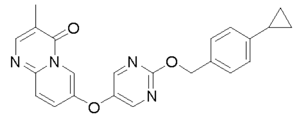

42

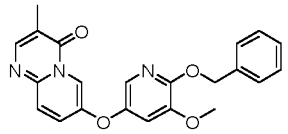

43

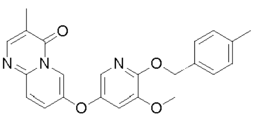

44

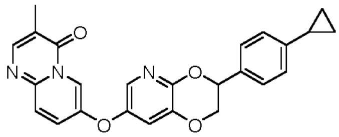

45

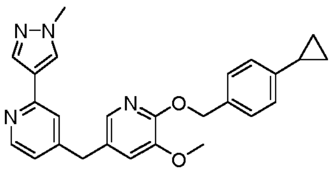

46

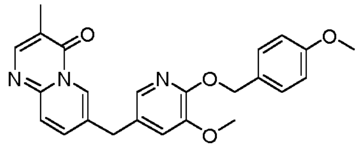

47

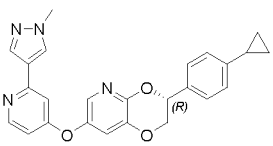

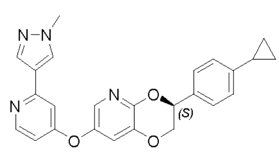

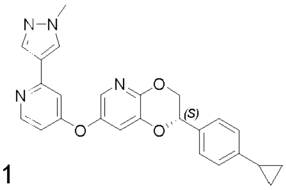

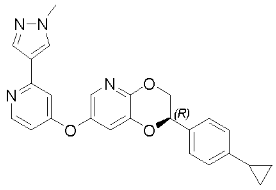

48-51

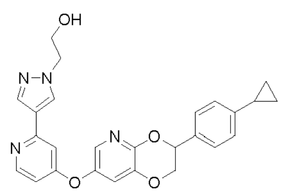

52

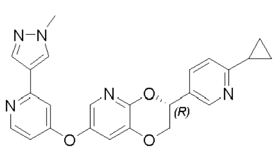

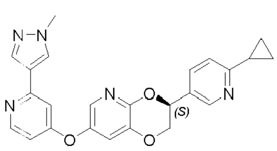

53 & 54

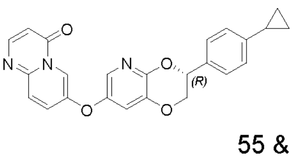

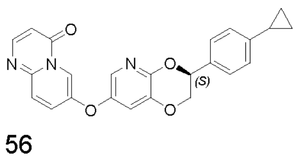

55 & 56

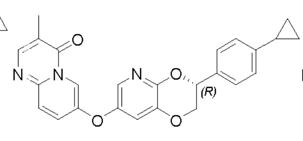

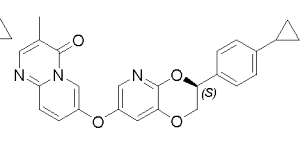

57 & 58

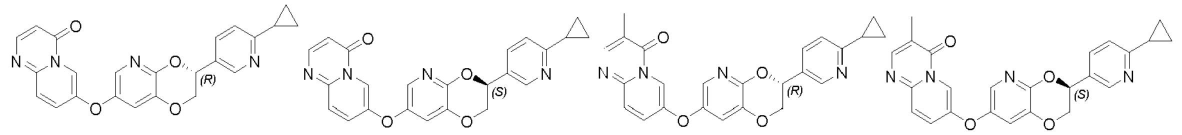

59 & 60

61 & 62

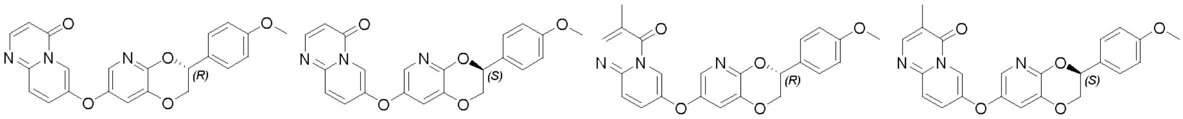

63 & 64

65 & 66

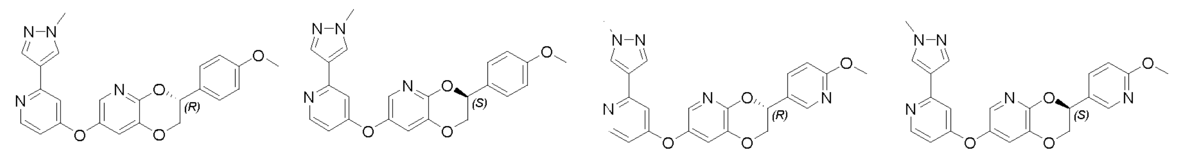

67 & 68

69 & 70

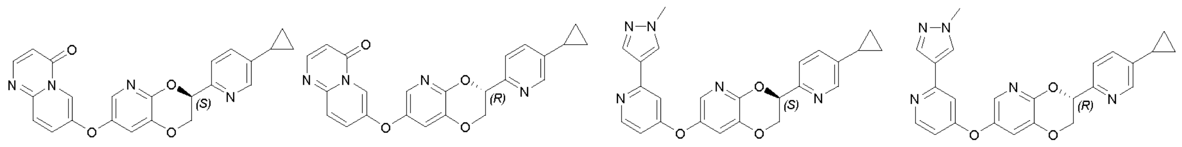

71 & 72

73 & 74

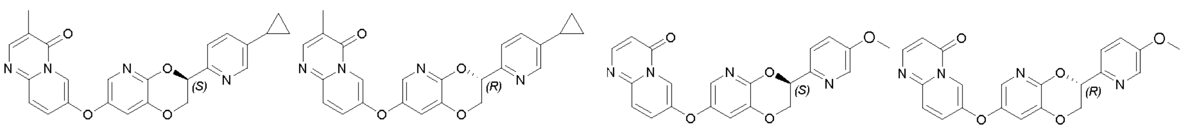

75 & 76

77 & 78

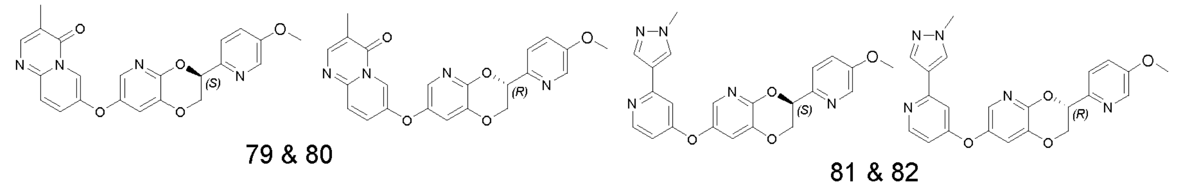

79 & 80

81 & 82

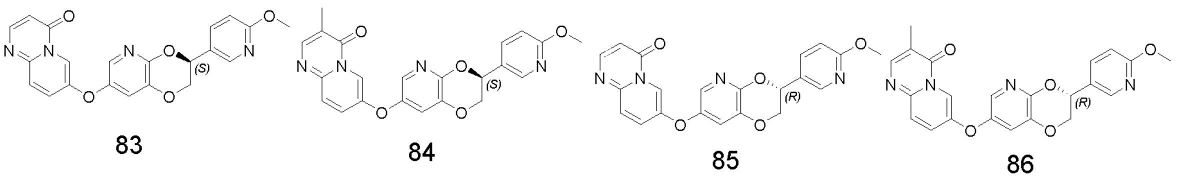

83

84

85

86

.

or a pharmaceutically acceptable salt thereof.

**13.** A pharmaceutical composition, comprising a compound or a pharmaceutically acceptable salt thereof according to any one of claims 1-12, and optionally comprising a pharmaceutically acceptable excipient.

**14.** A compound or a pharmaceutically acceptable salt thereof according to any one of claims 1-12 or a pharmaceutical composition according to claim 13, for use as a pharmaceutical, e.g. in treating a disease mediated by CSF-1R or at least in part by CSF-1R in a subject, wherein the disease is an autoimmune disease, an inflammatory disease, a

neurodegenerative disease, cancer, a metabolic disease, obesity, or an obesity-related disease; and preferably, wherein the autoimmune disease or inflammatory disease is chosen from rheumatoid arthritis, collagen-induced arthritis, osteoarthritis, pigmented villonodular synovitis (PVNS), systemic lupus erythematosus, multiple sclerosis, systemic scleroderma, autoimmune nephritis, inflammatory bowel disease, Crohn's disease, ulcerative colitis, psoriasis, atopic dermatitis, asthma, chronic obstructive pulmonary disease, Behcet's disease, idiopathic thrombocytopenic purpura, spinal arthritis, systemic juvenile idiopathic arthritis (SoJIA), pancreatitis, ischemia reperfusion injury of parenchymatous organs, organ-graft rejection, septicemia, systemic inflammatory response syndrome, and chemotherapy drugs induced organ injury; the neurodegenerative disease is chosen from Parkinson's disease (PD), multiple system atrophy, Alzheimer's disease (AD), frontotemporal lobar dementia, Huntington's disease (HD), corticobasal degeneration, spinocerebellar ataxia, amyotrophic lateral sclerosis (ALS), spinal muscular atrophy (SMA), and hereditary motor and sensory neuropathy (CMT); and the cancer is solid tumor or hematologic malignancy, such as ovarian cancer, lung cancer (including non-small cell lung cancer), brain tumor (including glioblastoma (GBM)), tenosynovial giant cell tumor, gastrointestinal stromal tumor (GIST), gastric cancer, esophageal cancer, colon cancer, colorectal cancer, pancreatic cancer, prostate cancer, breast cancer, cervical cancer, melanoma, mesothelioma, mesothelial carcinoma, renal cancer, liver cancer, thyroid carcinoma, head and neck cancer, urothelial carcinoma, bladder cancer, endometrial cancer, choriocarcinoma, adrenal carcinoma, sarcoma, leukemia, lymphoma, or myeloma.

**15.** A pharmaceutical combination, comprising a compound or a pharmaceutically acceptable salt thereof according to any one of claims 1-12, and at least one additional therapeutic agent; preferably, wherein the additional therapeutic agent is an antiinflammatory agent or an anti-neoplastic agent; and more preferably, the anti-neoplastic agent is chosen from a radiotherapeutic agent, a chemotherapeutic agent, an immune checkpoint inhibitor or agonist, and a targeted therapeutic agent.

## Patentansprüche

**1.** Verbindung der Formel (I):

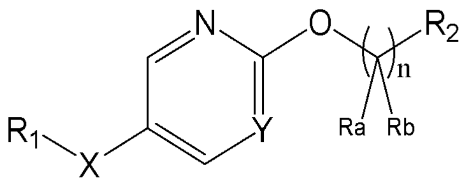

(I)

oder ein pharmazeutisch verträgliches Salz davon oder ein Solvat, ein racemisches Gemisch, ein Enantiomer, ein Diastereomer oder ein Tautomer davon, wobei

$R_1$ ausgewählt ist aus:

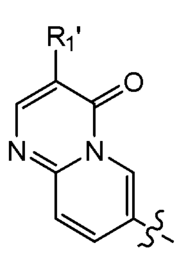 , 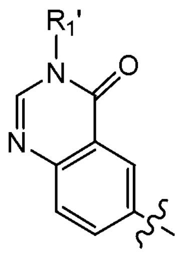 , 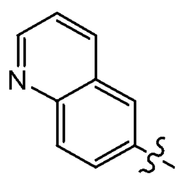 ,

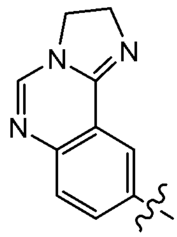 und 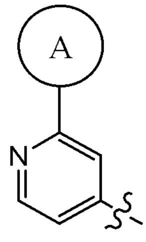;

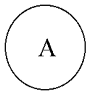

ausgewählt ist aus Phenyl und 5-6-gliedrigem Heteroaryl, das jeweils gegebenenfalls substituiert ist mit einer oder mehreren Gruppen, unabhängig ausgewählt aus: -CN, Halogen, $C_{1-6}$-Alkyl, -O($C_{1-6}$-Alkyl), $C_{1-6}$-Halogenalkyl, -O($C_{1-6}$-Halogenalkyl), - $C_{1-6}$-Alkylen-CN und -$C_{1-6}$-Alkylen-OH;

$R_1$' ausgewählt ist aus H, Halogen, -CN, $C_{1-6}$-Alkyl, $C_{1-6}$-Halogenalkyl, -$C_{1-6}$-Alkylen-CN, -$C_{1-6}$-Alkylen-OH, -O($C_{1-6}$-Alkyl), -O($C_{1-6}$-Halogenalkyl), $C_{3-8}$-Cycloalkyl, 4-8-gliedrigem Heterocyclyl und $NR_4R_5$; $R_4$ und $R_5$ jeweils unabhängig ausgewählt sind aus H, Halogen, -CN, $C_{1-6}$-Alkyl, $C_{1-6}$-Halogenalkyl, -$C_{1-6}$-Alkylen-CN, -$C_{1-6}$-Alkylen-OH und -O($C_{1-6}$-Alkyl); oder $R_4$ und $R_5$ zusammen mit dem N-Atom, an das sie gebunden sind, einen 4-8-gliedrigen heterocyclischen Ring bilden;

X O oder $CR_6R_7$ ist; $R_6$ und $R_7$ jeweils unabhängig ausgewählt sind aus H, Halogen, $C_{1-6}$-Alkyl, $C_{1-6}$-Halogenalkyl, -$C_{1-6}$-Alkylen-CN, - $C_{1-6}$-Alkylen-OH, -O($C_{1-6}$-Alkyl) und -O($C_{1-6}$-Halogenalkyl);

Y N oder $CR_3$ ist; $R_3$ ausgewählt ist aus H, -CN, Halogen, $C_{1-6}$-Alkyl, -O($C_{1-6}$-Alkyl), -$C_{1-6}$-Alkylen-CN, -$C_{1-6}$-Alkylen-OH, $C_{1-6}$-Halogenalkyl und -O($C_{1-6}$-Halogenalkyl);

$R_a$ und $R_a$ jeweils unabhängig ausgewählt sind aus H, Halogen, - CN, $C_{1-6}$-Alkyl, $C_{1-6}$-Halogenalkyl, -$C_{1-6}$-Alkylen-CN, -$C_{1-6}$-Alkylen-OH, -O($C_{1-6}$-Alkyl) und -O($C_{1-6}$-Halogenalkyl);

n 0, 1, 2, 3 oder 4 ist;

$R_2$ Phenyl oder 5-10-gliedriges Heteroaryl ist, das jeweils gegebenenfalls substituiert ist mit einer oder mehreren Gruppen, unabhängig ausgewählt aus: -CN, Halogen, $C_{1-6}$-Alkyl, $C_{2-6}$-Alkenyl, $C_{2-6}$-Alkinyl, -O($C_{1-6}$-Alkyl), $C_{1-6}$-Halogenalkyl, -O($C_{1-6}$-Halogenalkyl), -$C_{1-6}$-Alkylen-CN, -$C_{1-6}$-Alkylen-OH, $C_{3-8}$-Cycloalkyl, 4-8-gliedrigem Heterocyclyl und 5-6-gliedrigem Heteroaryl, wobei das $C_{3-8}$-Cycloalkyl, das 4-8-gliedrige Heterocyclyl oder das 5-6-gliedrige Heteroaryl, als ein Substituent von $R_2$, jeweils gegebenenfalls substituiert ist mit einer oder mehreren Gruppen, unabhängig ausgewählt aus: -CN, Halogen, $C_{1-6}$-Alkyl, -O($C_{1-6}$-Alkyl), $C_{1-6}$-Halogenalkyl, -O($C_{1-6}$-Halogenalkyl), -$C_{1-6}$-Alkylen-CN und -$C_{1-6}$-Alkylen-OH;

oder, wenn Y $CR_3$ ist und n nicht 0 ist, bilden $R_3$ und ein $R_a$ zusammen mit den Kohlenstoffatomen, an die sie gebunden sind, und dem/den Atom(en) zwischen den Kohlenstoffatomen einen 4-8-gliedrigen heterocyclischen Ring.

**2.** Verbindung oder das pharmazeutisch verträgliche Salz davon oder das Solvat, das racemische Gemisch, das Enantiomer, das Diastereomer oder das Tautomer davon nach Anspruch 1, wobei $R_1$ ausgewählt ist aus:

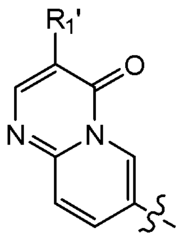;

$R_1$' ausgewählt ist aus H, $C_{1-6}$-Alkyl, $C_{3-8}$-Cycloalkyl und $NR_4R_5$; $R_4$ und $R_5$ beide H sind; oder $R_4$ und $R_5$ zusammen mit dem N-Atom, an das sie gebunden sind, einen 4-8-gliedrigen heterocyclischen Ring bilden; vorzugsweise $R_1$' ausgewählt ist aus H und $C_{1-6}$-Alkyl;

oder

$R_1$ ausgewählt ist aus

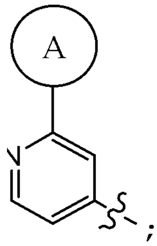

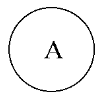

ein 5-6-gliedriges Heteroaryl ist, das gegebenenfalls substituiert ist mit einer oder mehreren Gruppen, unabhängig ausgewählt aus: $C_{1-6}$-Alkyl und -$C_{1-6}$-Alkylen-OH; vorzugsweise

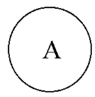

Pyrazolyl ist, das gegebenenfalls substituiert ist mit einer oder mehreren Gruppen, unabhängig ausgewählt aus: $C_{1-6}$-Alkyl und -$C_{1-6}$-Alkylen-OH; stärker bevorzugt

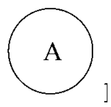

Pyrazolyl ist, das gegebenenfalls substituiert ist mit einer oder mehreren Gruppen, unabhängig ausgewählt aus: $C_{1-6}$-Alkyl.

**3.** Verbindung oder das pharmazeutisch verträgliche Salz davon oder das Solvat, das racemische Gemisch, das Enantiomer, das Diastereomer oder das Tautomer davon nach einem der Ansprüche 1 bis 2, wobei X O oder $CH_2$ ist; und vorzugsweise X O ist.

**4.** Verbindung oder das pharmazeutisch verträgliche Salz davon oder das Solvat, das racemische Gemisch, das Enantiomer, das Diastereomer oder das Tautomer davon nach einem der Ansprüche 1 bis 3, wobei Y N oder $CR_3$ ist; und $R_3$ ausgewählt ist aus H, -CN, Halogen, $C_{1-6}$-Alkyl, -O($C_{1-6}$-Alkyl) und -O($C_{1-6}$-Halogenalkyl); z. B. Y $CR_3$ ist und $R_3$ ausgewählt ist aus H, -CN, Halogen, $C_{1-6}$-Alkyl, -O($C_{1-6}$-Alkyl) oder -O($C_{1-6}$-Halogenalkyl); vorzugsweise $R_3$ -O($C_{1-6}$-Alkyl) ist; und stärker bevorzugt $R_3$ -O($C_{1-3}$-Alkyl) ist.

**5.** Verbindung oder das pharmazeutisch verträgliche Salz davon oder das Solvat, das racemische Gemisch, das Enantiomer, das Diastereomer oder das Tautomer davon nach einem der Ansprüche 1 bis 4, wobei $R_a$ und $R_b$ beide H sind und n 0, 1 oder 2 ist; und vorzugsweise $R_a$ und $R_b$ beide H sind und n 1 ist.

**6.** Verbindung oder das pharmazeutisch verträgliche Salz davon oder das Solvat, das racemische Gemisch, das Enantiomer, das Diastereomer oder das Tautomer davon nach einem der Ansprüche 1 bis 5, wobei $R_2$ Phenyl oder 5-6-gliedriges Heteroaryl ist, das jeweils gegebenenfalls substituiert ist mit einer oder mehreren Gruppen, unabhängig ausgewählt aus: Halogen, $C_{1-6}$-Alkyl, $C_{2-6}$-Alkinyl, -O($C_{1-6}$-Alkyl), -$C_{1-6}$-Alkylen-CN, -$C_{1-6}$-Alkylen-OH, $C_{3-8}$-Cycloalkyl und 5-6-gliedrigem Heteroaryl, wobei das $C_{3-8}$-Cycloalkyl oder 5-6-gliedrige Heteroaryl, als ein Substituent von $R_2$, jeweils gegebenenfalls mit einem oder mehreren Halogenen substituiert ist; und vorzugsweise $R_2$ Phenyl oder Pyridyl ist, das jeweils gegebenenfalls substituiert ist mit einer oder mehreren Gruppen, unabhängig ausgewählt aus: Halogen, $C_{1-6}$-Alkyl, -O($C_{1-6}$-Alkyl) und $C_{3-8}$-Cycloalkyl, wobei das $C_{3-8}$-Cycloalkyl, als ein Substituent von $R_2$, gegebenenfalls mit einem oder mehreren Halogenen substituiert ist.

**7.** Verbindung oder das pharmazeutisch verträgliche Salz davon oder das Solvat, das racemische Gemisch, das Enantiomer, das Diastereomer oder das Tautomer davon nach Anspruch 1, wobei die Verbindung eine Struktur der Formel (I-1a) aufweist:

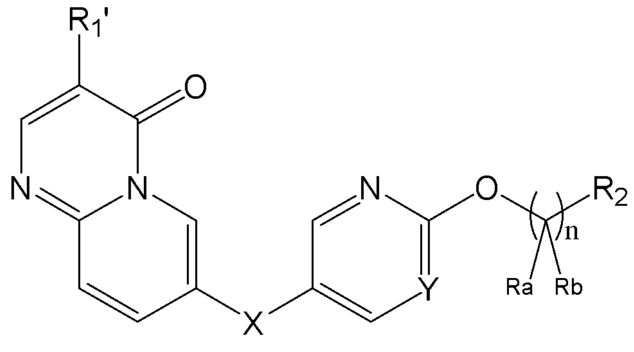

(I-1a)

wobei

$R_1$' ausgewählt ist aus H, $C_{1-6}$-Alkyl, $C_{3-8}$-Cycloalkyl und $NR_4R_5$; $R_4$ und $R_5$ beide H sind; oder $R_4$ und $R_5$ zusammen mit dem N-Atom, an das sie gebunden sind, einen 4-8-gliedrigen heterocyclischen Ring bilden; vorzugsweise einen 5- oder 6-gliedrigen heterocyclischen Ring bilden, der zusätzlich zu dem N-Atom, an das $R_4$ und $R_5$ gebunden sind, 0, 1 oder 2 Heteroatome enthält, die unabhängig ausgewählt sind aus N, O oder S, und stärker bevorzugt einen Morpholinring bilden;
X O oder $CH_2$ ist;
Y N oder $CR_3$ ist; $R_3$ ausgewählt ist aus H, -CN, Halogen, $C_{1-6}$-Alkyl, -O($C_{1-6}$-Alkyl) und -O($C_{1-6}$-Halogenalkyl) ;
$R_a$ und $R_b$ beide H sind;
n 0, 1 oder 2 ist; und
$R_2$ Phenyl ist, das gegebenenfalls substituiert ist mit einer oder mehreren Gruppen, unabhängig ausgewählt aus: Halogen, $C_{1-6}$-Alkyl, $C_{2-6}$-Alkinyl, -O($C_{1-6}$-Alkyl), -$C_{1-6}$-Alkylen-CN, -$C_{1-6}$-Alkylen-OH, $C_{3-8}$-Cycloalkyl und 5-6-gliedrigem Heteroaryl, wobei das $C_{3-8}$-Cycloalkyl oder 5-6-gliedrige Heteroaryl, als ein Substituent von $R_2$, jeweils gegebenenfalls mit einem oder mehreren Halogenen substituiert ist.

**8.** Verbindung oder das pharmazeutisch verträgliche Salz davon oder das Solvat, das racemische Gemisch, das Enantiomer, das Diastereomer oder das Tautomer davon nach Anspruch 7, wobei

$R_1$' ausgewählt ist aus H und $C_{1-6}$-Alkyl;
X O ist;
Y $CR_3$ ist; $R_3$ ausgewählt ist aus H, -CN, Halogen, $C_{1-6}$-Alkyl, - O($C_{1-6}$-Alkyl) und -O($C_{1-6}$-Halogenalkyl);
$R_a$ und $R_b$ beide H sind;
n 1 ist; und
$R_2$ Phenyl ist, das gegebenenfalls substituiert ist mit einer oder mehreren Gruppen, unabhängig ausgewählt aus: Halogen, $C_{1-6}$-Alkyl, -O($C_{1-6}$-Alkyl) und $C_{3-8}$-Cycloalkyl, wobei das $C_{3-8}$-Cycloalkyl, als ein Substituent von $R_2$, gegebenenfalls mit einem oder mehreren Halogenen substituiert ist.

**9.** Verbindung oder das pharmazeutisch verträgliche Salz davon oder das Solvat, das racemische Gemisch, das Enantiomer, das Diastereomer oder das Tautomer davon nach Anspruch 1, wobei die Verbindung eine Struktur der Formel (I-1e) aufweist:

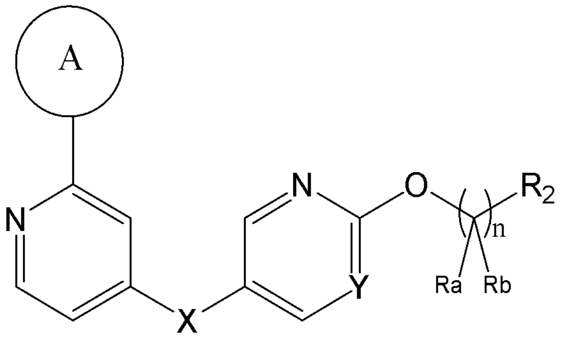

(I-1e)

wobei

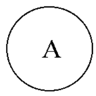

ein 5-6-gliedriges Heteroaryl ist, das gegebenenfalls substituiert ist mit einer oder mehreren Gruppen, unabhängig ausgewählt aus: $C_{1-6}$-Alkyl; vorzugsweise

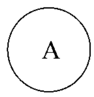

Pyrazolyl ist, das gegebenenfalls substituiert ist mit einer oder mehreren Gruppen, unabhängig ausgewählt aus: $C_{1-6}$-Alkyl;
X O oder $CH_2$ ist;
Y $CR_3$ ist; $R_3$ ausgewählt ist aus H, $C_{1-6}$-Alkyl, -O($C_{1-6}$-Alkyl) und -O($C_{1-6}$-Halogenalkyl);
$R_a$ und $R_b$ beide H sind;
n 1 oder 2 ist; und
$R_2$ Phenyl ist, das gegebenenfalls substituiert ist mit einer oder mehreren Gruppen, unabhängig ausgewählt aus: Halogen, $C_{1-6}$-Alkyl, -O($C_{1-6}$-Alkyl) und $C_{3-8}$-Cycloalkyl, wobei das $C_{3-8}$-Cycloalkyl, als ein Substituent von $R_2$, gegebenenfalls mit einem oder mehreren Halogenen substituiert ist.

**10.** Verbindung oder das pharmazeutisch verträgliche Salz davon oder das Solvat, das racemische Gemisch, das Enantiomer, das Diastereomer oder das Tautomer davon nach Anspruch 1, wobei die Verbindung eine Struktur der Formel (I-2) oder Formel (I-3) aufweist; und vorzugsweise die Verbindung eine Struktur der Formel (I-2) aufweist:

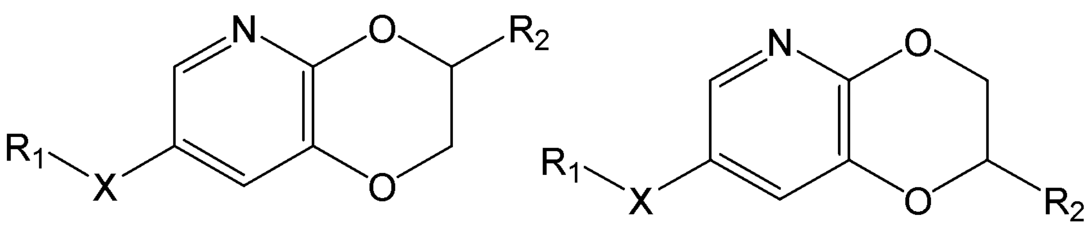

(I-2) (I-3)

wobei $R_1$, $R_2$ und X wie in Anspruch 1 definiert sind.

**11.** Verbindung oder das pharmazeutisch verträgliche Salz davon oder das Solvat, das racemische Gemisch, das Enantiomer, das Diastereomer oder das Tautomer davon nach Anspruch 10, wobei die Verbindung eine Struktur der Formel (I-2a) aufweist:

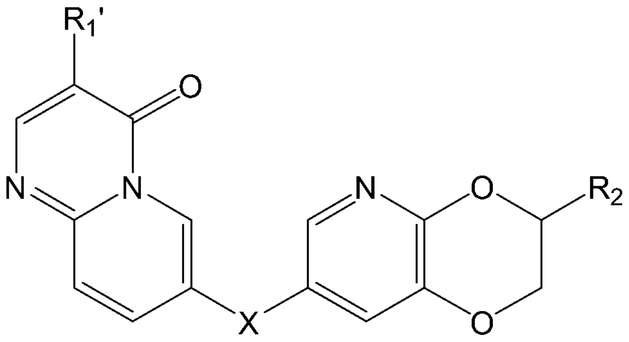

(I-2a)

wobei

$R_1$' ausgewählt ist aus H und $C_{1-6}$-Alkyl;
X O ist;
$R_2$ Phenyl oder 5-6-gliedriges Heteroaryl ist, das jeweils gegebenenfalls substituiert ist mit einer oder mehreren Gruppen, unabhängig ausgewählt aus: Halogen, $C_{1-6}$-Alkyl, -O($C_{1-6}$-Alkyl) und $C_{3-8}$-Cycloalkyl, wobei das $C_{3-8}$-Cycloalkyl, als ein Substituent von $R_2$, gegebenenfalls mit einem oder mehreren Halogenen substituiert ist; und vorzugsweise $R_2$ Phenyl oder Pyridyl ist, das jeweils gegebenenfalls substituiert ist mit einer oder mehreren Gruppen, unabhängig ausgewählt aus: -O($C_{1-6}$-Alkyl) und $C_{3-8}$-Cycloalkyl;
oder
die Verbindung eine Struktur der Formel (I-2b) aufweist:

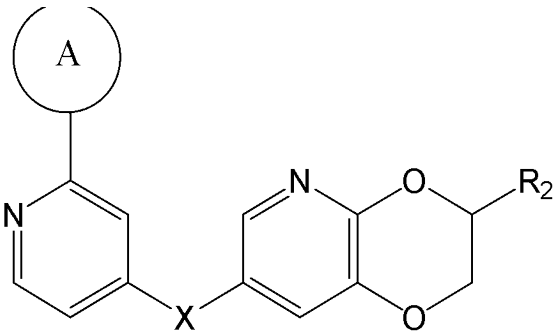

(I-2b)

wobei

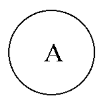

ein 5-6-gliedriges Heteroaryl ist, das gegebenenfalls substituiert ist mit einer oder mehreren Gruppen, unabhängig ausgewählt aus: $C_{1-6}$-Alkyl und -$C_{1-6}$-Alkylen-OH; vorzugsweise

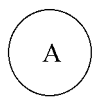

Pyrazolyl ist, das gegebenenfalls substituiert ist mit einer oder mehreren Gruppen, unabhängig ausgewählt aus: $C_{1-6}$-Alkyl und -$C_{1-6}$-Alkylen-OH; stärker bevorzugt

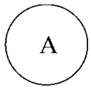

Pyrazolyl ist, das gegebenenfalls substituiert ist mit einer oder mehreren Gruppen, unabhängig ausgewählt aus: $C_{1-6}$-Alkyl;
X O ist;
$R_2$ Phenyl oder 5-6-gliedriges Heteroaryl ist, das jeweils gegebenenfalls substituiert ist mit einer oder mehreren Gruppen, unabhängig ausgewählt aus: Halogen, $C_{1-6}$-Alkyl, -O($C_{1-6}$-Alkyl) und $C_{3-8}$-Cycloalkyl, wobei das $C_{3-8}$-Cycloalkyl, als ein Substituent von $R_2$, gegebenenfalls mit einem oder mehreren Halogenen substituiert ist; vorzugsweise $R_2$ Phenyl oder Pyridyl ist, das jeweils gegebenenfalls substituiert ist mit einer oder mehreren Gruppen, unabhängig ausgewählt aus: -O($C_{1-6}$-Alkyl) und $C_{3-8}$-Cycloalkyl; und stärker bevorzugt $R_2$ Pyridyl ist, das gegebenenfalls substituiert ist mit einer oder mehreren Gruppen, unabhängig ausgewählt aus: -O($C_{1-6}$-Alkyl) und $C_{3-8}$-Cycloalkyl.

**12.** Verbindung der Formel (I) nach Anspruch 1, die ausgewählt ist aus den folgenden Verbindungen:

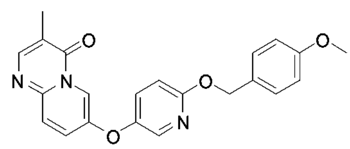

1

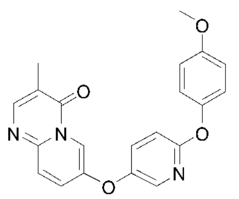

2

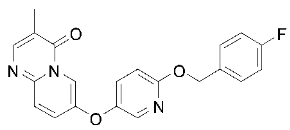

3

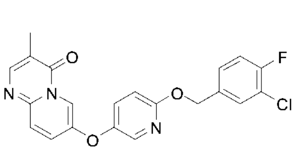

4

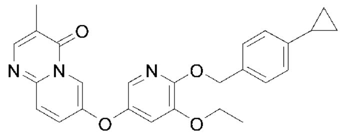

5

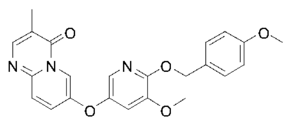

6

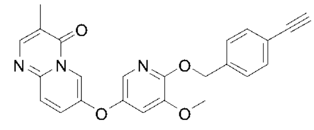

7

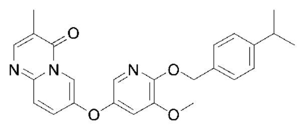

8

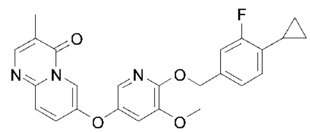

9

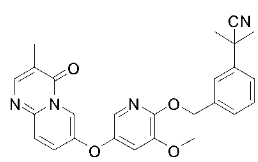

10

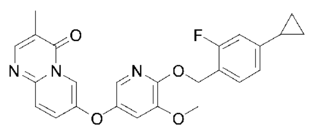

11

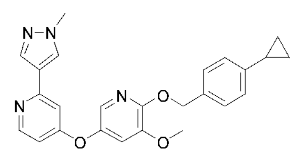

12

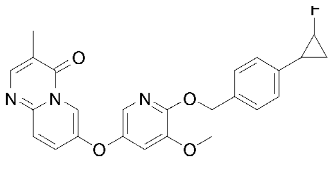

13

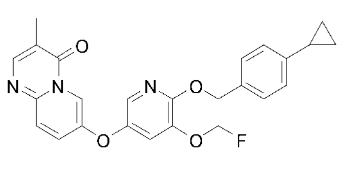

14

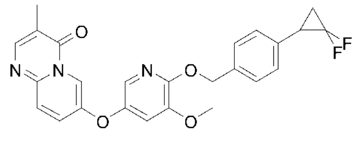

15

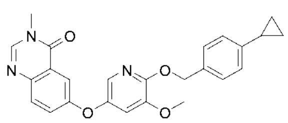

16

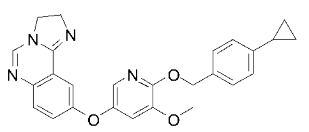

17

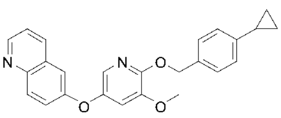

18

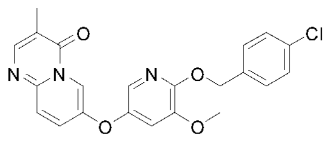

19

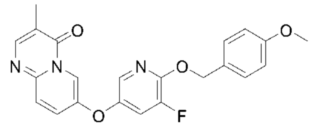

20

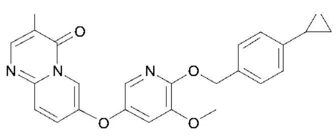

21

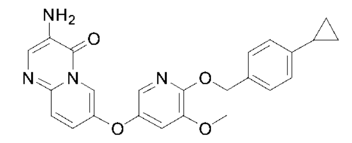

22

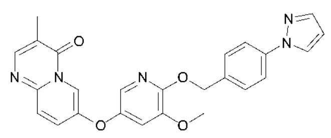

23

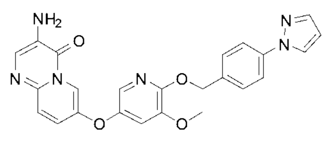

24

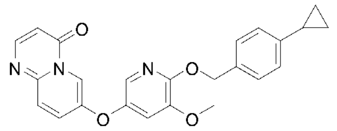

25

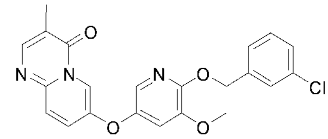

26

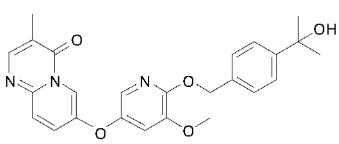

27

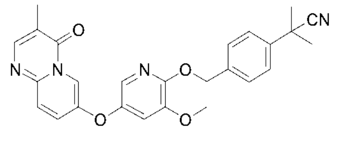

28

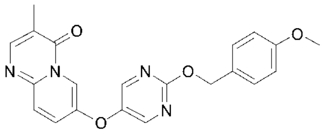

29

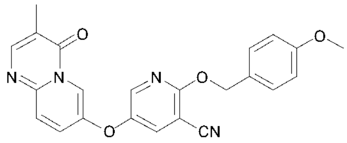

30

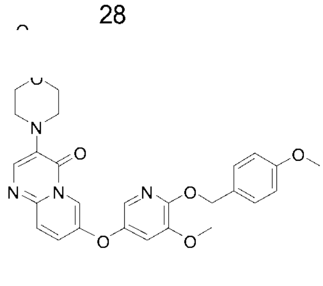

31

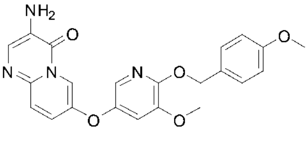

32

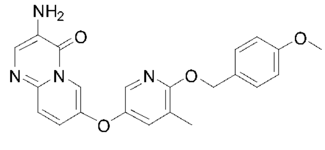

33

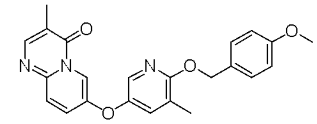

34

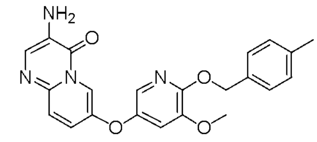

35

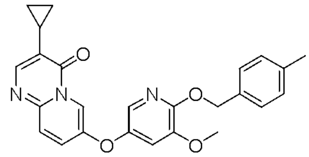

36

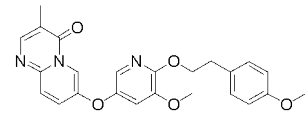

37

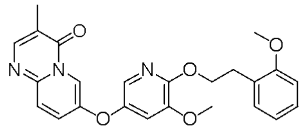

38

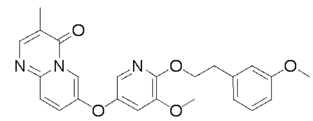

39

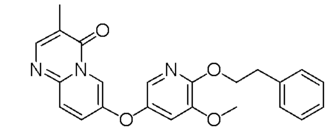

40

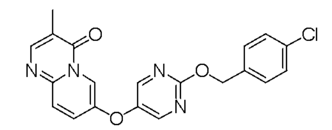

41

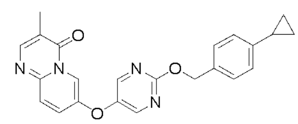

42

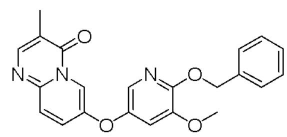

43

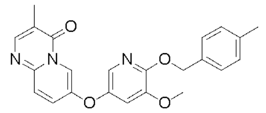

44

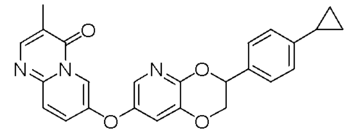

45

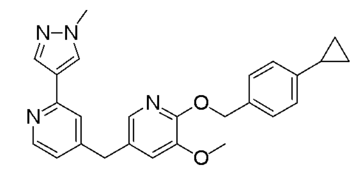

46

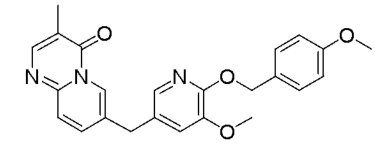

47

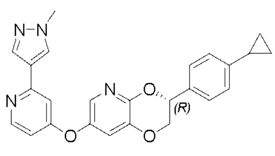

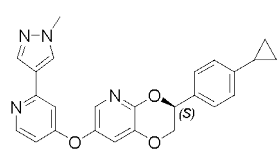

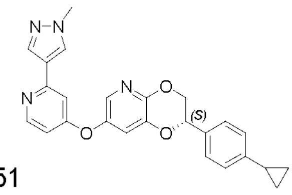

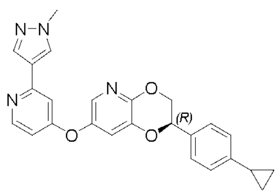

48-51

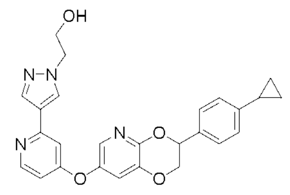

52

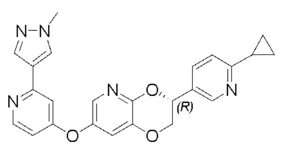

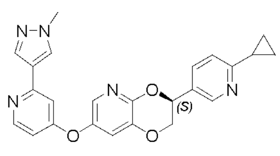

53 & 54

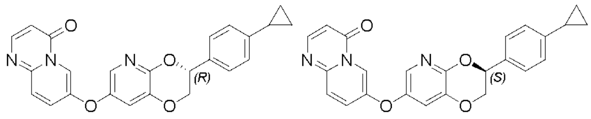

55 & 56

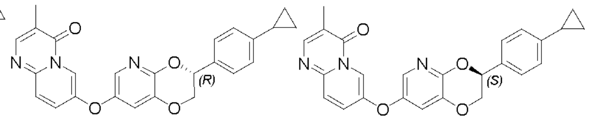

57 & 58

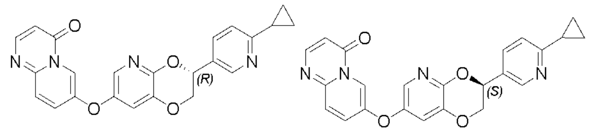

59 & 60

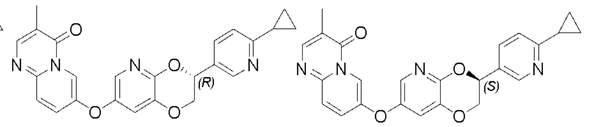

61 & 62

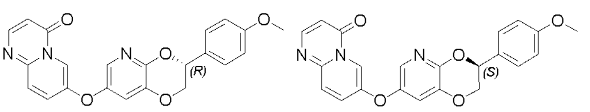

63 & 64

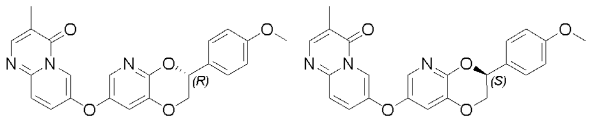

65 & 66

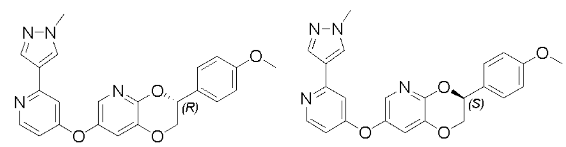

67 & 68

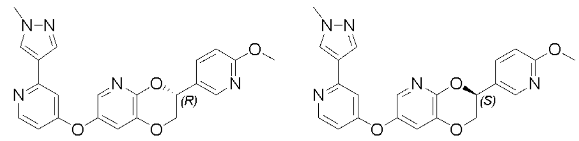

69 & 70

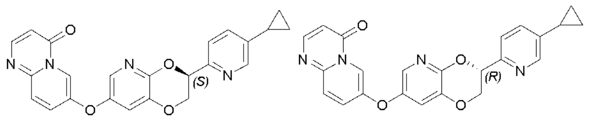

71 & 72

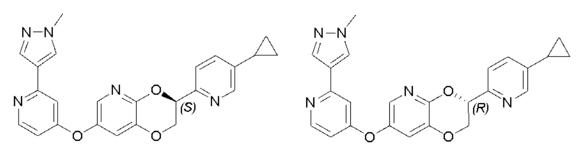

73 & 74

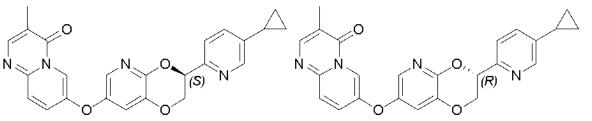

75 & 76

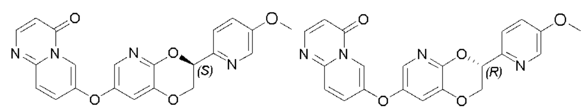

77 & 78

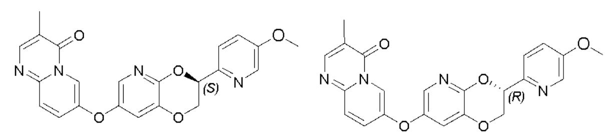

79 & 80

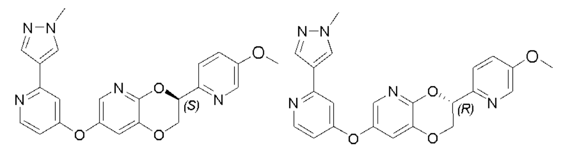

81 & 82

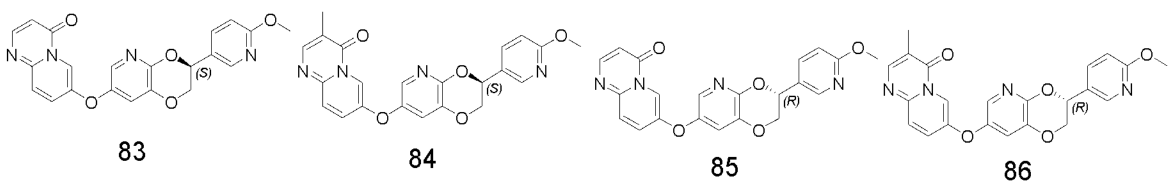

83 84 85 86

oder ein pharmazeutisch verträgliches Salz davon.

**13.** Pharmazeutische Zusammensetzung, umfassend eine Verbindung oder ein pharmazeutisch verträgliches Salz davon nach einem der Ansprüche 1 bis 12, und gegebenenfalls umfassend einen pharmazeutisch verträglichen Hilfsstoff.

**14.** Verbindung oder ein pharmazeutisch verträgliches Salz davon nach einem der Ansprüche 1 bis 12 oder eine pharmazeutische Zusammensetzung nach Anspruch 13, zur Verwendung als Pharmazeutikum, z. B. bei der Behandlung einer durch CSF-1R oder zumindest teilweise durch CSF-1R vermittelten Erkrankung bei einem Subjekt, wobei es sich bei der Erkrankung um eine Autoimmunerkrankung, eine entzündliche Erkrankung, eine neurodegenerative Erkrankung, Krebs, eine Stoffwechselerkrankung, Fettleibigkeit oder eine mit Fettleibigkeit zusammenhängende Erkrankung handelt; und vorzugsweise, wobei die Autoimmunerkrankung oder entzündliche Erkrankung ausgewählt ist aus rheumatoider Arthritis, kollageninduzierter Arthritis, Osteoarthritis, pigmentierter villonodulärer Synovitis (PVNS), systemischem Lupus erythematodes, multipler Sklerose, systemischer Sklerodermie, Autoimmunnephritis, entzündlicher Darmerkrankung, Morbus Crohn, Colitis ulcerosa, Psoriasis, atopischer Dermatitis, Asthma, chronisch-obstruktiver Lungenerkrankung, Morbus Behçet, idiopathischer thrombozytopenischer Purpura, Wirbelsäulenarthritis, systemischer juveniler idiopathischer Arthritis (SoJIA), Pankreatitis, Ischämie-Reperfusionsverletzung parenchymatöser Organe, Organtransplantatabstoßung, Septikämie, systemischem inflammatorischem Response-Syndrom und durch Chemotherapeutika induzierter Organverletzung; die neurodegenerative Erkrankung ausgewählt ist aus Parkinson-Krankheit (PD), Multisystematrophie, Alzheimer-Krankheit (AD), frontotemporaler lobärer Demenz, Huntington-Krankheit (HD), kortikobasaler Degeneration, spinozerebellärer Ataxie, amyotropher Lateralsklerose (ALS), spinaler Muskelatrophie (SMA) und hereditärer motorischer und sensorischer Neuropathie (CMT); und der Krebs ein solider Tumor oder eine hämatologische Malignität ist, wie Ovarialkarzinom, Lungenkrebs (einschließlich nichtkleinzelligem Lungenkrebs), Hirntumor (einschließlich Glioblastom (GBM)), tenosynovialer Riesenzelltumor, gastrointestinaler Stromatumor (GIST), Magenkrebs, Ösophaguskarzinom, Dickdarmkrebs, Kolorektalkarzinom, Pankreaskarzinom, Prostatakrebs, Brustkrebs, Zervixkarzinom, Melanom, Mesotheliom, Mesothelkarzinom, Nierenkrebs, Leberkrebs, Schilddrüsenkarzinom, Kopf- und Halskrebs, Urothelkarzinom, Blasenkrebs, Endometriumkarzinom, Chorionkarzinom, Nebennierenkarzinom, Sarkom, Leukämie, Lymphom oder Myelom.

**15.** Pharmazeutische Kombination, umfassend eine Verbindung oder ein pharmazeutisch verträgliches Salz davon nach einem der Ansprüche 1 bis 12 und mindestens einen zusätzlichen therapeutischen Wirkstoff; wobei vorzugsweise der zusätzliche therapeutische Wirkstoff ein entzündungshemmendes Mittel oder ein antineoplastisches Mittel ist; und stärker bevorzugt das antineoplastische Mittel ausgewählt ist aus einem Radiotherapeutikum, einem Chemotherapeutikum, einem Immun-Checkpoint-Inhibitor oder -Agonisten und einem zielgerichteten therapeutischen Mittel.

## Revendications

**1.** Composé de formule (I) :

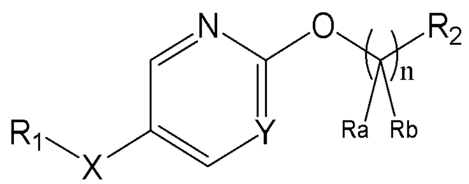

(I)

ou un de ses sels pharmaceutiquement acceptables, ou un de ses solvates, un mélange racémique, un énantiomère, un diastéréoisomère ou un tautomère de celui-ci, dans lequel

$R_1$ est choisi parmi :

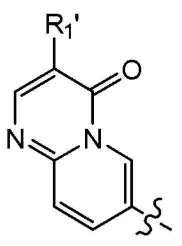 , 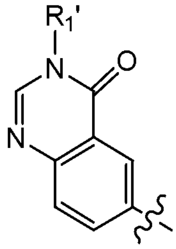 , 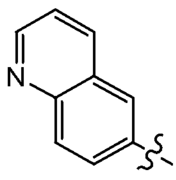 ,

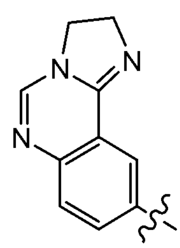 et 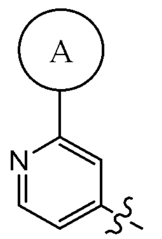 ;

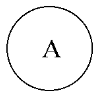

est choisi parmi le phényle et l'hétéroaryle à 5 à 6 chaînons, chacun étant éventuellement substitué par un ou plusieurs groupes choisis indépendamment parmi : -CN, halogène, alkyle en $C_{1-6}$ , -O(alkyle en $C_{1-6}$), haloalkyle en $C_{1-6}$, - O(haloalkyle en $C_{1-6}$), -alkylène-CN en $C_{1-6}$ et -alkylène-OH en $C_{1-6}$ ;

$R_1$' est choisi parmi H, halogène, -CN, alkyle en $C_{1-6}$, haloalkyle en $C_{1-6}$, -alkylène-CN en $C_{1-6}$, -alkylène-OH en $C_{1-6}$, -O(alkyle en $C_{1-6}$), -O(haloalkyle en $C_{1-6}$), cycloalkyle en $C_{3-8}$, hétérocycle à 4 à 8 chaînons et $NR_4R_5$ ; $R_4$ et $R_5$ sont chacun choisis indépendamment parmi H, halogène, -CN, alkyle en $C_{1-6}$, haloalkyle en $C_{1-6}$, -alkylène-CN en $C_{1-6}$, -alkylène-OH en $C_{1-6}$ et -O(alkyle en $C_{1-6}$) ; ou $R_4$ et $R_5$, ainsi que l'atome N auquel ils sont attachés, forment un cycle hétérocyclique à 4 à 8 chaînons ;

X est O ou $CR_6R_7$ ; $R_6$ et $R_7$ sont chacun choisis indépendamment parmi H, halogène, alkyle en $C_{1-6}$, haloalkyle en $C_{1-6}$, alkylène-CN en $C_{1-6}$, alkylène-OH en $C_{1-6}$, -O(alkyle en $C_{1-6}$) et -O(haloalkyle en $C_{1-6}$) ;

Y est N ou $CR_3$ ; $R_3$ est choisi parmi H, -CN, halogène, alkyle en $C_{1-6}$, -O(alkyle en $C_{1-6}$ ), alkylène-CN en $C_{1-6}$, alkylène-OH en $C_{1-6}$, haloalkyle en $C_{1-6}$ et -O(haloalkyle en $C_{1-6}$) ;

$R_a$ et $R_b$ sont chacun choisis indépendamment parmi H, halogène, - CN, alkyle en $C_{1-6}$, haloalkyle en $C_{1-6}$, alkylène-CN en $C_{1-6}$, alkylène-OH en $C_{1-6}$, -O(alkyle en $C_{1-6}$) et -O(haloalkyle en $C_{1-6}$) ; n est 0, 1, 2, 3, ou 4 ;

$R_2$ est un phényle ou un hétéroaryle à 5 à 10 chaînons, chacun pouvant être substitué éventuellement par un ou plusieurs groupes choisis indépendamment parmi : -CN, halogène, alkyle en $C_{1-6}$, alcényle en $C_{2-6}$, alcynyle en $C_{2-6}$, -O(alkyle en $C_{1-6}$), haloalkyle en $C_{1-6}$, -O(haloalkyle en $C_{1-6}$), alkylène-CN en $C_{1-6}$, alkylène-OH en $C_{1-6}$, cycloalkyle en $C_{3-8}$, hétérocyclyle à 4 à 8 chaînons et hétéroaryle à 5 à 6 chaînons, dans lequel le cycloalkyle en $C_{3-8}$, l'hétérocyclyle à 4 à 8 chaînons ou l'hétéroaryle à 5 à 6 chaînons, en tant que substituant de $R_2$, est chacun éventuellement subsitué par un ou plusieurs groupes choisis indépendamment parmi : -CN, halogène, alkyle en $C_{1-6}$, - O(alkyle en $C_{1-6}$), haloalkyle en $C_{1-6}$, -O(haloalkyle en $C_{1-6}$), alkylène-CN en $C_{1-6}$ et alkylène-OH en $C_{1-6}$ ;

ou, lorsque Y est $CR_3$ et n n'est pas 0, $R_3$ et un $R_a$ ainsi que les atomes de carbone auxquels ils sont attachés et le(s) atome(s) parmi les atomes de carbone forment un cycle hétérocyclique à 4 à 8 chaînons.

2. Composé, ou son sel pharmaceutiquement acceptable, ou son solvate, son mélange racémique, son énantiomère, son diastéréoisomère ou son tautomère selon la revendication 1, dans lequel $R_1$ est choisi parmi :

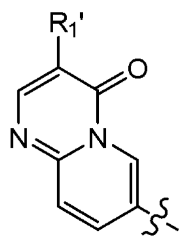 ;

$R_1$' est choisi parmi H, alkyle en $C_{1-6}$, cycloalkyle en $C_{3-8}$ et $NR_4R_5$ ; $R_4$ et $R_5$ sont tous deux H ; ou $R_4$ et $R_5$, ainsi que l'atome N auquel ils sont attachés, forment un cycle hétérocyclique à 4 à 8 chaînons ; de préférence, $R_1$' est choisi parmi H et alkyle en $C_{1-6}$ ;
ou

$R_1$ est choisi parmi

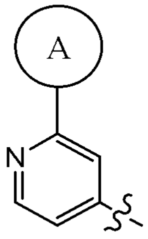 ;

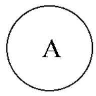

est un hétéroaryle à 5 à 6 chaînons, qui est éventuellement substitué par un ou plusieurs groupes choisis indépendamment parmi : alkyle en $C_{1-6}$ et alkylène-OH en $C_{1-6}$ ; de préférence,

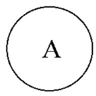

est un pyrazolyle, qui est éventuellement substitué par un ou plusieurs groupes choisis indépendamment parmi : alkyle en $C_{1-6}$ et -alkylène-OH en $C_{1-6}$ ; plus préférablement,

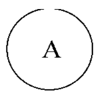

est un pyrazolyle, qui est éventuellement substitué par un ou plusieurs groupes choisis indépendamment parmi : alkyle en $C_{1-6}$.

**3.** Composé, ou son sel pharmaceutiquement acceptable, ou son solvate, son mélange racémique, son énantiomère, son diastéréoisomère ou son tautomère selon l'une quelconque des revendications 1 et 2, dans lequel X est O ou $CH_2$ ; et de préférence, X est O.

**4.** Composé, ou son sel pharmaceutiquement acceptable, ou son solvate, son mélange racémique, son énantiomère, son diastéréoisomère ou son tautomère selon l'une quelconque des revendications 1 à 3, dans lequel Y est N ou $CR_3$ ; et $R_3$ est choisi parmi H , -CN, halogène, alkyle en $C_{1-6}$, -O(alkyle en $C_{1-6}$) et -O(haloalkyle en $C_{1-6}$) ; par exemple, Y est $CR_3$ et $R_3$ est choisi parmi H, -CN, halogène, alkyle en $C_{1-6}$, -O(alkyle en $C_{1-6}$) ou -O(haloalkyle en $C_{1-6}$) ; de préférence, $R_3$ est -O(alkyle en $C_{1-6}$) ; et plus préférablement, $R_3$ est -O(alkyle en $C_{1-3}$).

**5.** Composé, ou son sel pharmaceutiquement acceptable, ou son solvate, son mélange racémique, son énantiomère, son diastéréoisomère ou son tautomère selon l'une quelconque des revendications 1 à 4, dans lequel $R_a$ et $R_b$ sont tous deux H, et n est 0, 1 ou 2 ; et de préférence, $R_a$ et $R_b$ sont tous deux H, et n est 1.

**6.** Composé, ou son sel pharmaceutiquement acceptable, ou son solvate, son mélange racémique, son énantiomère, son diastéréoisomère ou son tautomère selon l'une quelconque des revendications 1 à 5, dans lequel $R_2$ est un phényle ou un hétéroaryle à 5 à 6 chaînons, chacun étant éventuellement substitué par un ou plusieurs groupes choisis indépendamment parmi : halogène, alkyle en $C_{1-6}$, alcynyle en $C_{2-6}$, -O(alkyle en $C_{1-6}$), -alkylène-CN en $C_{1-6}$, -alkylène-OH en $C_{1-6}$, cycloalkyle en $C_{3-8}$ et hétéroaryle à 5 à 6 chaînons, dans lequel le cycloalkyle en $C_{3-8}$ ou l'hétéroaryle à 5 à 6 chaînons, en tant que substituant de $R_2$, est chacun éventuellement substitué par un ou plusieurs

halogènes ; et de préférence, $R_2$ est un phényle ou un pyridyle, chacun étant éventuellement substitué par un ou plusieurs groupes choisis indépendamment parmi : halogène, alkyle en $C_{1-6}$, -O(alkyle en $C_{1-6}$) et cycloalkyle en $C_{3-8}$, dans lequel le cycloalkyle en $C_{3-8}$, en tant que substituant de $R_2$, est éventuellement substitué par un ou plusieurs halogènes.

**7.** Composé, ou son sel pharmaceutiquement acceptable, ou son solvate, son mélange racémique, son énantiomère, son diastéréoisomère ou son tautomère selon la revendication 1, dans lequel le composé a une structure de formule (I-1a) :

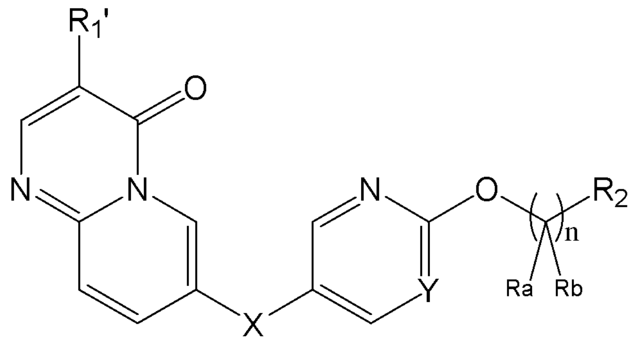

(I-1a)

dans lequel

$R_1$' est choisi parmi H, alkyle en $C_{1-6}$ , cycloalkyle en $C_{3-8}$ et $NR_4R_5$ ; $R_4$ et $R_5$ sont tous deux H ; ou $R_4$ et $R_5$, ainsi que l'atome N auquel ils sont attachés, forment un cycle hétérocyclique à 4 à 8 chaînons ; de préférence, ils forment un cycle hétérocyclique à 5 ou 6 chaînons qui, en plus de l'atome N auquel $R_4$ et $R_5$ sont attachés, contient 0, 1 ou 2 hétéroatomes choisis indépendamment parmi N, O ou S, et plus préférablement un cycle morpholine ;
X est O ou $CH_2$ ;
Y est N ou $CR_3$ ; $R_3$ est choisi parmi H, -CN, halogène, alkyle en $C_{1-6}$, -O(alkyle en $C_{1-6}$ ) et -O(haloalkyle en $C_{1-6}$) ;
$R_a$ et $R_b$ sont tous deux H ;
n est 0, 1 ou 2 ; et
$R_2$ est un phényle, qui est éventuellement substitué par un ou plusieurs groupes choisis indépendamment parmi : halogène, alkyle en $C_{1-6}$ , alcynyle en $C_{2-6}$, -O(alkyle en $C_{1-6}$), -alkylène-CN en $C_{1-6}$, -alkylène-OH en $C_{1-6}$, cycloalkyle en $C_{3-8}$ et hétéroaryle à 5 à 6 chaînons, dans lequel le cycloalkyle en $C_{3-8}$ ou l'hétéroaryle à 5 à 6 chaînons, en tant que substituant de $R_2$, est chacun éventuellement substitué par un ou plusieurs halogènes.

**8.** Composé, ou son sel pharmaceutiquement acceptable, ou son solvate, son mélange racémique, son énantiomère, son diastéréoisomère ou son tautomère selon la revendication 7, dans lequel

$R_1$' est choisi parmi H et alkyle en $C_{1-6}$ ;
X est O ;
Y est $CR_3$ ; $R_3$ est choisi parmi H, -CN, halogène, alkyle en $C_{1-6}$, -O(alkyle en $C_{1-6}$) et -O(haloalkyle en $C_{1-6}$) ;
$R_a$ et $R_b$ sont tous deux H ;
n est 1 ; et
$R_2$ est un phényle, qui est éventuellement substitué par un ou plusieurs groupes choisis indépendamment parmi : halogène, alkyle en $C_{1-6}$, -O(alkyle en $C_{1-6}$) et cycloalkyle en $C_{3-8}$, dans lequel le cycloalkyle en $C_{3-8}$, en tant que substituant de $R_2$, est éventuellement substitué par un ou plusieurs halogènes.

**9.** Composé, ou son sel pharmaceutiquement acceptable, ou son solvate, son mélange racémique, son énantiomère, son diastéréoisomère ou son tautomère selon la revendication 1, dans lequel le composé a une structure de formule (I-1e) :

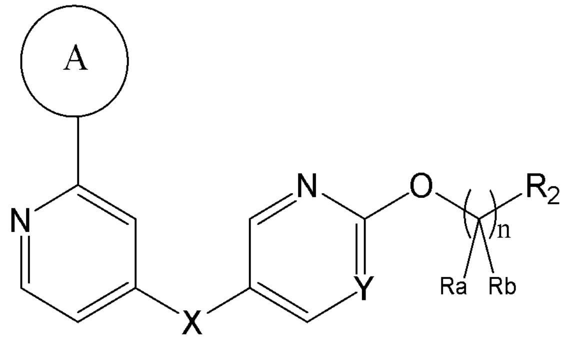

(I-1e)

dans lequel

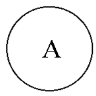

est un hétéroaryle à 5 à 6 chaînons, qui est éventuellement substitué par un ou plusieurs groupes choisis indépendamment parmi : alkyle en $C_{1-6}$ ; de préférence,

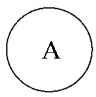

est un pyrazolyle, qui est éventuellement substitué par un ou plusieurs groupes choisis indépendamment parmi : alkyle en $C_{1-6}$ ;
X est O ou $CH_2$ ;
Y est $CR_3$ ; $R_3$ est choisi parmi H, alkyle en $C_{1-6}$, -O(alkyle en $C_{1-6}$) et -O(haloalkyle en $C_{1-6}$) ;
$R_a$ et $R_b$ sont tous deux H ;
n est 1 ou 2 ; et
$R_2$ est un phényle, qui est éventuellement substitué par un ou plusieurs groupes choisis indépendamment parmi : halogène, alkyle en $C_{1-6}$, -O(alkyle en $C_{1-6}$) et cycloalkyle en $C_{3-8}$, dans lequel le cycloalkyle en $C_{3-8}$, en tant que substituant de $R_2$, est éventuellement substitué par un ou plusieurs halogènes.

**10.** Composé, ou son sel pharmaceutiquement acceptable, ou son solvate, son mélange racémique, son énantiomère, son diastéréoisomère ou son tautomère selon la revendication 1, dans lequel le composé a une structure de formule (I-2) ou de formule (I-3) ; et de préférence, le composé a une structure de formule (I-2) :

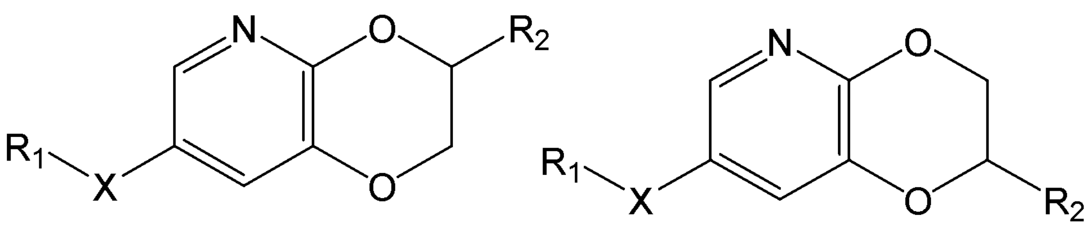

(I-2) (I-3)

dans lequel $R_1$, $R_2$ et X sont tels que définis dans la revendication 1.

**11.** Composé, ou son sel pharmaceutiquement acceptable, ou son solvate, son mélange racémique, son énantiomère, son diastéréoisomère ou son tautomère selon la revendication 10, dans lequel le composé a une structure de formule (I-2a) :

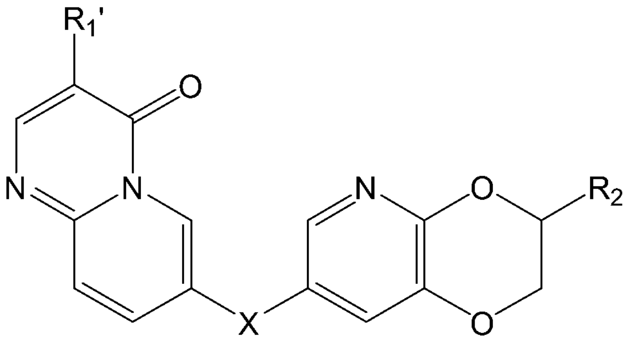

(I-2a)

dans lequel

$R_1$' est choisi parmi H et alkyle en $C_{1-6}$ ;
X est O ;
$R_2$ est un phényle ou un hétéroaryle à 5 à 6 chaînons, chacun est éventuellement substitué par un ou plusieurs groupes choisis indépendamment parmi : halogène, alkyle en $C_{1-6}$, -O(alkyle en $C_{1-6}$) et cycloalkyle en $C_{3-8}$, dans lequel le cycloalkyle en $C_{3-8}$, en tant que substituant de $R_2$, est éventuellement substitué par un ou plusieurs halogènes ; et de préférence, $R_2$ est un phényle ou un pyridyle, chacun est éventuellement substitué par un ou plusieurs groupes choisis indépendamment parmi : -O(alkyle en $C_{1-6}$) et cycloalkyle en $C_{3-8}$ ;
ou
le composé a une structure de formule (I-2b) :

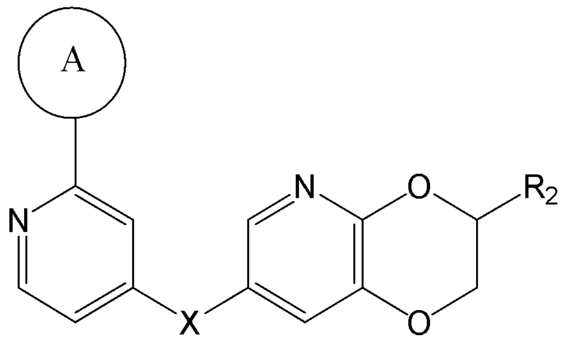

(I-2b)

dans lequel

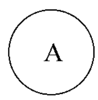

est un hétéroaryle à 5 à 6 chaînons, qui est éventuellement substitué par un ou plusieurs groupes choisis indépendamment parmi : alkyle en $C_{1-6}$ et alkylène-OH en $C_{1-6}$ ; de préférence,

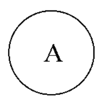

est un pyrazolyle, qui est éventuellement substitué par un ou plusieurs groupes choisis indépendamment parmi : alkyle en $C_{1-6}$ et -alkylène-OH en $C_{1-6}$ ; plus préférablement,

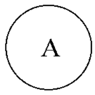

est un pyrazolyle, qui est éventuellement substitué par un ou plusieurs groupes choisis indépendamment parmi : alkyle en $C_{1-6}$ ;
X est O ;
$R_2$ est un phényle ou un hétéroaryle à 5 à 6 chaînons, chacun est éventuellement substitué par un ou plusieurs groupes choisis indépendamment parmi : halogène, alkyle en $C_{1-6}$, -O(alkyle en $C_{1-6}$) et cycloalkyle en $C_{3-8}$, dans lequel le cycloalkyle en $C_{3-8}$, en tant que substituant de $R_2$, est éventuellement substitué par un ou plusieurs halogènes ; et de préférence, $R_2$ est un phényle ou un pyridyle, chacun est éventuellement substitué par un ou plusieurs groupes choisis indépendamment parmi : -O(alkyle en $C_{1-6}$) et cycloalkyle en $C_{3-8}$ ; et plus préférablement, $R_2$ est pyridyle, qui est éventuellement substitué par un ou plusieurs groupes choisis indépendamment parmi : -O(alkyle en $C_{1-6}$) et cycloalkyle en $C_{3-8}$.

**12.** Composé de formule (I) selon la revendication 1, choisi parmi les composés suivants :

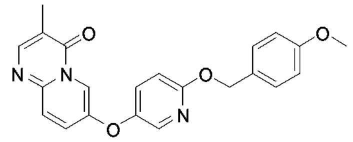

1

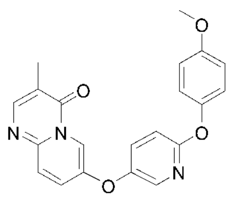

2

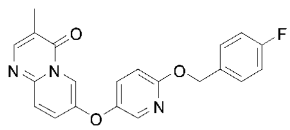

3

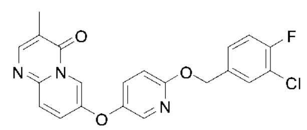

4

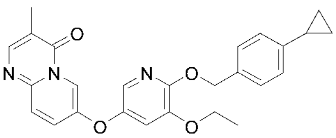

5

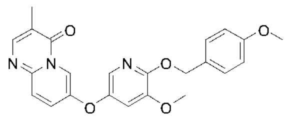

6

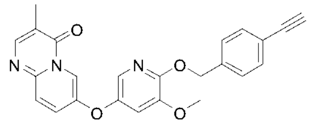

7

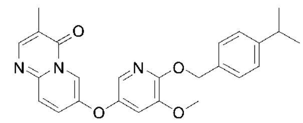

8

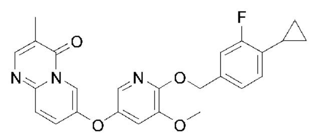

9

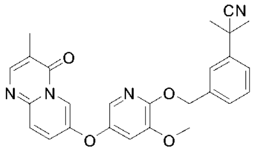

10

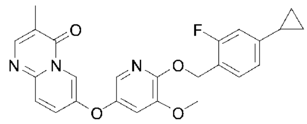

11

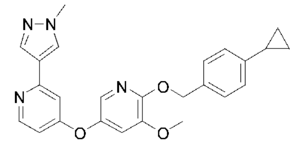

12

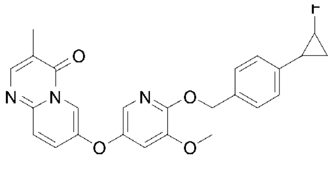

13

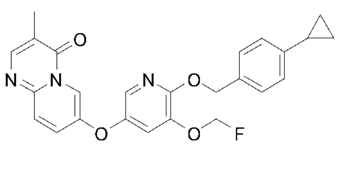

14

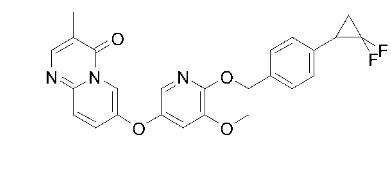

15

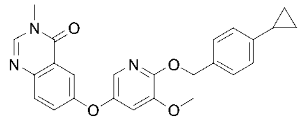

16

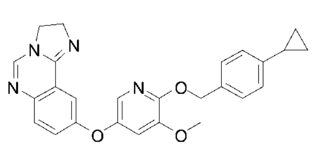

17

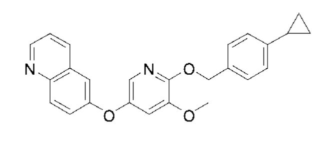

18

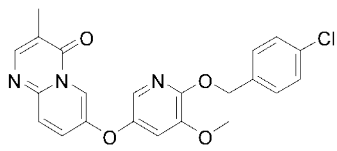

19

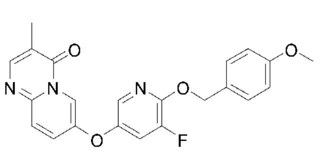

20

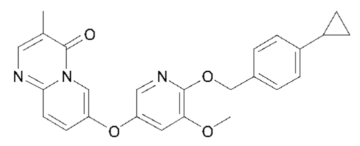

21

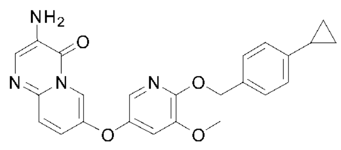

22

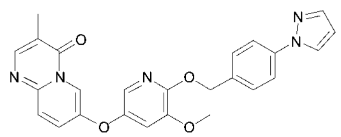

23

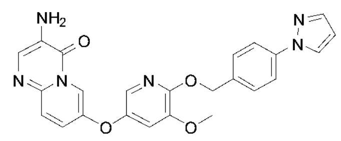

24

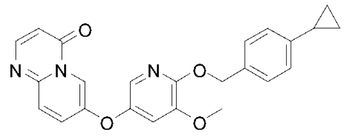

25

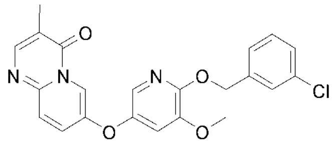

26

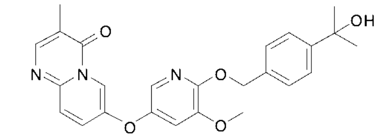

27

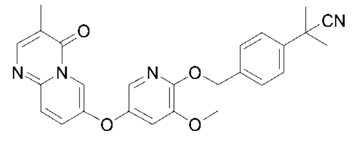

28

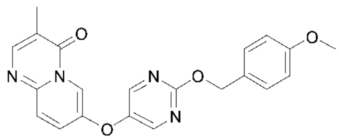

29

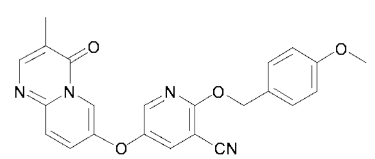

30

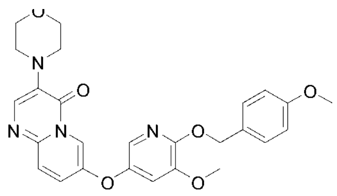

31

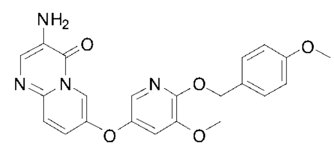

32

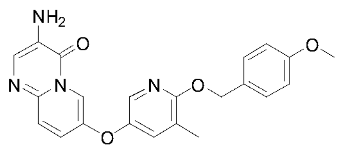

33

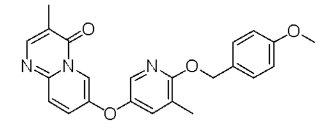

34

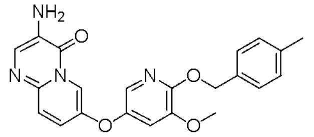

35

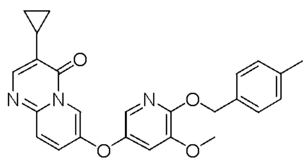

36

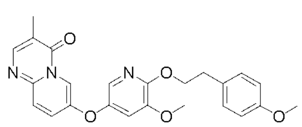

37

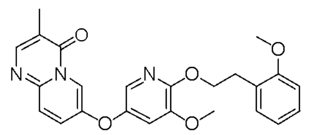

38

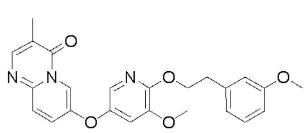

39

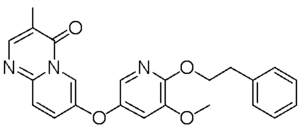

40

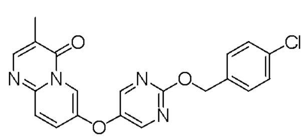

41

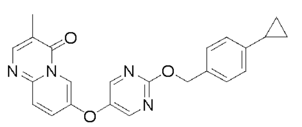

42

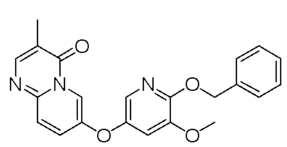

43

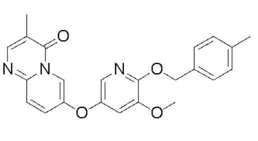

44

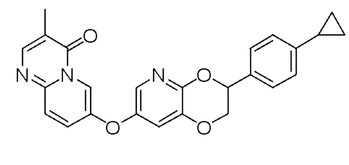

45

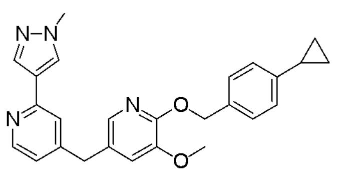

46

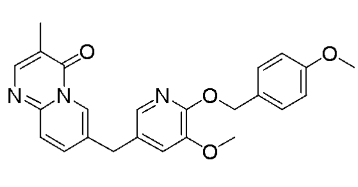

47

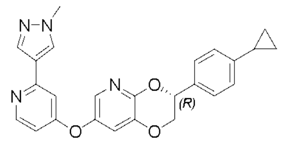 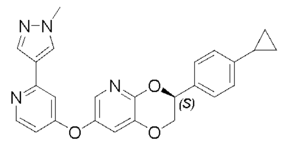 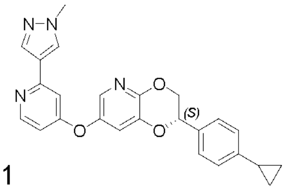 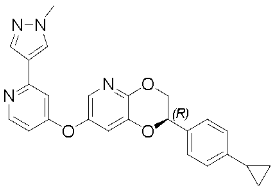

48-51

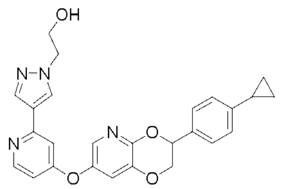

52

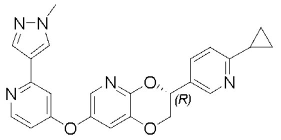 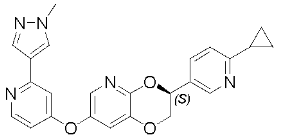

53 & 54

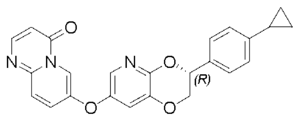 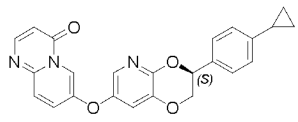

55 & 56

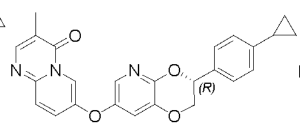 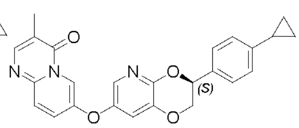

57 & 58

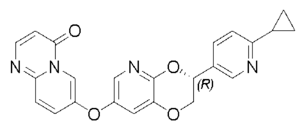 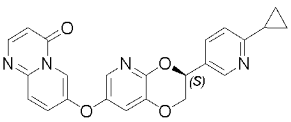

59 & 60

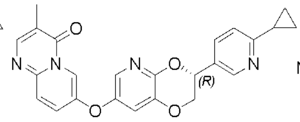 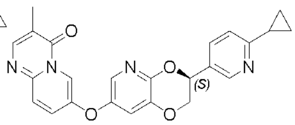

61 & 62

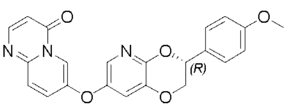 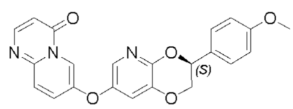

63 & 64

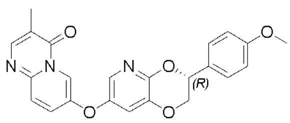 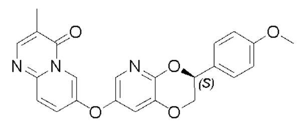

65 & 66

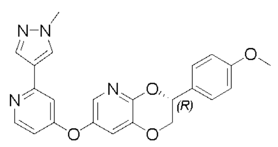 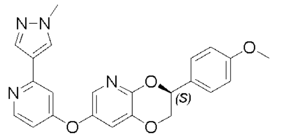

67 & 68

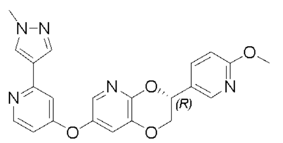 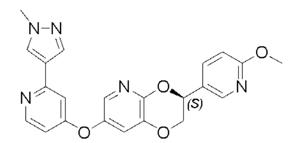

69 & 70

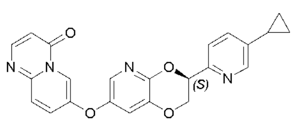 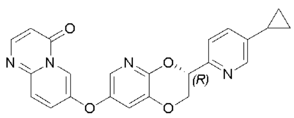

71 & 72

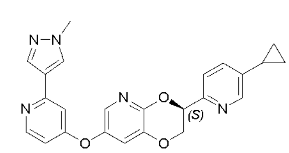 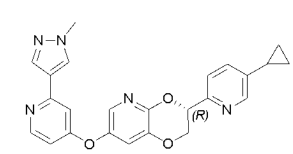

73 & 74

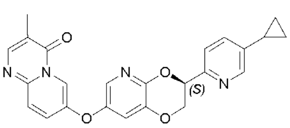 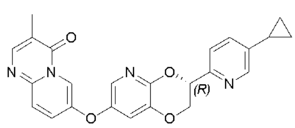

75 & 76

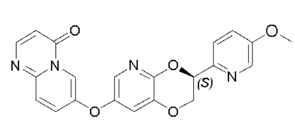 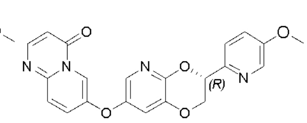

77 & 78

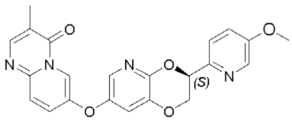 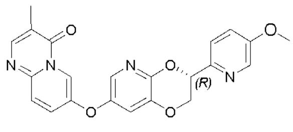

79 & 80

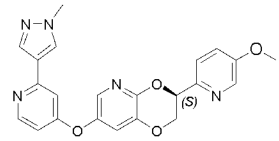 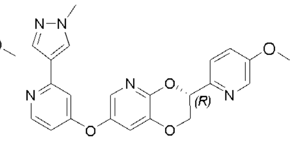

81 & 82

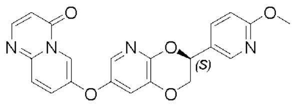

83

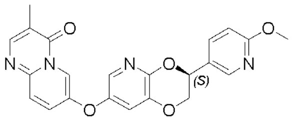

84

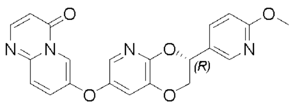

85

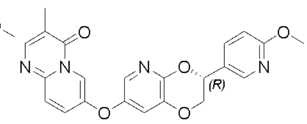

86

.

ou un sel pharmaceutiquement acceptable de ceux-ci.

**13.** Composition pharmaceutique, comprenant un composé ou un sel pharmaceutiquement acceptable de celui-ci selon l'une quelconque des revendications 1 à 12 et comprenant éventuellement un excipient pharmaceutiquement acceptable.

**14.** Composé ou sel pharmaceutiquement acceptable de celui-ci selon l'une quelconque des revendications 1 à 12 ou composition pharmaceutique selon la revendication 13, destiné(e) à être utilisé(e) comme produit pharmaceutique, par exemple dans le traitement d'une maladie médiée par le CSF-1R ou au moins en partie par le CSF-1R chez un sujet, dans lequel la maladie est une maladie auto-immune, une maladie inflammatoire, une maladie neurodégénérative, un cancer, une maladie métabolique, l'obésité ou une maladie liée à l'obésité ; et de préférence, dans lequel la maladie auto-immune ou la maladie inflammatoire est choisie parmi la polyarthrite rhumatoïde, l'arthrite induite par le collagène, l'arthrose, la synovite villonodulaire pigmentée (PVNS), le lupus érythémateux systémique, la sclérose en plaques, la sclérodermie systémique, la néphrite auto-immune, les maladies inflammatoires de l'intestin, la maladie de Crohn, la colite ulcéreuse, le psoriasis, la dermatite atopique, l'asthme, la bronchopneumopathie chronique obstructive, la maladie de Behçet, le purpura thrombopénique idiopathique, l'arthrite spinale, l'arthrite juvénile idiopathique systémique (AJIs), la pancréatite, les lésions d'ischémie-reperfusion des organes parenchymateux, le rejet de greffe d'organe, la septicémie, le syndrome de réponse inflammatoire systémique et les lésions organiques induites par les médicaments de chimiothérapie ; la maladie neurodégénérative est choisie parmi la maladie de Parkinson (MP), l'atrophie multisystémique, la maladie d'Alzheimer (MA), la démence lobaire frontotemporale, la maladie de Huntington (MH), la dégénérescence corticobasale, l'ataxie spinocérébelleuse, la sclérose latérale amyotrophique (SLA), l'amyotrophie spinale (SMA) et la neuropathie motrice et sensorielle héréditaire (CMT) ; et le cancer est une tumeur solide ou une hémopathie maligne, comme le cancer de l'ovaire, le cancer du poumon (y compris le cancer du poumon non à petites cellules), la tumeur cérébrale (y compris le glioblastome (GBM)), la tumeur à cellules géantes ténosynoviales, la tumeur stromale gastro-intestinale (GIST), le cancer de l'estomac, le cancer de l'œsophage, le cancer du côlon, le cancer colorectal, le cancer du pancréas, le cancer de la prostate, le cancer du sein, le cancer du col de l'utérus, le mélanome, le mésothéliome, le carcinome mésothélial, le cancer du rein, le cancer du foie, le carcinome de la thyroïde, le cancer de la tête et du cou, le carcinome urothélial, le cancer de la vessie, le cancer de l'endomètre, le choriocarcinome, le carcinome surrénalien, le sarcome, la leucémie, le lymphome ou le myélome.

**15.** Combinaison pharmaceutique, comprenant un composé ou un sel pharmaceutiquement acceptable de celle-ci selon l'une quelconque des revendications 1 à 12, et au moins un agent thérapeutique supplémentaire ; de préférence, dans lequel l'agent thérapeutique supplémentaire est un agent anti-inflammatoire ou un agent antinéoplasique ; et plus préférablement, l'agent antinéoplasique est choisi parmi un agent radiothérapeutique, un agent chimiothérapeutique, un inhibiteur ou un agoniste de point de contrôle immunitaire et un agent thérapeutique ciblé.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 3989816 A
- US 4444762 A


### Non-patent literature cited in the description


- *Nat Rev Cancer.*, 2012, vol. 12 (11), 753-66
- *J Pathol*, 2011, vol. 223 (2), 251-261
- *Biomark Insights*, 2015, vol. 10, 1-14
- *Cold Spring Harb Perspect Biol*, 2014, vol. 6 (6)
- *J Cell Biochem.*, 1988, vol. 38 (3), 179-87
- *Immunity*, 2014, vol. 41 (1), 49-61
- *Nat Med.*, 2013, vol. 19 (10), 1264-72
- *N Engl J Med.*, 2015, vol. 373 (5), 428-37
- *Arthritis Res Ther*, 2016, vol. 18, 75
- *Nat Rev Immunol.*, 2008, vol. 8 (7), 533-44
- *J Immunother Cancer*, 2017, vol. 5 (1), 53
- *Neurobiol Aging.*, 2000, vol. 21, 383-421
- *Brain Res*, 1994, vol. 639, 171-4
- *Brain*, 2016, vol. 139, 891-907
- *Sci Rep*, 2016, vol. 6, 25663
- *Bioorg. Med. Chem. Lett.*, 2010, vol. 20, 1543-1547
- **ALLINGER N. L** ; **ELIEL E. L**. Topics in Stereochemistry. Wiley Interscience, 1971, vol. 6
- **S. M. BERGE et al.** Pharmaceutical Salts. *J. Pharm. Sci*, 1977, vol. 66, 1-19
- Handbook of Pharmaceutical Salts, Properties, Selection, and Use. Wiley-VCH and VHCA, 2002
- **R. LAROCK**. Comprehensive Organic Transformations. VCH Publishers, 1989
- **L. FIESER** ; **M. FIESER**. Fieser and Fieser's Reagents for Organic Synthesis. John Wiley and Sons, 1994
- Encyclopedia of Reagents for Organic Synthesis. John Wiley and Sons, 1995
- *CHEMICAL ABSTRACTS*, 192139-92-7